(19) Europäisches Patentamt / European Patent Office / Office européen des brevets

(11) **EP 4 640 680 A1**

(12) **EUROPEAN PATENT APPLICATION**
published in accordance with Art. 153(4) EPC


(43) Date of publication:
**29.10.2025 Bulletin 2025/44**

(21) Application number: **23906645.9**

(22) Date of filing: **30.11.2023**

(51) International Patent Classification (IPC):
***C07D 403/14*** *(2006.01)* ***C07D 405/14*** *(2006.01)*
***C09K 11/06*** *(2006.01)* ***H10K 50/00*** *(2023.01)*
***H10K 50/10*** *(2023.01)* ***H10K 50/11*** *(2023.01)*
***H10K 50/12*** *(2023.01)* ***H10K 59/00*** *(2023.01)*
***H10K 59/10*** *(2023.01)* ***H10K 85/00*** *(2023.01)*
***H10K 85/60*** *(2023.01)* ***H10K 101/10*** *(2023.01)*

(52) Cooperative Patent Classification (CPC):
**C07D 403/14; C07D 405/14; C09K 11/06; H10K 50/00; H10K 50/10; H10K 50/11; H10K 50/12; H10K 59/00; H10K 59/10; H10K 85/00; H10K 85/60;** H10K 2101/10

(86) International application number:
**PCT/JP2023/042938**

(87) International publication number:
**WO 2024/135277 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.12.2022 JP 2022205830**



(71) Applicant: **NIPPON STEEL CHEMICAL & MATERIAL CO., LTD.**
**Tokyo 103-0027 (JP)**

(72) Inventors:
- **INOUE Munetomo**
**Tokyo 103-0027 (JP)**
- **OKUYAMA Masataka**
**Tokyo 103-0027 (JP)**
- **SAGARA Yuta**
**Tokyo 103-0027 (JP)**

(74) Representative: **Kraus & Lederer PartGmbB**
**Thomas-Wimmer-Ring 15**
**80539 München (DE)**


(54) **ORGANIC ELECTROLUMINESCENT ELEMENT**


(57) Provided is an organic EL device having characteristics of high efficiency and a long lifetime, and a compound suitable for the device. The compound is represented by the following general formula (1), and the organic EL device uses the compound. $Ar^1$ represents the following general formula (2). $R^1$ and $R^{11}$ are independently deuterium, an aliphatic hydrocarbon group having 1 to 10 carbon atoms, or a substituted or unsubstituted aromatic hydrocarbon group having 6 to 18 carbon atoms, $a^0$ represents an integer of 0 to 3, s represents an integer of 0 to 3, and c represents an integer of 0 to 4. n and m are an integer of 1 to 3, and at least one of n or m is 2 or 3. "*" is a bonding site to the general formula (1). $X^1$ is N or C-$R^2$, and at least one $X^1$ is N. $R^2$, $Ar^0$, $Ar^2$, and $Ar^3$ independently hydrogen, deuterium, an aliphatic hydrocarbon group having 1 to 10 carbon atoms, an aromatic hydrocarbon group having 6 to 18 carbon atoms, or the like.


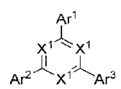
(1)

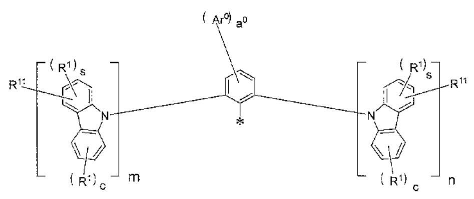
(2)

EP 4 640 680 A1

Fig. 1

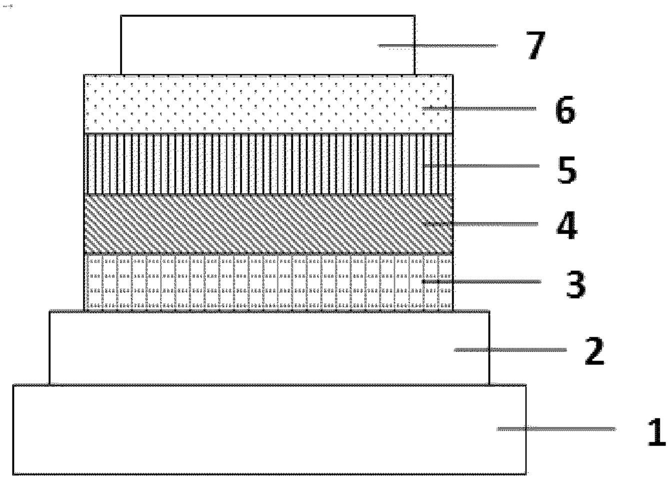

## Description

Technical Field

The present invention relates to a compound, a material for organic electroluminescent device, as well as an organic electroluminescent device or element (also referred to as an organic EL device or element).

When a voltage is applied to an organic EL device, holes and electrons are injected from the anode and the cathode, respectively, into the light emitting layer. Then, the injected holes and electrons are recombined in the light emitting layer to thereby generate excitons. At this time, according to the electron spin statistics theory, singlet excitons and triplet excitons are generated at a ratio of 1:3. In the fluorescent organic EL device that uses emission caused by singlet excitons, the limit of the internal quantum efficiency is said to be 25%. On the other hand, it has been known that, in the phosphorescent organic EL device that uses emission caused by triplet excitons, the internal quantum efficiency can be enhanced up to 100% when intersystem crossing efficiently occurs from singlet excitons.

However, the phosphorescent organic EL device has a technical problem of further extending the lifetime.

Further, a highly efficient organic EL device utilizing delayed fluorescence has been developed, in recent years. For example, Patent Literature 1 discloses an organic EL device utilizing the Triplet-Triplet Fusion (TTF) mechanism, which is one of the mechanisms of delayed fluorescence. The TTF mechanism utilizes a phenomenon in which a singlet exciton is generated by the collision of two triplet excitons, and it is believed that the internal quantum efficiency can be enhanced up to 40%, in theory. However, its efficiency is low as compared with the efficiency of the phosphorescent organic EL device, and thus further improvement in efficiency is desired.

On the other hand, Patent Literature 2 discloses an organic EL device utilizing the Thermally Activated Delayed Fluorescence (TADF) mechanism. The TADF mechanism utilizes a phenomenon in which reverse intersystem crossing occurs from the triplet exciton to the singlet exciton in a material having a small energy difference between the singlet level and the triplet level, and it is believed that the internal quantum efficiency can be enhanced up to 100%, in theory. However, further improvement in lifetime characteristics is desired as in the phosphorescent device, and for a blue light emitting organic EL device, improvement in lifetime characteristics is particularly demanded.

Citation List

Patent Literature

Patent Literature 1: WO2010/134350
Patent Literature 2: WO2011/070963
Patent Literature 3: WO2015/102118
Patent Literature 4: WO2017/115833
Patent Literature 5: WO2018/212169
Patent Literature 6: WO2018/181188
Patent Literature 7: WO2020/040298
Patent Literature 8: JP2020-120096 A
Patent Literature 9: WO2016/159479
Patent Literature 10: US2011/0309345
Patent Literature 11: WO2008/117826
Patent Literature 12: US2022/0177492
Patent Literature 13: WO2016/158191
Patent Literature 14: WO2012/077520
Patent Literature 15: US2014/0158992
Patent Literature 16: WO2016/158540
Patent Literature 17: US2020/0168812


Patent Literature 4 discloses an organic EL device containing two host materials exemplified by compounds shown below, and a TADF material as light emitting dopants in a light emitting layer.

[C1]

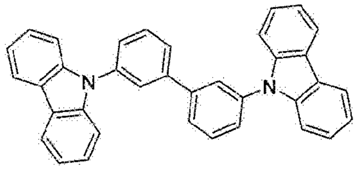

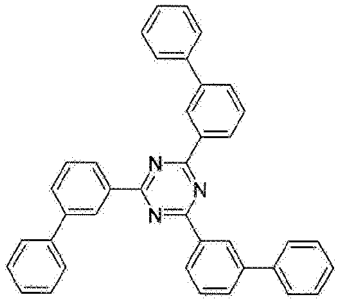

Patent Literature 3 and Patent Literature 5 disclose an organic EL device in which a TADF material including a polycyclic aromatic compound exemplified by the following compound is used as a light emitting dopant.

[C2]

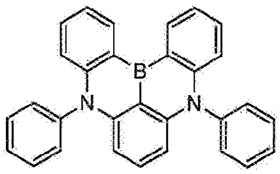

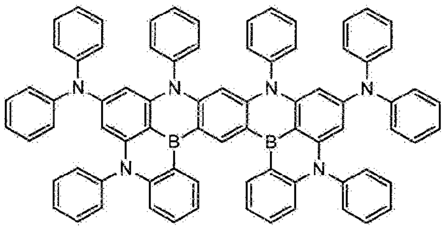

Patent Literatures 6 and 7 disclose an organic EL device in which a mixture of a boron-based compound, a TADF material, and the following carbazole compound a3, is used in a light emitting layer.

[C3]

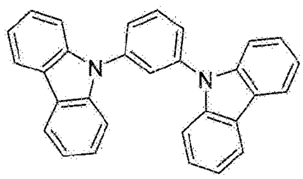

a3

Patent Literature 8 discloses an organic EL device in which a mixture of the following boron-based compound a7, nitrogen-containing six-membered ring compound a8, and carbazole compound a9, is used in a light-emitting layer.

[C4]

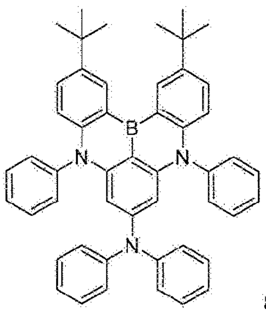

a7

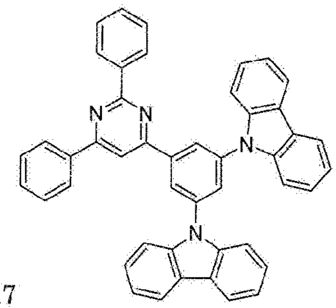

a8

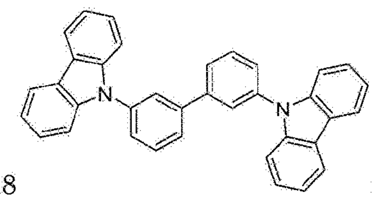

a9

Patent Literature 9 discloses an organic EL device in which a TADF material exemplified by the following compound is used as a light emitting dopant.

[C5]

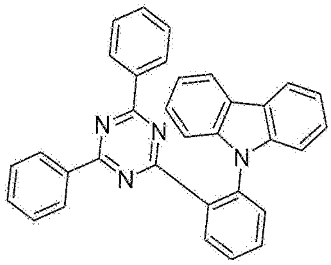

H1

Patent Literature 10 discloses a phosphorescent organic EL device in which a compound having a nitrogen-containing six-membered ring and biscarbazole or tricarbazole linked to each other and exemplified by the following compound is used as a host material.

[C6]

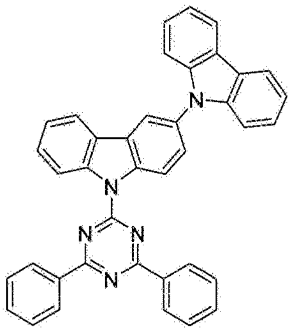

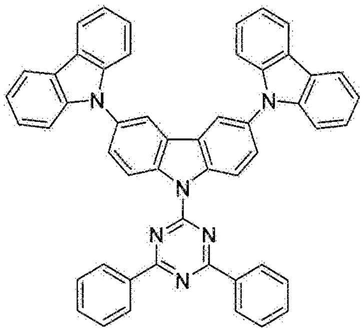

Patent Literature 11 discloses a phosphorescent organic EL device in which a compound having a nitrogen-containing six-membered ring and carbazole linked to each other and exemplified by the following compound is used as a host material.

[C7]

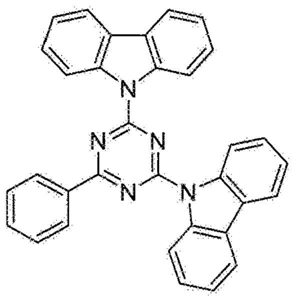

Patent Literature 12 discloses a phosphorescent organic EL device in which a compound having a nitrogen-containing six-membered ring and carbazole linked to each other and exemplified by the following compound and a deuterated compound of the compound are used as host materials.

[C8]

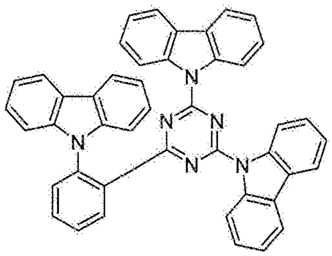

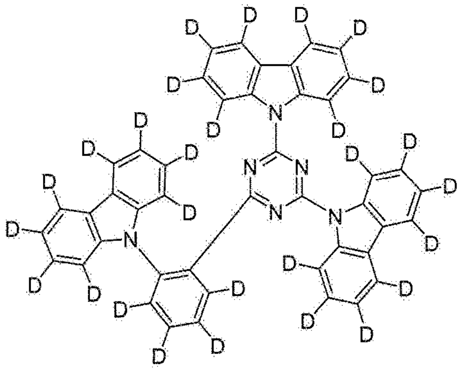

Patent Literature 13 discloses a phosphorescent organic EL device containing two host materials exemplified by the following compounds, and a phosphorescent material as light emitting dopants in a light emitting layer.

[C9]

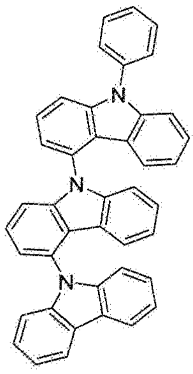

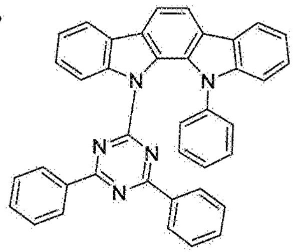

Patent Literature 14 discloses a phosphorescent organic EL device in which a compound having a nitrogen-containing six-membered ring and biscarbazole linked to each other and exemplified by the following compound is used as a host material.

[C10]

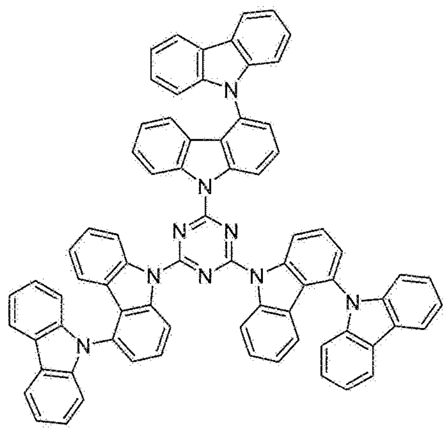

Patent Literature 15 discloses an organic EL device in which a TADF material exemplified by the following compound is used as a light emitting dopant.

[C11]

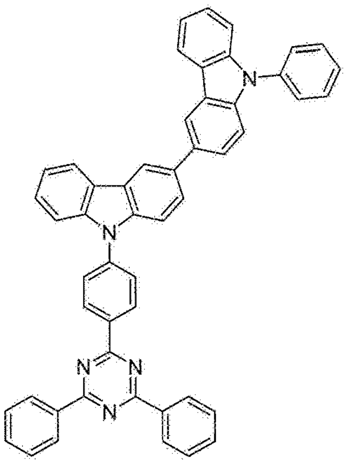

Patent Literature 16 discloses an organic EL device in which a mixture of a boron-based compound and the following compound H1 or H2 having a nitrogen-containing six-membered ring and carbazole linked to each other is used in a light emitting layer.

[C12]

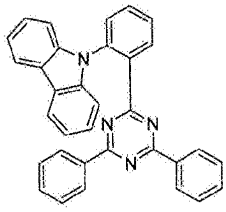

H 1

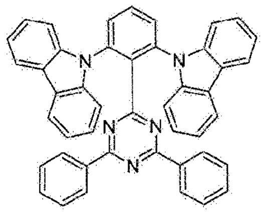

H 2

Patent Literature 17 discloses an organic EL device in which the following compound H7 having a nitrogen-containing six-membered ring and carbazole linked to each other is used.

[C13]

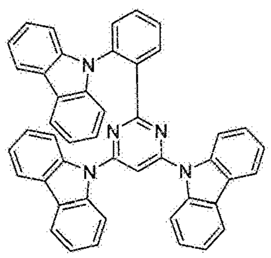

H 7

In all the patent literatures, however, there is still room for improvement in organic EL devices that exhibit sufficient lifetime characteristics.

Summary of Invention

Technical Problem

In order to apply an organic EL device to a display device or a light source for a flat panel display and the like, it is necessary to improve emission efficiency of the device and simultaneously to ensure sufficient stability upon driving. An object of the present invention is to provide a practically useful organic EL device that has high efficiency and long lifetime characteristics, and a compound suitable for the device.

Solution to Problem

The present invention is a material for organic electroluminescent devices, i.e., a compound represented by the following general formula (1).

[C14]

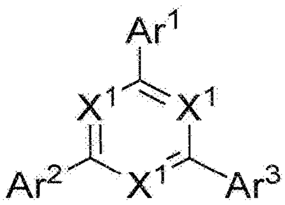

(1)

wherein $Ar^1$ represents the following general formula (2).

[C15]

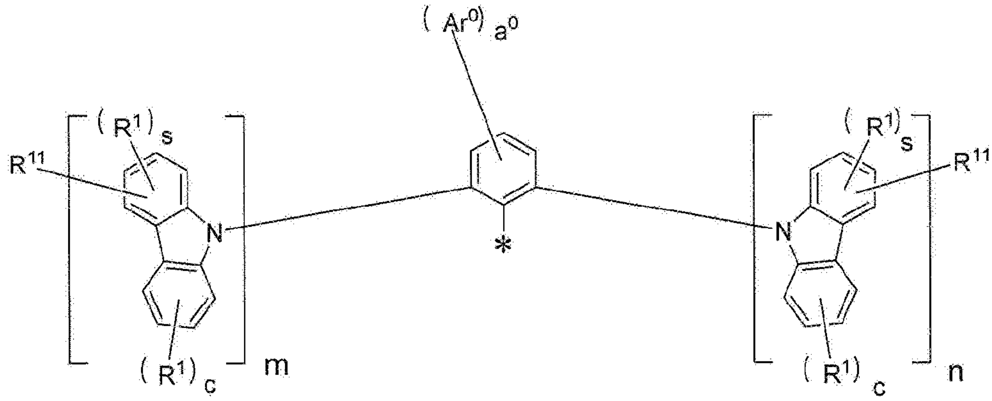

(2)

wherein $R^1$ independently represents deuterium, an aliphatic hydrocarbon group having 1 to 10 carbon atoms, or a substituted or unsubstituted aromatic hydrocarbon group having 6 to 18 carbon atoms. $R^{11}$ independently represents hydrogen, deuterium, an aliphatic hydrocarbon group having 1 to 10 carbon atoms, or a substituted or unsubstituted aromatic hydrocarbon group having 6 to 18 carbon atoms. $a^0$ represents the number of substitutions and represents an integer of 0 to 3. s and c represent the number of substitutions, s independently represents an integer of 0 to 3, and c independently represents an integer of 0 to 4. n and m represent the number of repetitions and each independently represent an integer of 1 to 3, and at least one of n or m represents 2 or 3. "*" represents a bonding site to the general formula (1) above.

$X^1$ independently represents N or C-$R^2$, and at least one $X^1$ represents N. $R^2$ independently represents hydrogen, deuterium, an aliphatic hydrocarbon group having 1 to 10 carbon atoms, a substituted or unsubstituted aromatic hydrocarbon group having 6 to 18 carbon atoms, or a substituted or unsubstituted linked aromatic group formed by linking 2 to 8 of the aromatic hydrocarbon groups.

$Ar^2$ and Ar3 represent hydrogen, deuterium, an aliphatic hydrocarbon group having 1 to 10 carbon atoms, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups.

$Ar^0$ represents deuterium, an aliphatic hydrocarbon group having 1 to 10 carbon atoms, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups.

In a preferred example of the general formula (1) above, each $X^1$ in formula (1) is N.

In a preferred example of the general formula (1) above, $Ar^1$ in the formula (1) contains at least one linked carbazole group represented by any of the following general formulae (4a) to (4c).

[C16]

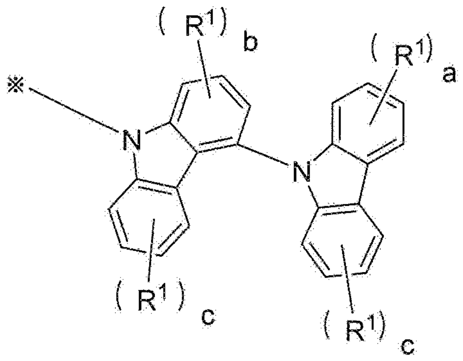

(4 a)

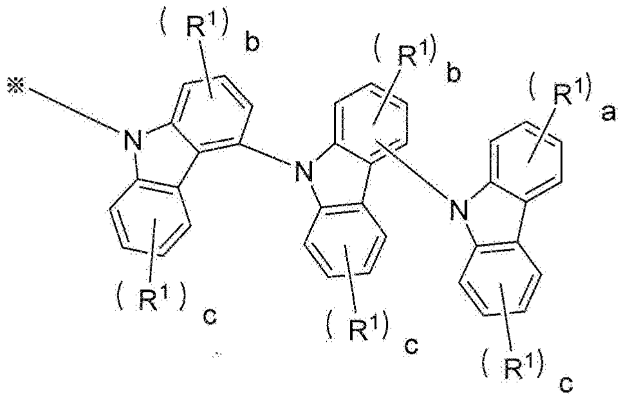

(4 b)

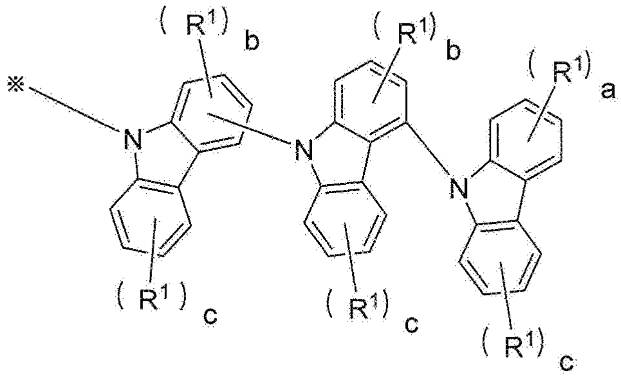

(4 c)

wherein, a and b represent the number of substitutions, a independently represents an integer of 0 to 4, and b independently represents an integer of 0 to 3. "※" represents a bonding site to the benzene ring in the general formula (2) above. $R^1$ and c are as defined for the general formula (2) above.

In a preferred example of the general formula (1) above, $Ar^1$ in the formula (1) is represented by any of the following general formulae (5a) to (5e).

[C17]

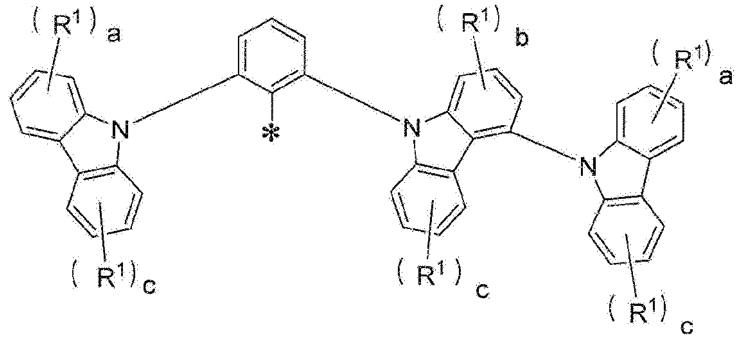

(5 a)

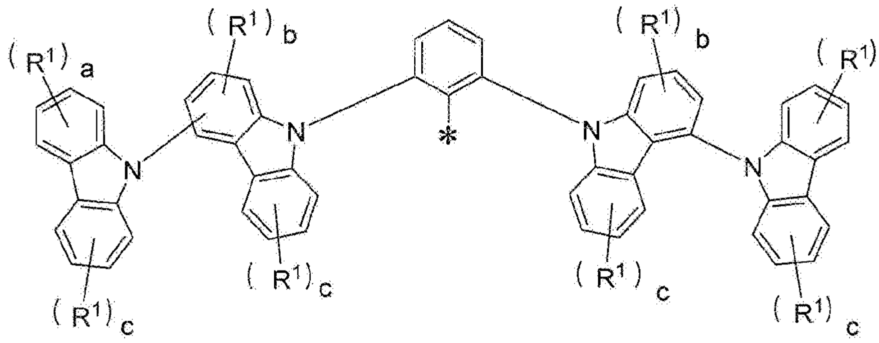

(5 b)

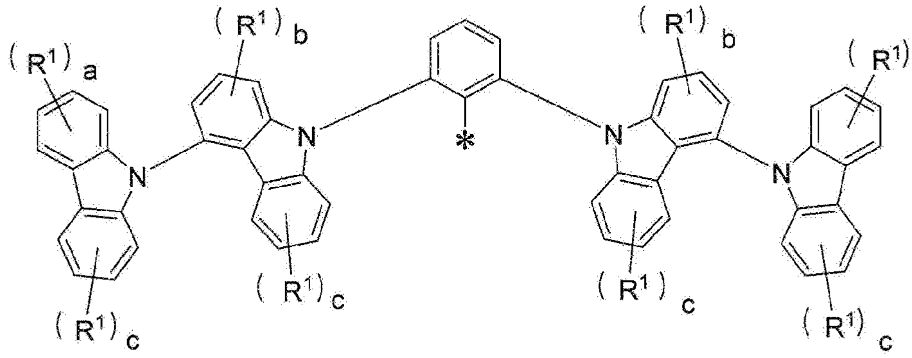

(5 c)

[C18]

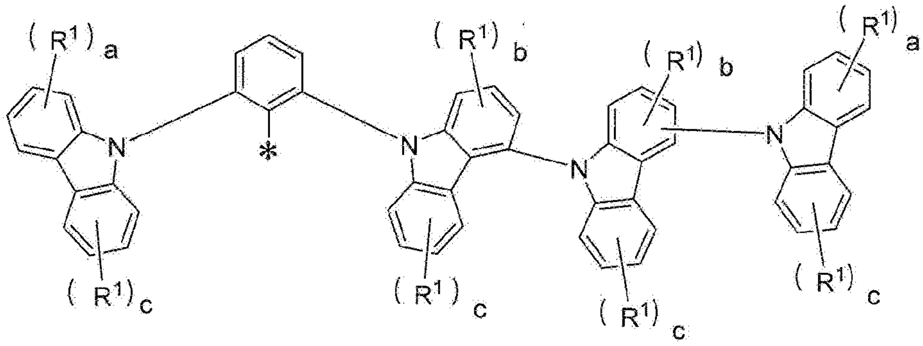

(5d)

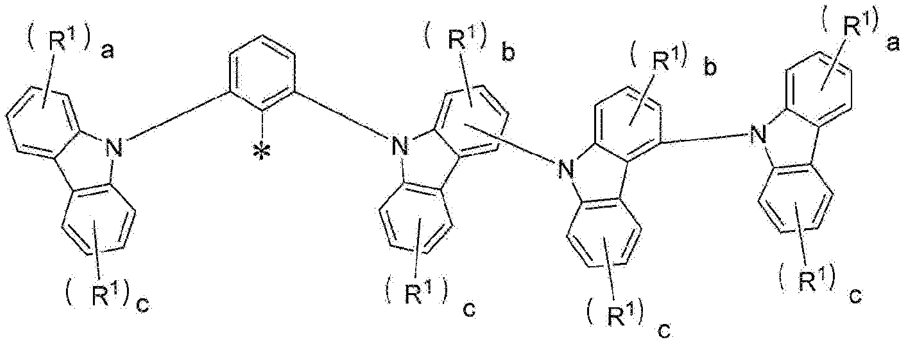

(5e)

wherein $R^1$, c, and "*" are as defined for the general formula (2) above. a and b are as defined for the general formulae (4a) to (4c) above.

In a preferred example of the general formula (1) above, $Ar^2$ and $Ar^3$ in the formula (1) are represented by hydrogen, deuterium, a substituted or unsubstituted aromatic hydrocarbon group having 6 to 18 carbon atoms, or a substituted or unsubstituted linked aromatic group formed by linking 2 to 8 of the aromatic hydrocarbon groups.

In a preferred example of the general formula (1) above, the general formula (1) above is represented by any of the following general formulae (6a) to (6c).

[C19]

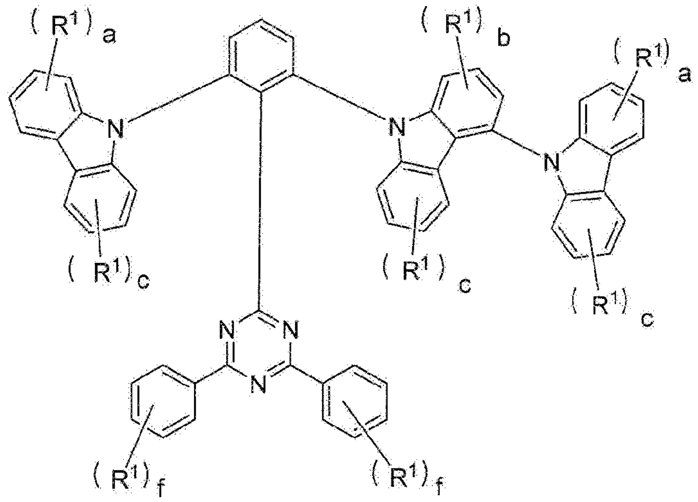

(6 a)

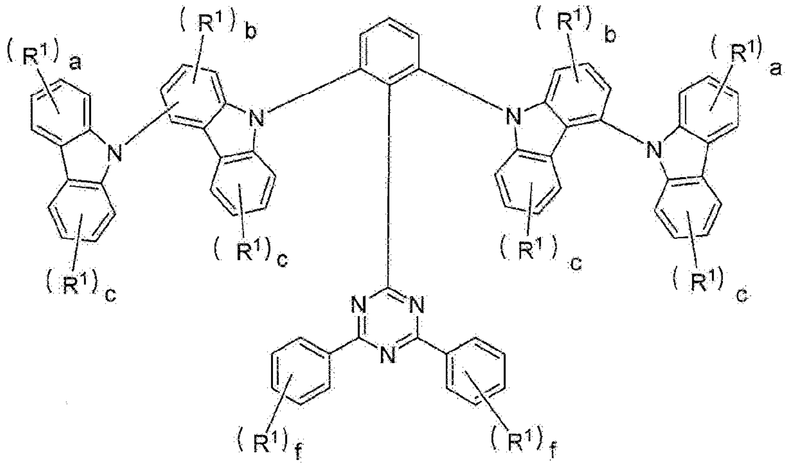

(6 b)

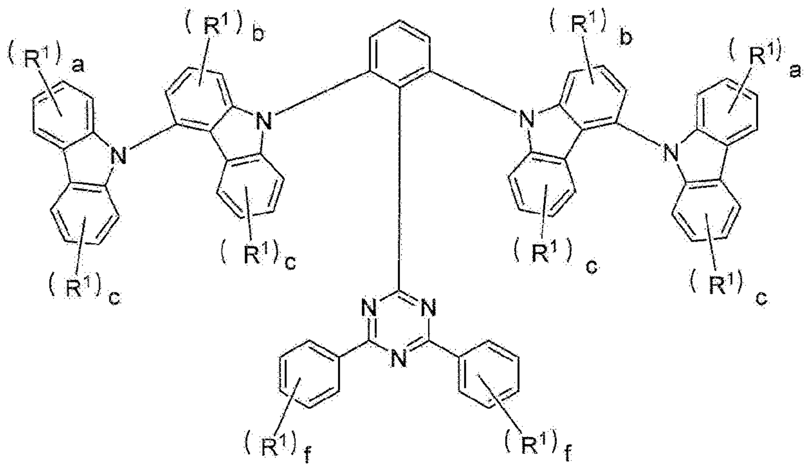

(6 c)

wherein, $R^1$ and c are as defined for the general formula (2) above and are independent of each other. a and b are as defined for the general formulae (4a) to (4c) above. Moreover, f independently represents the number of substitutions and represents an integer of 0 to 5.

In a preferred example of the general formula (1) above, at least one of $Ar^1$ to $Ar^3$ contains a deuterium atom, and in a more preferred example, at least one of $Ar^2$ and $Ar^3$ contains a deuterium atom.

In a preferred example of the general formula (1) above, at least one $R^1$ is deuterium.

The compound represented by the general formula (1) above is preferably used as a material for an organic

electroluminescent device.

In an organic electroluminescent device that includes one or more light emitting layers between an anode and a cathode opposite to each other, at least one light emitting layer preferably comprises a host selected from the compounds represented by the general formula (1) above and a light emitting dopant, and the light emitting dopant is preferably a polycyclic aromatic compound represented by the following general formula (7a) or (7b), or a phosphorescent dopant.

[C20]

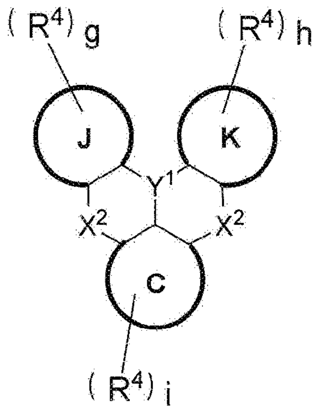

(7 a)

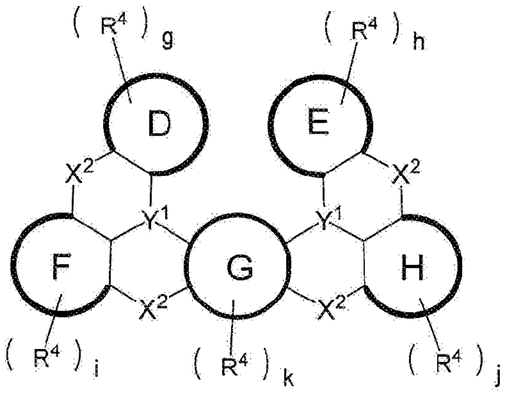

(7 b)

Here, a ring J, a ring K, a ring C, a ring D, a ring E, a ring F, a ring G, and a ring H are each independently a substituted or unsubstituted aromatic hydrocarbon ring having 6 to 24 carbon atoms, or a substituted or unsubstituted aromatic heterocyclic ring having 3 to 17 carbon atoms.

$Y^1$ is each independently B, P, P=O, P=S, Al, Ga, As, Si-$R^3$ or Ge-$R^3$, preferably B, P, P=O or P=S, and more preferably B.

$R^3$ is each independently an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.

$X^2$ is each independently O, N-$Ar^4$, S or Se, preferably O, N-$Ar^4$ or S, and more preferably O or N-$Ar^4$.

$Ar^4$ is each independently a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these groups.

N-$Ar^4$ is optionally bonded to any of the ring J, the ring K, the ring C, the ring D, the ring E, the ring F, the ring G, or the ring H to form a heterocyclic ring containing N.

$R^4$ each independently represents a cyano group, deuterium, a diarylamino group having 12 to 44 carbon atoms, an arylheteroarylamino group having 12 to 44 carbon atoms, a diheteroarylamino group having 12 to 44 carbon atoms, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.

g and h represent the number of substitutions and each independently represent an integer of 0 to 4, i and j represent the number of substitutions and each independently represent an integer of 0 to 3, and k represents the number of substitutions and represents an integer of 0 to 2.

A preferred example of the polycyclic aromatic compound represented by the general formula (7a) above includes a boron-containing polycyclic aromatic compound represented by the following formula (8a), and an preferred example of the polycyclic aromatic compound represented by the general formula (7b) above includes a boron-containing polycyclic aromatic compound represented by the following formula (8b).

[C21]

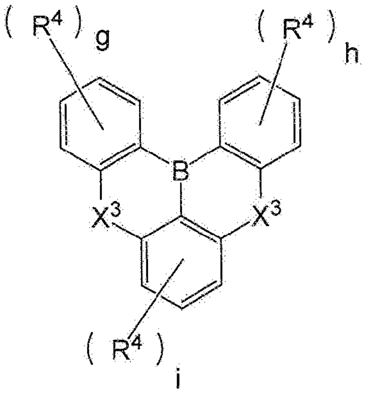

(8 a)

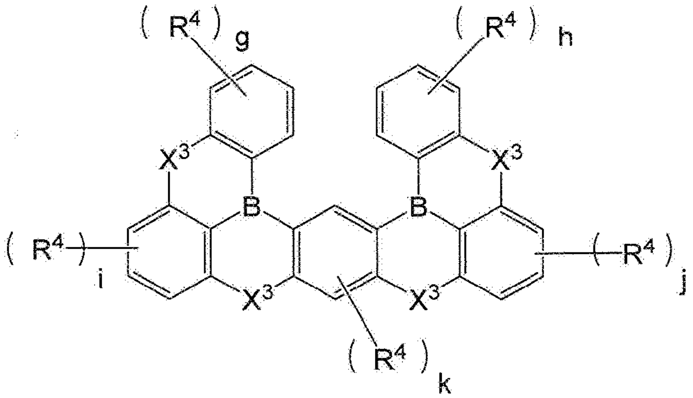

(8 b)

Here, $X^3$ each independently represents N-$Ar^4$, O, or S, and at least one $X^3$ represents N-$Ar^4$. $Ar^4$, $R^4$, g, h, i, j and k are as defined for the general formulae (7a) or (7b).

The aforementioned polycyclic aromatic compound preferably has a difference between excited singlet energy (S1) and excited triplet energy (T1) (ΔEST) of 0.20 eV or less and more preferably 0.10 eV or less.

The organic electroluminescent device of the present invention preferably contains a first host selected from the compounds represented by the general formula (1) above, a light emitting dopant, and a second host, and among these, it is more preferable to contain a compound represented by the following general formula (9), or (12), or (13) as the second host. First, the general formula (9) is as follows.

[C22]

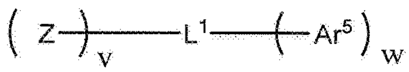

(9)

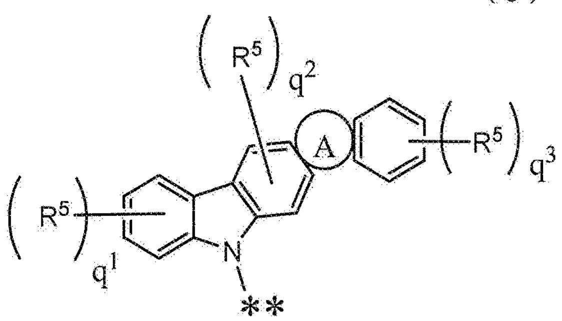

(1 0)

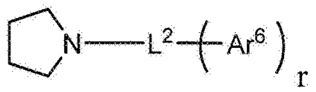

(1 1)

wherein Z in general formula (9) is an indolocarbazole ring-containing group represented by general formula (10), "* *" represents a bonding site to $L^1$. The ring A in general formula (10) is also a heterocyclic ring represented by general formula (11), and this ring A is condensed with the adjacent ring at arbitrary position thereof.

$L^1$ and $L^2$ are each independently a substituted or unsubstituted aromatic hydrocarbon group having 6 to 18 carbon atoms, or a substituted or unsubstituted aromatic heterocyclic group having 3 to 17 carbon atoms.

$Ar^5$ and $Ar^6$ are each independently deuterium, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups.

Furthermore, $R^5$ is each independently deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.

Furthermore, v represents the number of substitutions and represents an integer of 1 to 3, w represents the number of substitutions and represents an integer of 0 to 3, $q^1$ and $q^3$ represent the number of substitutions and each independently represent an integer of 0 to 4, $q^2$ represents the number of substitutions and represents an integer of 0 to 2, and r represents the number of substitutions and represents an integer of 0 to 3.

The general formula (9) may also be represented by the following general formula (9a) or (9b).

[C23]

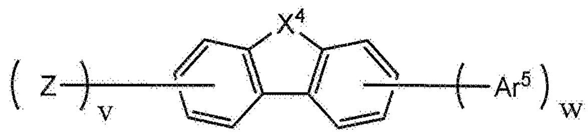

(9a)

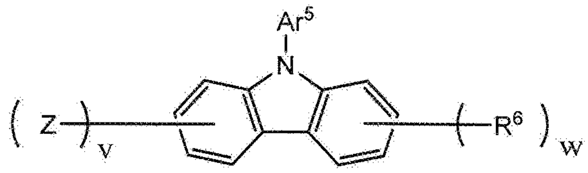

(9b)

wherein Z, $Ar^5$, v, and w are as defined for the general formula (9), and $X^4$ represents O or S. $R^6$ is each independently deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.

Also, the general formulae (12) and (13) are as follows.

[C24]

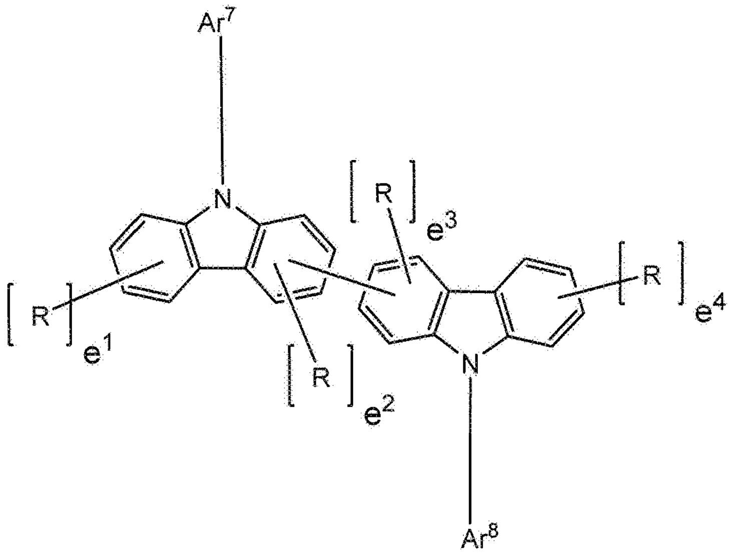

(12)

wherein $Ar^7$ and $Ar^8$ represent each independently a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 aromatic rings of these aromatic groups.

R each independently represents deuterium, or an aliphatic hydrocarbon group having 1 to 10 carbon atoms.
$e^1$ to $e^4$ represent the number of substitutions, $e^1$ and $e^4$ independently represent an integer of 0 to 4, and $e^2$ and $e^3$ independently represent an integer of 0 to 3.

Furthermore, as another example, the organic electroluminescent device of the present invention preferably contains a compound represented by the following general formula (13) as the second host.

[C25]

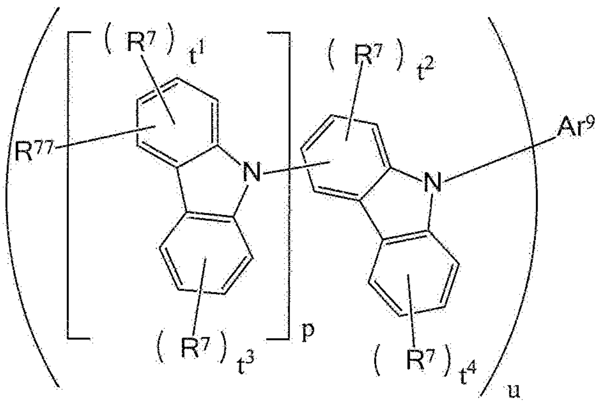

(13)

wherein $Ar^9$ represents a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 aromatic rings thereof.

$R^7$ each independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.
$R^{77}$ independently represents hydrogen, deuterium, an aliphatic hydrocarbon group having 1 to 10 carbon atoms, or a substituted or unsubstituted aromatic hydrocarbon group having 6 to 18 carbon atoms.
$t^1$ to $t^4$ each represent the number of substitutions, $t^3$ and $t^4$ independently represent an integer of 0 to 4, and $t^1$ and $t^2$ independently represent an integer of 0 to 3. p represents the number of repetitions and independently represents an integer of 1 to 4, and u represents the number of substitutions and represents an integer of 1 or 2. When u is 2, the general formula (13) may be symmetric or asymmetric.

The first host and the second host in the present invention have different structures.

Advantageous Effects of Invention

The organic EL device using the compound of the present invention can be an organic EL device with high emission efficiency and a long lifetime.

The reason for the organic EL device of the present invention having high emission efficiency is considered to be that in particular when the compound of the general formula (1) above is used as a host, excitons generated on a host move quickly to a light emitting dopant, resulting in a small energy loss, and excitons generated on a light emitting dopant are less likely to move to the host, causing a small energy loss. Also, the reason for the high emission efficiency is conjectured as follows: The biscarbazole ring of the compound represented by the general formula (1) above is characteristic in that holes are easily injected thereinto, and the nitrogen-containing six-membered ring of the compound represented by the general formula (1) above is characteristic in that electrons are easily injected thereinto, whereby the balance between holes and electrons are maintained in the light emission layer. In addition the reason for the long lifetime of the organic EL device of the present invention is probably as follows: when a voltage is applied to the organic EL device, holes are preferentially injected into the biscarbazole ring of the compound of the present invention represented by the general formula (1) above

and electrons are preferentially injected into the compound of the present invention including the nitrogen-containing 6-membered ring compound of the compound of the present invention represented by the general formula (1) above, whereby the electrochemical load on the light emitting dopant is reduced. Moreover, the use of compound represented by the general formula (9), (12), or (13) above as the second host is presumed to further reduce the electrochemical load on the light emitting dopant.

Incidentally, the polycyclic aromatic compound represented by the general formula (7a) or (7b) above, specifically the polycyclic aromatic compound represented by the general formula (8a) or (8b) above, can exhibit blue light emission with high efficiency by utilizing the TADF mechanism, however, they have low resistance to holes and electrons, making it difficult to ensure a practical device lifetime when used in combination with a conventionally known host material. On the contrary, the compound represented by the general formula (1) above according to the present invention has higher resistance to holes and electrons than these known compounds, as a result of which it can be used as an organic EL device with a longer lifetime.

Brief Description of Drawing

[Figure 1] Figure 1 shows a cross-sectional view of one example of the organic EL device.

Description of Embodiments

The present invention relates to the compound represented by the general formula (1) above, and particularly relates to a compound represented by the general formula (1) used as a material for an organic electroluminescent device (organic EL device).

Also, the organic EL device of the present invention includes one or more light emitting layers between an anode and a cathode opposite to each other, wherein at least one of the emitting layers contains a host selected from the compounds represented by the aforementioned general formula (1), and a light emitting dopant, and preferably includes a first host selected from the compounds represented by the aforementioned general formula (1), a second host, and a light emitting dopant. More preferably, at least one of the emitting layers contains a second host selected from the compound represented by the above-described general formula (9), the compound represented by the above-described general formula (12), or the compounds represented by the above-described general formula (13), and also contains the polycyclic aromatic compound represented by the above-described general formula (7a) or (7b), more specifically the polycyclic aromatic compound represented by the above-described general formula (8a) or (8b), as a light emitting dopant, or contains a phosphorescent dopant as a light emitting dopant.

First, in the present invention, the compound represented by the general formula (1) above will be described.

In the general formula (1), $Ar^1$ represents the general formula (2) above. n and m represent the number of repetitions and each independently represent an integer of 1 to 3, with at least one of n and m representing 2 or 3. "*" represents a bonding site to the general formula (1) above.

$R^1$ independently represents deuterium, an aliphatic hydrocarbon group having 1 to 10 carbon atoms, or a substituted or unsubstituted aromatic hydrocarbon group having 6 to 18 carbon atoms. Preferably, $R^1$ is deuterium, an aliphatic hydrocarbon group having 1 to 8 carbon atoms, or a substituted or unsubstituted aromatic hydrocarbon group having 6 to 12 carbon atoms. More preferably, $R^1$ is deuterium or a substituted or unsubstituted aromatic hydrocarbon group having 6 to 10 carbon atoms.

When $R^1$ is an aliphatic hydrocarbon group having 1 to 10 carbon atoms, it may be any of linear, branched, and cyclic aliphatic hydrocarbon groups, and specific examples thereof can include a linear saturated hydrocarbon group such as a methyl group, an ethyl group, a n-propyl group, a n-butyl group, a n-pentyl group, a n-hexyl group, a n-octyl group, a n-dodecyl group, a n-tetradecyl group, or a n-octadecyl group, a branched saturated hydrocarbon group such as an isopropyl group, an isobutyl group, a tert-butyl group, a neopentyl group, a 2-ethylhexyl group, or a 2-hexyloctyl group, and a saturated alicyclic hydrocarbon group such as a cyclopentyl group, a cyclohexyl group, a cyclooctyl group, a 4-butylcyclohexyl group, or a 4-dodecylcyclohexyl group. Preferably, $R^1$ is a methyl group, an ethyl group, a n-propyl group, a n-butyl group, a tert-butyl group, a neopentyl group, or a cyclohexyl group.

When $R^1$ is an unsubstituted aromatic hydrocarbon group having 6 to 18 carbon atoms, specific examples thereof include a group produced from benzene, naphthalene, acenaphthene, acenaphthylene, anthracene, chrysene, pyrene, phenanthrene, triphenylene, fluorene, or benzo[a]anthracene. Preferred examples thereof include a group produced from benzene, naphthalene, anthracene, chrysene, pyrene, phenanthrene, triphenylene, or fluorene, and more preferably a group produced from benzene or naphthalene.

s and c represent the number of substitutions, s independently represents an integer of 0 to 3, c independently represents an integer of 0 to 4, s is preferably 0 to 2, and c is preferably 0 to 2. Here, when $R^1$ is deuterium, s is preferably 2 or 3, and c is preferably 2 to 4.

$R^{11}$ independently represents hydrogen, deuterium, an aliphatic hydrocarbon group having 1 to 10 carbon

atoms, or a substituted or unsubstituted aromatic hydrocarbon group having 6 to 18 carbon atoms. Preferably, $R^{11}$ is hydrogen, deuterium, an aliphatic hydrocarbon group having 1 to 8 carbon atoms, or a substituted or unsubstituted aromatic hydrocarbon group having 6 to 12 carbon atoms. More preferably, $R^{11}$ is hydrogen, deuterium, or a substituted or unsubstituted aromatic hydrocarbon group having 6 to 10 carbon atoms.

When $R^{11}$ is an aliphatic hydrocarbon group having 1 to 10 carbon atoms and an unsubstituted aromatic hydrocarbon group having 6 to 18 carbon atoms, specific examples thereof are the same as described for $R^1$ above, respectively.

Of these, preferred examples thereof include a methyl group, an ethyl group, an n-propyl group, an n-butyl group, a tert-butyl group, a neopentyl group, a cyclohexyl group, or a group produced from benzene, naphthalene, anthracene, chrysene, pyrene, phenanthrene, triphenylene, or fluorene. A more preferred example thereof includes a group produced from benzene or naphthalene.

$Ar^0$ represents deuterium, an aliphatic hydrocarbon group having 1 to 10 carbon atoms, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups. Preferably, $Ar^0$ represents deuterium, a substituted or unsubstituted aromatic hydrocarbon group having 6 to 12 carbon atoms, or a substituted or unsubstituted linked aromatic group formed by linking 2 to 4 aromatic rings thereof. More preferably, $Ar^0$ is deuterium, a substituted or unsubstituted aromatic hydrocarbon group having 6 to 10 carbon atoms, or a substituted or unsubstituted linked aromatic group formed by linking 2 to 3 aromatic rings thereof.

When $Ar^0$ is an aliphatic hydrocarbon group having 1 to 10 carbon atoms and an unsubstituted aromatic hydrocarbon group having 6 to 18 carbon atoms, specific examples thereof are the same as described for $R^1$ above, respectively.

Of these, preferred examples thereof include a methyl group, an ethyl group, an n-propyl group, an n-butyl group, a tert-butyl group, a neopentyl group, a cyclohexyl group, or a group produced from an aromatic hydrocarbon group such as benzene, naphthalene, anthracene, chrysene, pyrene, phenanthrene, triphenylene, or fluorene. A more preferred example thereof includes a group produced from benzene or naphthalene.

When $Ar^0$ is an unsubstituted aromatic heterocyclic group having 3 to 17 carbon atoms, specific examples thereof include a group produced from a nitrogen-containing aromatic compound having a pyrrole ring such as pyrrole, pyrrolopyrrole, indole, isoindole, pyrroloisoindole, or carboline, thiophene, benzothiophene, dibenzothiophene, furan, benzofuran, dibenzofuran, carbazole, pyridine, pyrimidine, triazine, quinoline, isoquinoline, quinazoline, or quinoxaline. Preferred examples thereof include a group produced from thiophene, benzothiophene, dibenzothiophene, furan, benzofuran, dibenzofuran, or carbazole, and more preferred examples thereof include a group produced from dibenzothiophene, dibenzofuran, or carbazole.

When $Ar^0$ is an unsubstituted linked aromatic group, specific examples thereof include a group formed by linking 2 to 8 aromatic groups described in the aforementioned specific examples of the unsubstituted aromatic hydrocarbon group having 6 to 18 carbon atoms and the unsubstituted aromatic heterocyclic group having 3 to 17 carbon atoms.

$a^0$ represents the number of substitutions and represents an integer of 0 to 3, and is preferably an integer of 0 or 1. Here, when $A^0$ is deuterium, $a^0$ is preferably an integer of 2 or 3.

$X^1$ independently represents N or C-$R^2$, and it is preferred that at least one $X^1$ represents N, and that the each $X^1$ represents N.

$R^2$ independently represents hydrogen, deuterium, an aliphatic hydrocarbon group having 1 to 10 carbon atoms, a substituted or unsubstituted aromatic hydrocarbon group having 6 to 18 carbon atoms, or a substituted or unsubstituted linked aromatic group formed by linking 2 to 8 of the aromatic hydrocarbon groups. Preferably, $R^2$ is hydrogen, deuterium, an aliphatic hydrocarbon group having 1 to 8 carbon atoms, a substituted or unsubstituted aromatic hydrocarbon group having 6 to 12 carbon atoms, or a substituted or unsubstituted linked aromatic group formed by linking 2 to 3 of the aromatic hydrocarbon groups. More preferably, $R^2$ is hydrogen, deuterium, a substituted or unsubstituted aromatic hydrocarbon group having 6 to 10 carbon atoms, or a substituted or unsubstituted linked aromatic group formed by linking 2 to 3 of the aromatic hydrocarbon groups.

When $R^2$ is an aliphatic hydrocarbon group having 1 to 10 carbon atoms and an unsubstituted aromatic hydrocarbon group having 6 to 18 carbon atoms, specific examples thereof are the same as described for $R^1$ above.

Of these, preferred examples thereof include a methyl group, an ethyl group, an n-propyl group, an n-butyl group, a tert-butyl group, a neopentyl group, a cyclohexyl group, or an aromatic hydrocarbon group produced by removing one hydrogen atom from an aromatic hydrocarbon selected from benzene, naphthalene, anthracene, chrysene, pyrene, phenanthrene, triphenylene, or fluorene. A more preferred example thereof includes a group produced from benzene or naphthalene.

When $R^2$ is an unsubstituted linked aromatic group, specific examples thereof include a group formed by linking the 2 to 8 aromatic groups described in the aforementioned specific examples of the unsubstituted aromatic hydrocarbon group having 6 to 18 carbon atoms.

$Ar^2$ and $Ar^3$ represent hydrogen, deuterium, an aliphatic hydrocarbon group having 1 to 10 carbon atoms, or a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups. Preferably, $Ar^2$ and $Ar^3$ represent a substituted or unsubstituted aromatic hydrocarbon group having 6 to 12 carbon atoms, a substituted or unsubstituted aromatic heterocyclic group having 6 to 17 carbon atoms, or a substituted or unsubstituted linked aromatic group formed by linking 2 to 4 aromatic rings thereof. More preferably, $Ar^2$ and $Ar^3$ represent a substituted or unsubstituted aromatic hydrocarbon group having 6 to 10 carbon atoms, a substituted or unsubstituted aromatic heterocyclic group having 3 to 17 carbon atoms, or a substituted or unsubstituted linked aromatic group formed by linking 2 to 3 aromatic rings thereof.

When $Ar^2$ and $Ar^3$ are each an aliphatic hydrocarbon group having 1 to 10 carbon atoms and an unsubstituted aromatic hydrocarbon group having 6 to 18 carbon atoms, specific examples thereof are the same as described for $R^1$ above, respectively.

Of these, preferred examples thereof include a methyl group, an ethyl group, an n-propyl group, an n-butyl group, a tert-butyl group, a neopentyl group, a cyclohexyl group, or a group produced from an aromatic hydrocarbon group such as benzene, naphthalene, anthracene, chrysene, pyrene, phenanthrene, triphenylene, or fluorene. A more preferred example thereof includes a group produced from benzene or naphthalene.

When $Ar^2$ and $Ar^3$ are each an unsubstituted aromatic heterocyclic group having 3 to 17 carbon atoms, specific examples thereof are the same as described for $Ar^0$ above.

Among these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

When $Ar^2$ and $Ar^3$ are each an unsubstituted linked aromatic group, specific examples thereof are the same as described for $Ar^0$ above.

$Ar^1$ preferably includes at least one linked carbazole structure represented by any of the formulae (4a) to (4c) above. a and b represent the number of substitutions, a independently represents an integer of 0 to 4, and b independently represents an integer of 0 to 3. a is preferably an integer of 0 to 3, and b is preferably an integer of 0 to 2. Here, when $R^1$ is deuterium, a is preferably an integer of 2 to 4, and b is preferably an integer of 2 or 3. "※" represents a bonding site to the benzene ring in the general formula (2) above.

$Ar^1$ is preferably represented by any of the formulae (5a) to (5e) above.

The general formula (1) above is more preferably represented by any of the formulae (6a) to (6c) above. f in these formulae independently represents the number of substitutions and represents an integer of 0 to 5, and f is preferably 0 to 2. Here, when $R^1$ is deuterium, f is preferably 2 to 5.

Among $Ar^1$ to $Ar^3$ in the general formula (1) above, at least one thereof preferably contains a deuterium atom, and at least one of $Ar^2$ and $Ar^3$ more preferably contains a deuterium atom. Also, at least one $R^1$ is preferably deuterium.

The compound of the present invention is suitable as a material for an organic EL device. It is excellent as a host used in a light emitting layer of an organic EL device, but can also be used in a hole blocking layer, a light emitting material, or the like. The organic EL device of the present invention contains a host selected from the compounds represented by the general formula (1) and the light emitting dopant. In the organic EL device of the present invention, the compound represented by the general formula (7a) or (7b) above suitably contained in a light emitting layer as a dopant, will be described.

The ring J, the ring K, the ring C, the ring D, the ring E, the ring F, the ring G, and the ring H are each independently a substituted or unsubstituted aromatic hydrocarbon ring having 6 to 24 carbon atoms, or a substituted or unsubstituted aromatic heterocyclic ring having 3 to 17 carbon atoms, preferably an aromatic hydrocarbon ring having 6 to 20 carbon atoms, or an aromatic heterocyclic ring having 3 to 15 carbon atoms. The ring J, the ring K, the ring C, the ring D, the ring E, the ring F, the ring G, and the ring H are the aromatic hydrocarbon rings or aromatic heterocyclic rings as described above, and therefore are also referred to as aromatic rings.

Specific examples of the aromatic ring include a ring including benzene, naphthalene, acenaphthene, acenaphthylene, azulene, anthracene, chrysene, pyrene, phenanthrene, triphenylene, fluorene, benzo[a]anthracenepyridine, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, or carbazole. The aromatic ring is more preferably a benzene ring, a naphthalene ring, an anthracene ring, a triphenylene ring, a phenanthrene ring, a pyrene ring, a pyridine ring, a dibenzofuran ring, a dibenzothiophene ring, or a carbazole ring.

$Y^1$ each independently is B, P, P=O, P=S, Al, Ga, As, Si-$R^3$ or Ge-$R^3$, preferably B, P, P=O or P=S, and more preferably B.

$R^3$ each independently is an aliphatic hydrocarbon group having 1 to 10 carbon atoms, a substituted or

unsubstituted aromatic hydrocarbon group having 6 to 18 carbon atoms, or a substituted or unsubstituted aromatic heterocyclic group having 3 to 17 carbon atoms, and preferably an aliphatic hydrocarbon group having 1 to 8 carbon atoms, a substituted or unsubstituted aromatic hydrocarbon group having 6 to 12 carbon atoms, or a substituted or unsubstituted aromatic heterocyclic group having 3 to 15 carbon atoms. $R^3$ is more preferably a substituted or unsubstituted aromatic hydrocarbon group having 6 to 10 carbon atoms, or a substituted or unsubstituted aromatic heterocyclic group having 3 to 12 carbon atoms.

When $R^3$ is an aliphatic hydrocarbon group having 1 to 10 carbon atoms and an unsubstituted aromatic hydrocarbon group having 6 to 18 carbon atoms, specific examples thereof each are the same as described for $R^1$ above.

Of these, preferred examples thereof include a methyl group, an ethyl group, an n-propyl group, a n-butyl group, a tert-butyl group, a neopentyl group, a cyclohexyl group, or a group produced from benzene, naphthalene, anthracene, chrysene, pyrene, phenanthrene, triphenylene, or fluorene, and, more preferably a group produced from benzene, naphthalene.

When $R^3$ is an unsubstituted aromatic heterocyclic group having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

$X^2$ is each independently O, N-$Ar^4$, S or Se, preferably O, N-$Ar^4$ or S, and more preferably O or N-$Ar^4$.

$Ar^4$ is each independently a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these groups, and preferably a substituted or unsubstituted aromatic hydrocarbon group having 6 to 12 carbon atoms, a substituted or unsubstituted aromatic heterocyclic group having 3 to 15 carbon atoms, or a substituted or unsubstituted linked aromatic group formed by linking 2 to 6 aromatic rings thereof. More preferably $Ar^4$ is a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 4 aromatic rings thereof. Still more preferably, $Ar^4$ is a substituted or unsubstituted aromatic hydrocarbon group having 6 to 10 carbon atoms.

When $Ar^4$ is an unsubstituted aromatic hydrocarbon group having 6 to 18 carbon atoms, specific examples thereof are the same as described for $R^1$ above.

Of these, preferred examples thereof include a group produced from benzene, naphthalene, anthracene, chrysene, pyrene, phenanthrene, triphenylene, or fluorene. More preferred examples thereof include a group produced from benzene, naphthalene.

When $Ar^4$ is an unsubstituted aromatic heterocyclic group having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

When $Ar^4$ is an unsubstituted linked aromatic group, specific examples thereof are the same as described for $Ar^0$.

N-$Ar^4$ is optionally bonded to any of the ring J, the ring K, the ring C, the ring D, the ring E, the ring F, the ring G, or the ring H to form a heterocyclic ring containing N.

$R^4$ each independently represents a cyano group, deuterium, a diarylamino group having 12 to 44 carbon atoms, an arylheteroarylamino group having 12 to 44 carbon atoms, an aliphatic hydrocarbon group having 1 to 10 carbon atoms, a substituted or unsubstituted aromatic hydrocarbon group having 6 to 18 carbon atoms, a substituted or unsubstituted aromatic heterocyclic group having 3 to 17 carbon atoms. $R^4$ is preferably a diarylamino group having 12 to 36 carbon atoms, an arylheteroarylamino group having 12 to 36 carbon atoms, a diheteroarylamino group having 12 to 36 carbon atoms, a substituted or unsubstituted aromatic hydrocarbon group having 6 to 12 carbon atoms, or a substituted or unsubstituted aromatic heterocyclic group having 3 to 15 carbon atoms. $R^4$ is more preferably a diarylamino group having 12 to 24 carbon atoms, an arylheteroarylamino group having 12 to 24 carbon atoms, a diheteroarylamino group having 12 to 24 carbon atoms, a substituted or unsubstituted aromatic hydrocarbon group having 6 to 10 carbon atoms, or a substituted or unsubstituted aromatic heterocyclic group having 3 to 12 carbon atoms.

When $R^4$ representing a diarylamino group having 12 to 44 carbon atoms, an arylheteroarylamino group having 12 to 44 carbon atoms, a diheteroarylamino group having 12 to 44 carbon atoms, an aliphatic hydrocarbon group having 1 to 10 carbon atoms, specific examples thereof include diphenylamino, dibiphenylamino, phenylbiphenylamino, naphthylphenylamino, dinaphthylamino, dianthranylamino, diphenanthrenylamino, dipyrenylamino, dibenzofuranylphenylamino, dibenzofuranylbiphenylamino, dibenzofuranylnaphthylamino, dibenzofuranylanthranylamino, dibenzofuranylphenanthrenylamino, dibenzofuranylpyrenylamino, bis-dibenzofuranylamino, carbazolylphenylamino, carbazolylnaphthylamino, carbazolylanthranylamino, carbazolylphenanthrenylamino, carbazolylpyrenylamino, dicarbazolylamino, methyl, ethyl, propyl, butyl, pentyl, hexyl, heptyl, octyl, nonyl. $R^4$ is preferably diphenylamino, dibiphenylamino, phenylbiphenylamino, naphthylphenylamino, dinaphthylamino, dianthranylamino, diphenanthrenylamino, dipyrenylamino. $R^4$ is more preferably diphenylamino, dibiphenylamino, phenylbiphenylamino, naphthylphenylamino, dinaphthylamino, dibenzofuranylphenylamino, or carbazolylphenylamino.

When $R^4$ is an unsubstituted aromatic hydrocarbon group having 6 to 18 carbon atoms, specific examples thereof each are the same as described for $R^1$ above.

Of these, preferred examples thereof include a group produced from benzene, naphthalene, anthracene, chrysene, pyrene, phenanthrene, triphenylene, or fluorene. More preferred examples thereof include a phenyl or a naphthyl group.

When $R^4$ is an unsubstituted aromatic heterocyclic group having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

g and h represent the number of substitutions, and each independently represent an integer of 0 to 4, preferably an integer of 0 to 2, and more preferably an integer of 0 or 1. i and j represent the number of substitutions, and each independently represent an integer of 0 to 3, preferably an integer of 0 to 2, and more preferably an integer of 0 or 1. k represents the number of substitutions, and an integer of 0 to 2 and preferably an integer of 0 or 1.

A preferred example of the polycyclic aromatic compound represented by the general formula (7a) above includes a boron-containing polycyclic aromatic compound represented by the following formula (8a), and a preferred example of the polycyclic aromatic compound represented by the general formula (7b) above includes a boron-containing polycyclic aromatic compound represented by the following formula (8b).

In the compound represented by the general formulae (8a) and (8b) above, $X^3$ each independently represents N-$Ar^4$, O, or S, and at least one $X^3$ represents N-$Ar^4$. The symbol common to the general formula (7a) or general formula (7b) has the same meaning.

The organic EL device of the present invention can include the compound represented by the general formula (9) above, the general formula (9a) above, the general formula (9b) above, the general formula (12) above or the general formula (13) above, as the second host.

In the general formula (9), Z is an indolocarbazole ring-containing group represented by formula (10), and "* *" in the formula represents a bonding site to $L^1$. The ring A in the formula is a heterocyclic ring represented by the general formula (11), and the ring A is condensed with the adjacent ring at arbitrary position thereof. Z is preferably an indolocarbazole ring-containing group represented by the following general formula (101).

[C26]

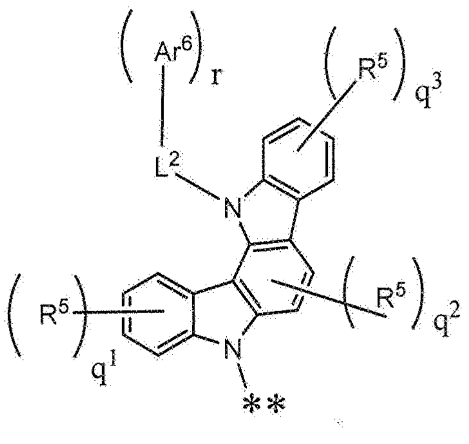

(101)

wherein "* *" in the formula (101) represents a bonding site to $L^1$.

$L^1$ and $L^2$ are each independently a substituted or unsubstituted aromatic hydrocarbon group having 6 to 18 carbon atoms or a substituted or unsubstituted aromatic heterocyclic group having 3 to 17 carbon atoms, preferably a substituted or unsubstituted aromatic hydrocarbon group having 6 to 12 carbon atoms or a substituted or unsubstituted aromatic heterocyclic group having 3 to 15 carbon atoms. More preferably $L^1$ and $L^2$ are each a substituted or unsubstituted aromatic hydrocarbon group having 6 to 10 carbon atoms or a substituted or unsubstituted aromatic heterocyclic group having 3 to 12 carbon atoms.

When $L^1$ and $L^2$ are unsubstituted aromatic hydrocarbon groups having 6 to 18 carbon atoms, specific examples

thereof are the same as described for $R^1$ above.

Of these, preferred examples thereof include a group produced from benzene, naphthalene, anthracene, chrysene, pyrene, phenanthrene, triphenylene, or fluorene. More preferred examples thereof include a group produced from benzene, naphthalene. $L^1$ is a $v^1$+$v^2$-valent group, and $L^2$ is a w+1-valent group.

When $L^1$ and $L^2$ are unsubstituted aromatic heterocyclic groups having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

$Ar^5$ and $Ar^6$ are each independently deuterium, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups, preferably a substituted or unsubstituted aromatic hydrocarbon group having 6 to 12 carbon atoms, a substituted or unsubstituted aromatic heterocyclic group having 3 to 15 carbon atoms, or a substituted or unsubstituted linked aromatic group formed by linking 2 to 4 of these groups, and more preferably a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 3 of these groups.

Still more preferred examples thereof include a substituted or unsubstituted aromatic hydrocarbon group having 6 to 10 carbon atoms or a substituted or unsubstituted aromatic heterocyclic group having 3 to 12 carbon atoms.

When $Ar^5$ and $Ar^6$ are unsubstituted aromatic hydrocarbon groups having 6 to 18 carbon atoms, specific examples thereof are the same as described for $R^1$ above.

Of these, preferred examples thereof include a group produced from benzene, naphthalene, anthracene, chrysene, pyrene, phenanthrene, triphenylene, or fluorene. More preferred examples thereof include a group produced from benzene, naphthalene.

When $Ar^5$ and $Ar^6$ are each an unsubstituted aromatic heterocyclic group having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

When $Ar^5$ and $Ar^6$ are each an unsubstituted linked aromatic group, specific examples thereof are the same as described for $Ar^0$ above.

$R^5$ independently is deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms, and is preferably deuterium, a substituted or unsubstituted aromatic hydrocarbon group having 6 to 12 carbon atoms, or a substituted or unsubstituted aromatic heterocyclic group having 3 to 15 carbon atoms. More preferably, $R^5$ is deuterium, a substituted or unsubstituted aromatic hydrocarbon group having 6 to 10 carbon atoms or a substituted or unsubstituted aromatic heterocyclic group having 3 to 12 carbon atoms.

When $R^5$ is an aliphatic hydrocarbon group having 1 to 10 carbon atoms and an unsubstituted aromatic hydrocarbon group having 6 to 18 carbon atoms, specific examples thereof are the same as described for $R^1$ above, respectively.

Of these, preferred examples thereof include a methyl group, an ethyl group, an n-propyl group, an n-butyl group, a tert-butyl group, a neopentyl group, a cyclohexyl group, or a group produced from benzene, naphthalene, anthracene, chrysene, pyrene, phenanthrene, triphenylene, or fluorene. A more preferred example thereof includes a group produced from benzene or naphthalene.

When $R^5$ is an unsubstituted aromatic heterocyclic group having 3 to 17 carbon atoms, specific examples thereof are the same as described for $Ar^0$ above.

Among them, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

v represents the number of substitutions, represents an integer of 1 to 3, and is preferably represented by 1 or 2. w represents the number of substitutions, represents an integer of 0 to 3, and is preferably 0 to 2. $q^1$ and $q^3$ represent the number of substitutions and each independently represent an integer of 0 to 4, and is preferably represented by an integer of 0 to 2. $q^2$ represents the number of substitutions and represents an integer of 0 to 2, and is preferably represented by 0 or 1. r represents the number of substitutions and represents an integer of 0 to 3, and is preferably represented by 0 to 2.

The general formula (9) may be represented by the following general formula (9a) or (9b), wherein $X^4$ represents O or S. The symbols common to the general formula (9) have the same meaning.

$R^6$ is each independently deuterium, an aliphatic hydrocarbon group having 1 to 10 carbon atoms, a substituted or unsubstituted aromatic hydrocarbon group having 6 to 18 carbon atoms, or a substituted or unsubstituted aromatic

heterocyclic group having 3 to 17 carbon atoms, preferably deuterium, a substituted or unsubstituted aromatic hydrocarbon group having 6 to 12 carbon atoms, or a substituted or unsubstituted aromatic heterocyclic group having 3 to 15 carbon atoms. More preferably $R^6$ is deuterium, a substituted or unsubstituted aromatic hydrocarbon group having 6 to 10 carbon atoms, or a substituted or unsubstituted aromatic heterocyclic group having 3 to 12 carbon atoms.

When $R^6$ is an aliphatic hydrocarbon group having 1 to 10 carbon atoms and an unsubstituted aromatic hydrocarbon group having 6 to 18 carbon atoms, specific examples thereof each are the same as described for $R^1$ above.

Of these, preferred examples thereof include a methyl group, an ethyl group, an n-propyl group, a n-butyl group, a tert-butyl group, a neopentyl group, a cyclohexyl group, or a group produced from benzene, naphthalene, anthracene, chrysene, pyrene, phenanthrene, triphenylene, or fluorene. More preferred examples thereof include a group produced from benzene, naphthalene.

When $R^6$ is an unsubstituted aromatic heterocyclic group having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

Next, as another example, the compound represented by the general formula (12) above that is suitably contained as the second host in the organic EL element of the present invention, will be described. It is to be noted that the general formula (12) above is preferably represented by the following general formula (121).

[C27]

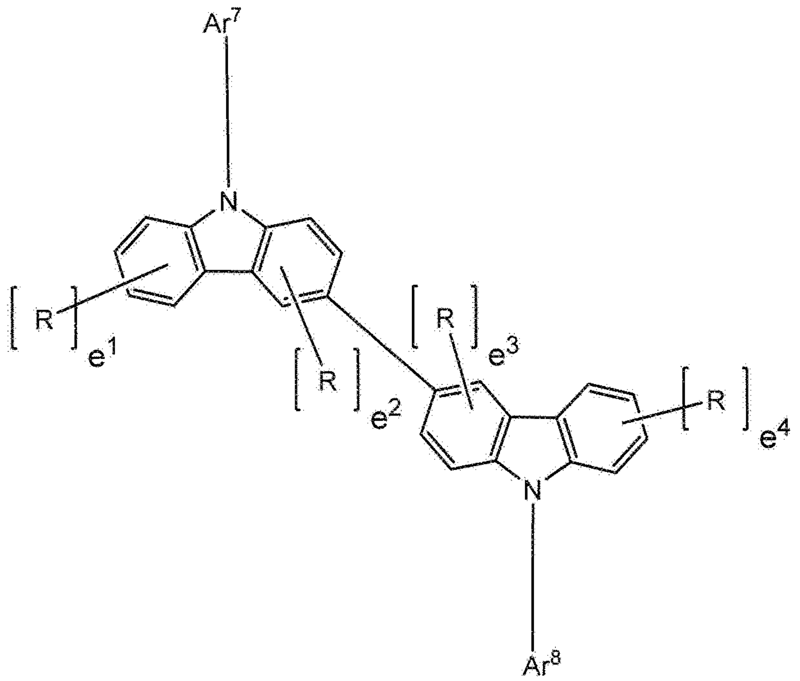

(1 2 1)

$Ar^7$ and $Ar^8$ are each independently a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 aromatic rings of these aromatic groups, preferably a substituted or unsubstituted aromatic hydrocarbon group having 6 to 12 carbon atoms, a substituted or unsubstituted aromatic heterocyclic group having 3 to 15 carbon atoms, or a substituted or unsubstituted linked aromatic group formed by linking 2 to 4 of these groups, and more preferably a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 3 of these groups.

When $Ar^7$ and $Ar^8$ are unsubstituted aromatic hydrocarbon groups having 6 to 18 carbon atoms, specific examples thereof are the same as described for $R^1$ above.

Of these, preferred examples thereof include a group produced from benzene, naphthalene, anthracene, chrysene, pyrene, phenanthrene, triphenylene, or fluorene. More preferred examples thereof include a group produced from benzene, naphthalene.

When $Ar^7$ and $Ar^8$ are unsubstituted aromatic heterocyclic groups having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

When $Ar^7$ and $Ar^8$ are unsubstituted linked aromatic groups, specific examples thereof are the same as described for $Ar^0$ above.

R each independently represents deuterium or an aliphatic hydrocarbon group having 1 to 10 carbon atoms.

When R is an aliphatic hydrocarbon group having 1 to 10 carbon atoms, specific examples thereof are the same as described for $R^1$ above.

Of these, preferred examples thereof include a methyl group, an ethyl group, an n-propyl group, an n-butyl group, a tert-butyl group, a neopentyl group, or a cyclohexyl group.

$e^1$ and $e^4$ represent the number of substitutions, and represent an integer of 0 to 4 and preferably represent an integer of 0 to 2. $e^2$ and $e^3$ represent the number of substitutions, and represent an integer of 0 to 3 and preferably represent an integer of 0 to 2.

Next, as another example, the compound represented by the general formula (13) above that is suitably contained as the second host in the organic EL element of the present invention, will be described.

Here, $Ar^9$ is a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 aromatic rings thereof, preferably a substituted or unsubstituted aromatic hydrocarbon group having 6 to 12 carbon atoms, a substituted or unsubstituted aromatic heterocyclic group having 6 to 15 carbon atoms, or a substituted or unsubstituted linked aromatic group formed by linking 2 to 4 of these groups, more preferably a substituted or unsubstituted aromatic hydrocarbon group having 6 to 10 carbon atoms, a substituted or unsubstituted aromatic heterocyclic group having 6 to 12 carbon atoms, or a substituted or unsubstituted linked aromatic group formed by linking 2 to 3 of these groups, and still more preferably, a substituted or unsubstituted aromatic hydrocarbon group having 6 to 10 carbon atoms, or a substituted or unsubstituted linked aromatic group formed by linking 2 to 3 of these groups.

When $Ar^9$ is an unsubstituted aromatic hydrocarbon group having 6 to 18 carbon atoms, specific examples thereof are the same as described for $R^1$ above.

Of these, preferred examples thereof include a group produced from benzene, naphthalene, anthracene, chrysene, pyrene, phenanthrene, triphenylene, or fluorene. More preferred examples thereof include a group produced from benzene, naphthalene.

When $Ar^9$ is an unsubstituted aromatic heterocyclic group having 3 to 17 carbon atoms, specific examples thereof can include a group produced from benzothiophene, dibenzothiophene, benzofuran, dibenzofuran, carbazole, quinoline, isoquinoline, quinazoline, or quinoxaline. Preferred examples thereof include a group produced from benzothiophene, dibenzothiophene, benzofuran, dibenzofuran, or carbazole, and more preferred examples thereof include dibenzothiophene, dibenzofuran, or carbazole.

When $Ar^9$ is an unsubstituted linked aromatic group, specific examples thereof are the same as described for $Ar^0$ above.

$R^7$ is each independently deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms, preferably deuterium, a substituted or unsubstituted aromatic hydrocarbon group having 6 to 12 carbon atoms, or a substituted or unsubstituted aromatic heterocyclic group having 3 to 15 carbon atoms. More preferably $R^7$ is deuterium, a substituted or unsubstituted aromatic hydrocarbon group having 6 to 10 carbon atoms, or a substituted or unsubstituted aromatic heterocyclic group having 3 to 12 carbon atoms.

When $R^7$ is an aliphatic hydrocarbon group having 1 to 10 carbon atoms and an unsubstituted aromatic hydrocarbon group having 6 to 18 carbon atoms, specific examples thereof each are the same as described for $R^1$ above.

Of these, preferred examples thereof include a methyl group, an ethyl group, an n-propyl group, a n-butyl group, a tert-butyl group, a neopentyl group, a cyclohexyl group, or a group produced from benzene, naphthalene, anthracene, chrysene, pyrene, phenanthrene, triphenylene, or fluorene. More preferred examples thereof include a group produced from benzene, naphthalene.

When $R^7$ is an unsubstituted aromatic heterocyclic group having 3 to 17 carbon atoms, specific examples thereof are the same as described for $Ar^0$ above.

Of these, preferred examples thereof include a group produced from thiophene, benzothiophene, dibenzothiophene, furan, benzofuran, dibenzofuran, or carbazole. More preferred examples thereof include a group produced from dibenzothiophene, dibenzofuran, or carbazole.

$R^{77}$ independently represents hydrogen, deuterium, an aliphatic hydrocarbon group having 1 to 10 carbon atoms, or a substituted or unsubstituted aromatic hydrocarbon group having 6 to 18 carbon atoms. Preferably $R^{77}$ is

hydrogen, deuterium, an aliphatic hydrocarbon group having 1 to 8 carbon atoms, or a substituted or unsubstituted aromatic hydrocarbon group having 6 to 12 carbon atoms. More preferably $R^{77}$ is hydrogen, deuterium, or a substituted or unsubstituted aromatic hydrocarbon group having 6 to 10 carbon atoms.

When $R^{77}$ is an aliphatic hydrocarbon group having 1 to 10 carbon atoms and an unsubstituted aromatic hydrocarbon group having 6 to 18 carbon atoms, specific examples thereof each are the same as described for $R^1$ above.

Of these, preferred examples thereof include a methyl group, an ethyl group, an n-propyl group, an n-butyl group, a tert-butyl group, a neopentyl group, a cyclohexyl group, or a group produced from benzene, naphthalene, anthracene, chrysene, pyrene, phenanthrene, triphenylene, or fluorene. More preferred examples thereof include a group produced from benzene, naphthalene.

$t^3$ and $t^4$ represent the number of substitutions, and independently represent an integer of 0 to 4, and are preferably represented by an integer of 0 to 2, and $t^1$ and $t^2$ represent the number of substitutions, and independently represent an integer of 0 to 3, and are preferably represented by an integer of 0 to 2, and p represents the number of repetitions, and independently represents an integer of 1 to 4, and is preferably represented by an integer of 1 or 2, and u represents the number of substitutions, and represents an integer of 1 or 2. When u is 2, the general formula (13) is symmetric or asymmetric.

The linked aromatic group as used herein refers to a group in which aromatic rings of an aromatic hydrocarbon group or an aromatic heterocyclic group are linked by a single bond, which may be linked in a linear or branched manner. The linked aromatic rings may be the same or different. When a group falls within the linked aromatic group, the group is considered not to be a substituted aromatic hydrocarbon group or aromatic heterocyclic group.

In the general formulae (1), (7a), (7b), (8a), (8b), (9), (9a), (9b), (10), (12) and (13), when $Ar^0$, $Ar^2$ to $Ar^9$, $R^1$ to $R^7$, $R^{11}$, $R^{77}$, R, $L^1$, and $L^2$ are aromatic hydrocarbon groups, aromatic heterocyclic groups, or linked aromatic groups, they may have a substituent, and the substituent is preferably deuterium, a cyano group, a triarylsilyl group having 18 to 36 carbon atoms, an aliphatic hydrocarbon group having 1 to 10 carbon atoms, a diarylamino group having 12 to 44 carbon atoms. Here, the substituent is the aliphatic hydrocarbon group having 1 to 10 carbon atoms, the substituent may be linear, branched, or cyclic. The number of substituents is 0 to 5, and preferably 0 to 2. When each of the aromatic hydrocarbon groups and aromatic heterocyclic groups has a substituent, the number of carbon atoms of the substituent is not included in the calculation of the number of carbon atoms. However, it is preferred that the total number of carbon atoms including the number of carbon atoms of the substituent satisfy the above range.

Specific examples of the above substituent include deuterium, cyano, methyl, ethyl, propyl, i-propyl, butyl, t-butyl, pentyl, cyclopentyl, hexyl, cyclohexyl, heptyl, octyl, nonyl, decyl, diphenylamino, naphthylphenylamino, dinaphthylamino, dianthranylamino, diphenanthrenylamino, dipyrenylamino, and triphenylsilyl groups. Preferred examples thereof include deuterium, cyano, methyl, ethyl, propyl, butyl, pentyl, hexyl, heptyl, octyl, diphenylamino, naphthylphenylamino, and dinaphthylamino groups.

Here, the hydrogen may be understood to be deuterium. Namely, a hydrogen atom and a hydrogen atom in the substituent in the compounds represented by the general formulae (1), (7a), (7b), (9), (9a), (9b), (12), and (13), may be partially or totally a deuterium atom.

An average deuteration ratio in the present invention will also be described, and for example, in the case of a compound represented by the general formula (1), the compound includes both a single compound and a compound composed of a mixture of two or more compounds represented by the general formula (1). Namely, when specifically described, an average deuteration ratio of 50% means that an average half of all hydrogen atoms is replaced with a deuterium atom, and it may be formed by a single compound or formed by a mixture of compounds with different deuteration ratios.

Specific examples of the compounds represented by the general formula (1) above are shown below, but are not limited to these exemplified compounds.

[C28]

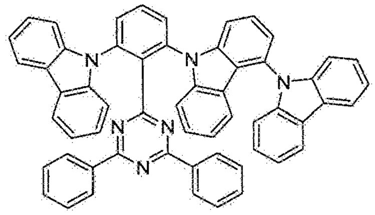

1-1

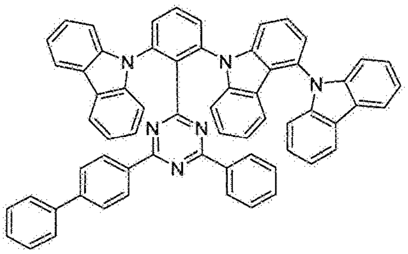

1-2

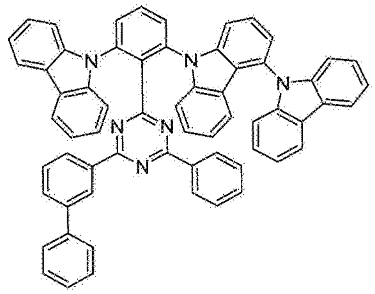

1-3

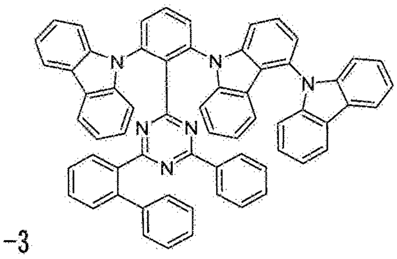

1-4

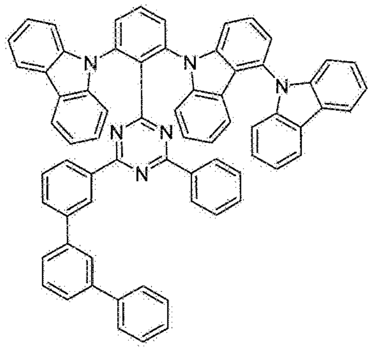

1-5

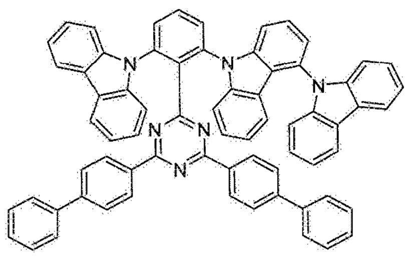

1-6

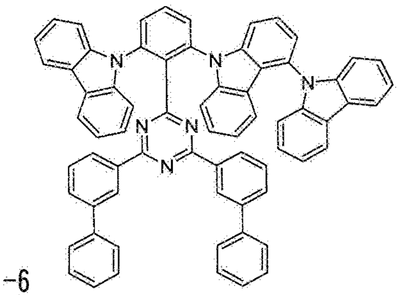

1-7

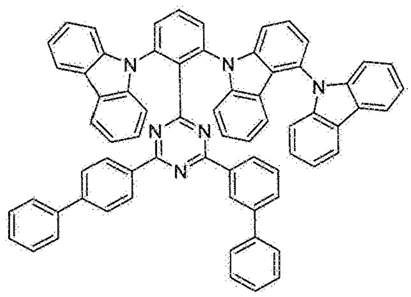

1-8

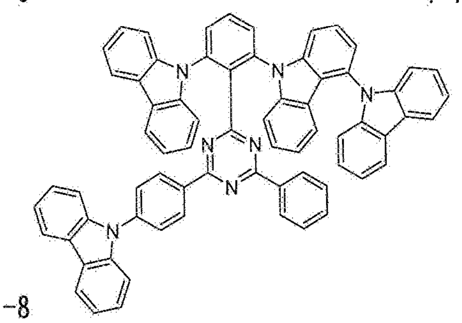

1-9

[C29]

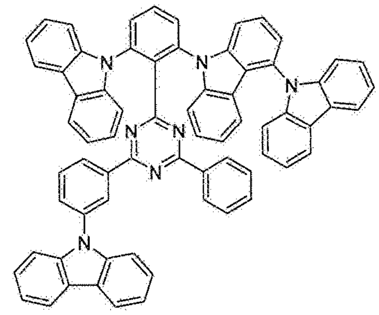

1-10

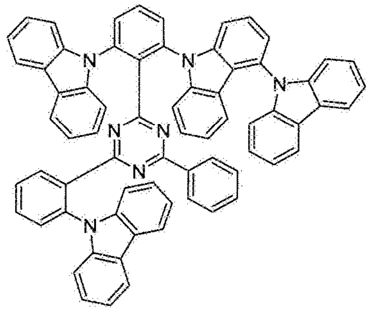

1-11

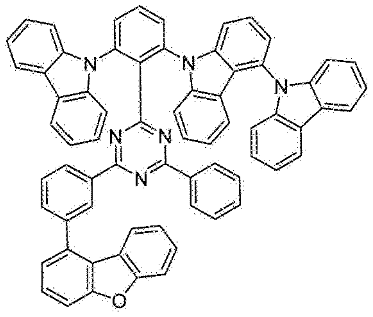

1-12

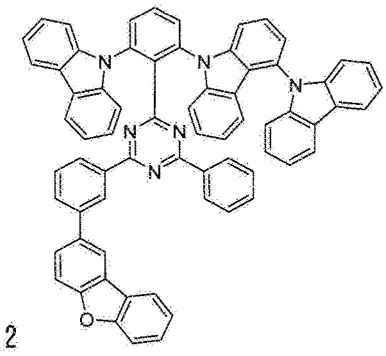

1-13

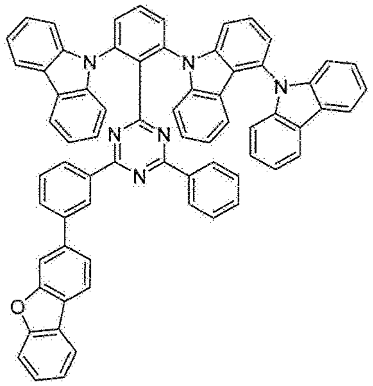

1-14

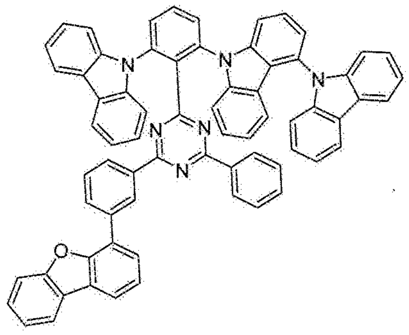

1-15

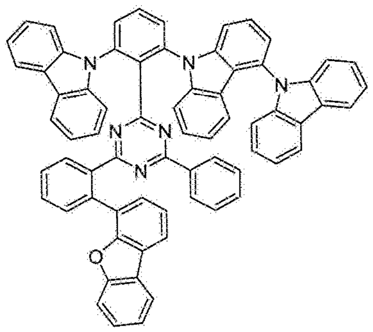

1-16

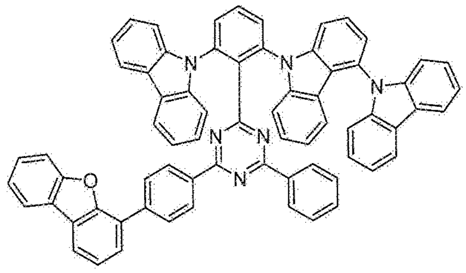

1-17

[C30]

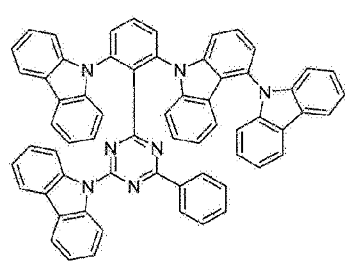

1-18

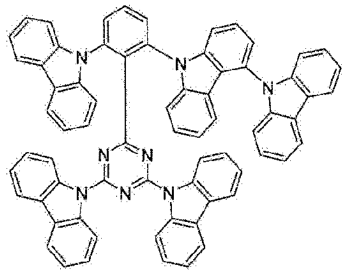

1-19

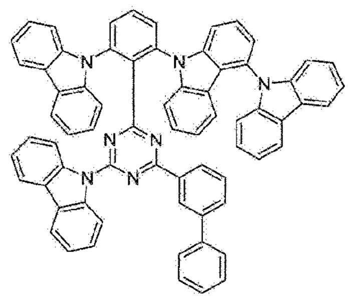

1-20

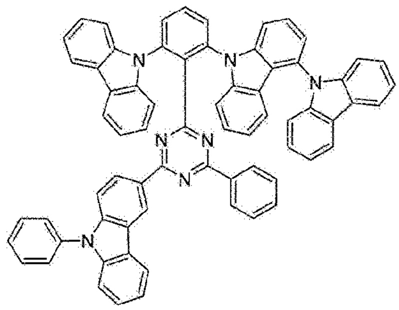

1-21

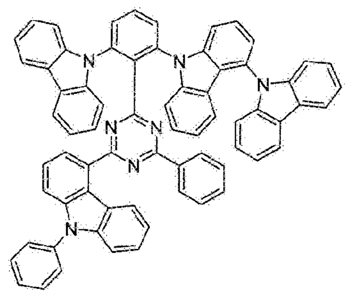

1-22

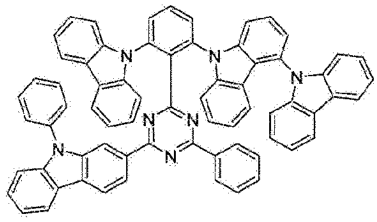

1-23

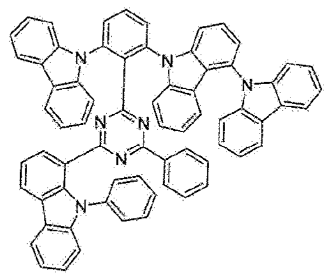

1-24

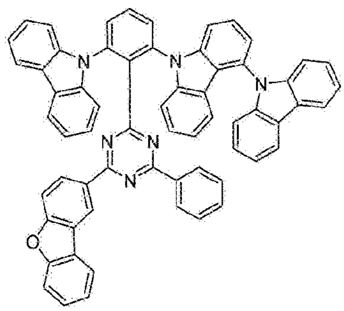

1-25

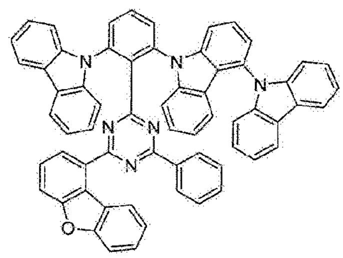

1-26

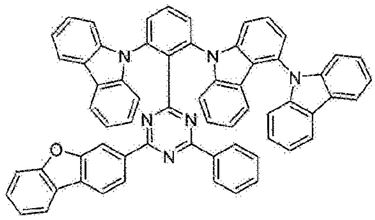

1-27

[C31]

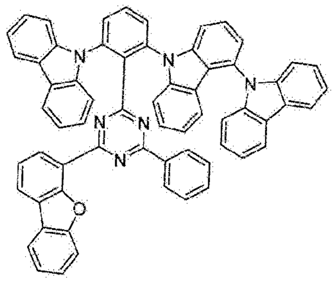

1-28

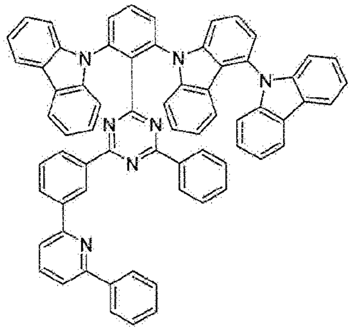

1-29

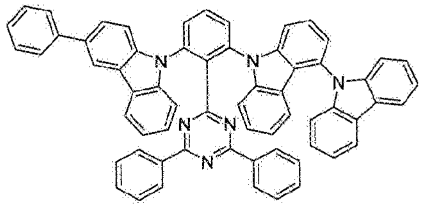

1-30

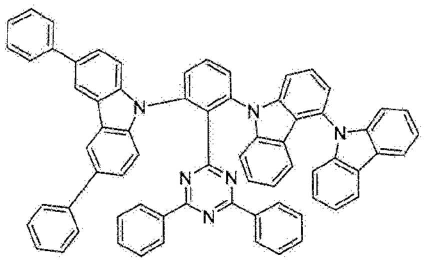

1-31

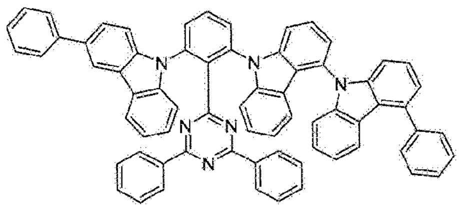

1-32

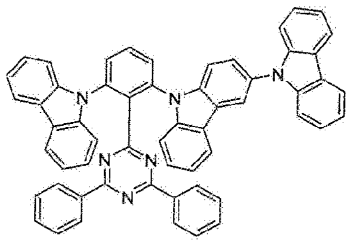

1-33

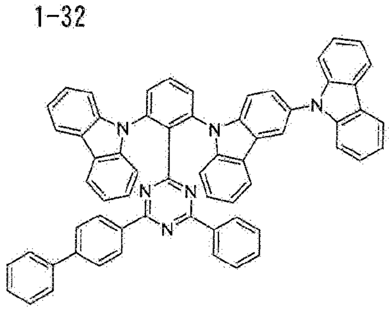

1-34

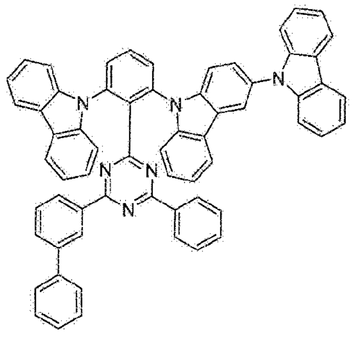

1-35

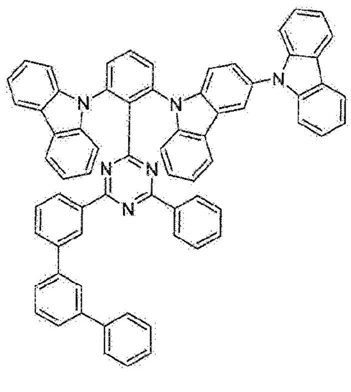

1-36

[C32]

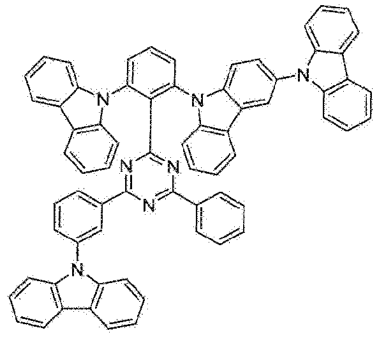

1-37

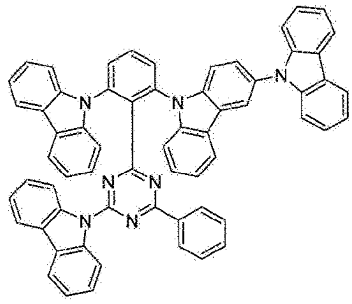

1-38

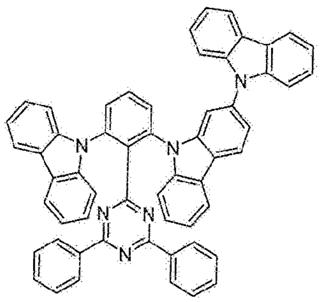

1-39

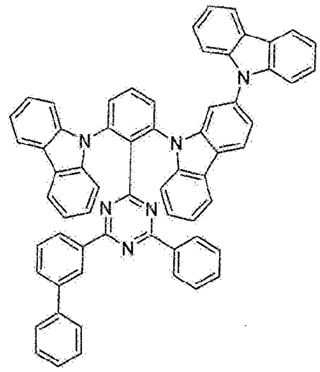

1-40

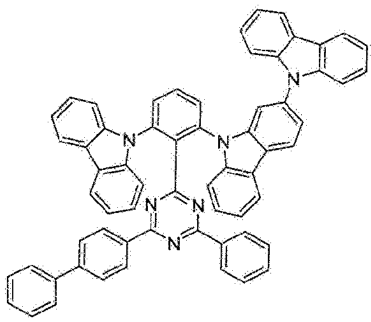

1-41

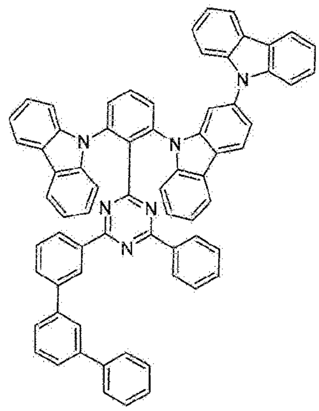

1-42

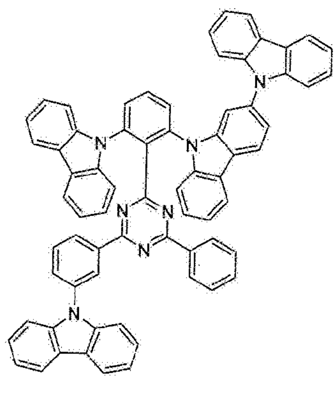

1-43

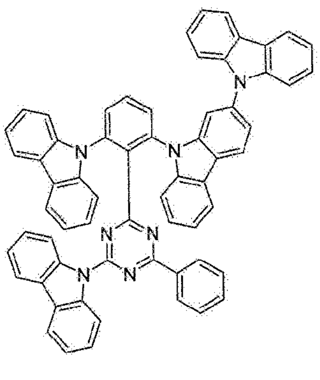

1-44

[C33]

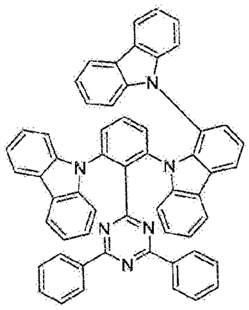

1-45

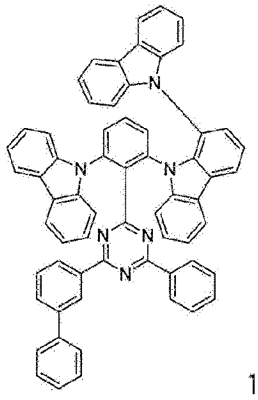

1-46

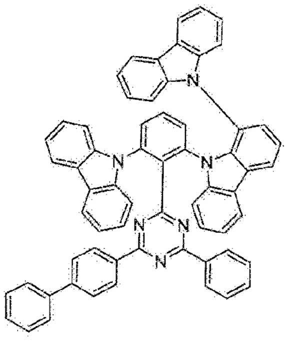

1-47

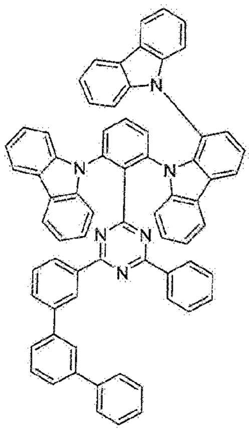

1-48

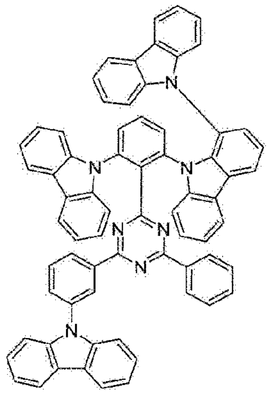

1-49

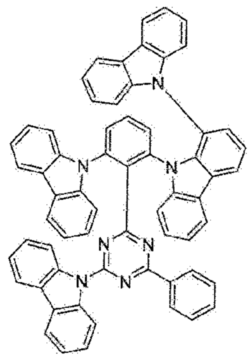

1-50

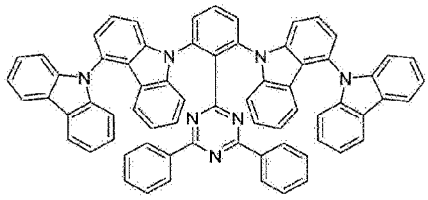

1-51

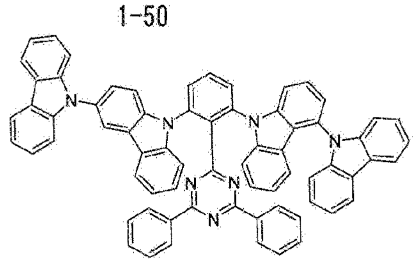

1-52

[C34]

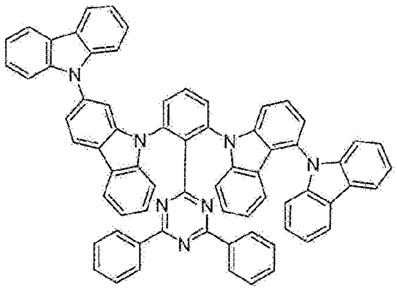
1-53

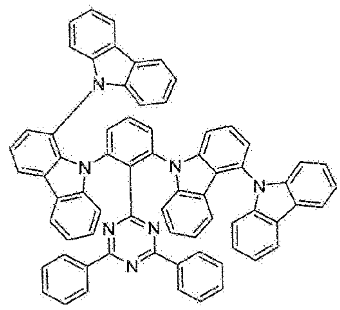
1-54

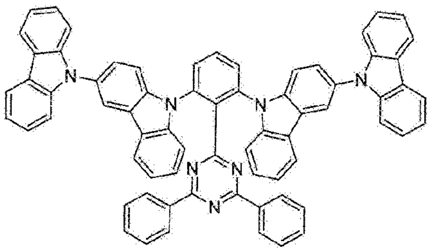
1-55

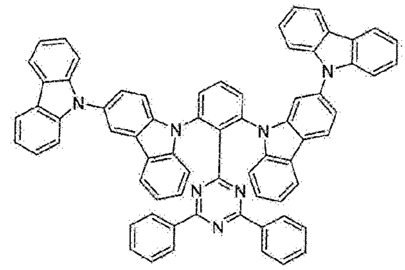
1-56

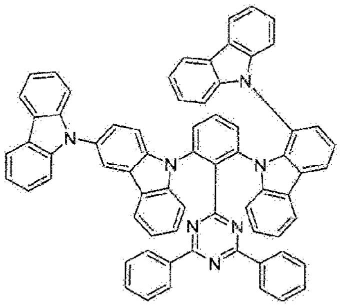
1-57

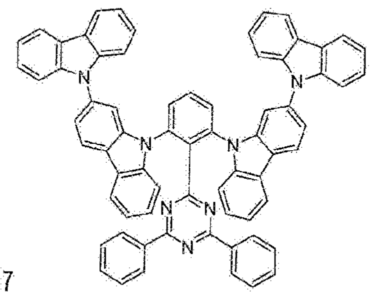
1-58

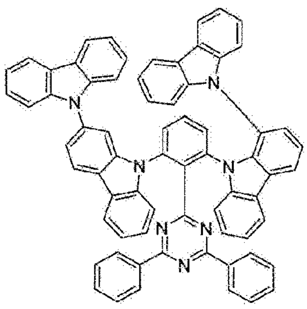
1-59

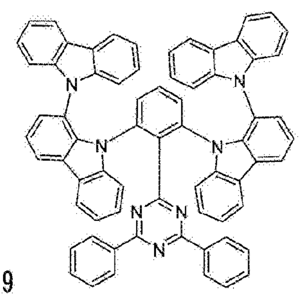
1-60

[C35]

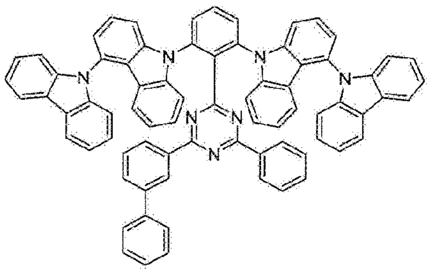

1-61

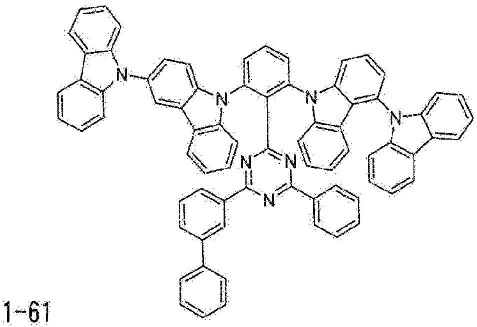

1-62

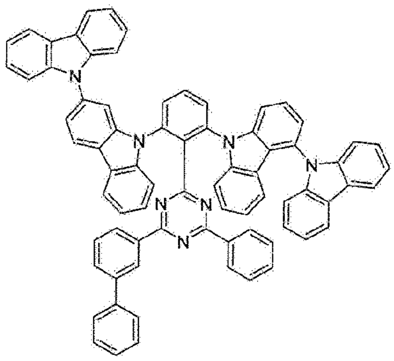

1-63

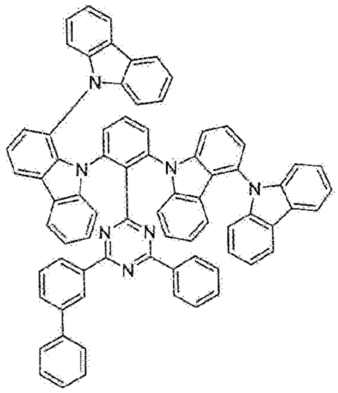

1-64

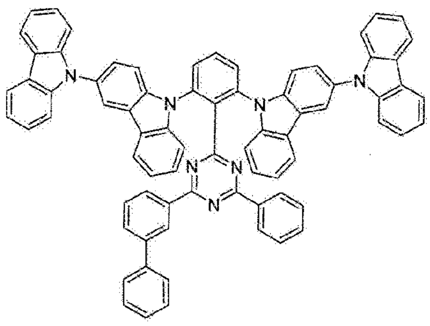

1-65

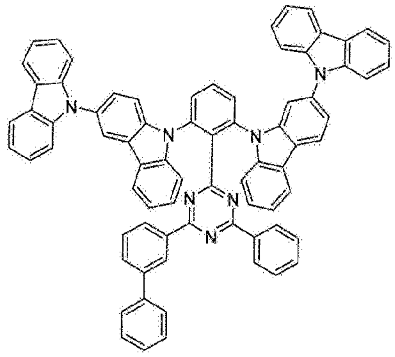

1-66

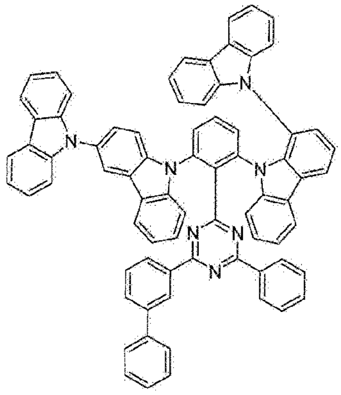

1-67

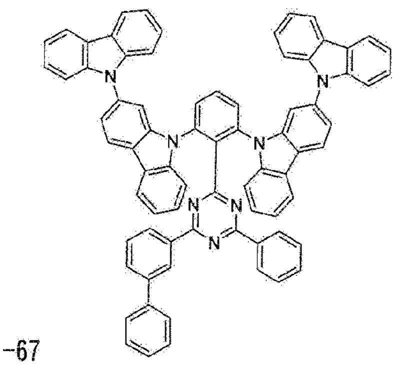

1-68

[C36]

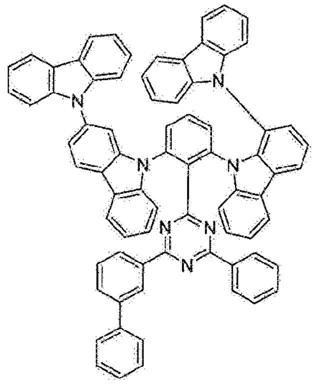
1-69

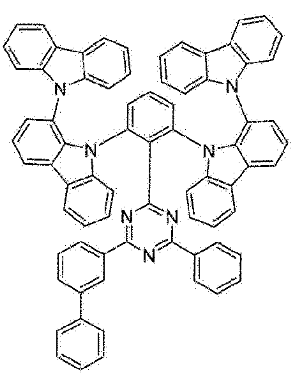
1-70

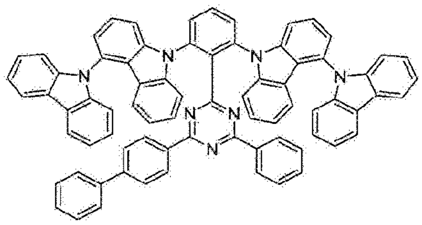
1-71

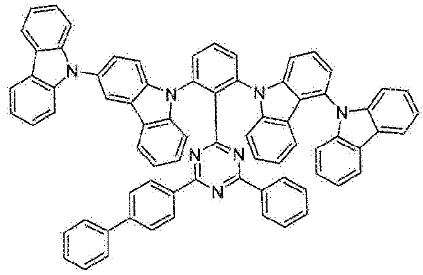
1-72

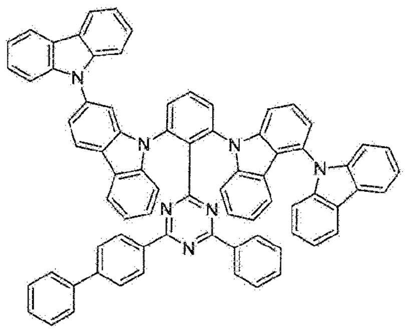
1-73

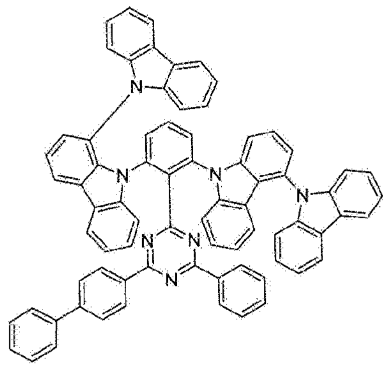
1-74

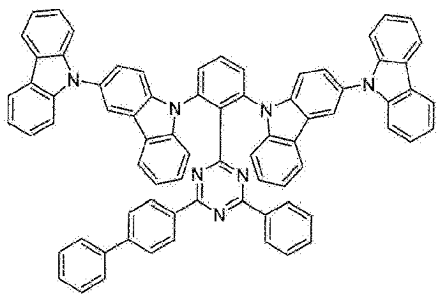
1-75

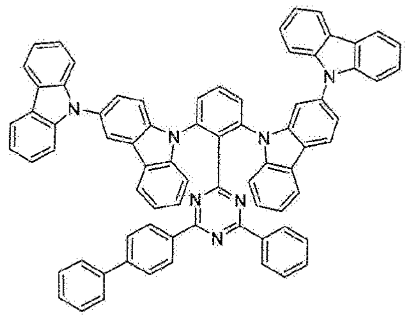
1-76

[C37]

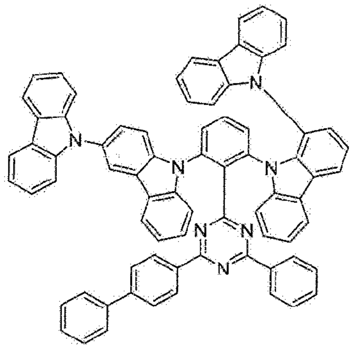

1-77

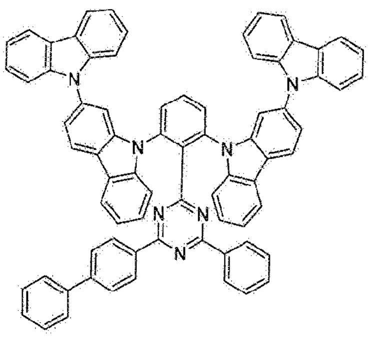

1-78

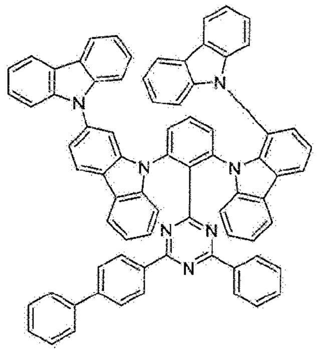

1-79

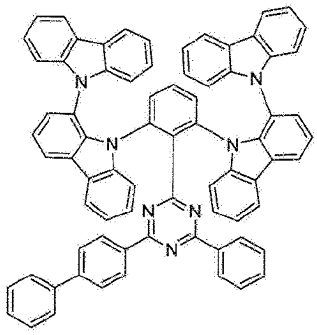

1-80

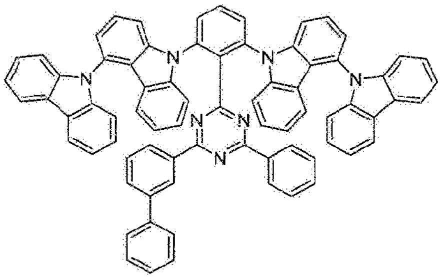

1-81

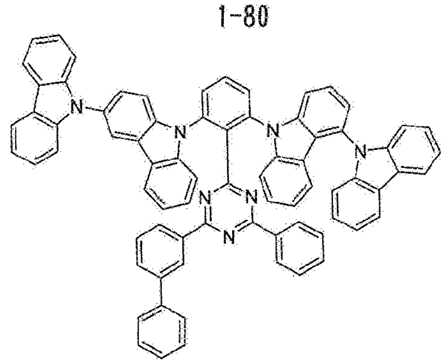

1-82

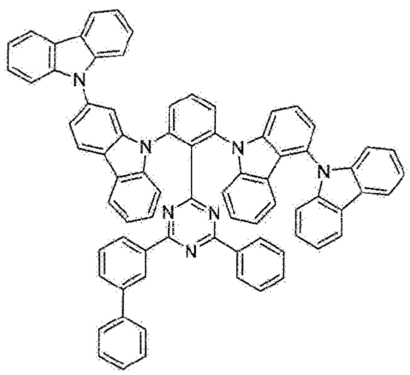

1-83

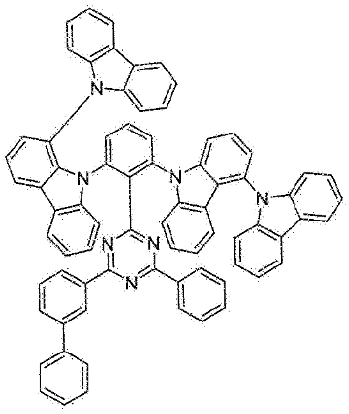

1-84

[C38]

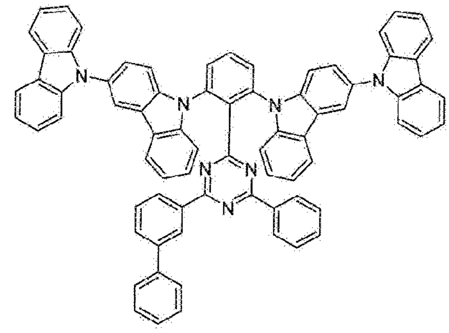

1-85

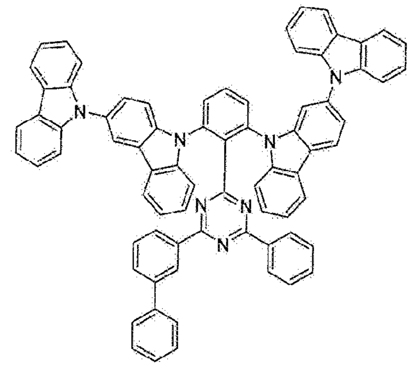

1-86

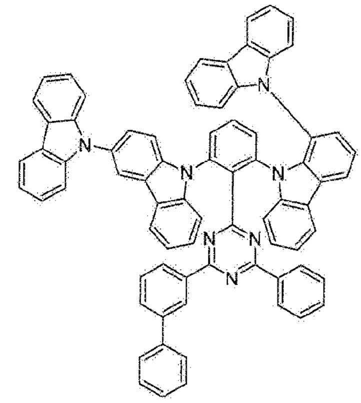

1-87

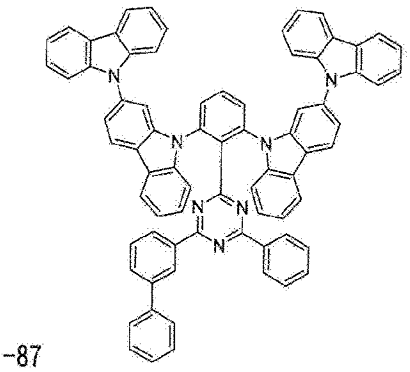

1-88

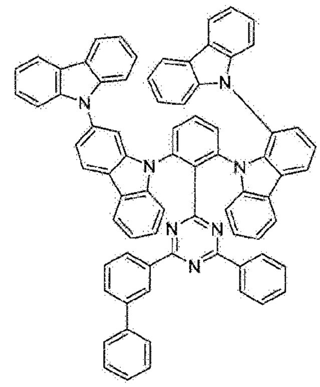

1-89

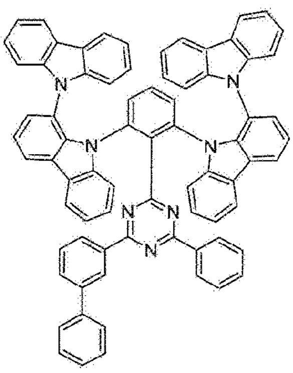

1-90

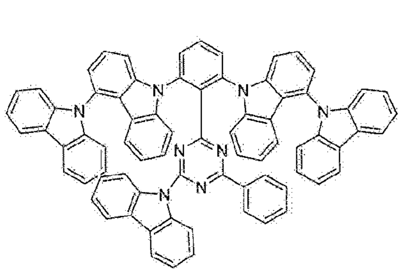

1-91

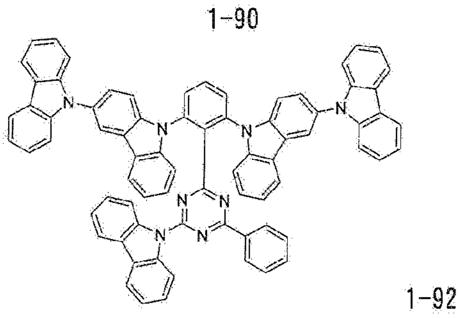

1-92

[C39]

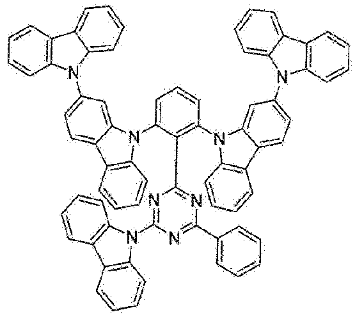

1-93

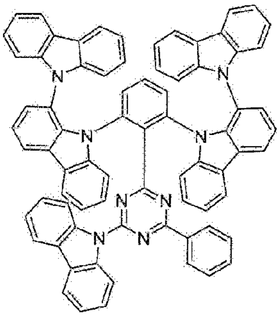

1-94

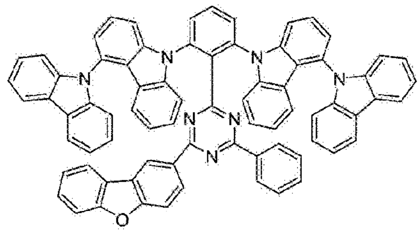

1-95

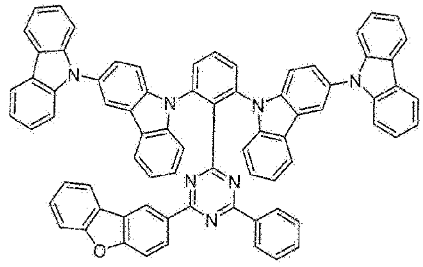

1-96

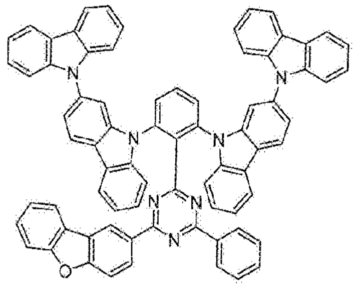

1-97

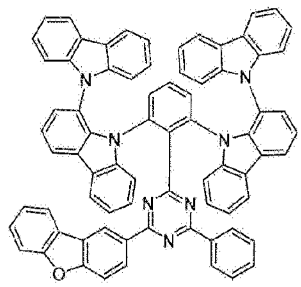

1-98

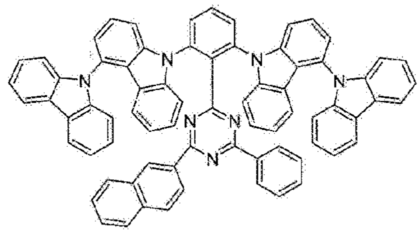

1-99

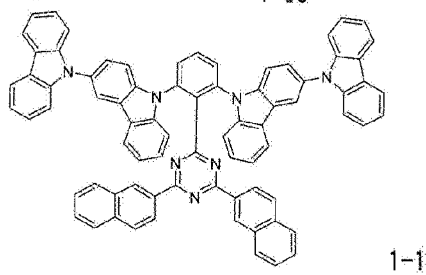

1-100

[C40]

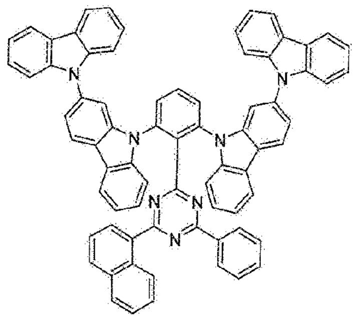

1-101

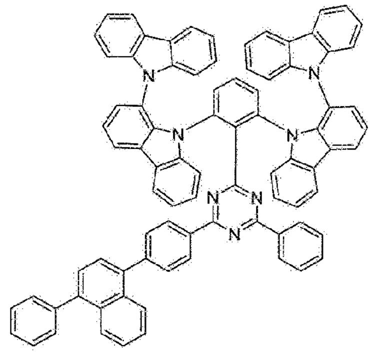

1-102

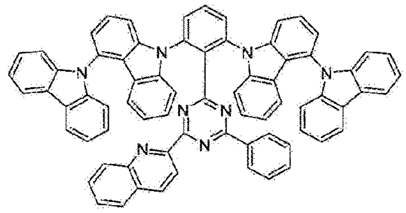

1-103

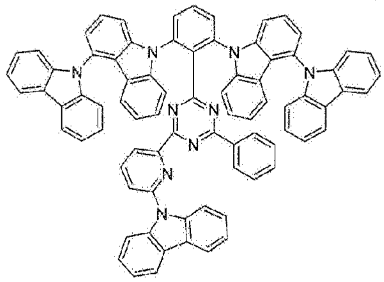

1-104

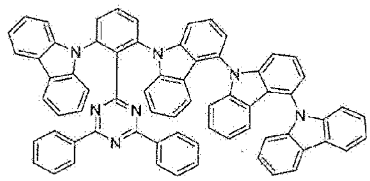

1-105

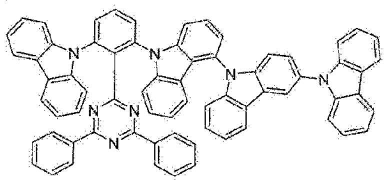

1-106

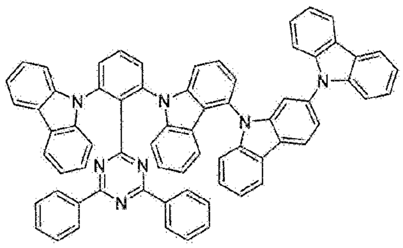

1-107

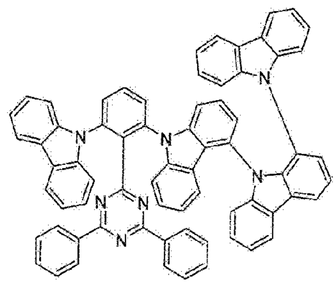

1-108

[C41]

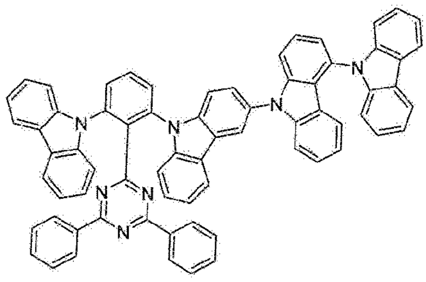

1-109

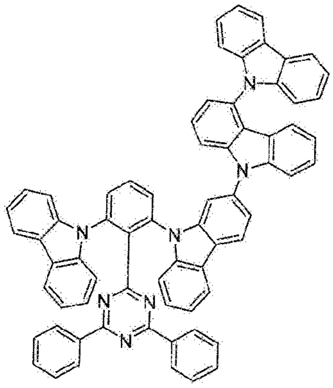

1-110

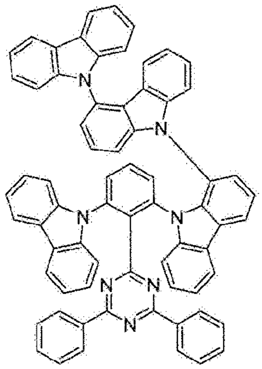

1-111

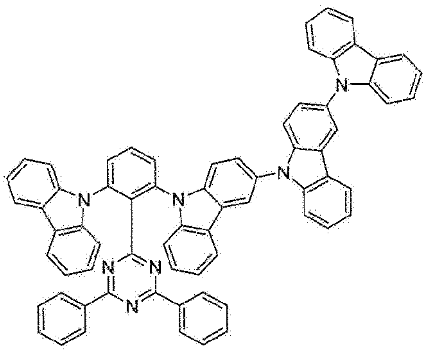

1-112

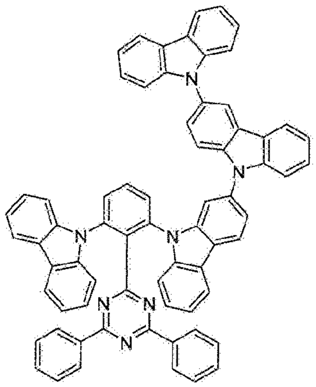

1-113

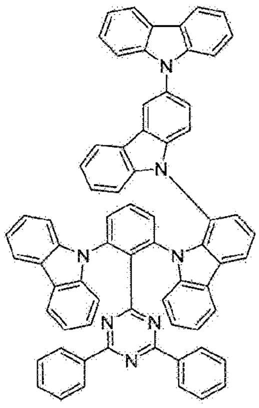

1-114

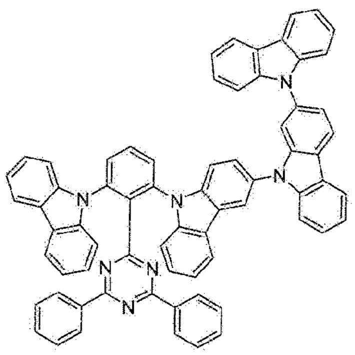

1-115

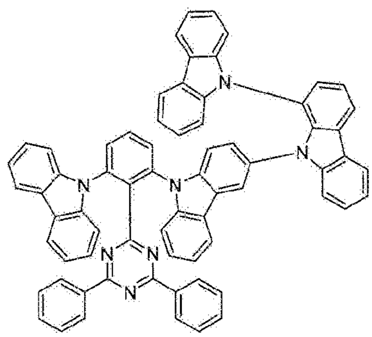

1-116

[C42]

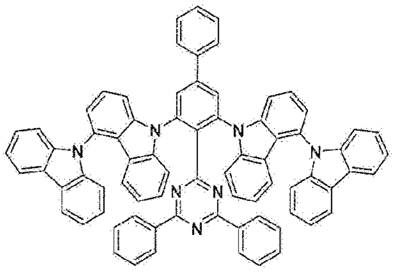

1-117

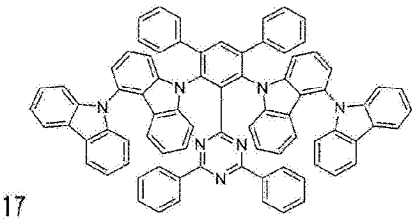

1-118

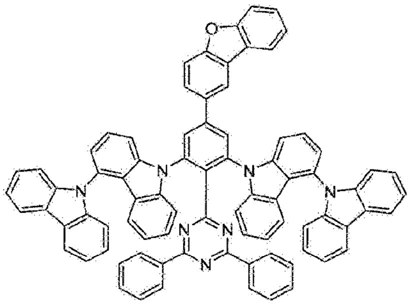

1-119

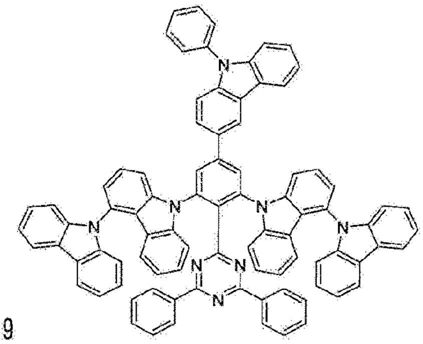

1-120

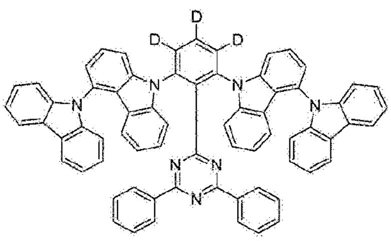

1-121

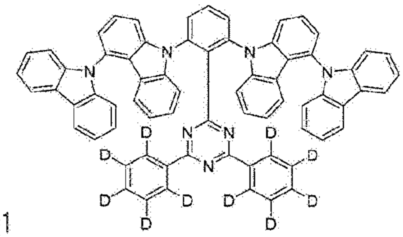

1-122

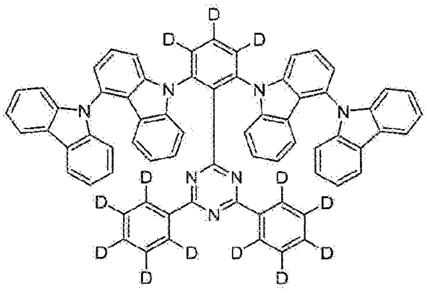

1-123

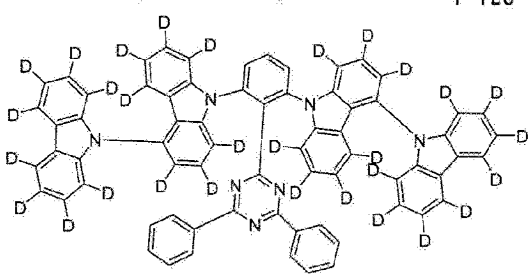

1-124

[C43]

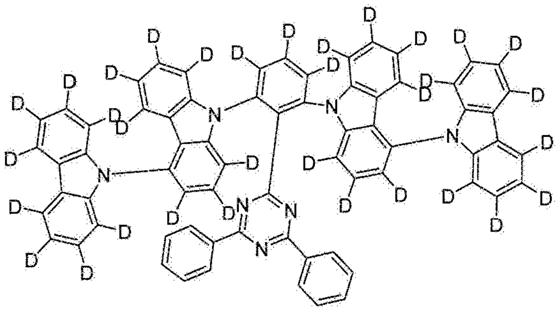

1-125

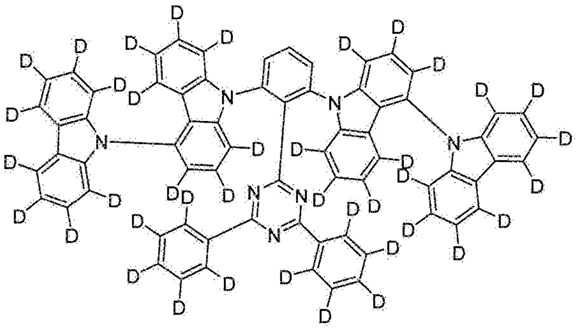

1-126

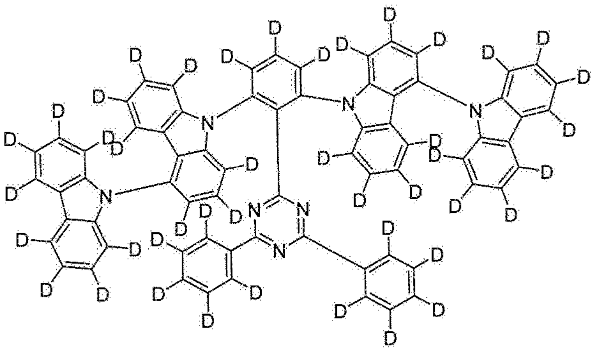

1-127

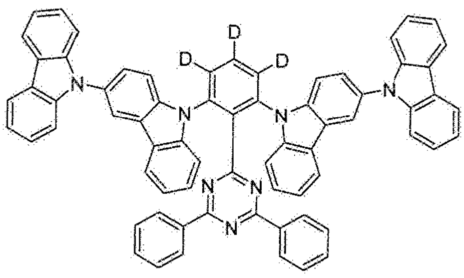

1-128

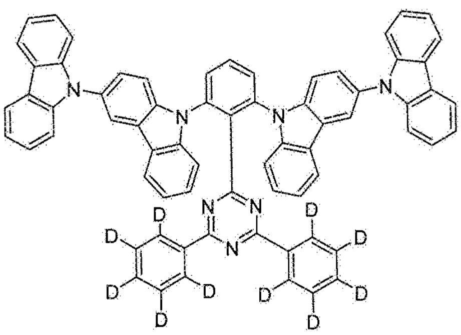

1-129

[C44]

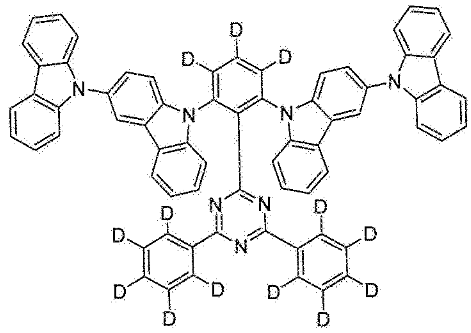

1-130

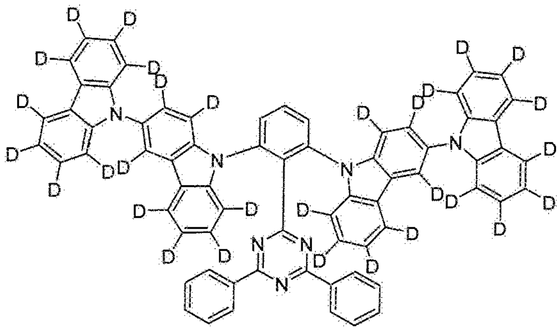

1-131

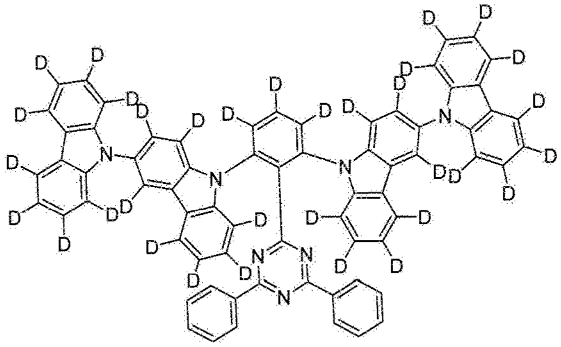

1-132

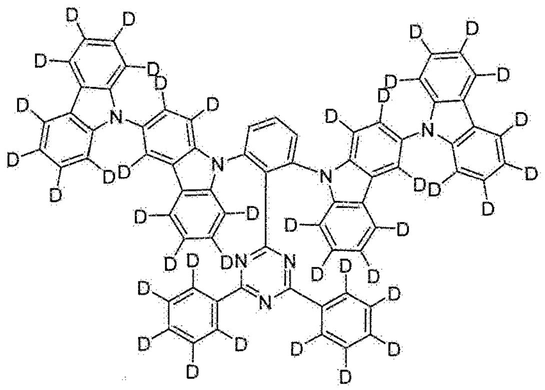

1-133

[C45]

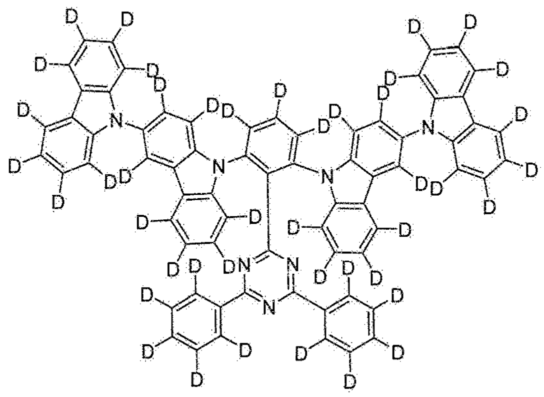

1-134

Specific examples of the compounds represented by the general formula (7a), (7b), (8a), or (8c) above are shown below, but are not limited to these exemplified compounds.

[C46]

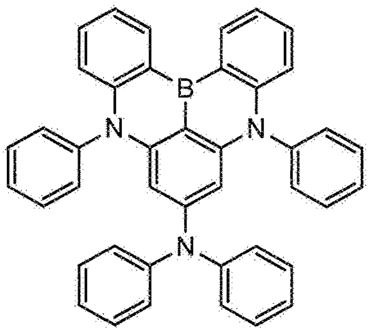

2-1

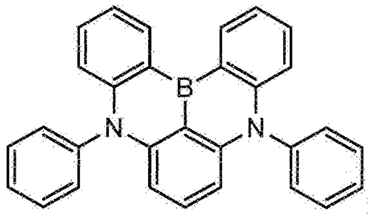

2-2

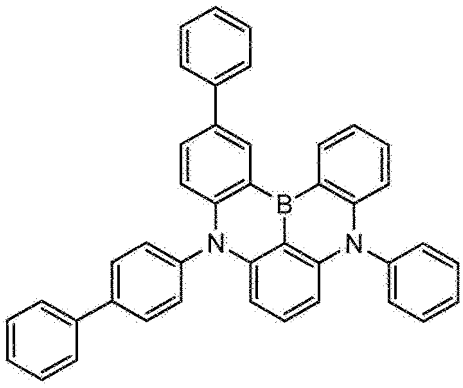

2-3

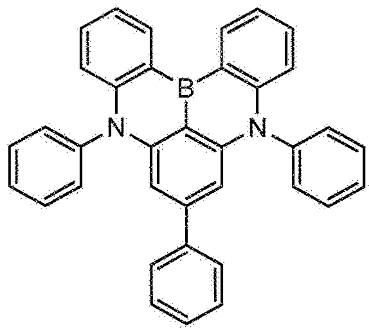

2-4

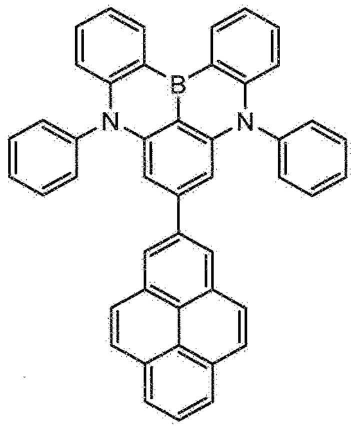

2-5

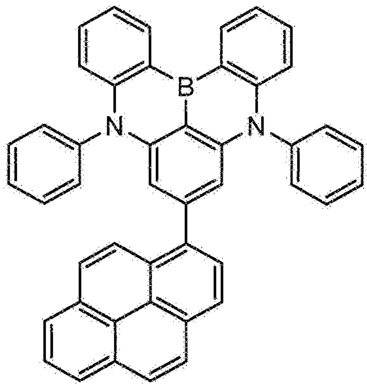

2-6

[C47]

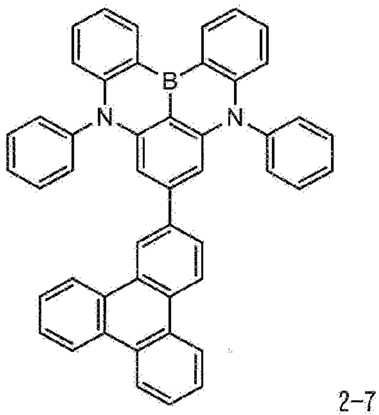

2-7

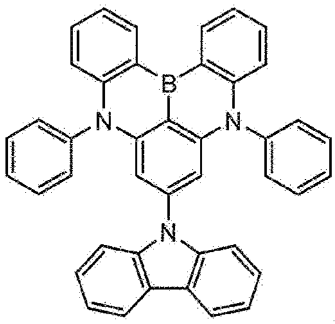

2-8

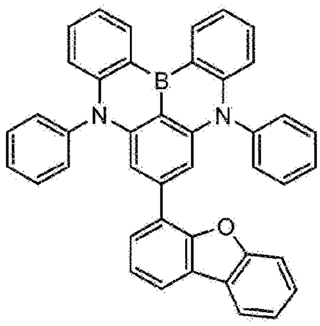

2-9

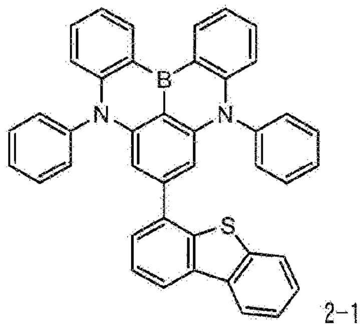

2-10

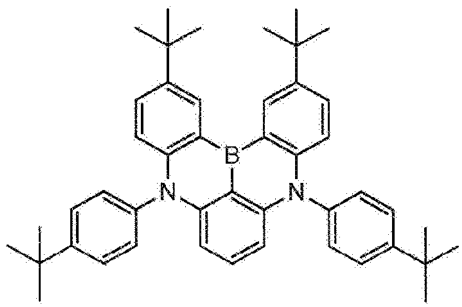

2-11

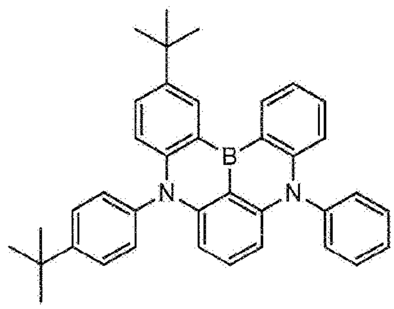

2-12

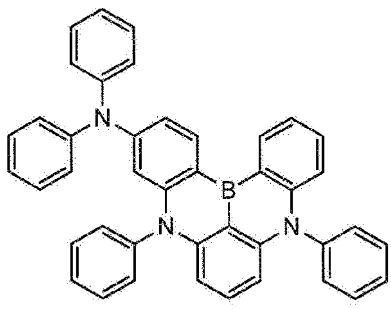

2-13

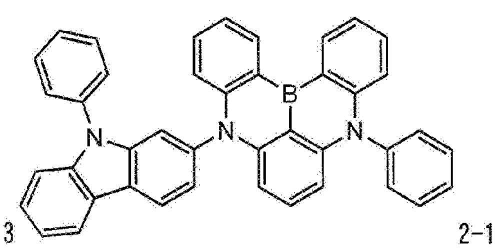

2-14

[C48]

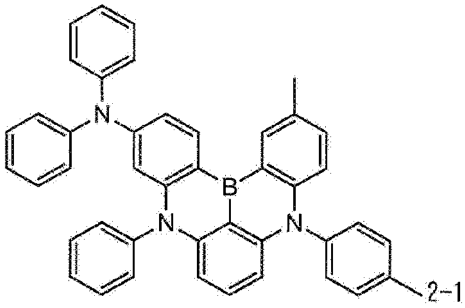
2-15

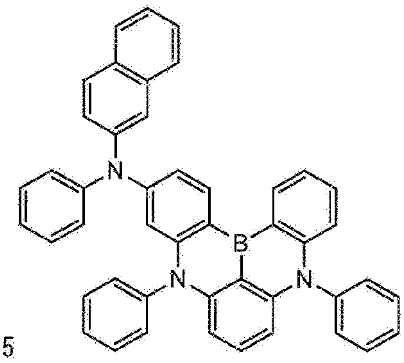
2-16

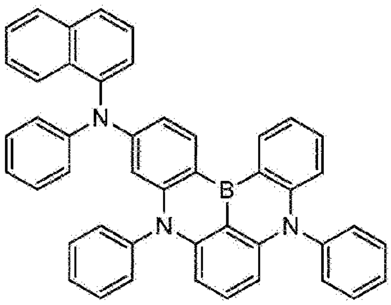
2-17

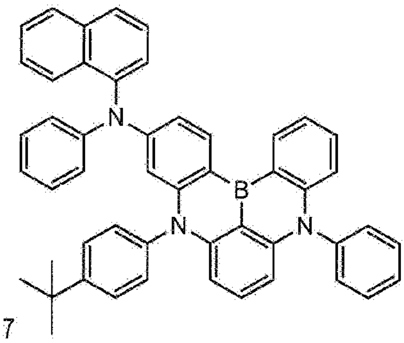
2-18

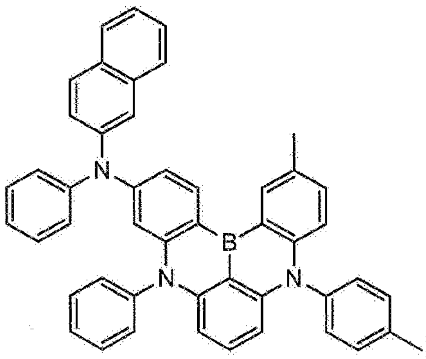
2-19

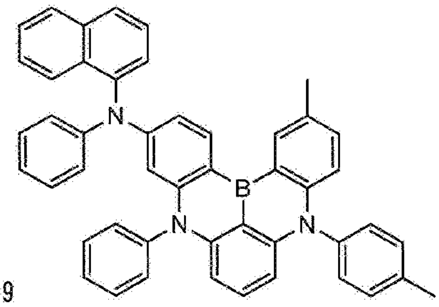
2-20

[C49]

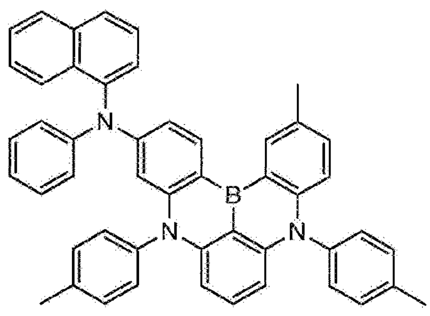

2-21

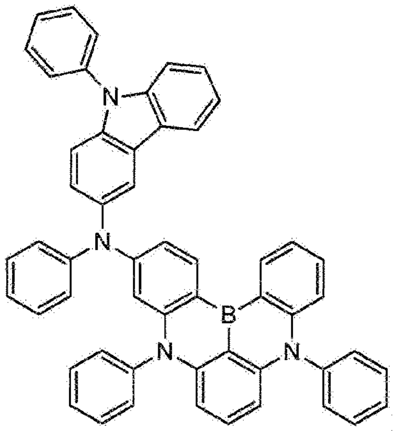

2-22

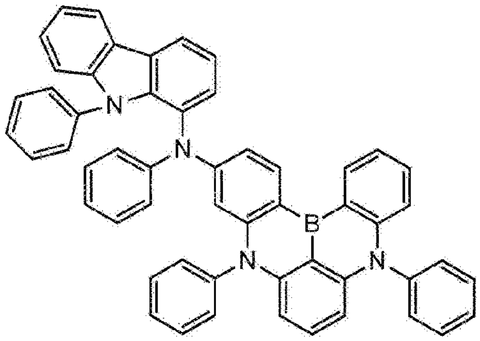

2-23

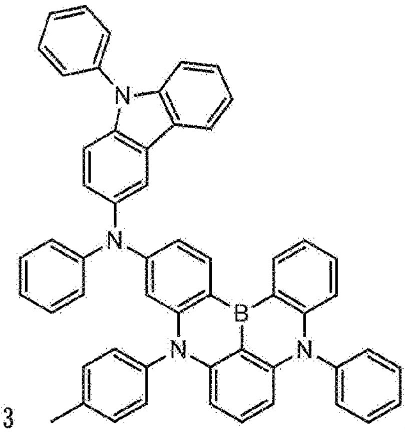

2-24

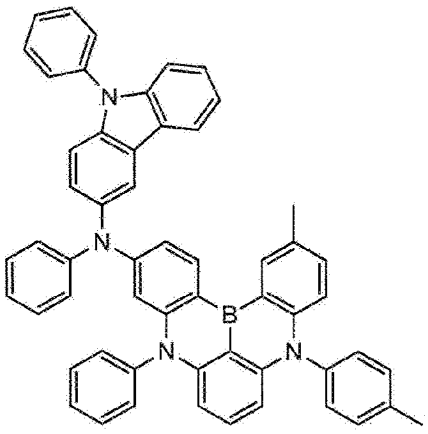

2-25

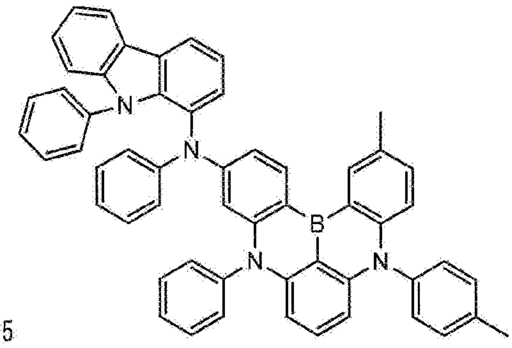

2-26

[C50]

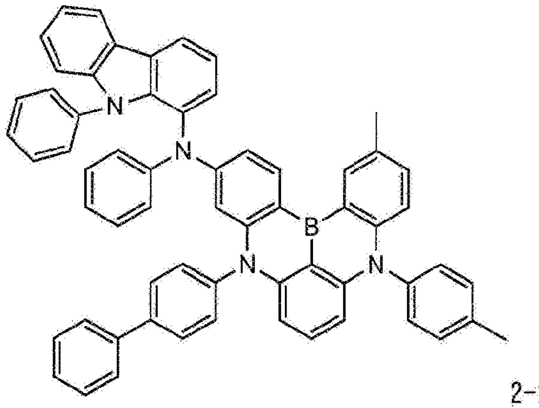
2-27

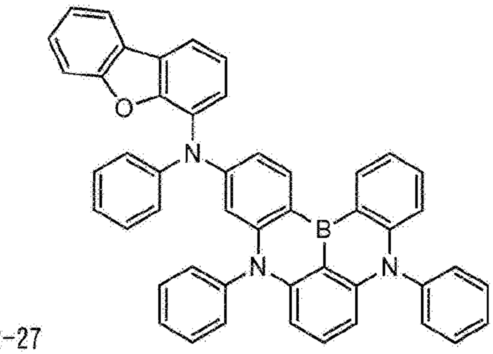
2-28

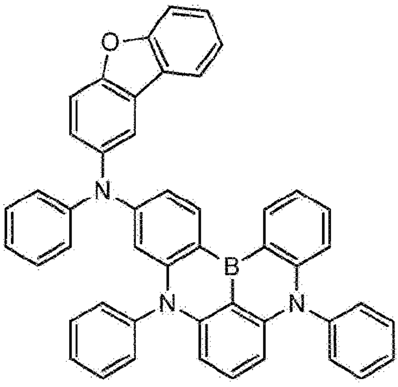
2-29

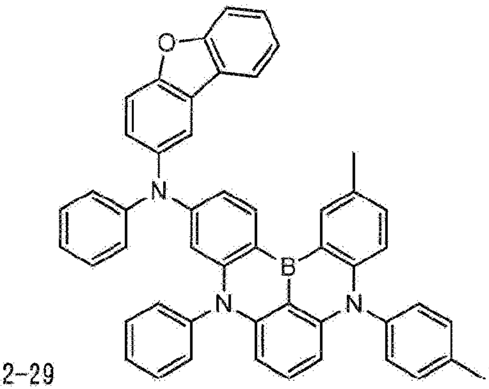
2-30

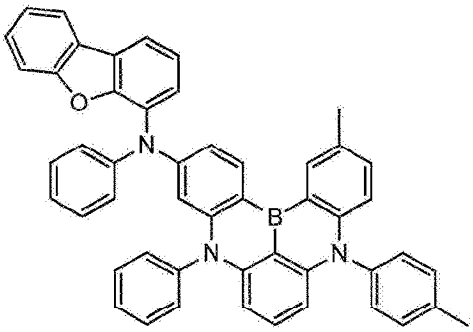
2-31

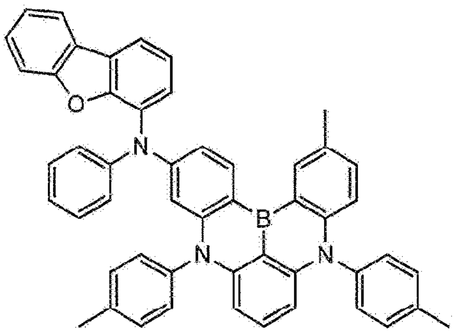
2-32

[C51]

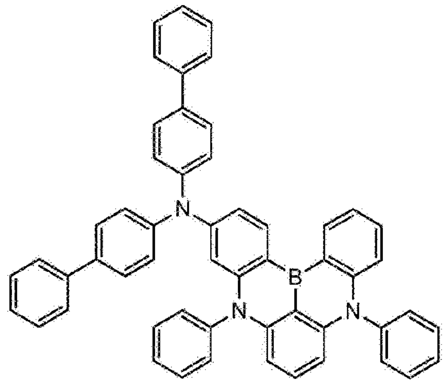

2-33

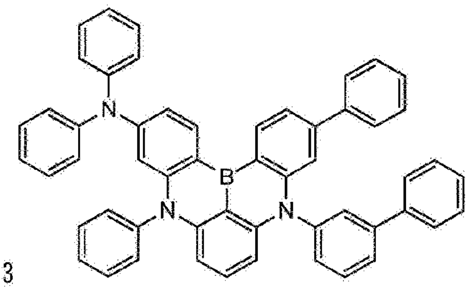

2-34

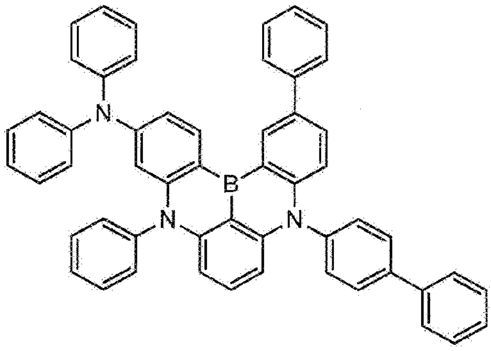

2-35

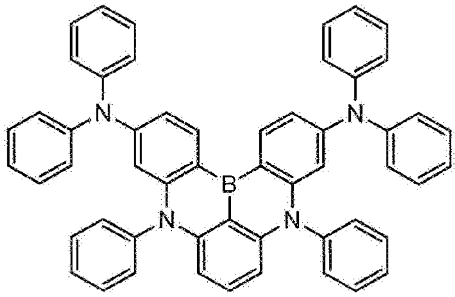

2-36

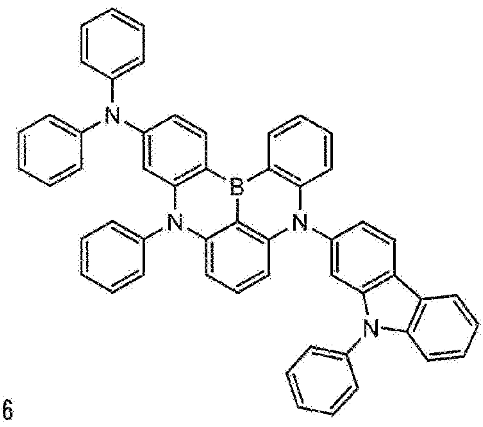

2-37

[C52]

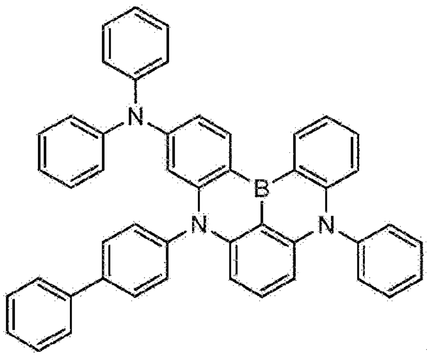

2-38

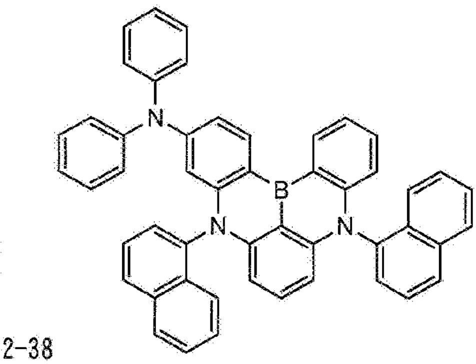

2-39

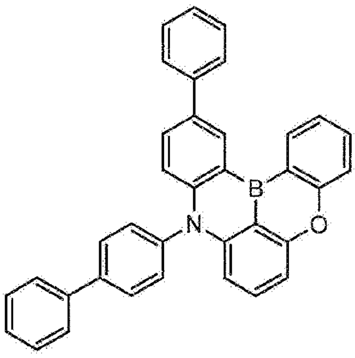

2-40

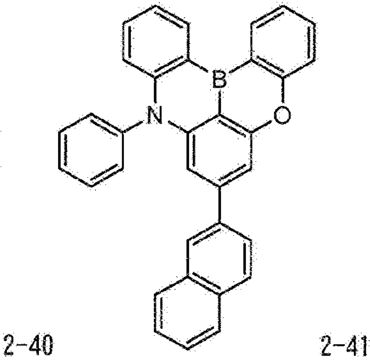

2-41

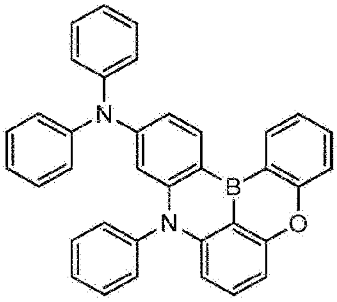

2-42

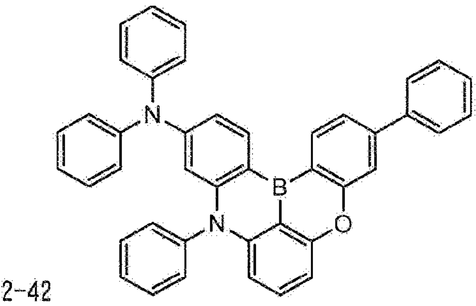

2-43

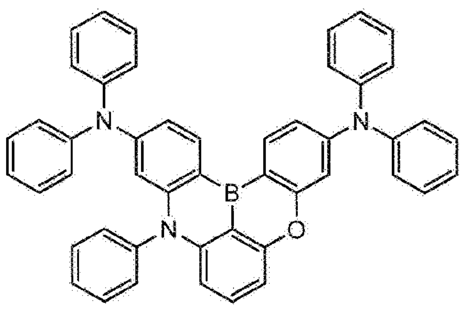

2-44

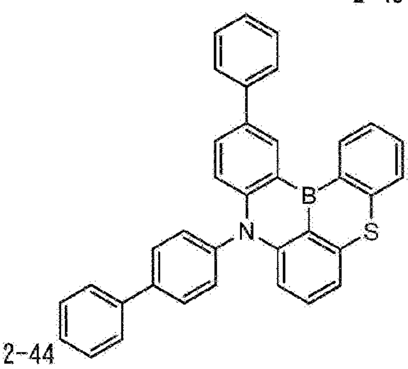

2-45

[C53]

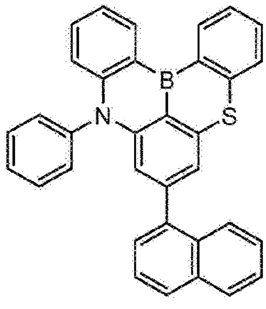

2-46

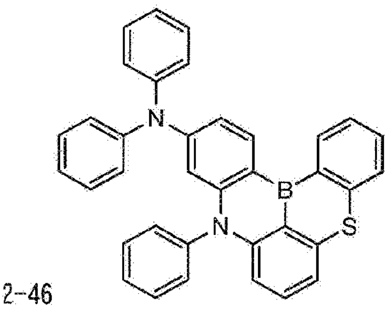

2-47

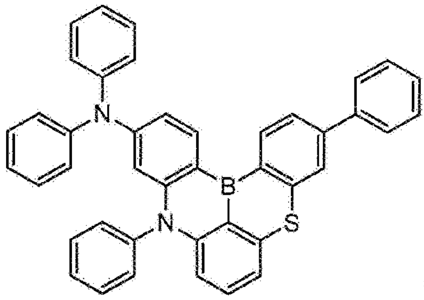

2-48

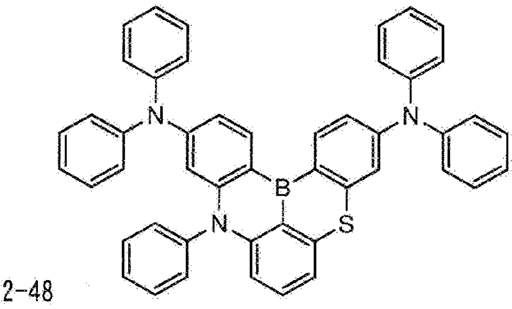

2-49

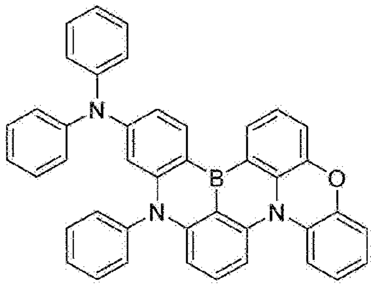

2-50

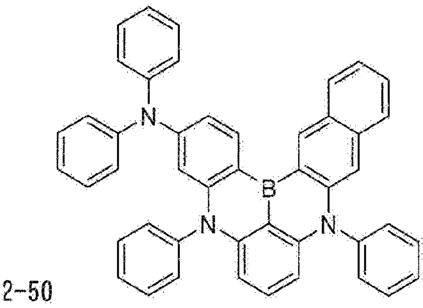

2-51

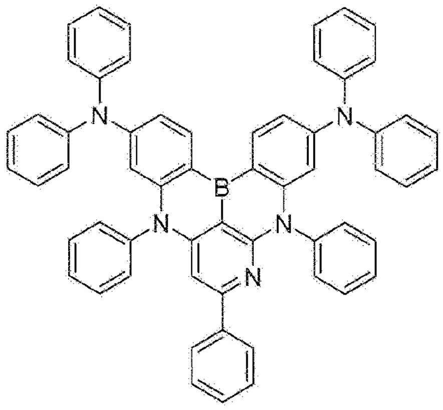

2-52

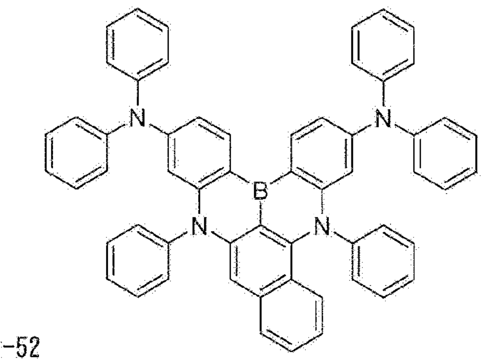

2-53

[C54]

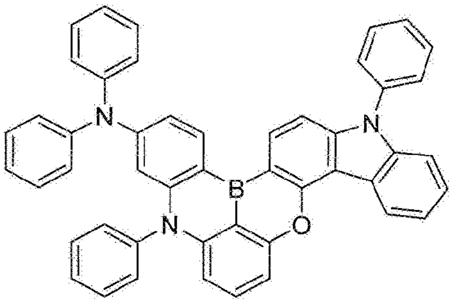
2-54

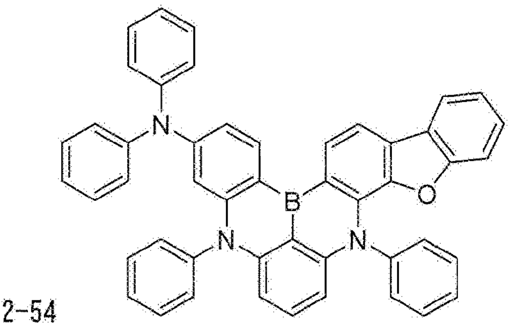
2-55

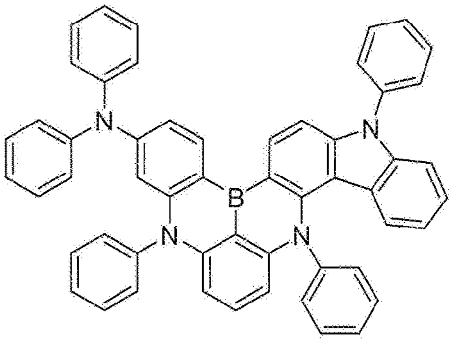
2-56

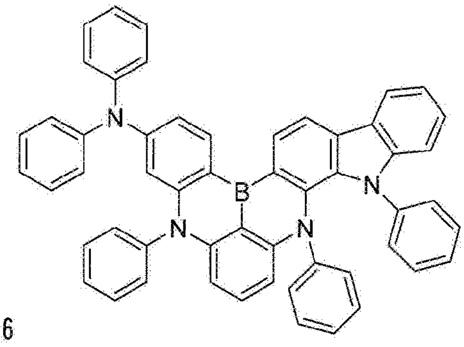
2-57

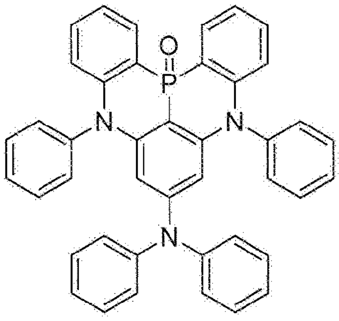
2-58

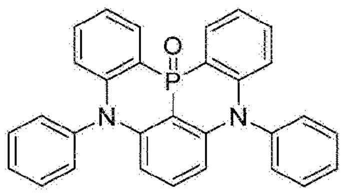
2-59

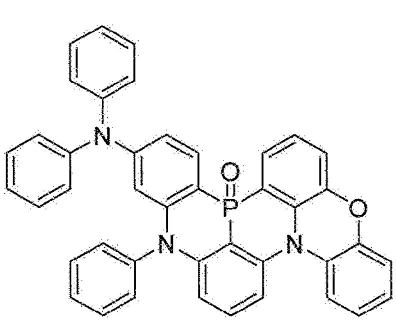
2-60

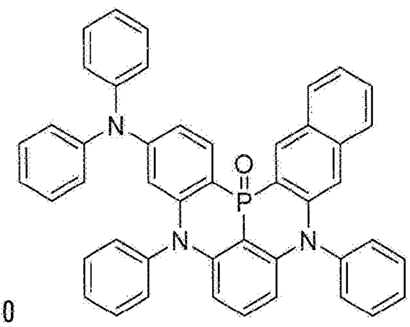
2-61

[C55]

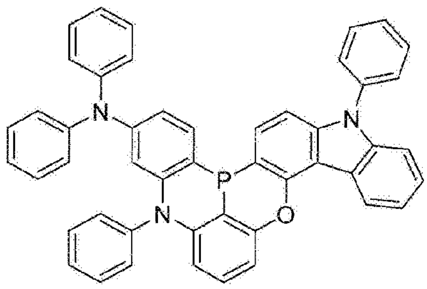

2-62

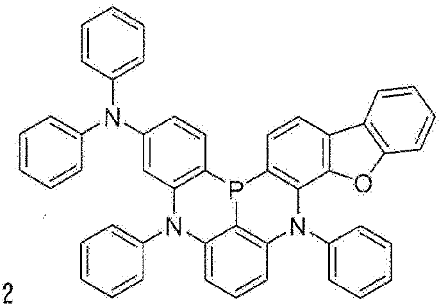

2-63

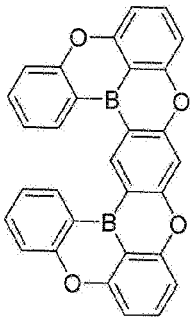

2-69

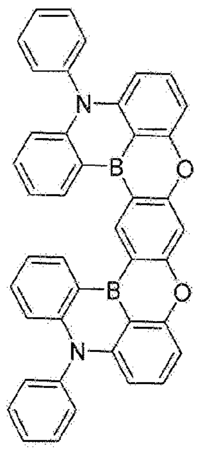

2-80

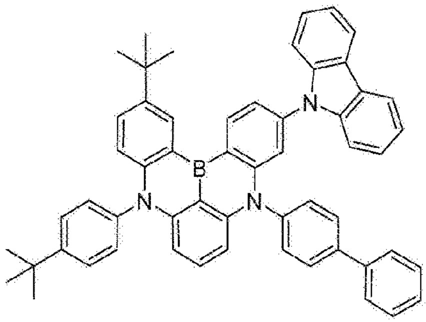

2-86

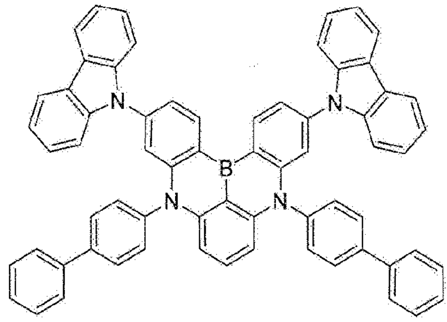

2-87

[C56]

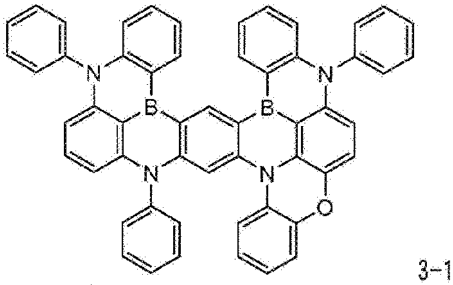

3-1

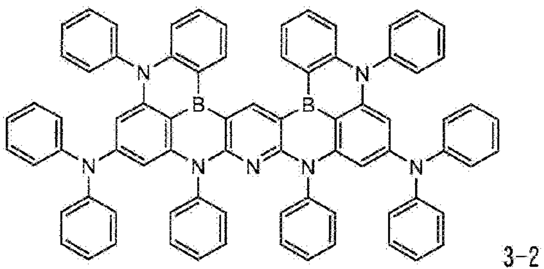

3-2

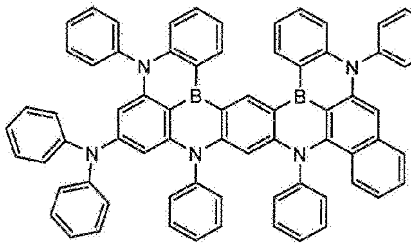

3-3

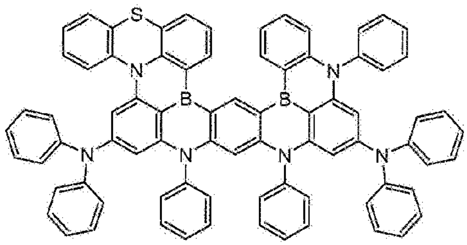

3-4

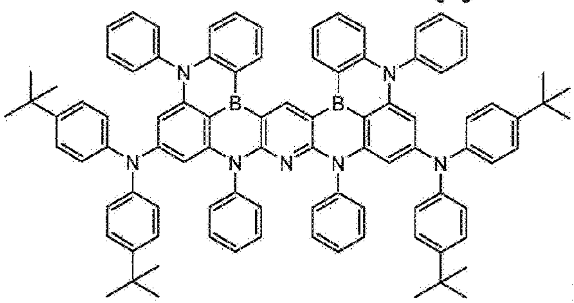

3-5

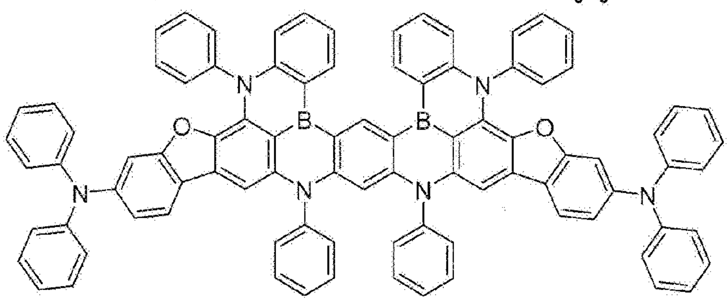

3-6

[C57]

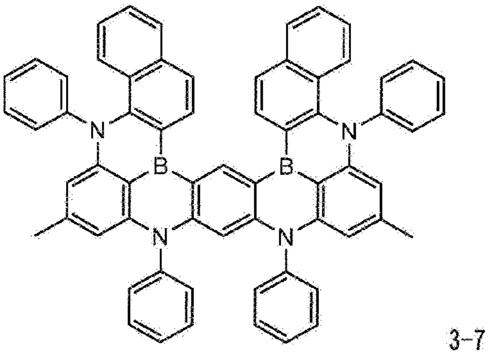

3-7

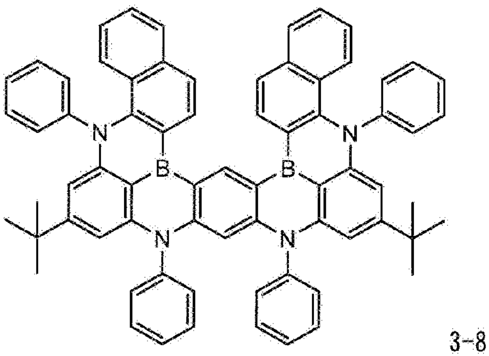

3-8

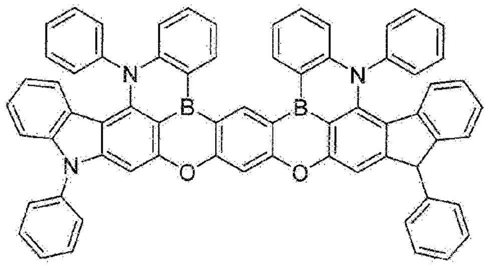

3-9

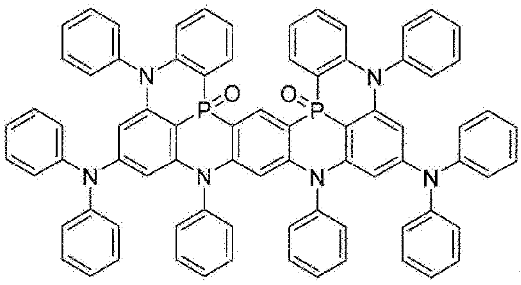

3-10

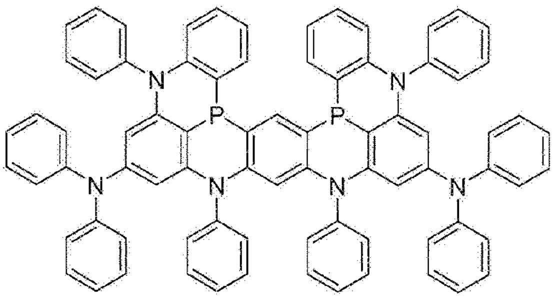

3-11

[C58]

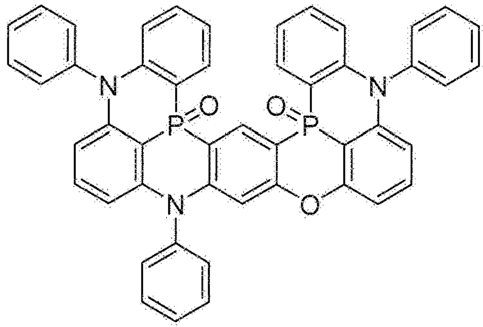

3-12

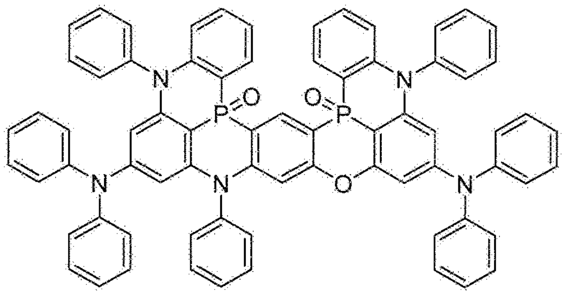

3-13

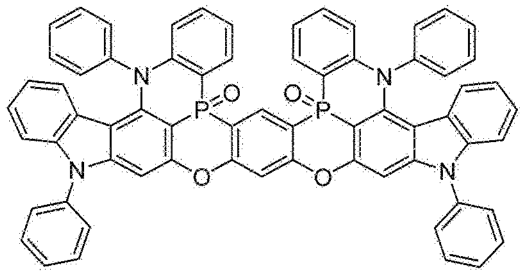

3-14

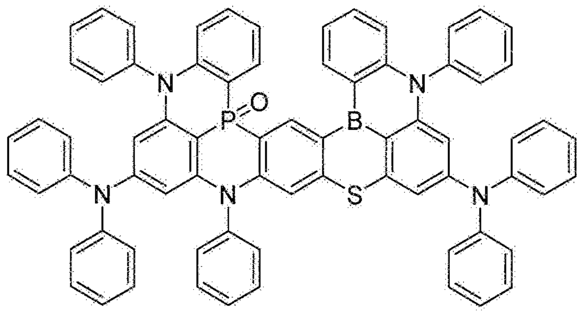

3-15

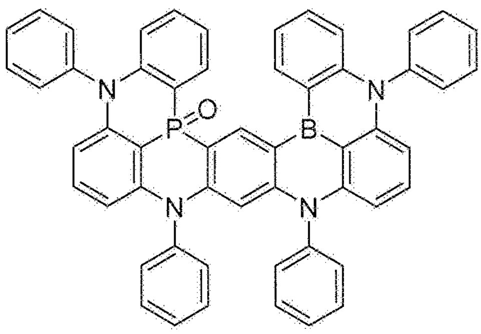

3-16

[C59]

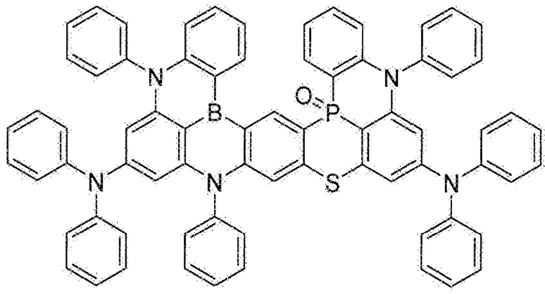

3-17

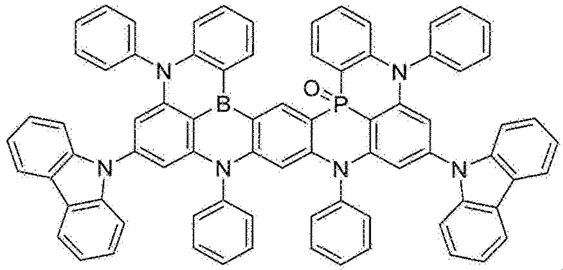

3-18

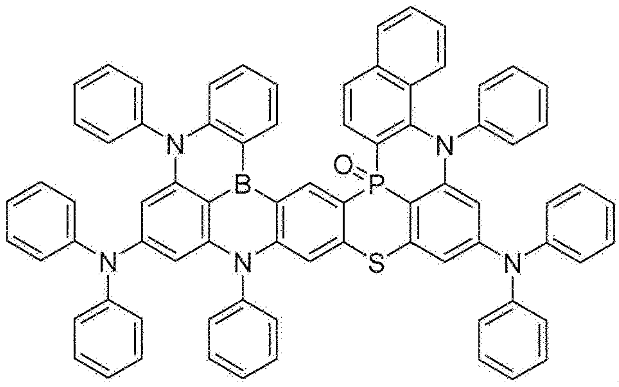

3-19

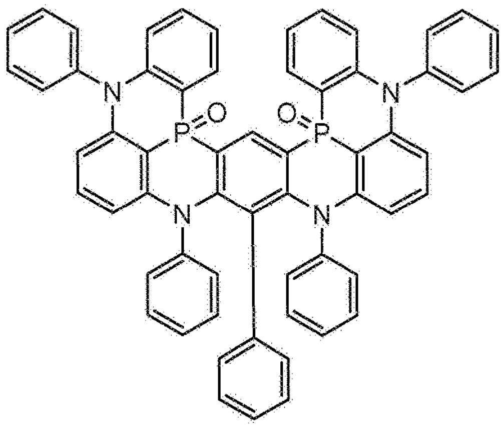

3-20

[C60]

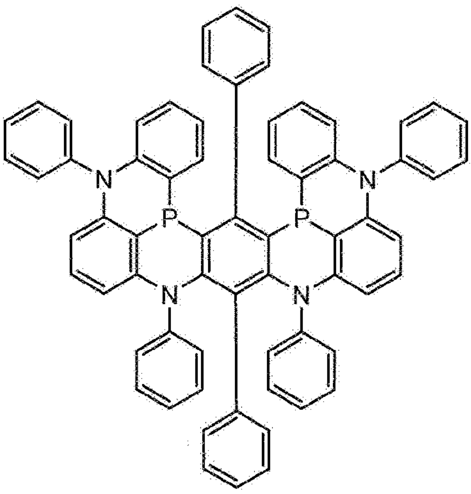

3-21

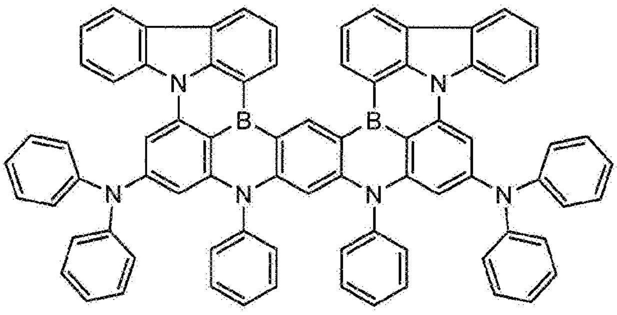

3-22

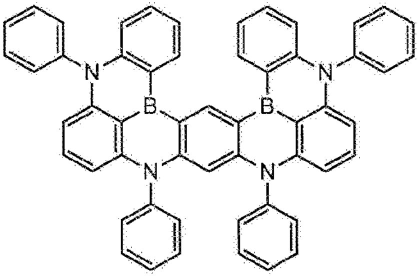

4-1

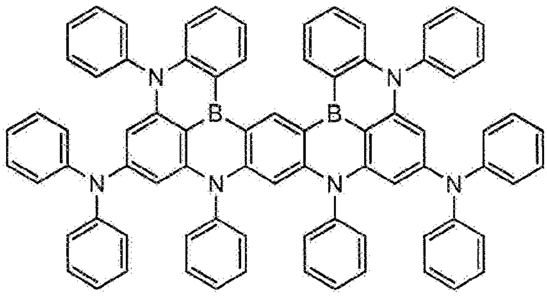

4-2

[C61]

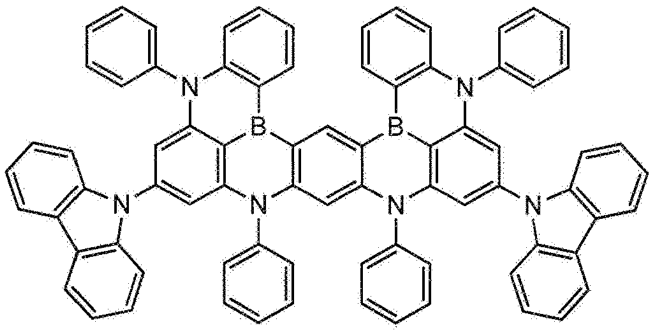

4-3

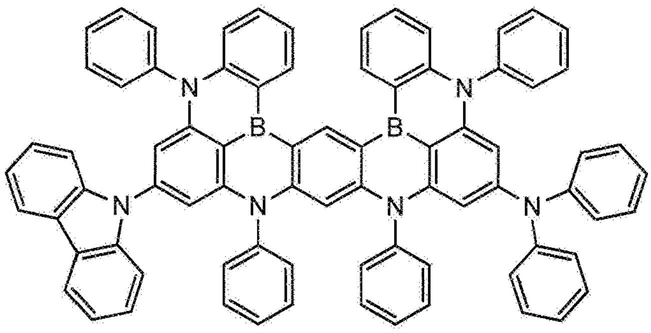

4-4

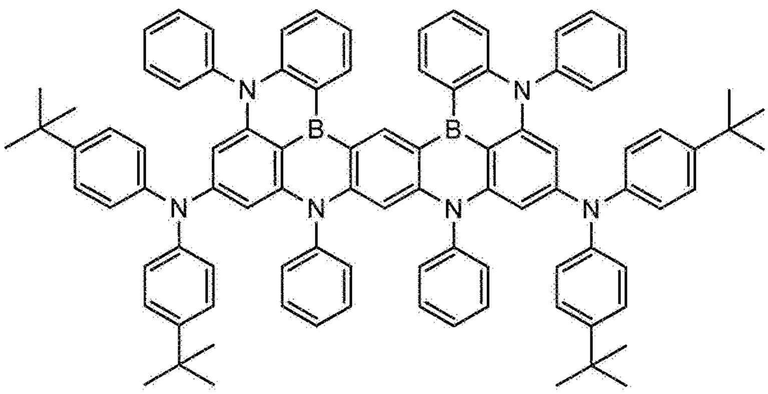

4-5

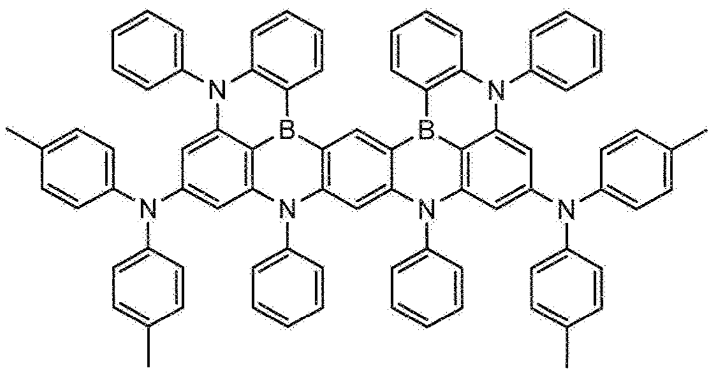

4-6

[C62]

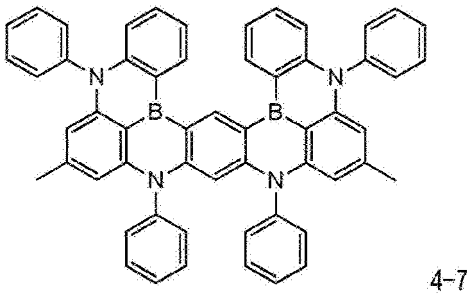

4-7

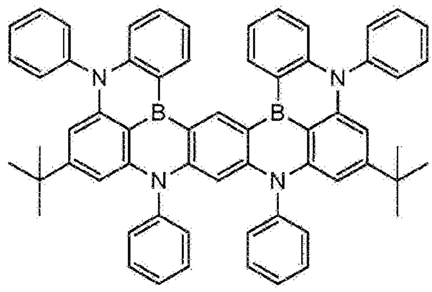

4-8

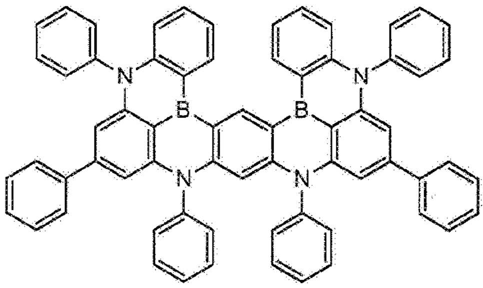

4-9

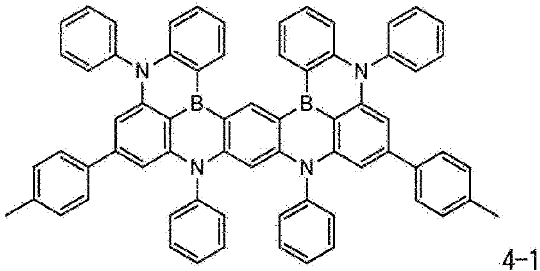

4-10

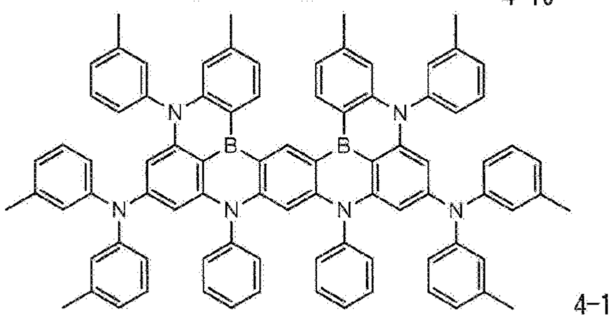

4-11

[C63]

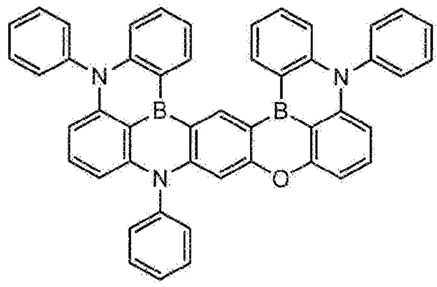

4-12

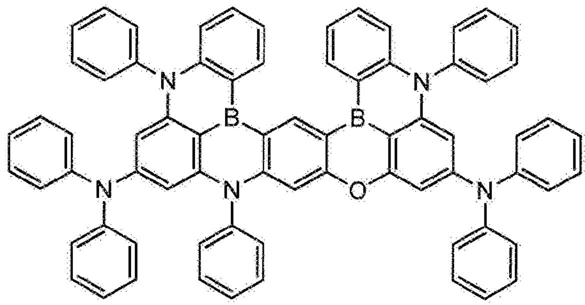

4-13

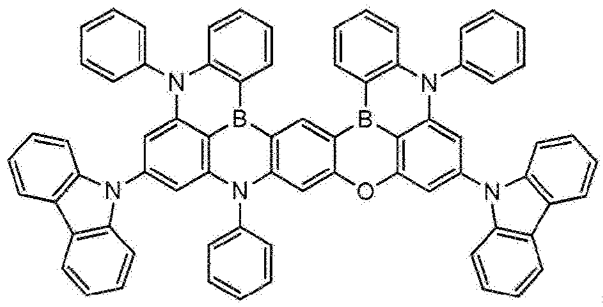

4-14

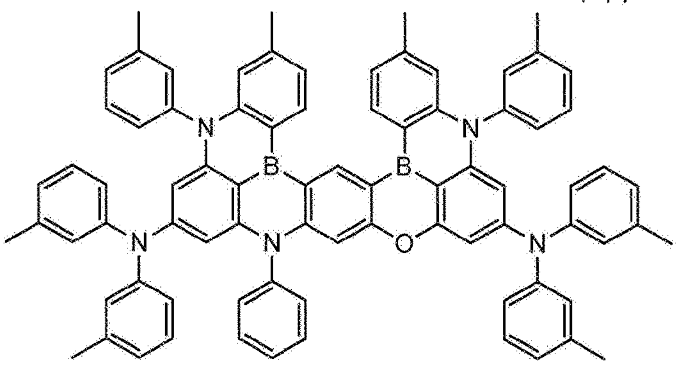

4-15

[C64]

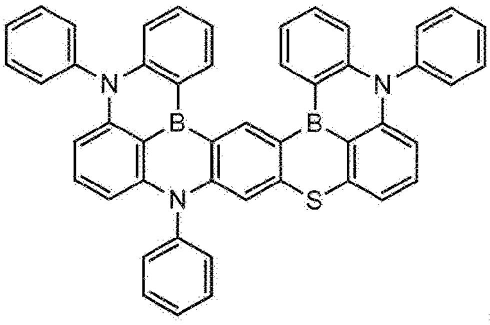

4-16

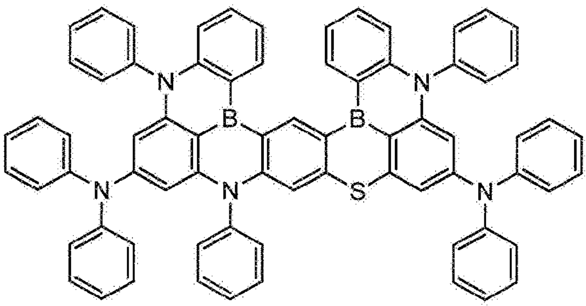

4-17

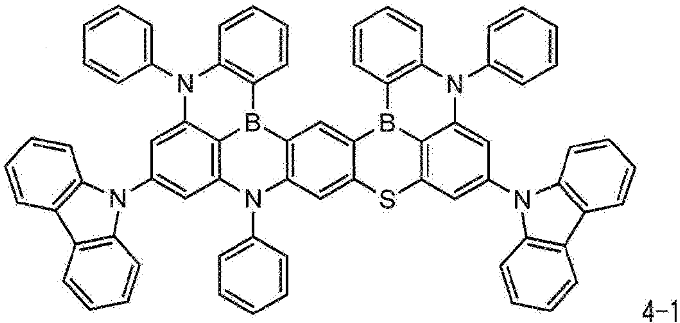

4-18

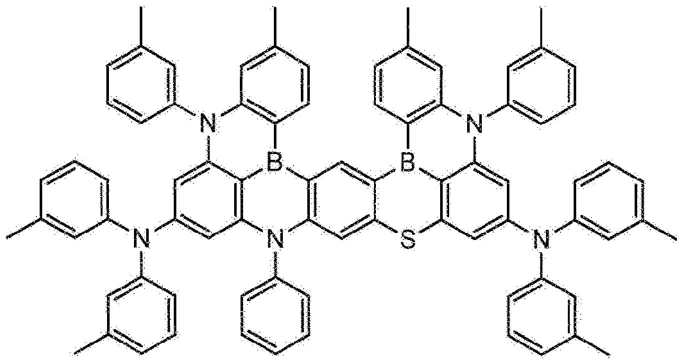

4-19

[C65]

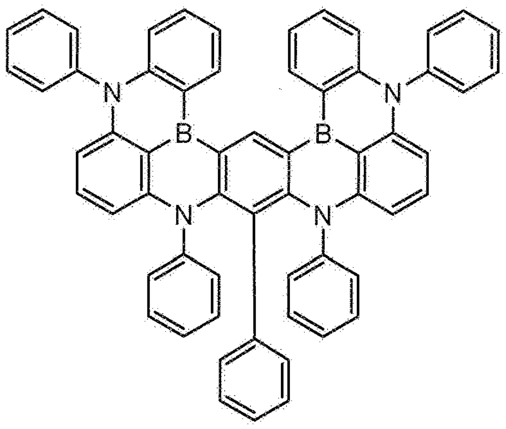

4-20

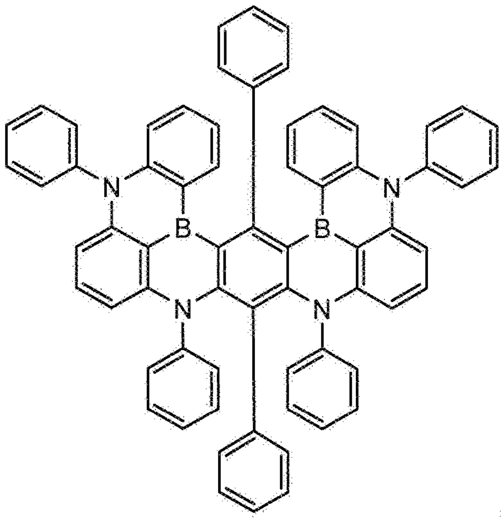

4-21

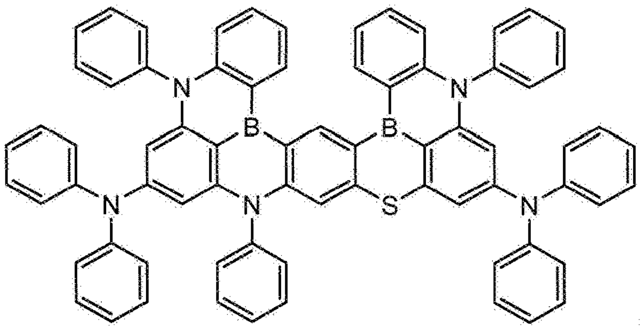

4-22

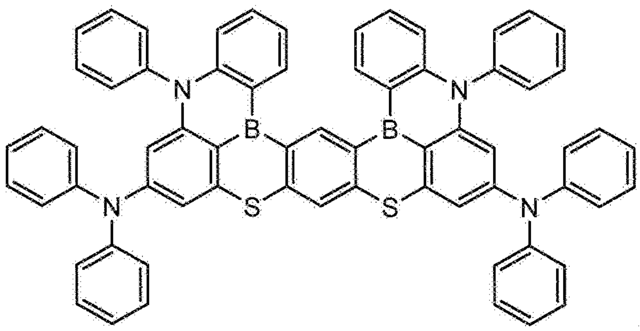

4-23

[C66]

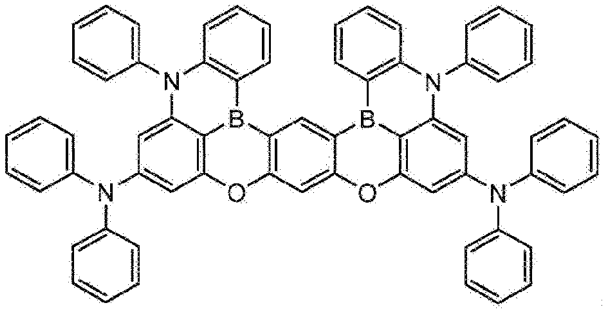

4-24

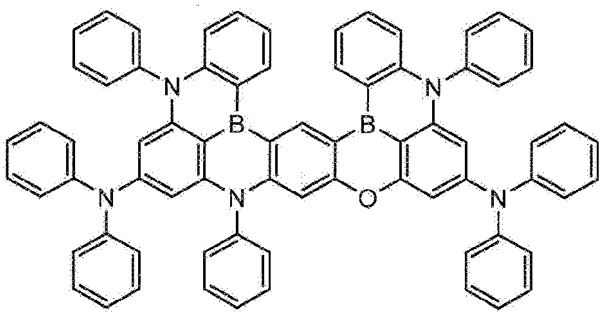

4-25

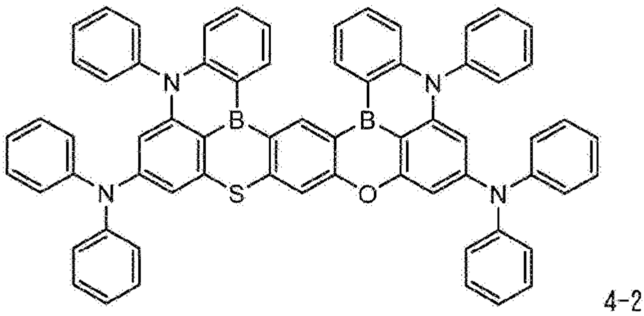

4-26

Specific examples of the compounds represented by the general formula (9) above are shown below, but are not limited to these exemplified compounds.

[C67]

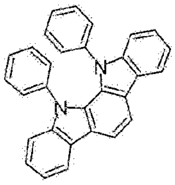

5-1

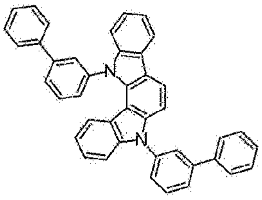

5-2

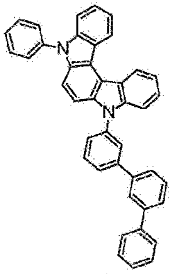

5-3

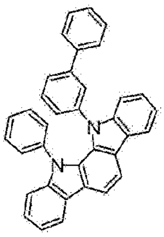

5-4

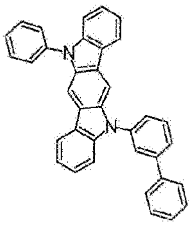

5-5

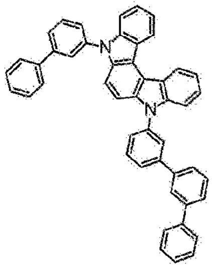

5-6

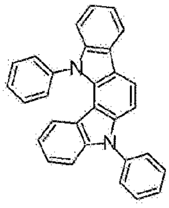

5-7

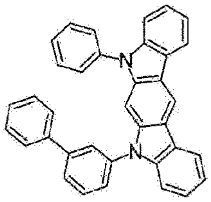

5-8

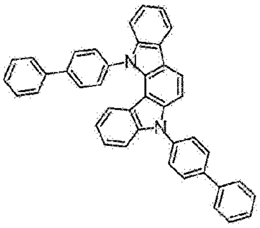

5-10

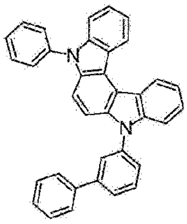

5-11

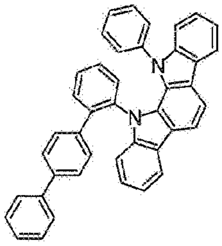

5-12

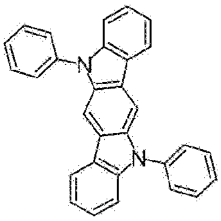

5-13

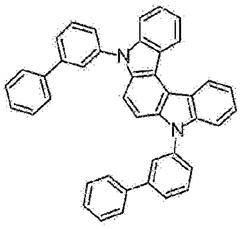

5-14

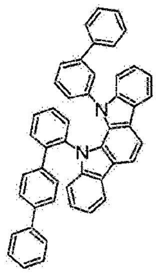

5-15

[C68]

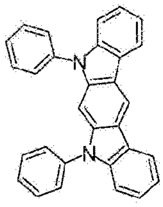
5-16

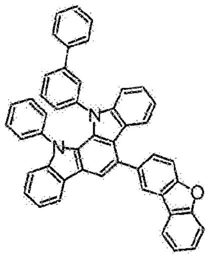
5-17

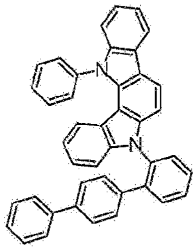
5-18

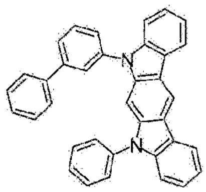
5-19

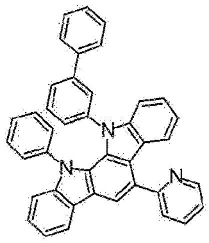
5-20

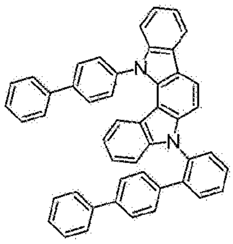
5-21

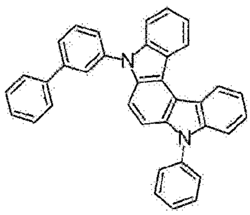
5-22

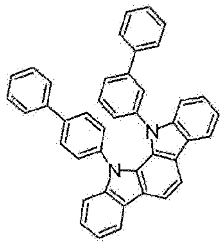
5-23

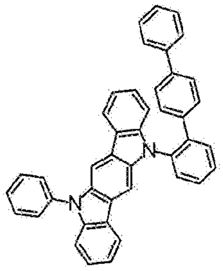
5-24

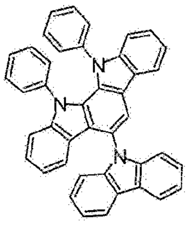
5-25

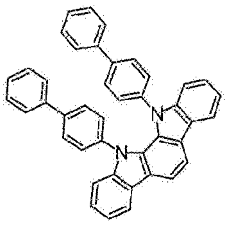
5-26

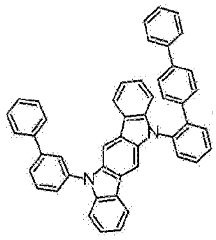
5-27

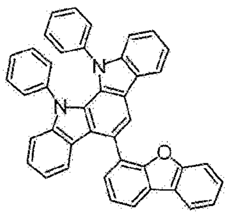
5-28

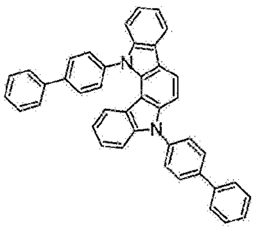
5-29

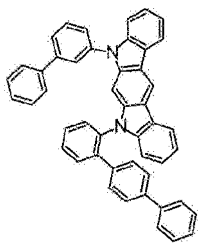
5-30

[C69]

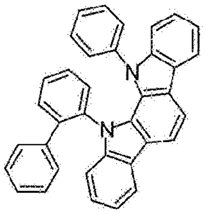
5-31

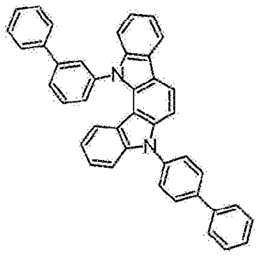
5-32

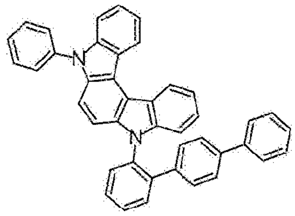
5-33

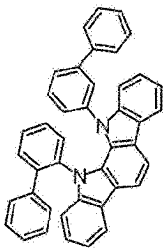
5-34

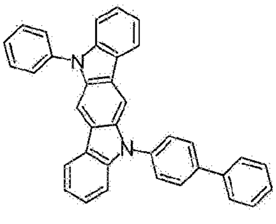
5-3

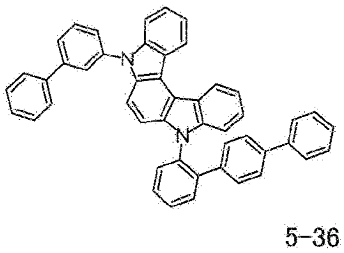
5-36

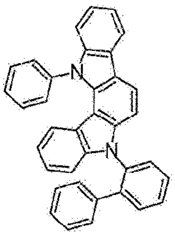
5-37

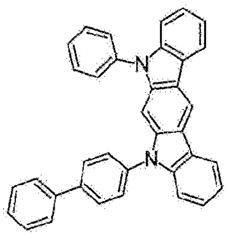
5-38

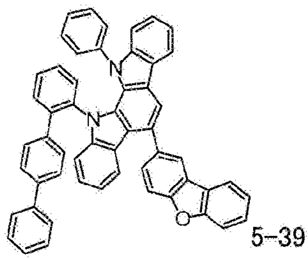
5-39

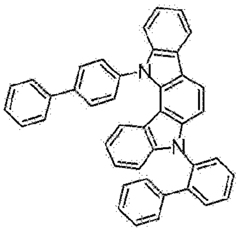
5-40

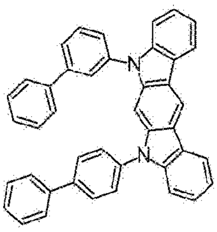
5-41

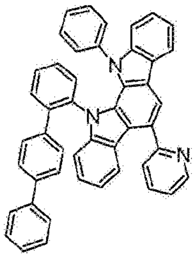
5-42

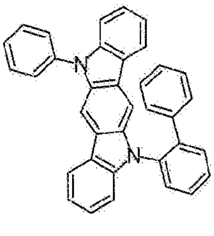
5-43

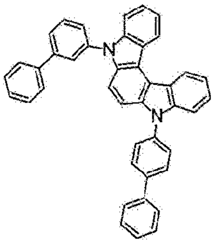
5-44

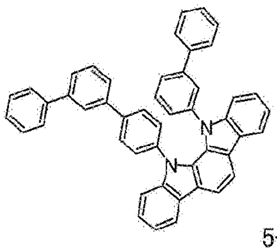
5-45

[C70]

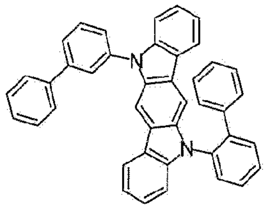
5-46

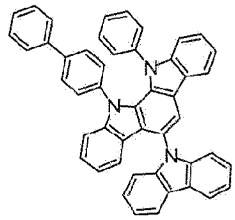
5-47

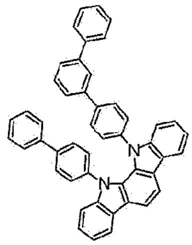
5-48

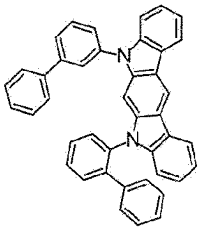
5-49

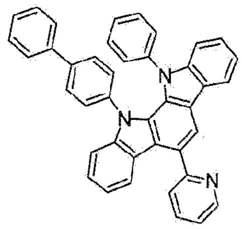
5-50

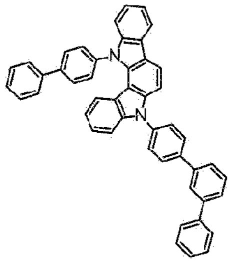
5-51

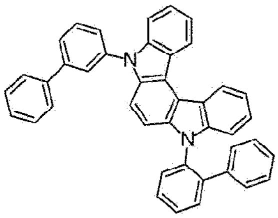
5-52

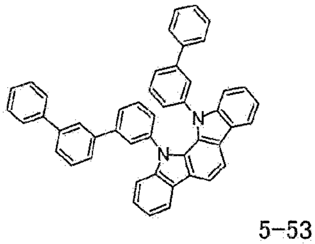
5-53

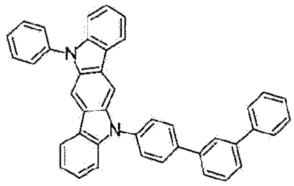
5-54

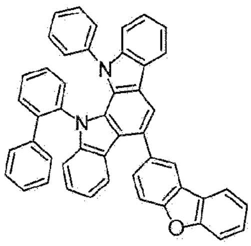
5-55

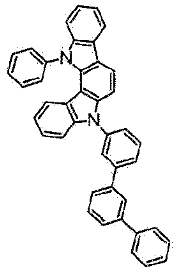
5-56

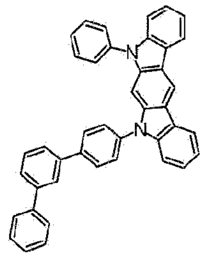
5-57

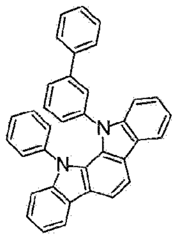
5-58

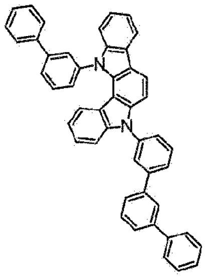
5-59

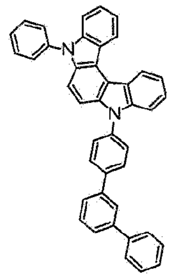
5-60

[C71]

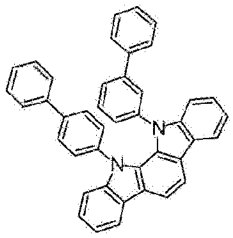
5-61

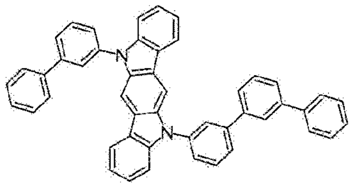
5-62

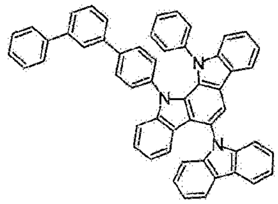
5-63

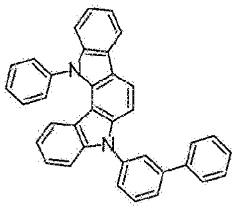
5-64

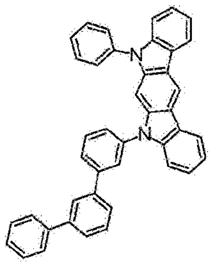
5-65

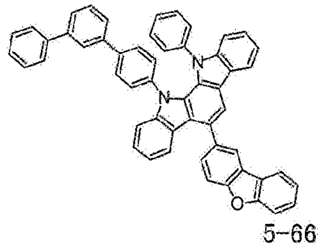
5-66

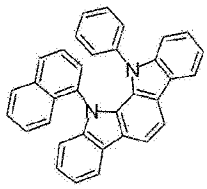
5-67

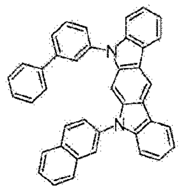
5-68

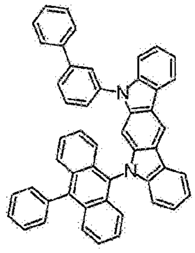
5-69

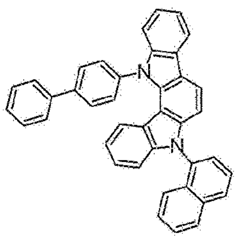
5-70

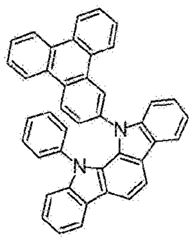
5-71

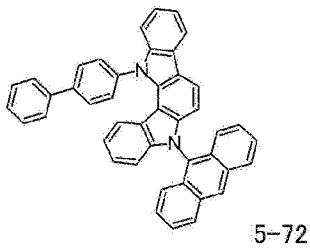
5-72

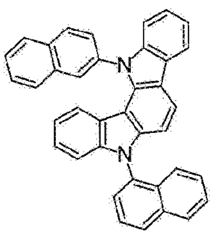
5-73

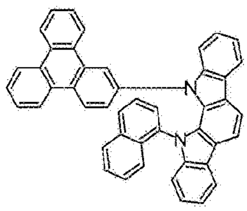
5-74

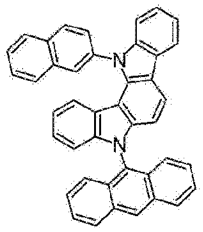
5-75

[C72]

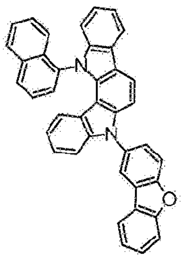
5-76

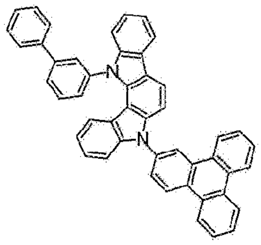
5-77

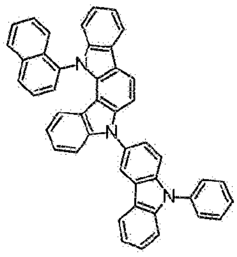
5-79

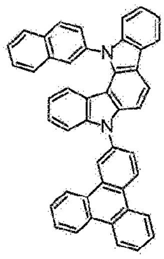
5-80

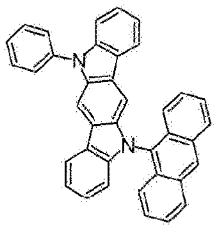
5-81

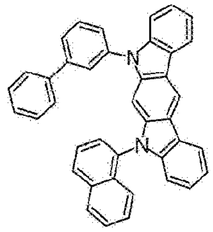
5-82

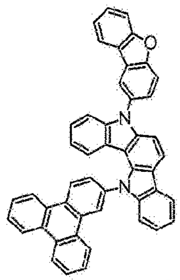
5-83

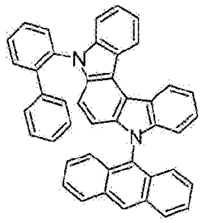
5-84

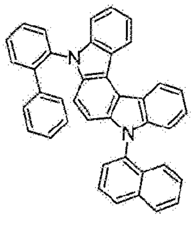
5-85

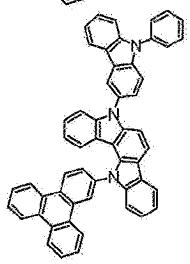
5-86

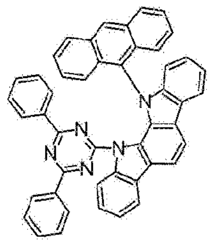
5-87

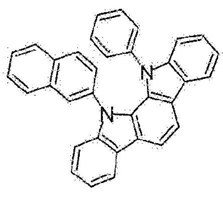
5-88

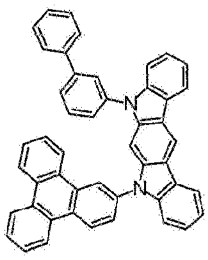
5-89

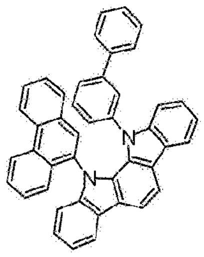
5-90

[C73]

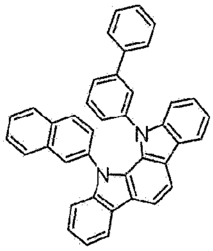
5-91

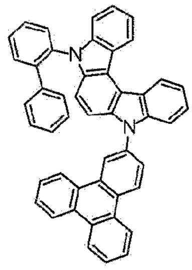
5-92

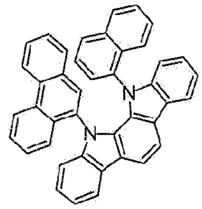
5-93

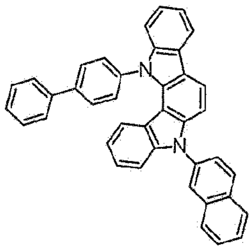
5-94

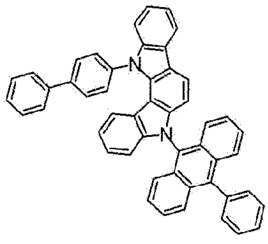
5-95

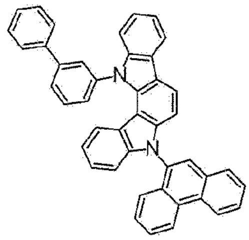
5-96

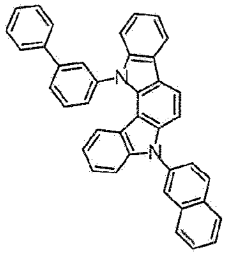
5-97

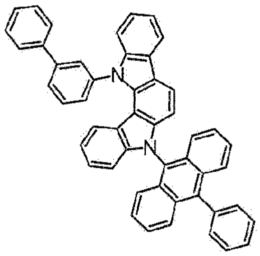
5-98

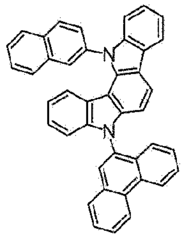
5-99

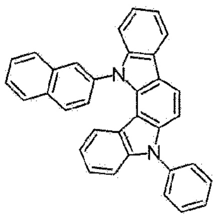
5-100

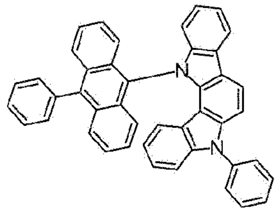
5-101

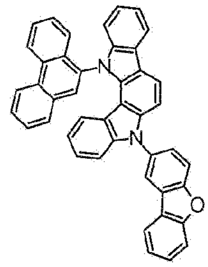
5-102

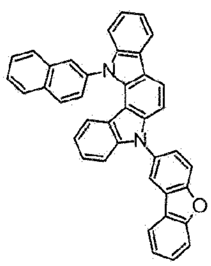
5-103

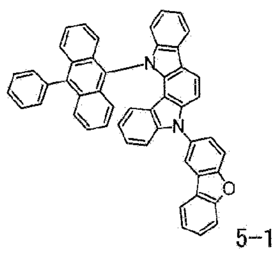
5-104

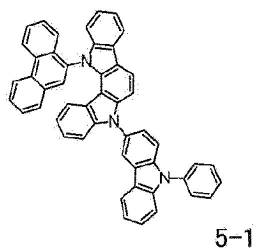
5-105

[C74]

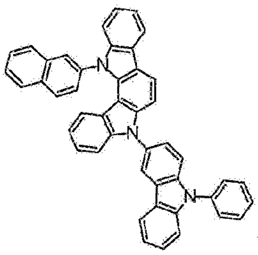
5-106

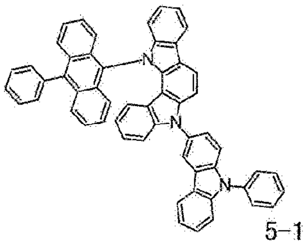
5-107

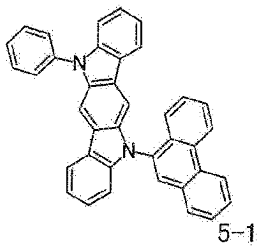
5-108

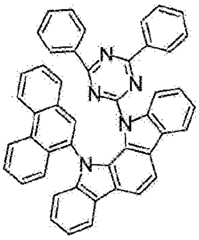
5-109

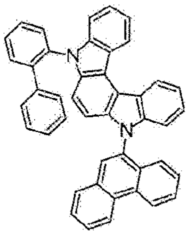
5-110

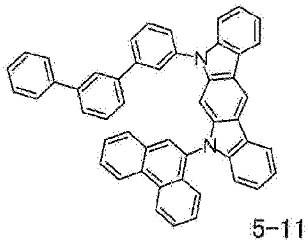
5-111

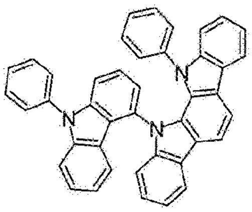
5-112

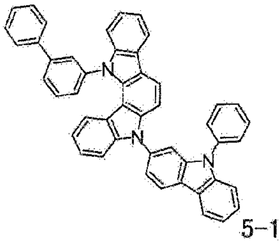
5-113

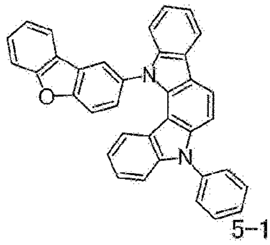
5-114

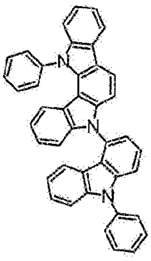
5-115

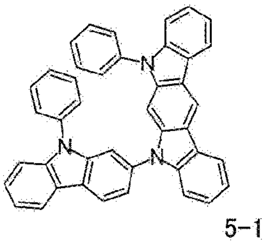
5-116

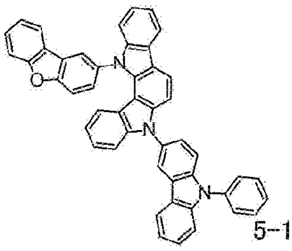
5-117

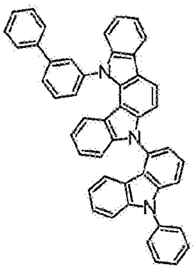
5-118

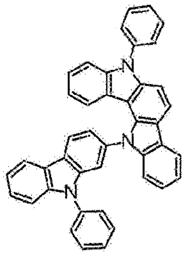
5-119

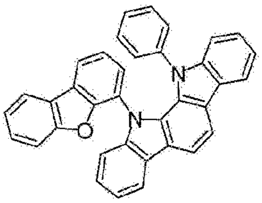
5-120

[C75]

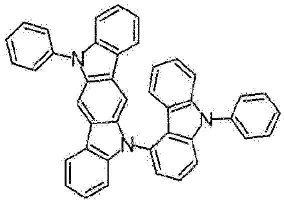
5-121

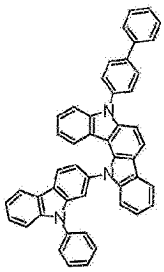
5-122

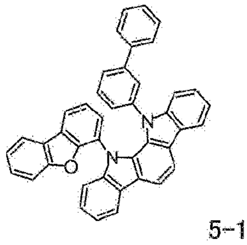
5-123

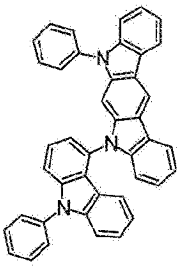
5-124

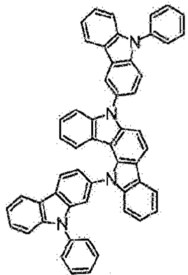
5-125

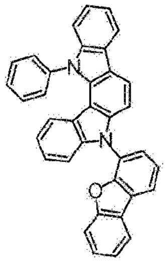
5-126

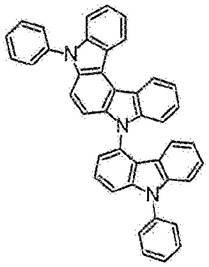
5-127

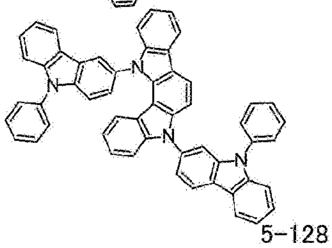
5-128

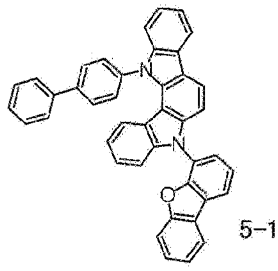
5-129

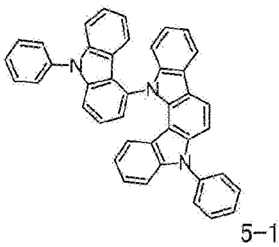
5-130

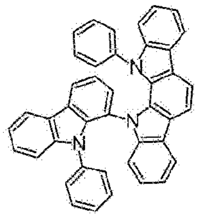
5-131

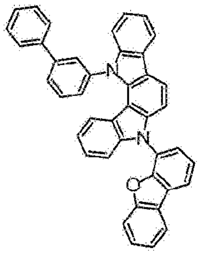
5-132

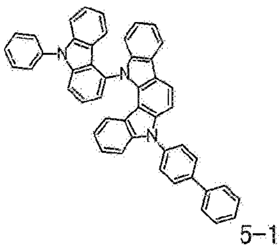
5-133

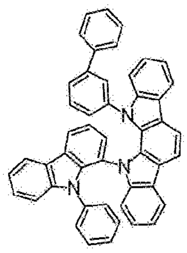
5-134

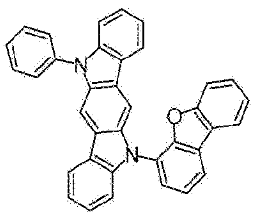
5-135

[C76]

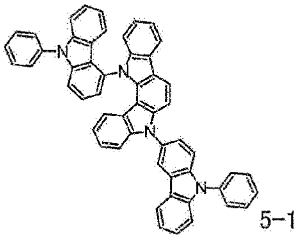
5-136

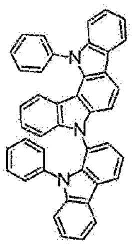
5-137

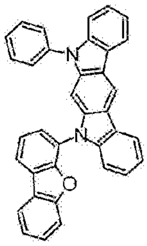
5-138

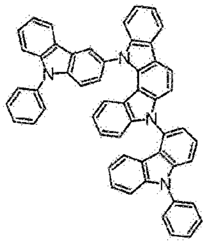
5-139

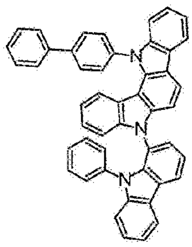
5-140

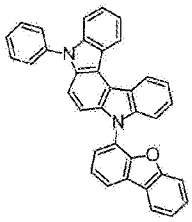
5-141

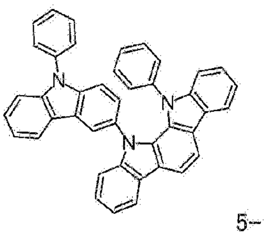
5-142

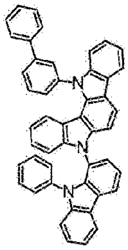
5-143

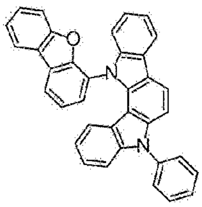
5-144

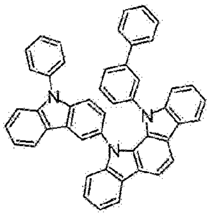
5-145

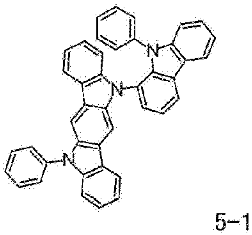
5-146

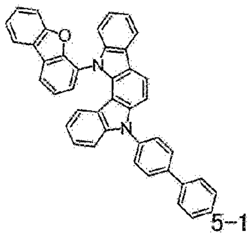
5-147

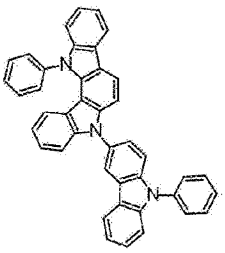
5-148

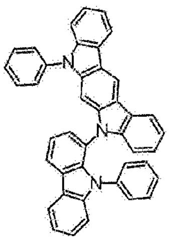
5-149

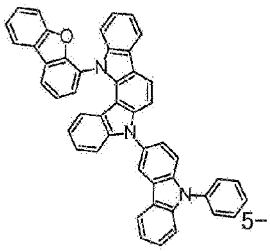
5-150

[C77]

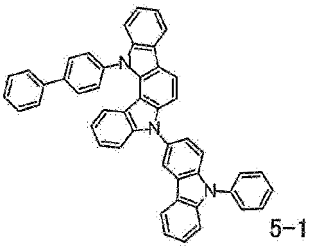
5-151

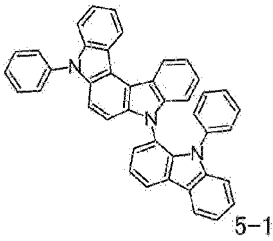
5-152

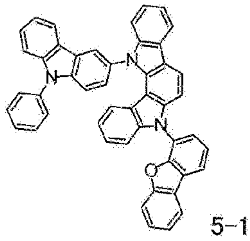
5-153

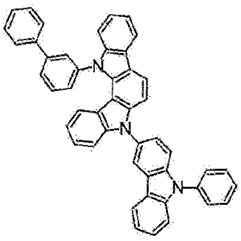
5-154

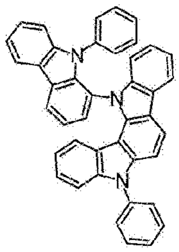
5-155

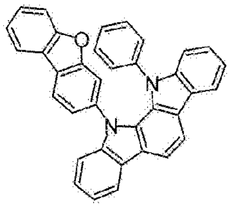
5-156

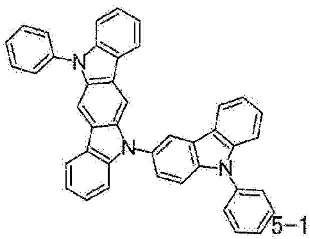
5-157

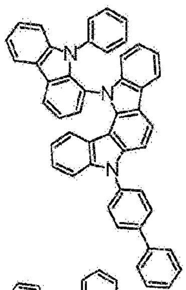
5-158

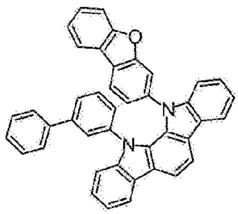
5-159

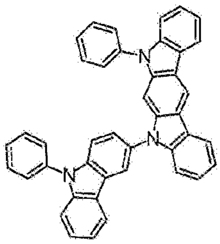
5-160

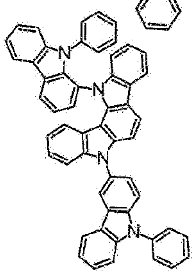
5-161

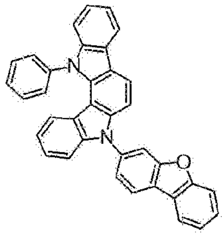
5-162

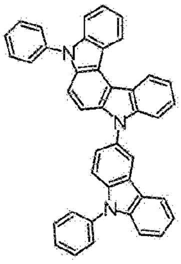
5-163

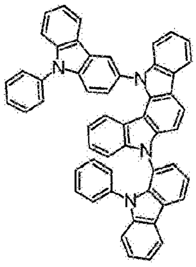
5-164

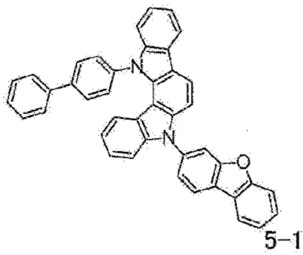
5-165

[C78]

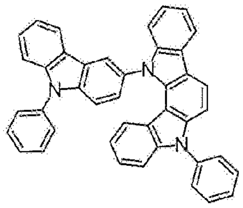
5-166

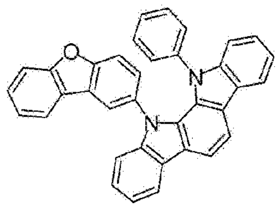
5-167

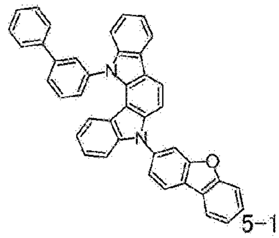
5-168

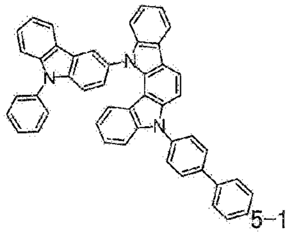
5-169

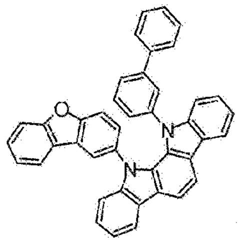
5-170

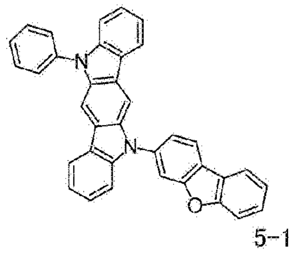
5-171

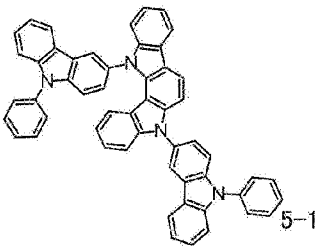
5-172

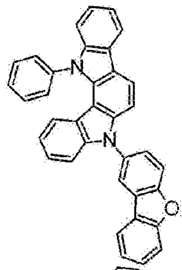
5-173

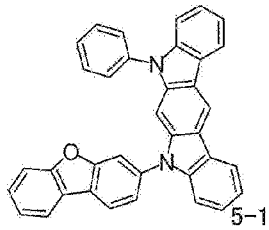
5-174

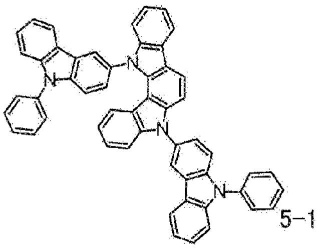
5-175

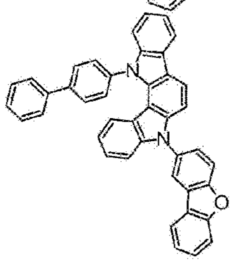
5-176

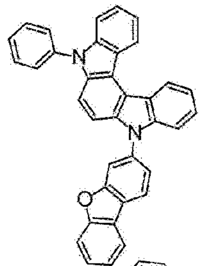
5-177

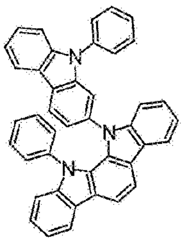
5-178

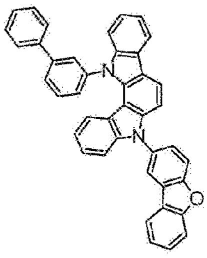
5-179

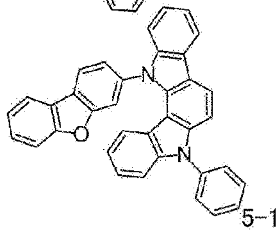
5-180

[C79]

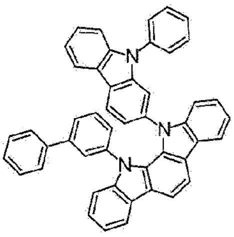
5-181

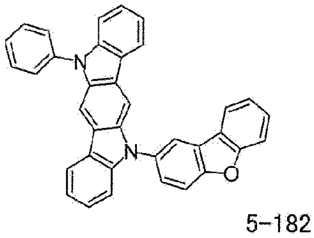
5-182

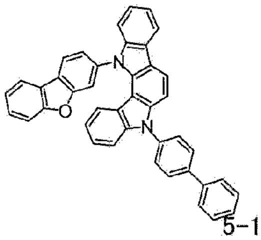
5-183

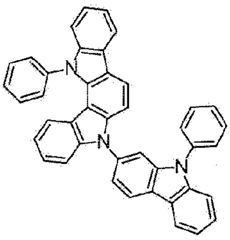
5-184

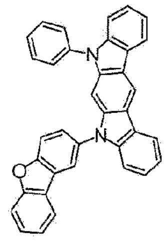
5-185

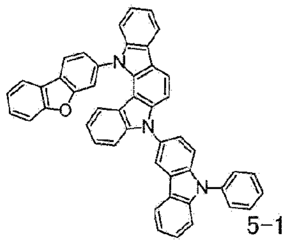
5-186

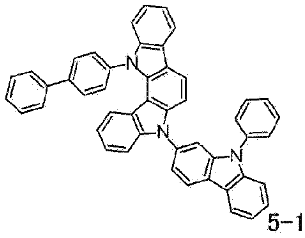
5-187

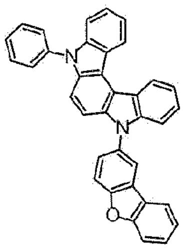
5-188

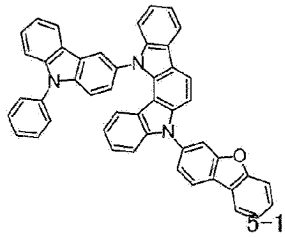
5-189

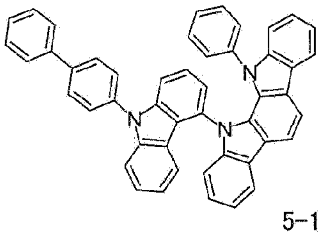
5-190

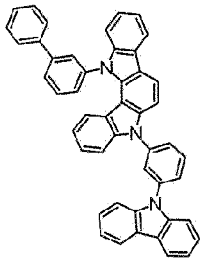
5-191

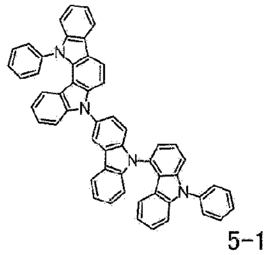
5-192

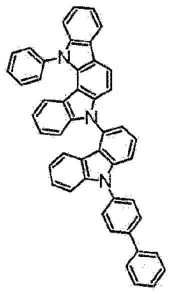
5-193

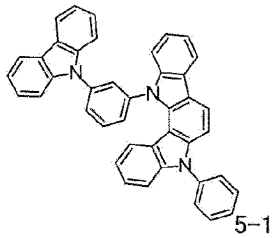
5-194

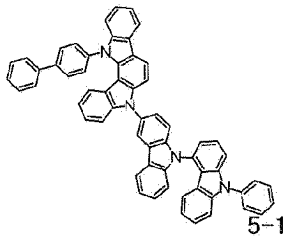
5-195

[C80]

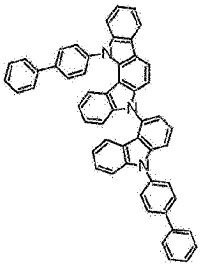
5-196

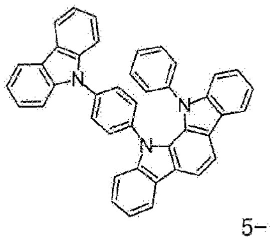
5-197

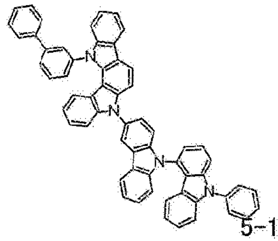
5-198

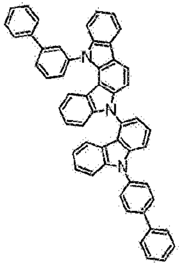
5-199

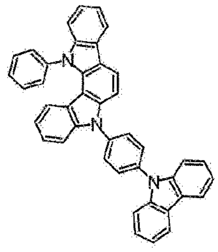
5-200

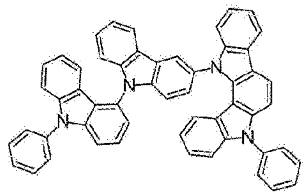
5-201

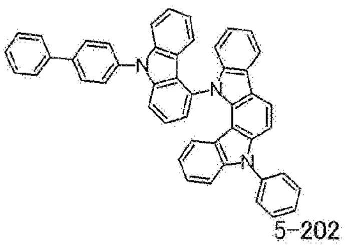
5-202

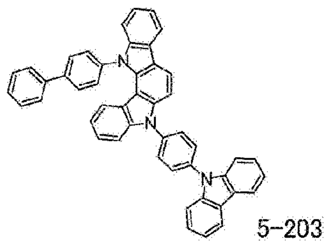
5-203

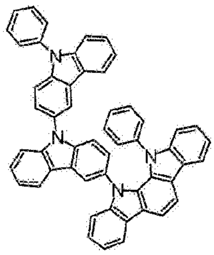
5-204

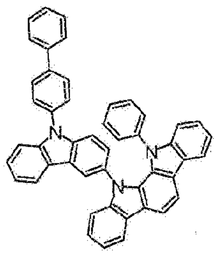
5-205

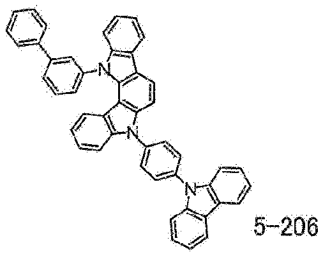
5-206

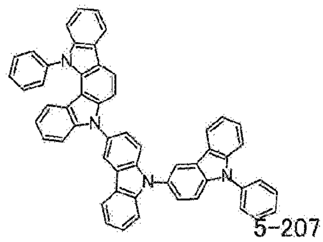
5-207

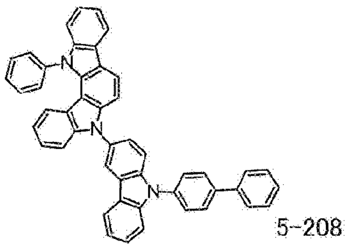
5-208

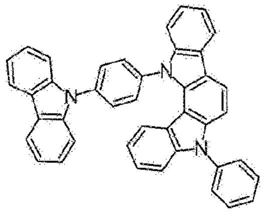
5-209

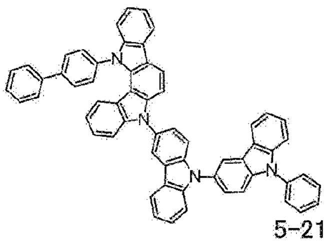
5-210

[C81]

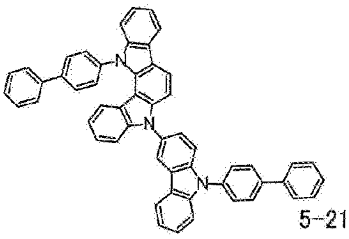
5-211

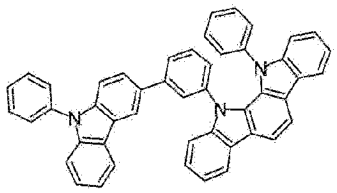
5-212

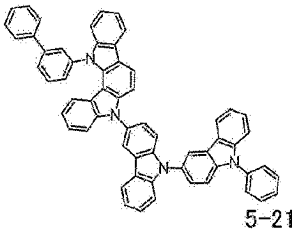
5-213

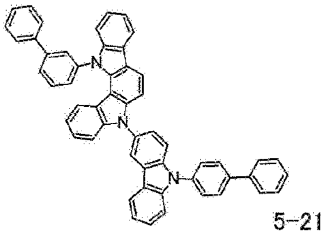
5-214

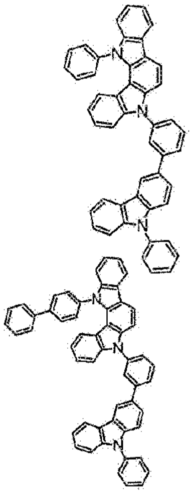
5-215

5-218

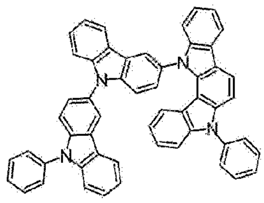
5-216

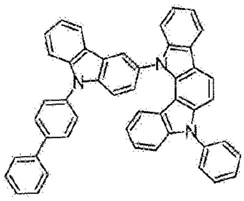
5-217

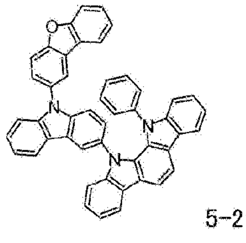
5-219

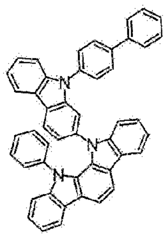
5-220

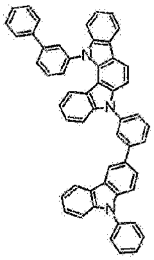
5-221

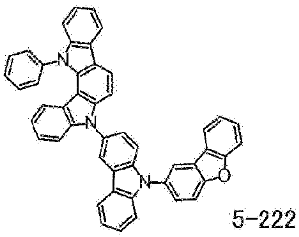
5-222

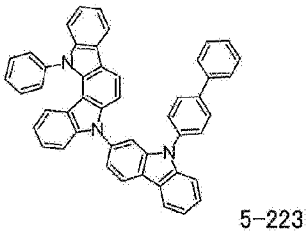
5-223

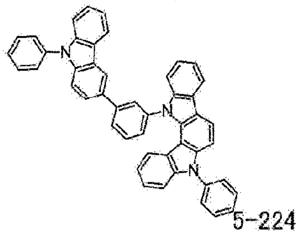
5-224

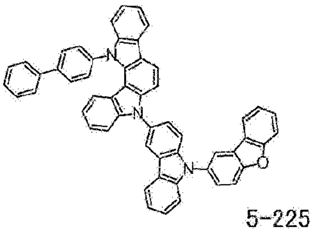
5-225

[C82]

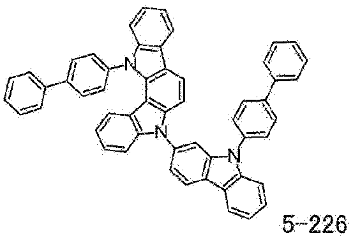

5-226

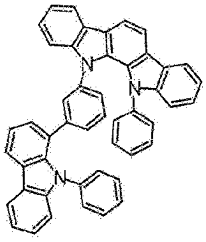

5-227

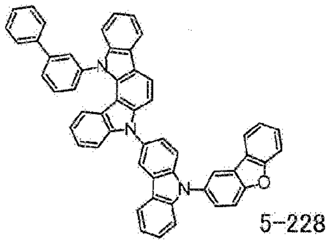

5-228

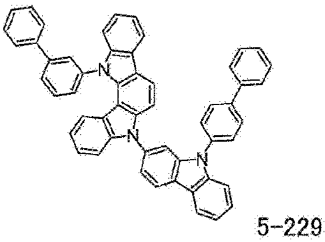

5-229

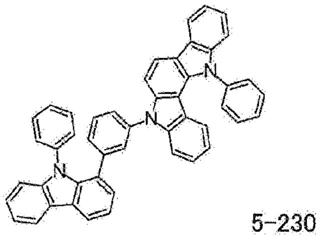

5-230

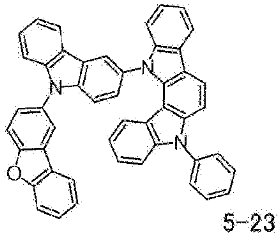

5-231

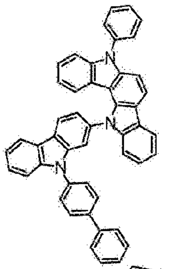

5-232

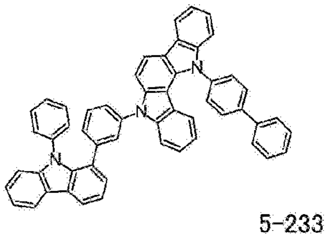

5-233

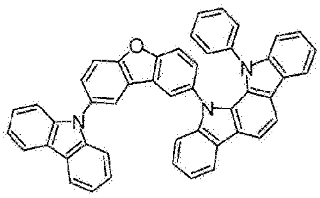

5-234

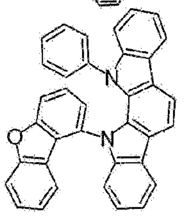

5-235

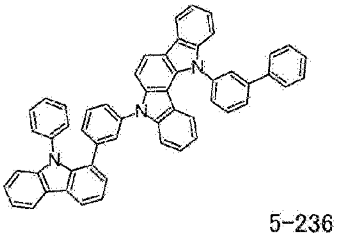

5-236

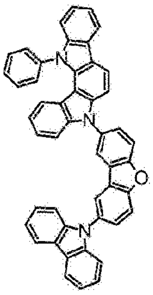

5-237

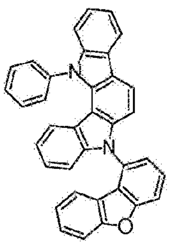

5-238

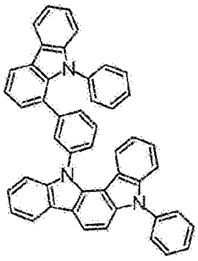

5-239

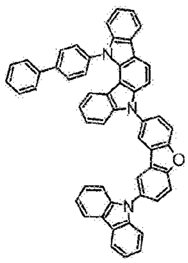

5-240

[C83]

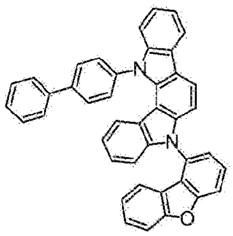
5-241

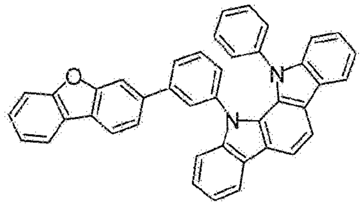
5-242

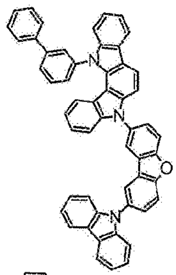
5-243

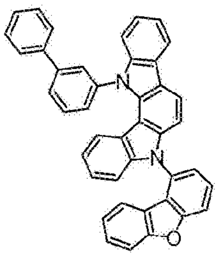
5-244

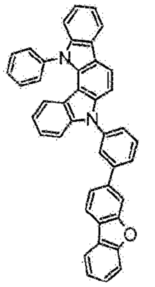
5-245

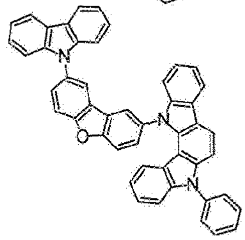
5-246

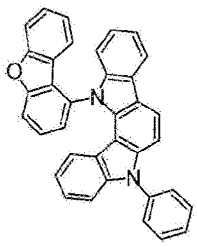
5-247

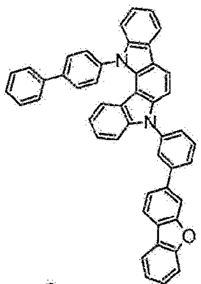
5-248

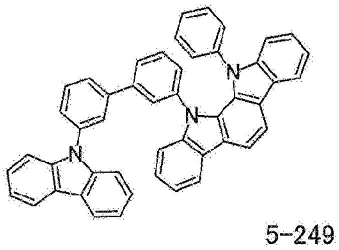
5-249

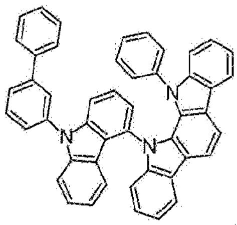
5-250

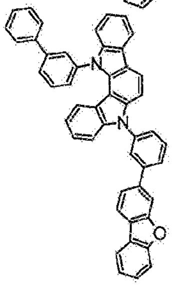
5-251

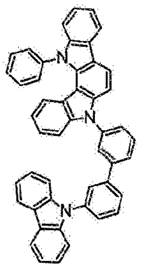
5-252

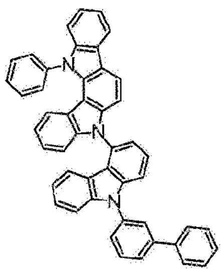
5-253

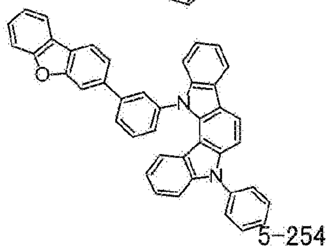
5-254

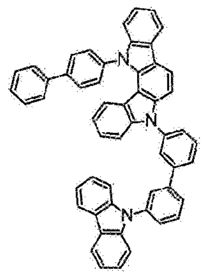
5-255

[C84]

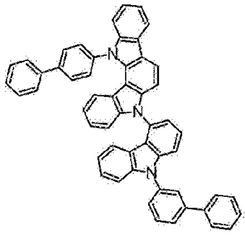
5-256

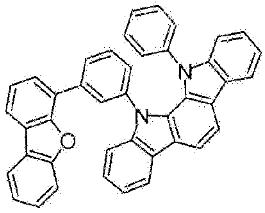
5-257

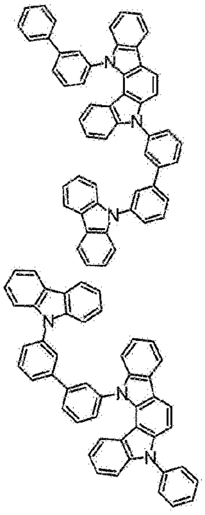
5-258

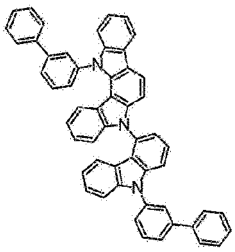
5-259

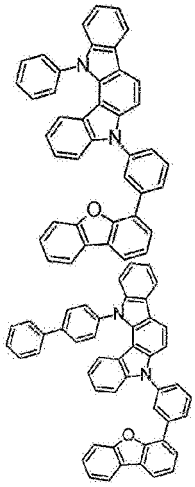
5-260

5-261

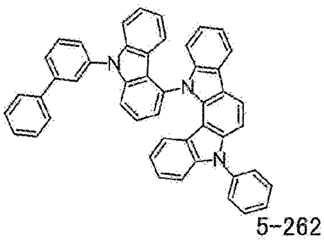
5-262

5-263

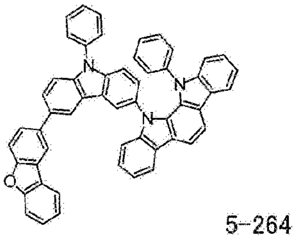
5-264

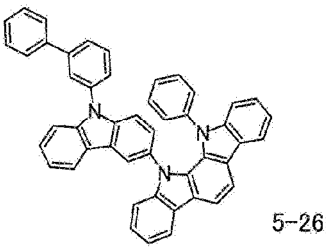
5-265

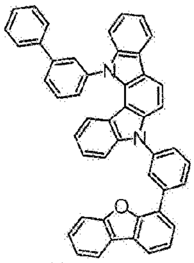
5-266

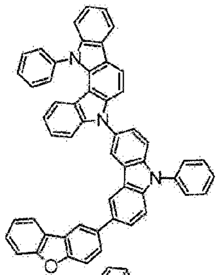
5-267

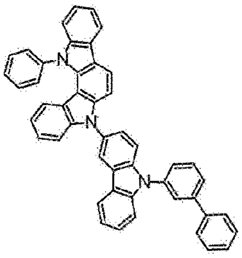
5-268

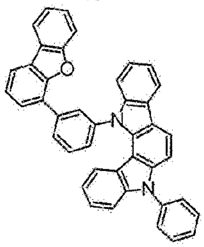
5-269

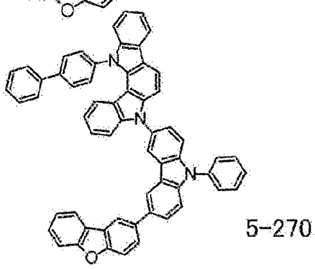
5-270

[C85]

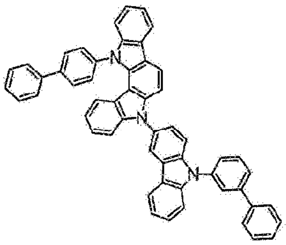
5-271

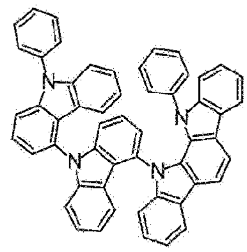
5-272

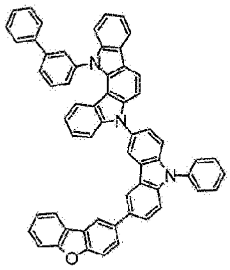
5-273

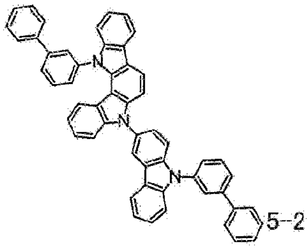
5-274

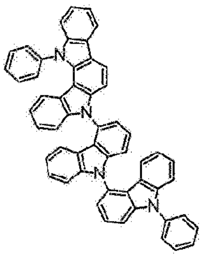
5-275

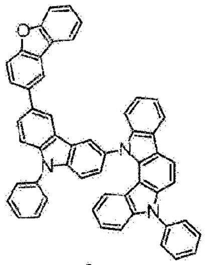
5-276

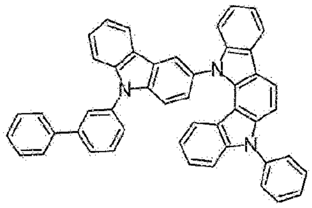
5-277

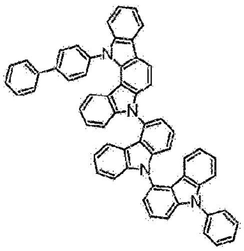
5-278

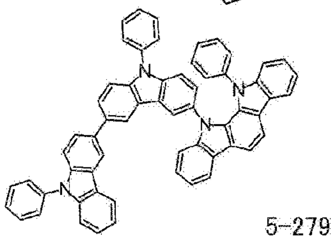
5-279

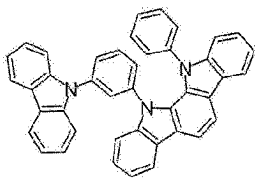
5-280

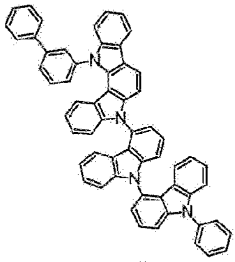
5-281

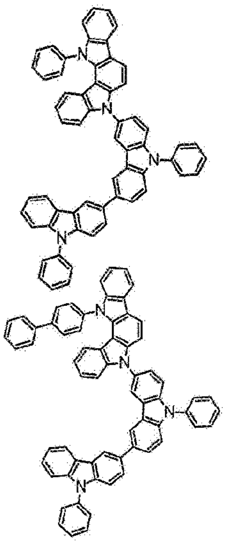
5-282

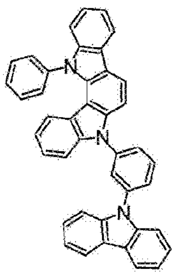
5-283

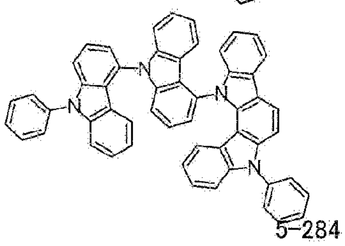
5-284

5-285

[C86]

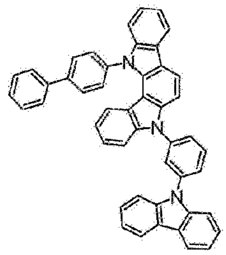

5-286

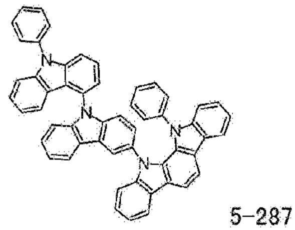

5-287

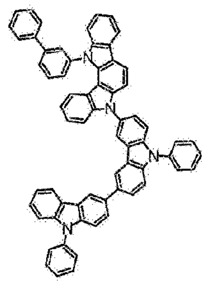

5-288

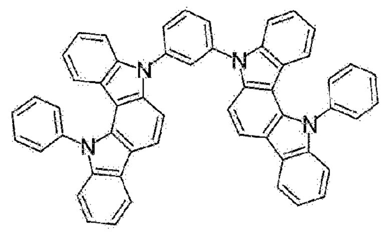

5-289

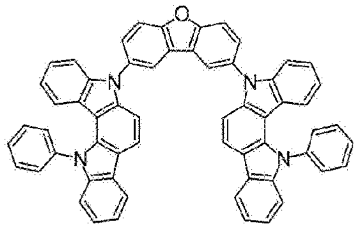

5-290

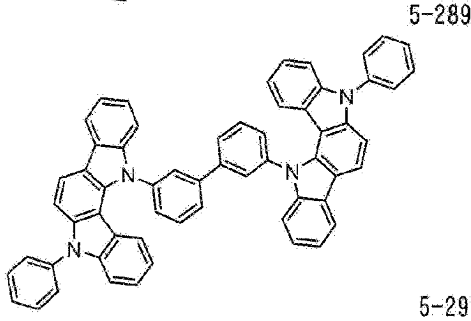

5-291

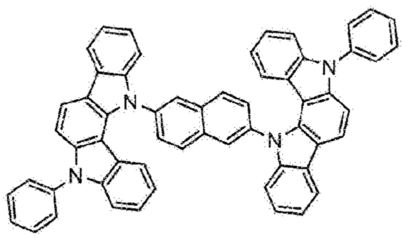

5-292

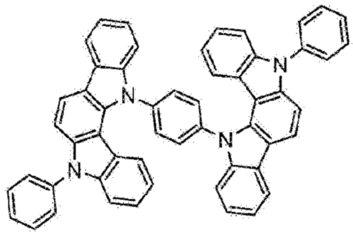

5-293

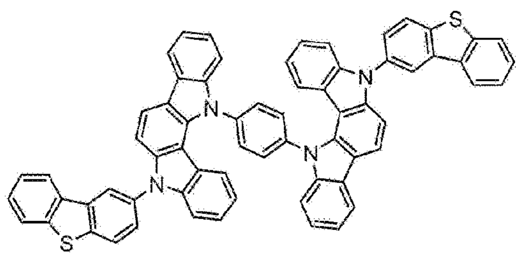

5-294

[C87]

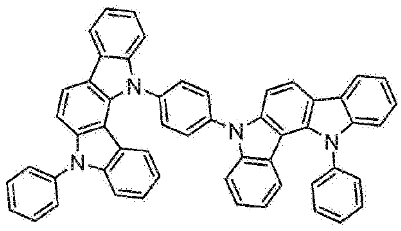

5-295

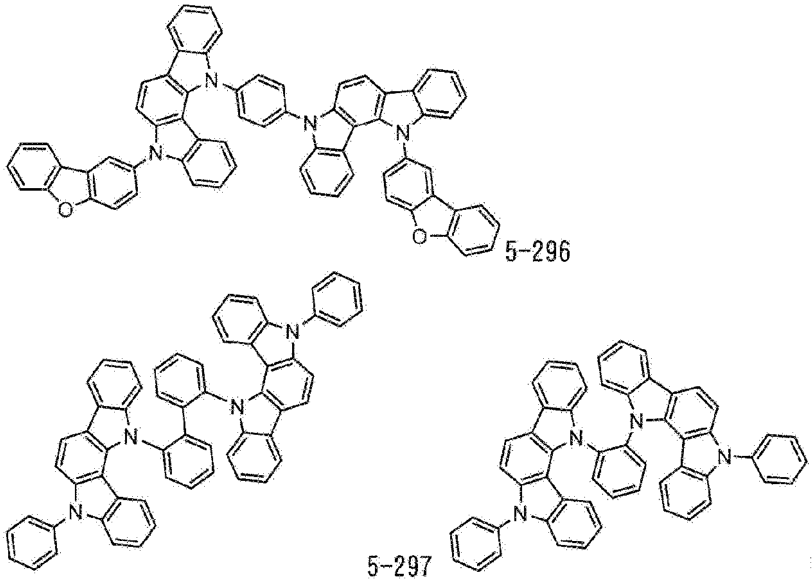

5-296

5-297

5-298

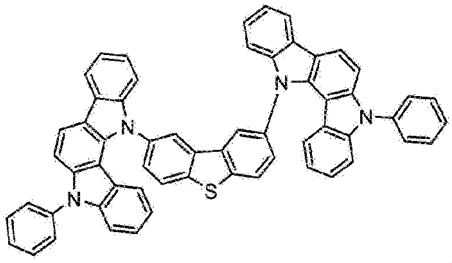

5-299

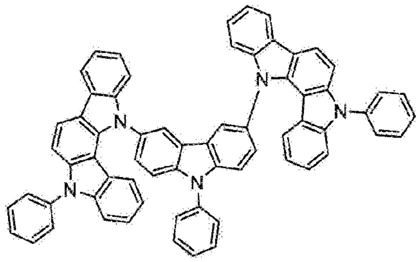

5-300

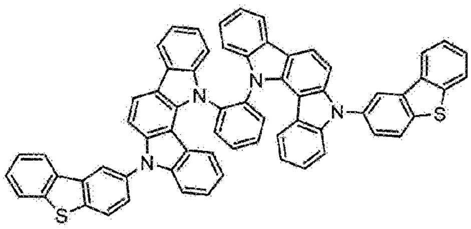

5-301

[C88]

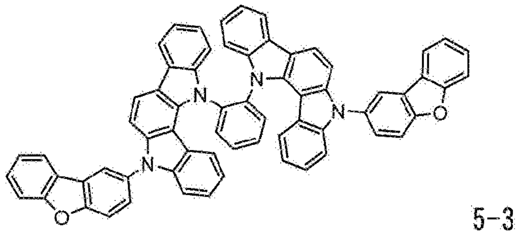

5-302

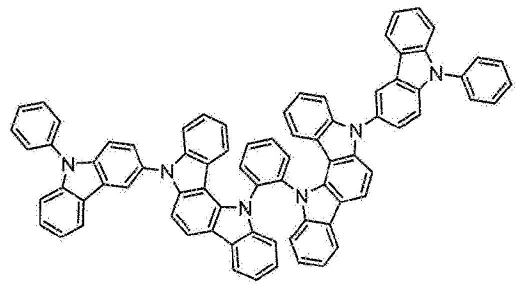

5-303

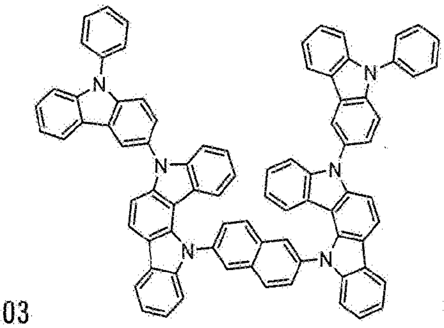

5-304

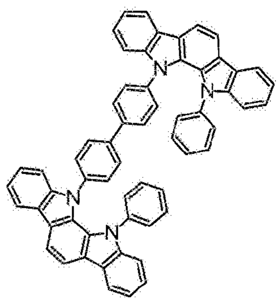

5-305

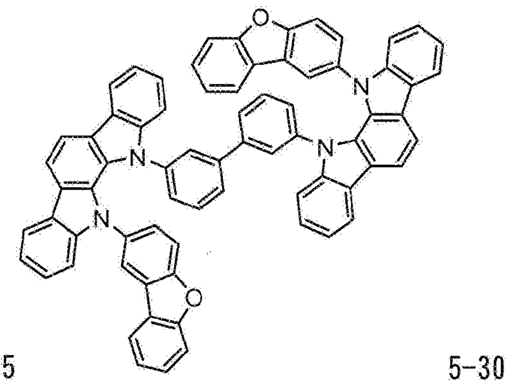

5-306

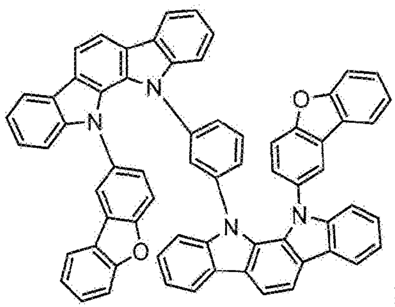

5-307

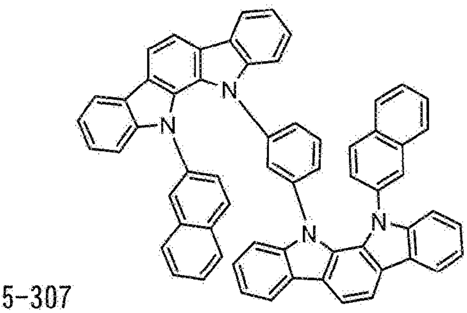

5-308

[C89]

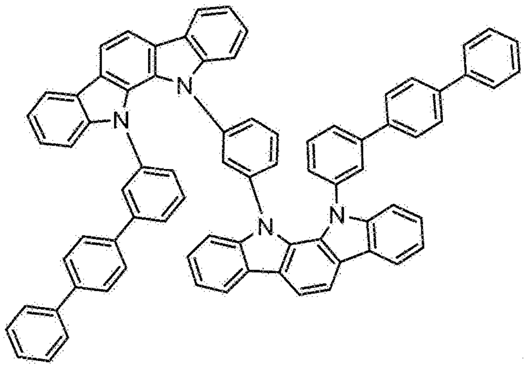

5-309

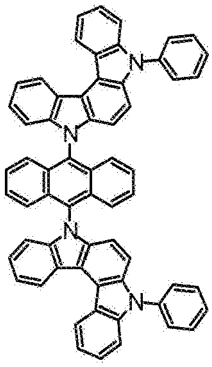

5-310

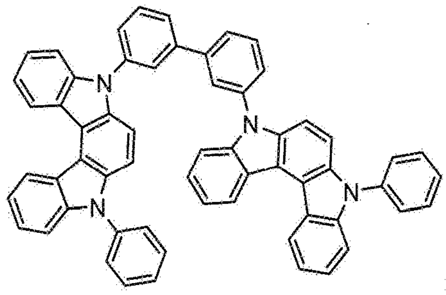

5-311

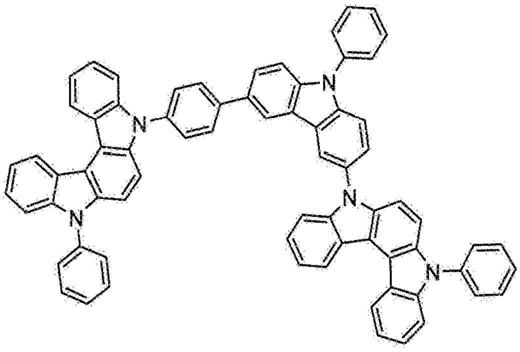

5-312

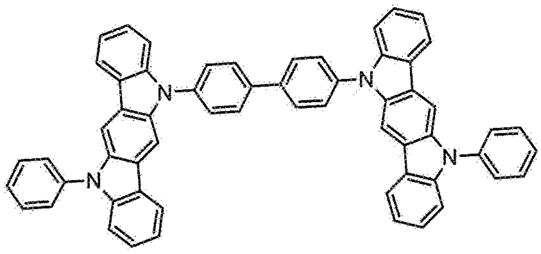

5-313

[C90]

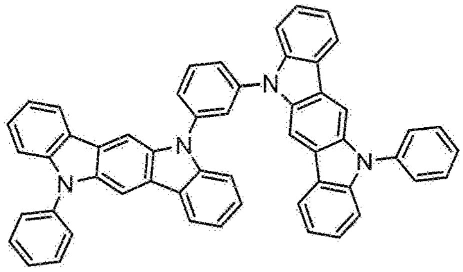

5-314

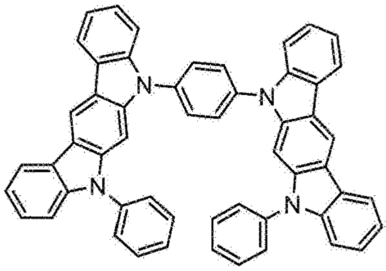

5-315

Specific examples of the compounds represented by the general formula (12) above are shown below, but are not limited to these exemplified compounds.

[C91]

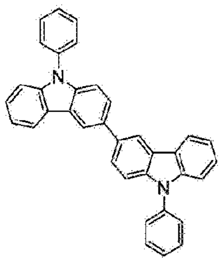
6-1

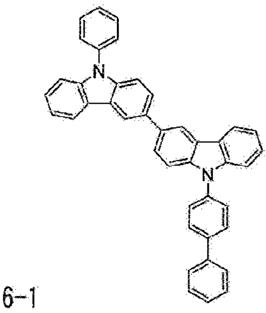
6-2

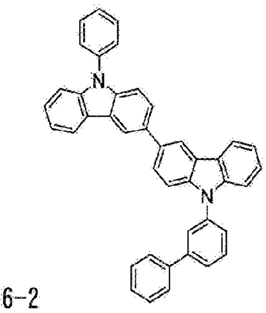
6-3

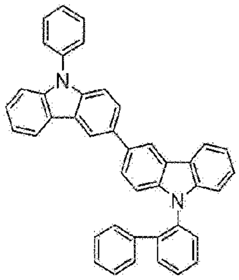
6-4

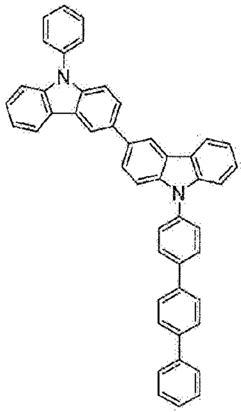
6-5

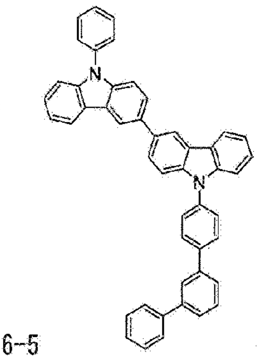
6-6

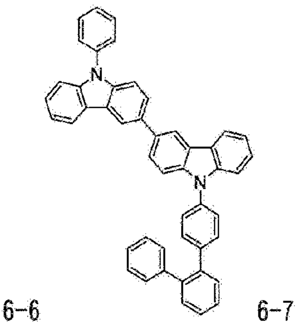
6-7

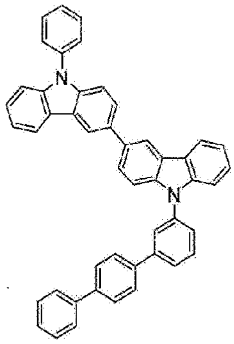
6-8

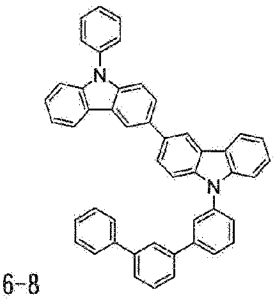
6-9

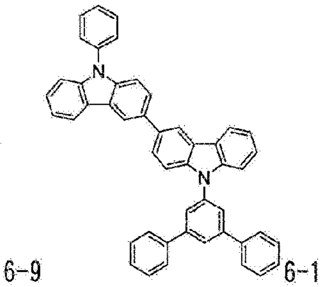
6-10

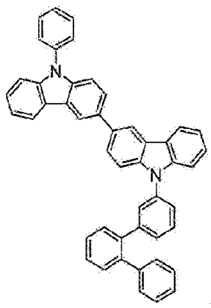
6-11

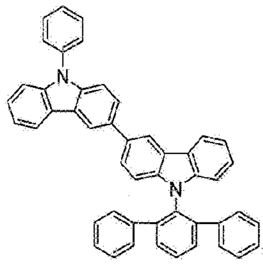
6-12

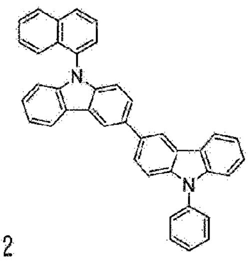
6-13

[C92]

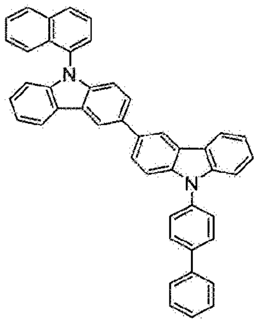

6-14

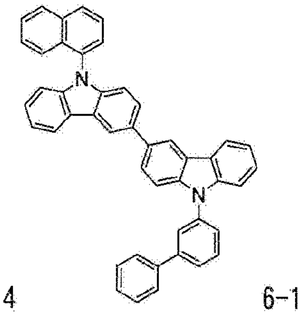

6-15

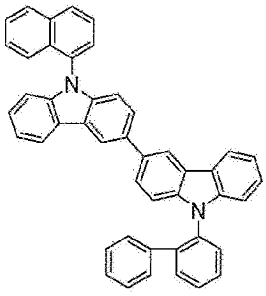

6-16

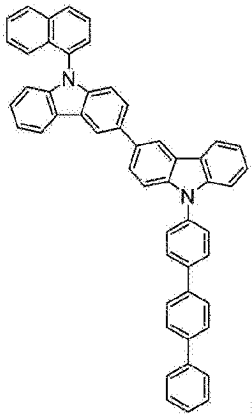

6-17

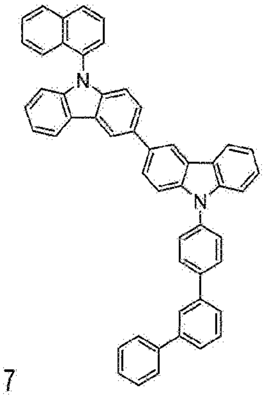

6-18

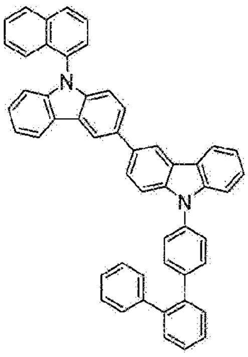

6-19

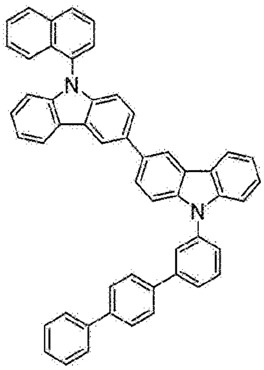

6-20

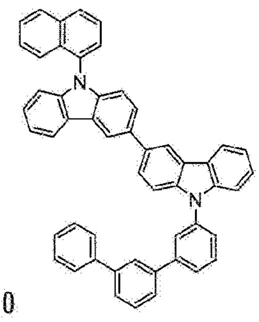

6-21

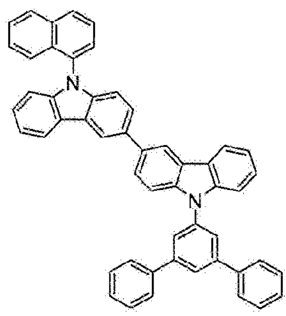

6-22

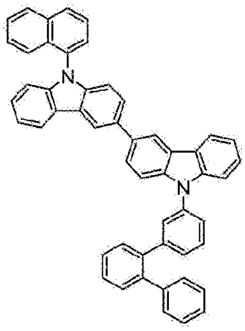

6-23

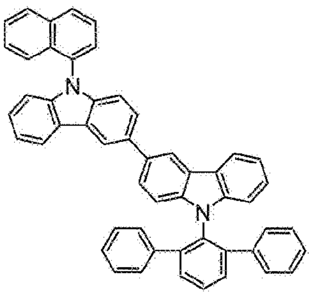

6-24

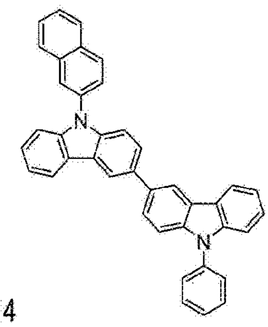

6-25

[C93]

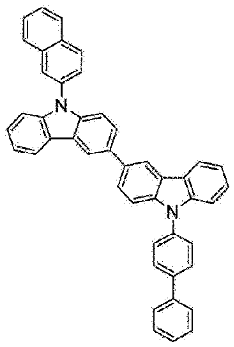
6-26

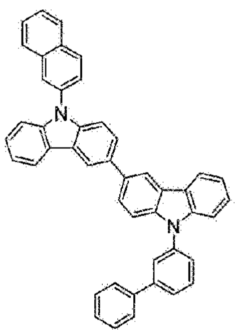
6-27

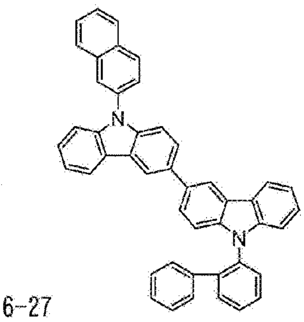
6-28

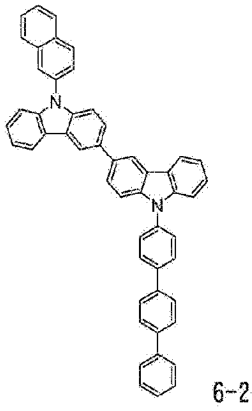
6-29

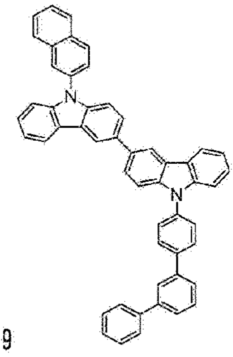
6-30

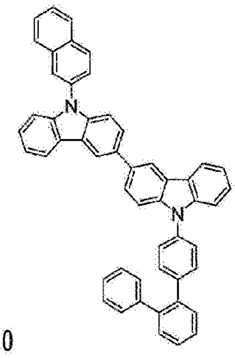
6-31

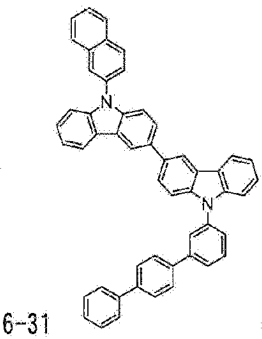
6-32

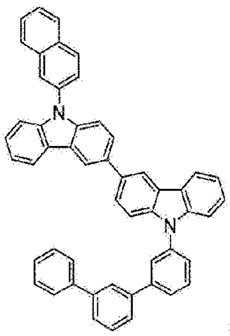
6-33

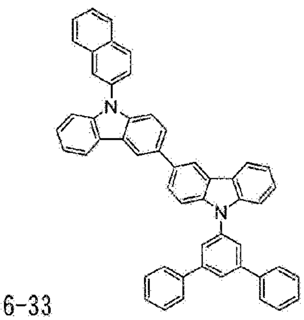
6-34

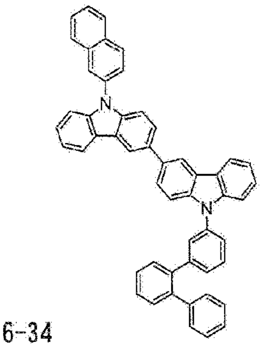
6-35

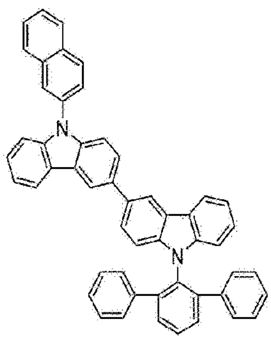
6-36

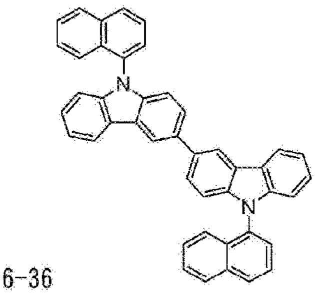
6-37

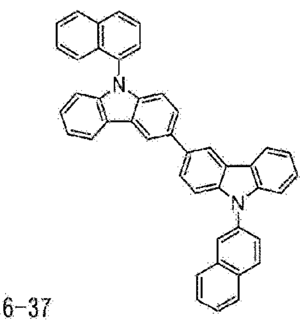
6-38

[C94]

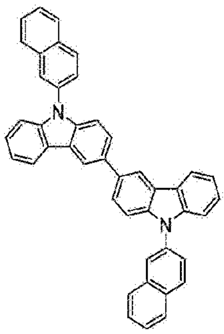
6-39

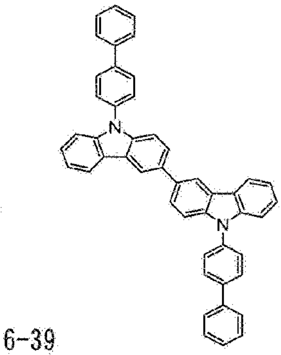
6-40

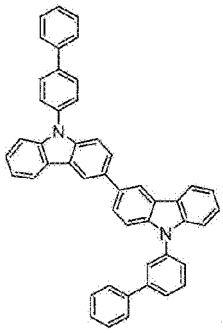
6-41

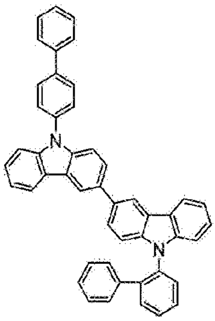
6-42

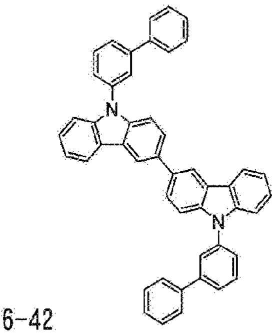
6-43

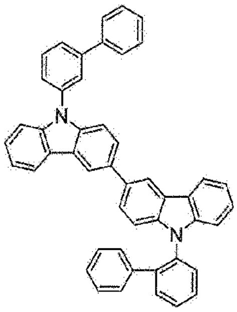
6-44

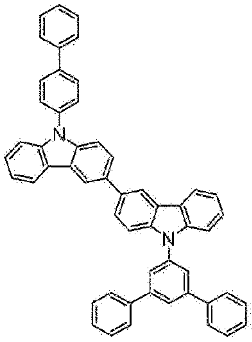
6-45

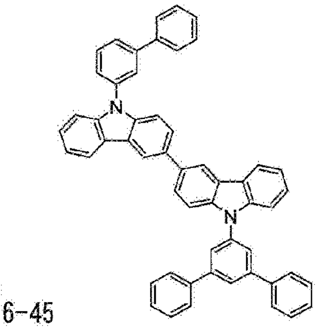
6-46

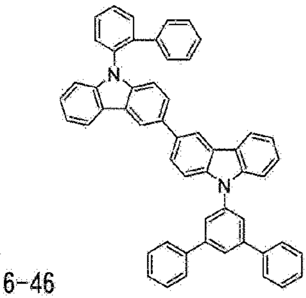
6-47

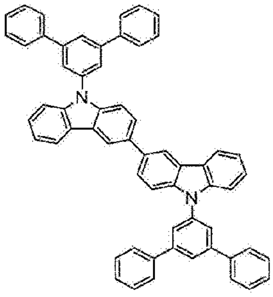
6-48

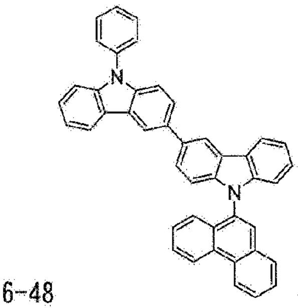
6-49

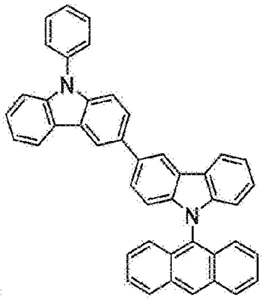
6-50

[C95]

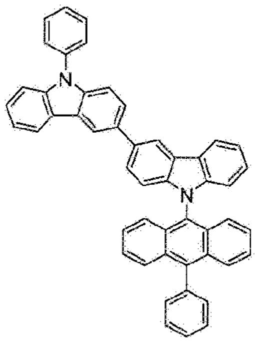
6-51

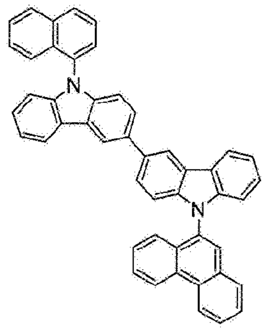
6-52

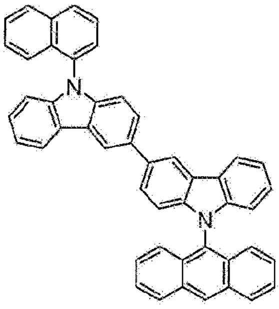
6-53

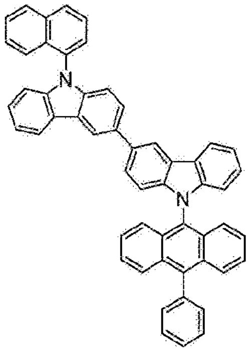
6-54

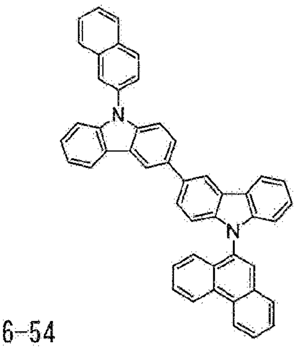
6-55

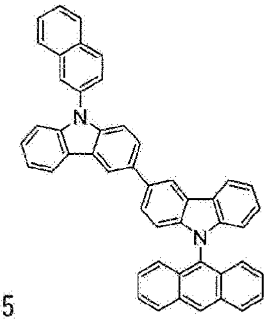
6-56

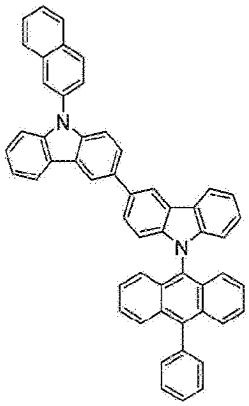
6-57

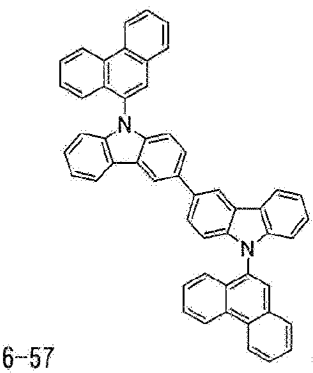
6-58

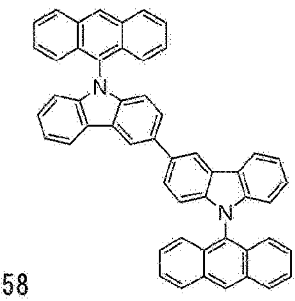
6-59

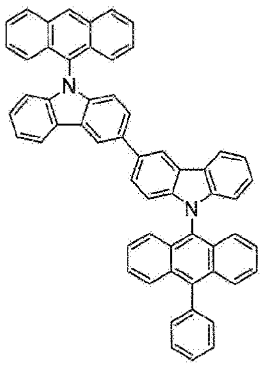
6-60

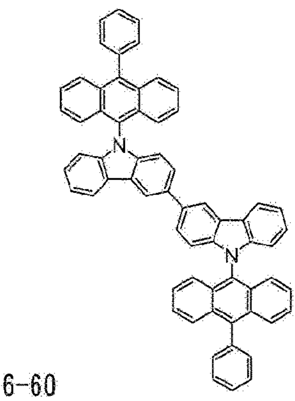
6-61

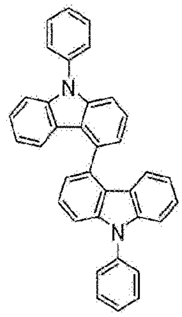
6-62

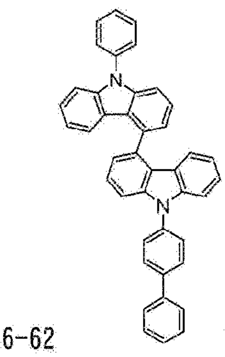
6-63

[C96]

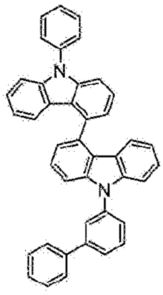
6-64
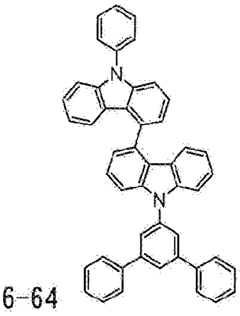
6-65
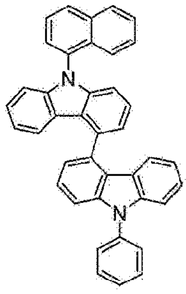
6-66
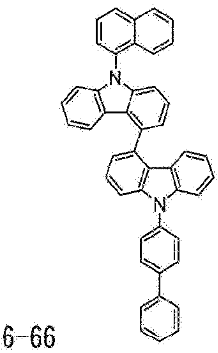
6-67

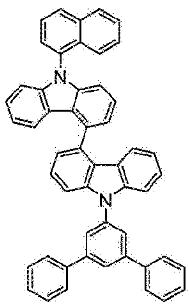
6-68
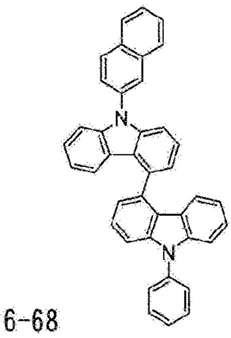
6-69
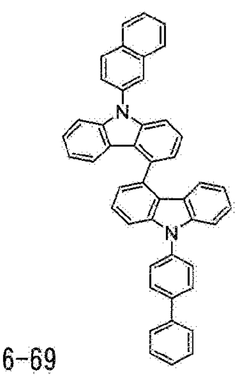
6-70
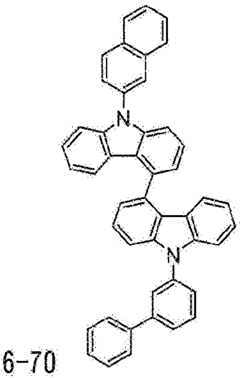
6-71

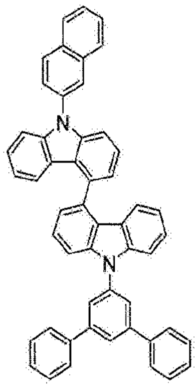
6-72
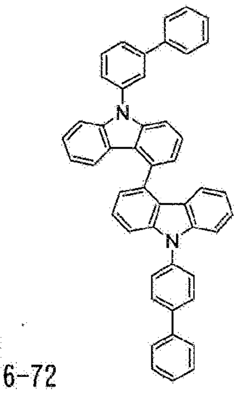
6-73
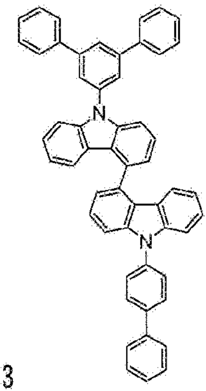
6-74
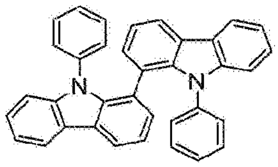
6-75

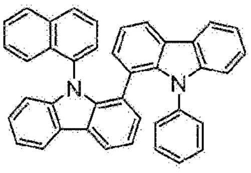
6-76
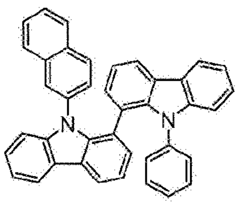
6-77
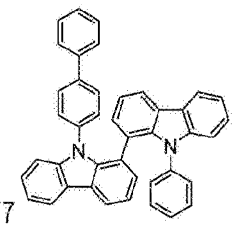
6-78

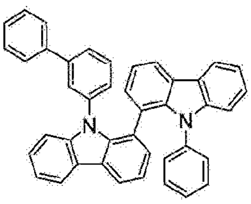
6-79
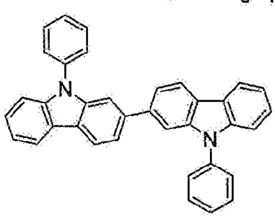
6-80
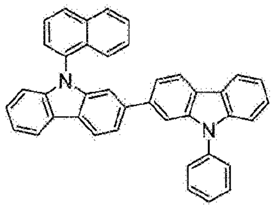
6-81

[C97]

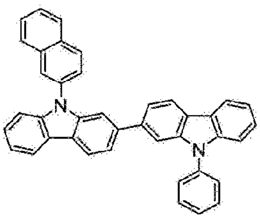
6-82
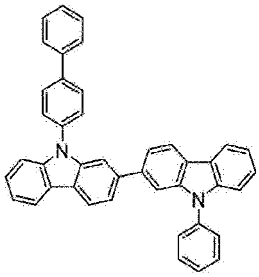
6-83
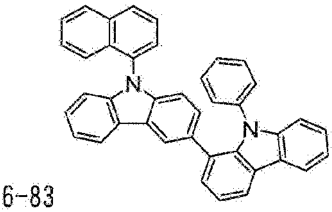
6-84
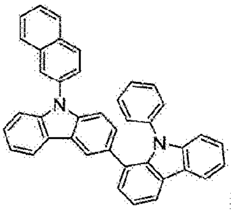
6-85
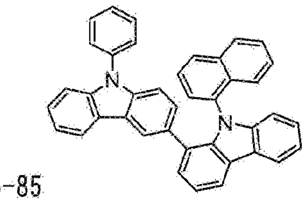
6-86
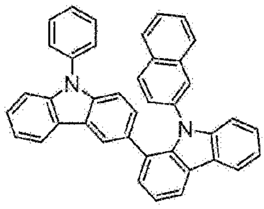
6-87
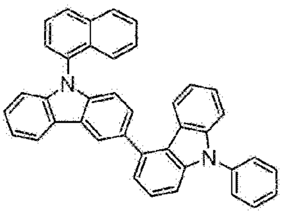
6-88
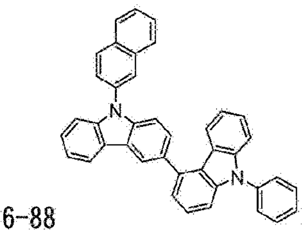
6-89
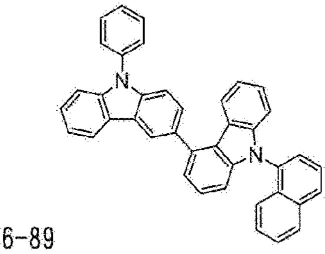
6-90
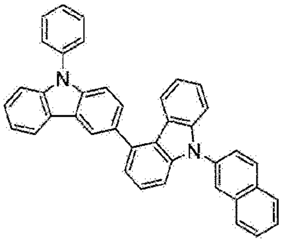
6-91
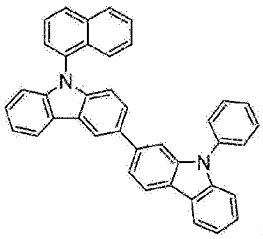
6-92
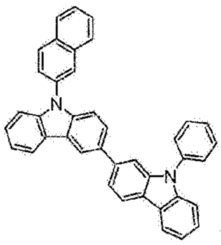
6-93
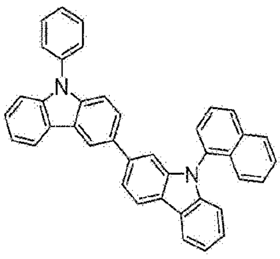
6-94
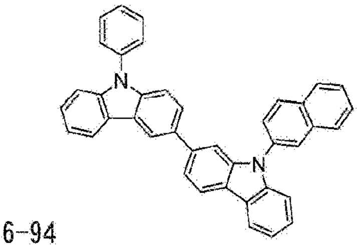
6-95
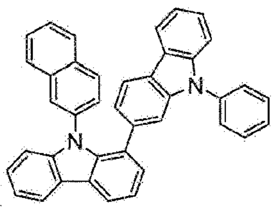
6-96
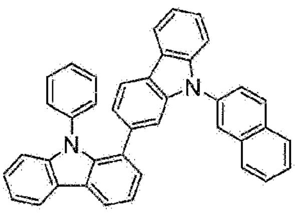
6-97
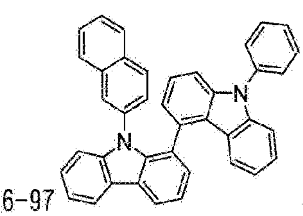
6-98
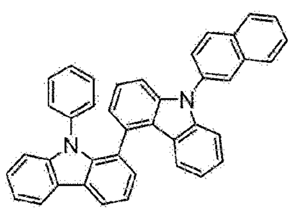
6-99

[C98]

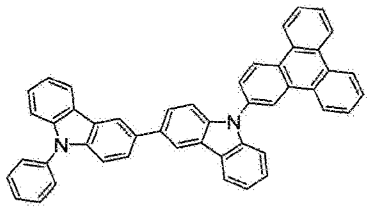
6-100

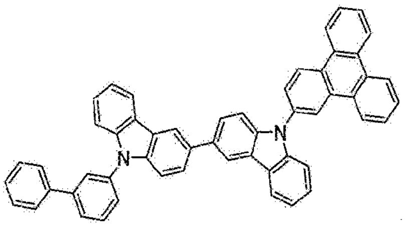
6-101

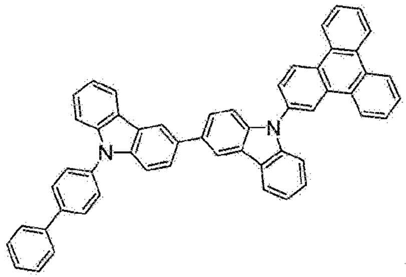
6-102

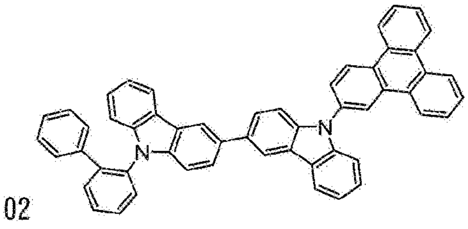
6-103

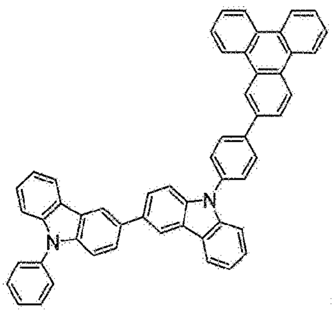
6-104

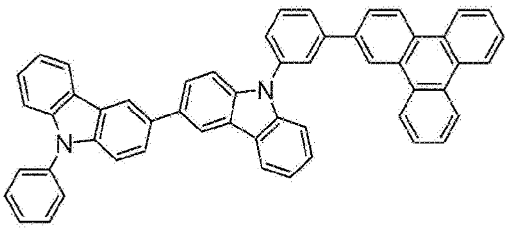
6-105

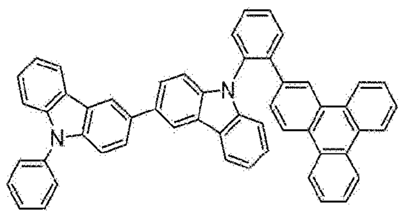
6-106

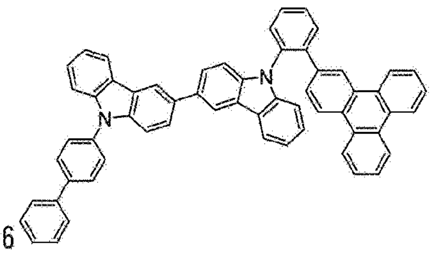
6-107

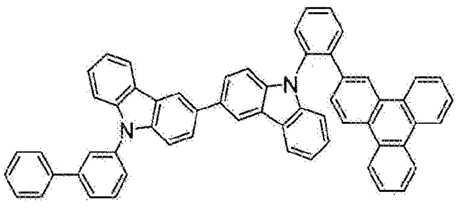
6-108

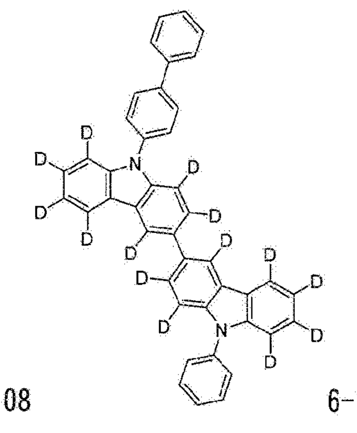
6-109

[C99]

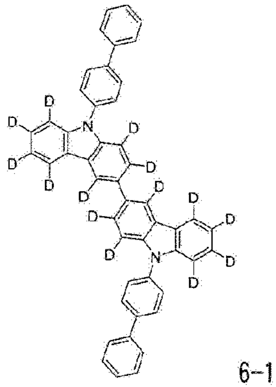
6-110

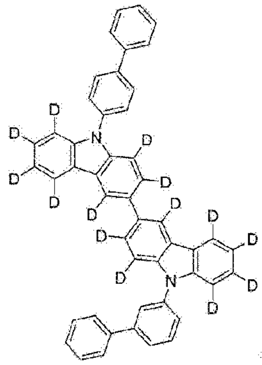
6-111

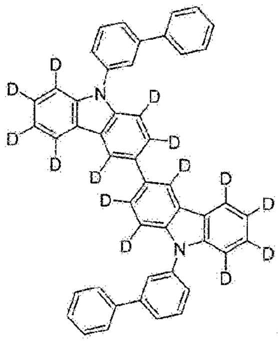
6-112

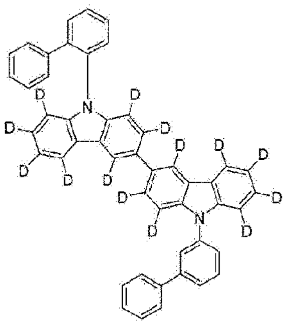
6-113

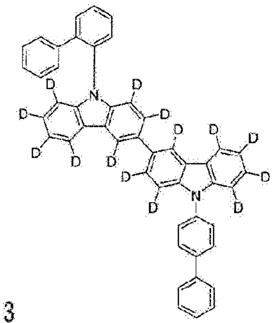
6-114

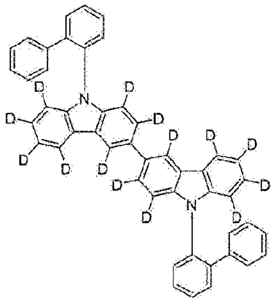
6-115

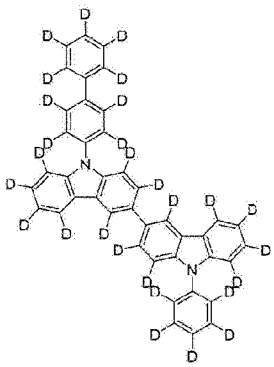
6-116

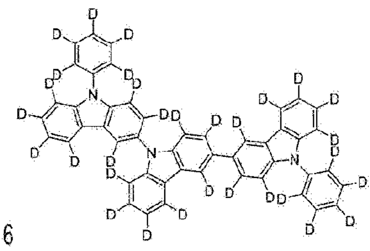
6-117

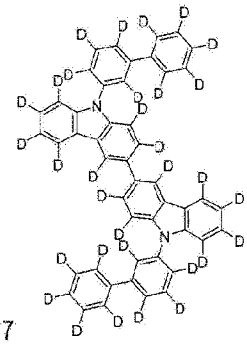
6-118

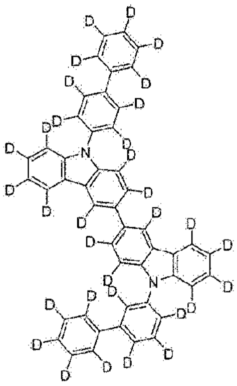
6-119

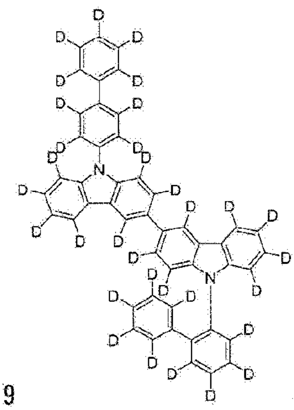
6-120

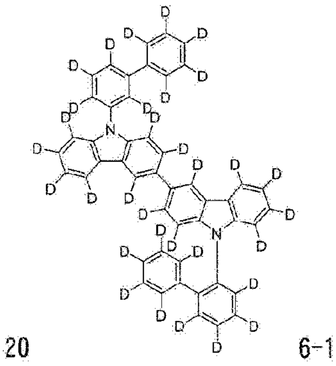
6-121

Specific examples of the compounds represented by the general formula (13) above are shown below, but are not limited to these exemplified compounds.

[C100]

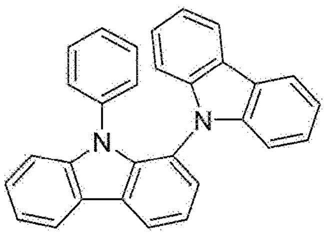
7-1

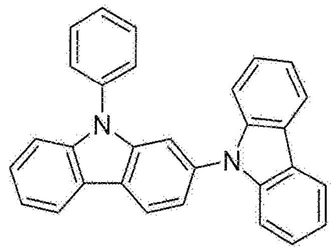
7-2

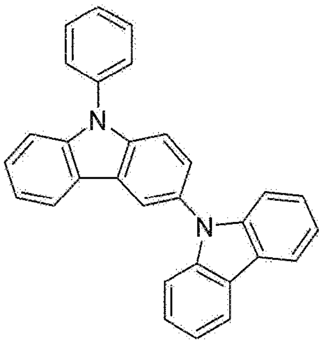
7-3

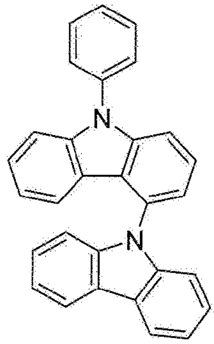
7-4

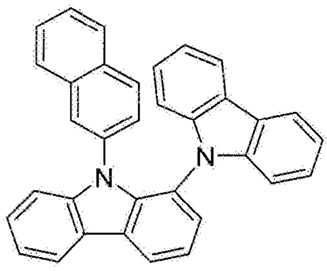
7-5

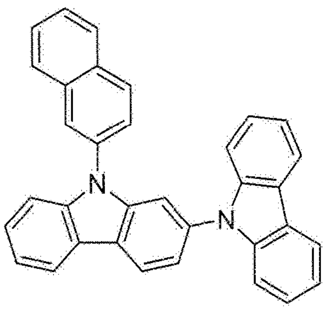
7-6

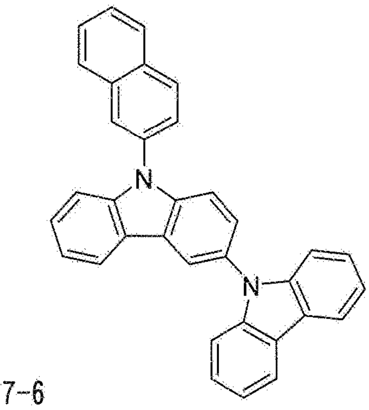
7-7

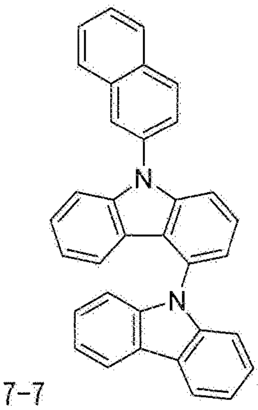
7-8

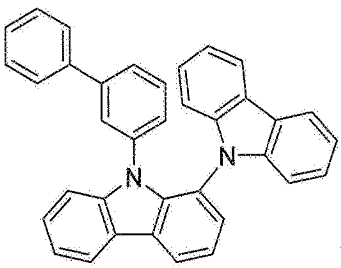
7-9

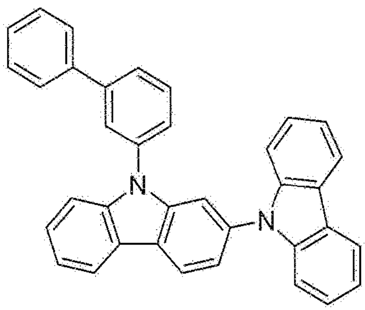
7-10

[C101]

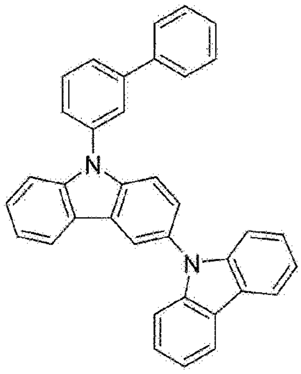
7-11
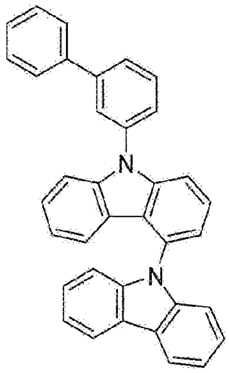
7-12
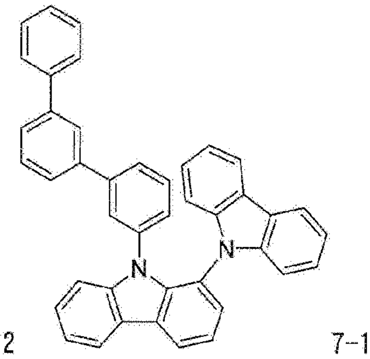
7-13
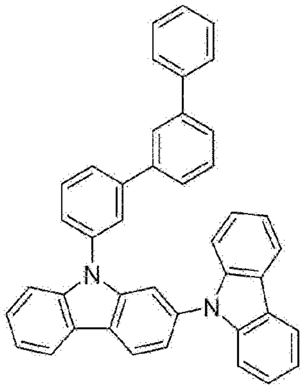
7-14
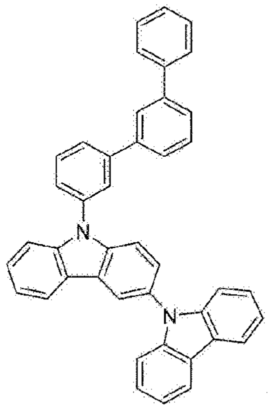
7-15
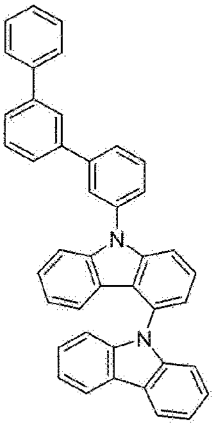
7-16
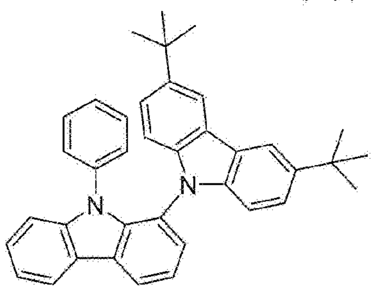
7-17
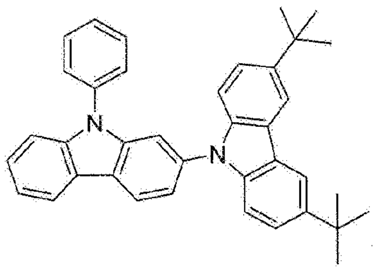
7-18
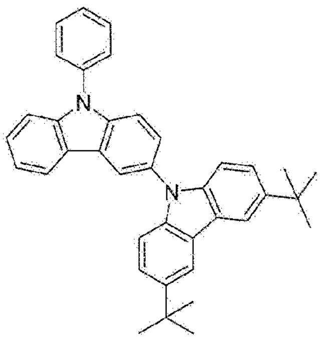
7-19
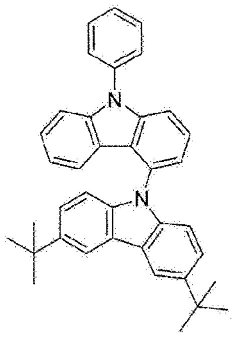
7-20

[C102]

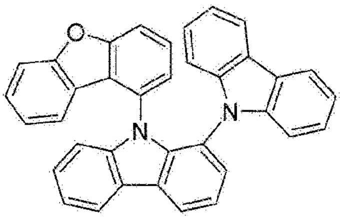

7-21

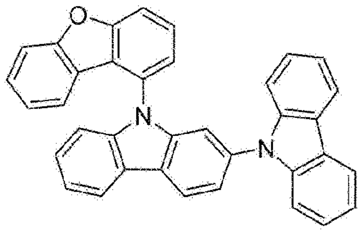

7-22

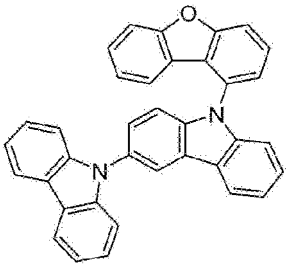

7-23

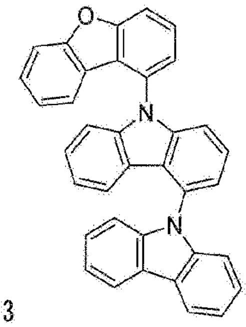

7-24

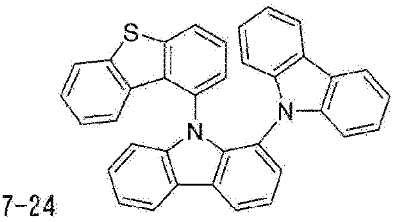

7-25

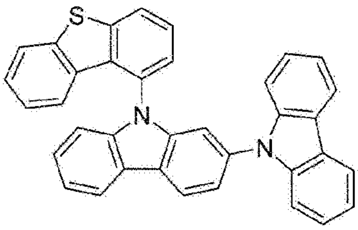

7-26

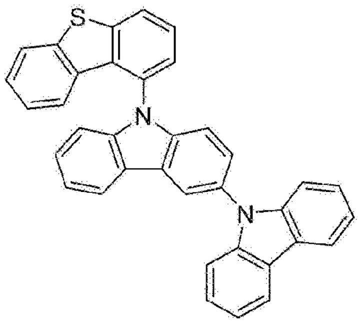

7-27

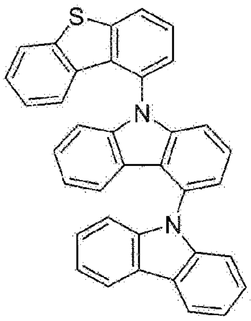

7-28

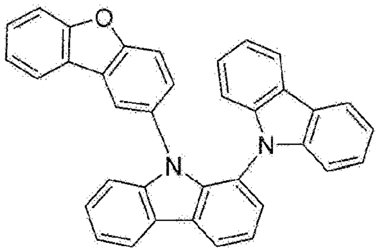

7-29

[C103]

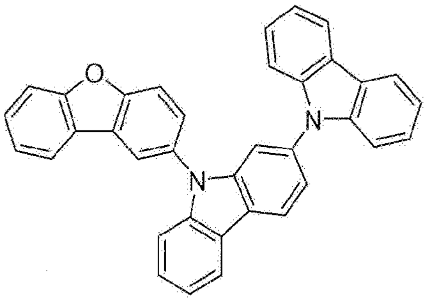

7-30

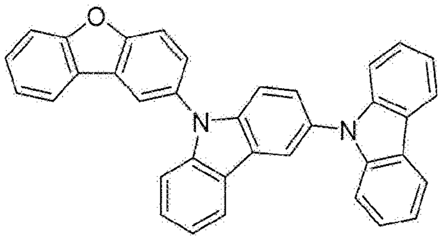

7-31

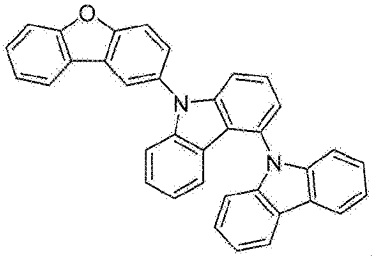

7-32

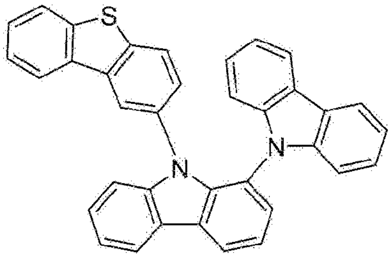

7-33

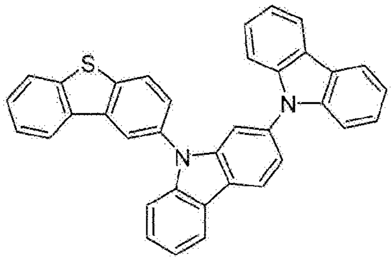

7-34

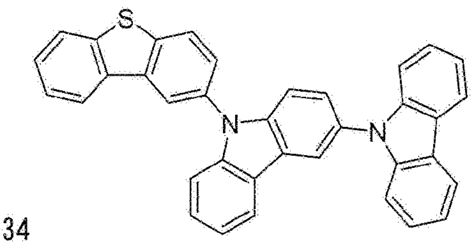

7-35

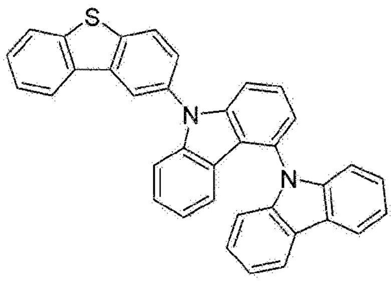

7-36

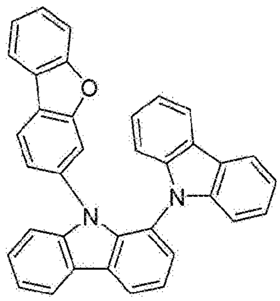

7-37

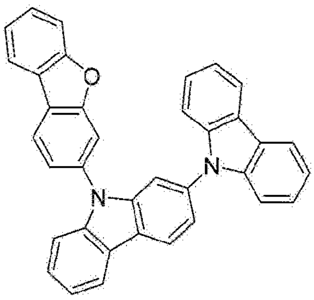

7-38

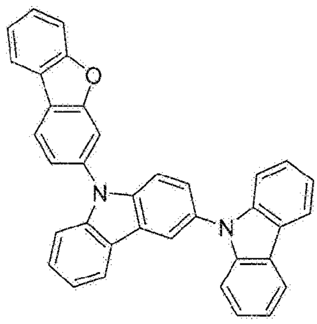

7-39

[C104]

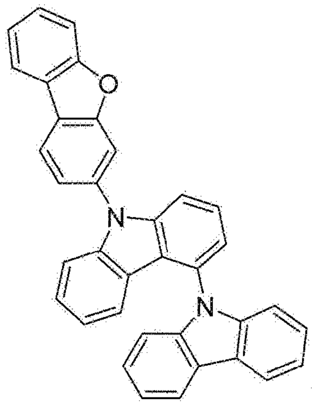

7-40

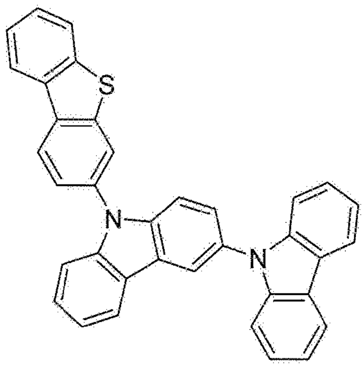

7-41

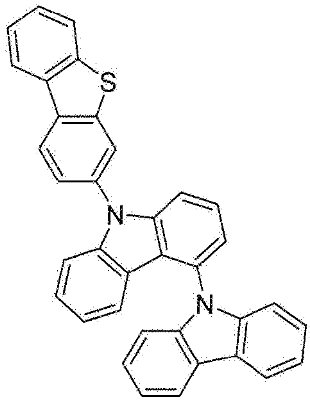

7-42

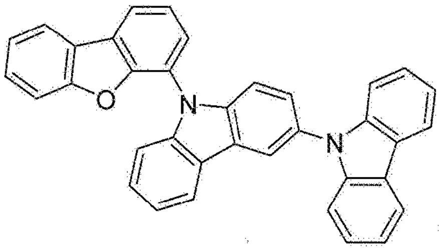

7-43

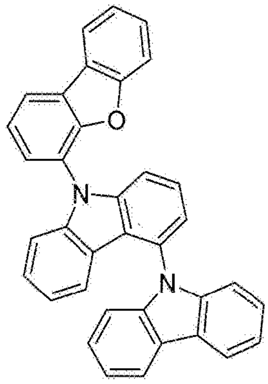

7-44

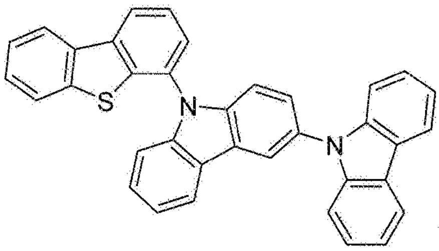

7-45

[C105]

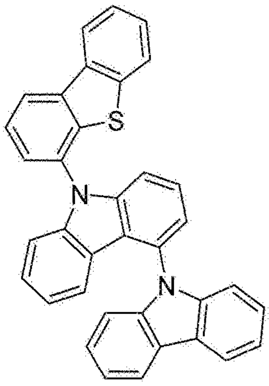

7-46

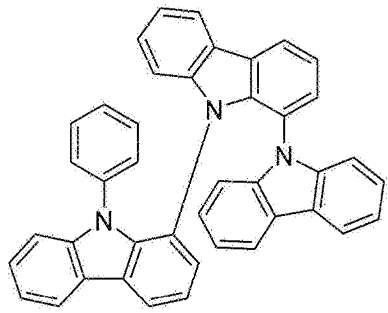

7-47

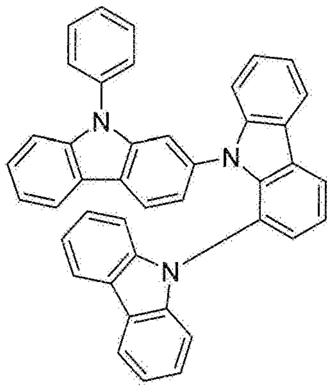

7-48

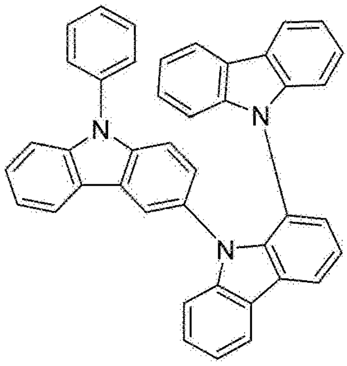

7-49

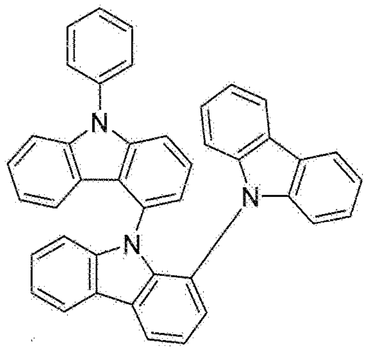

7-50

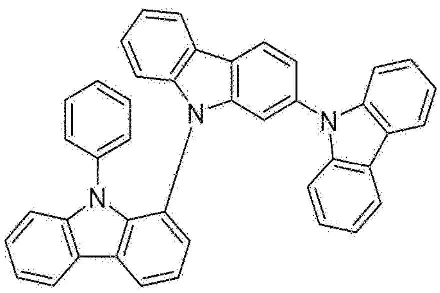

7-51

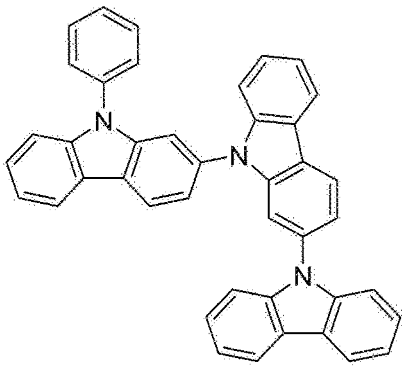

7-52

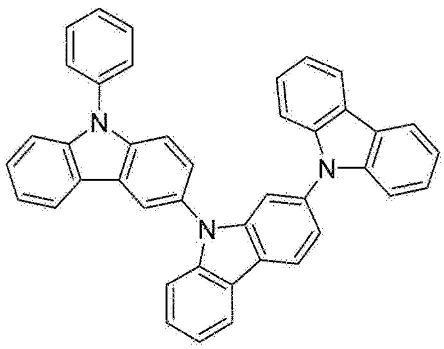

7-53

[C106]

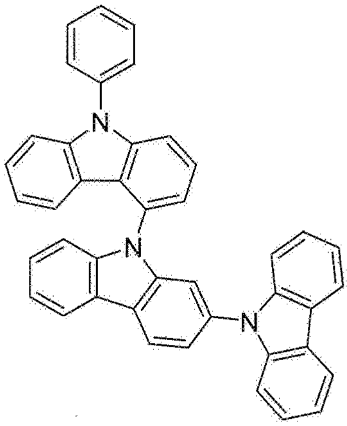

7-54

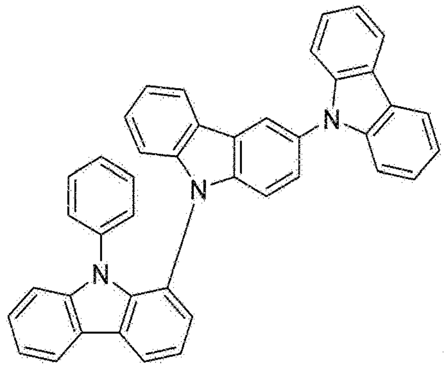

7-55

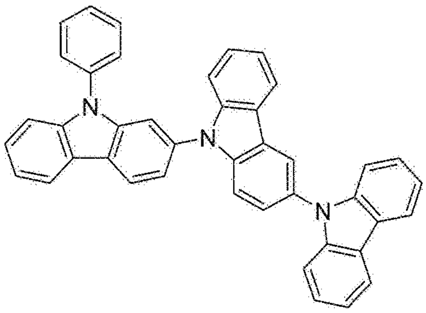

7-56

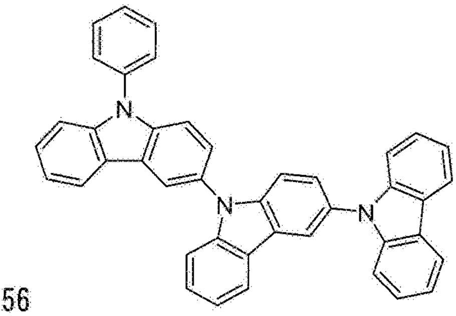

7-57

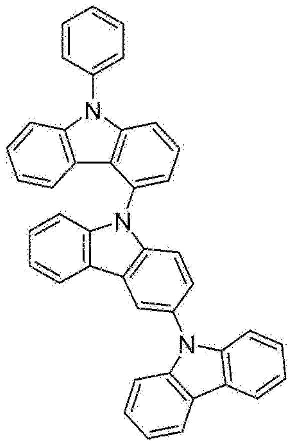

7-58

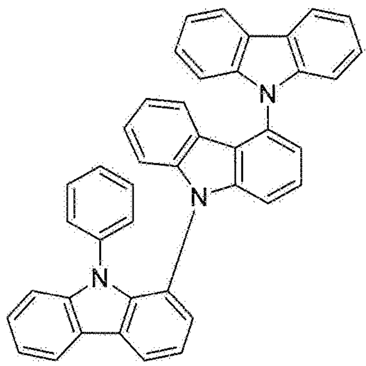

7-59

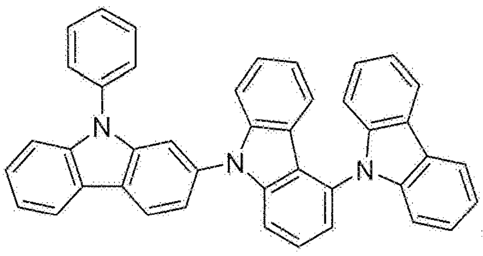

7-60

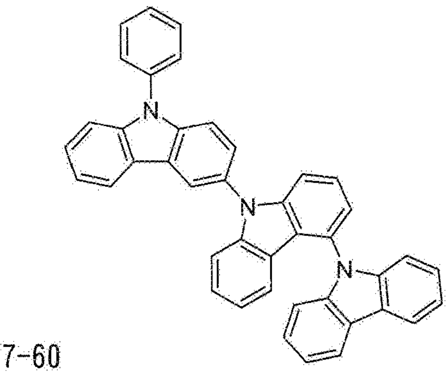

7-61

[C107]

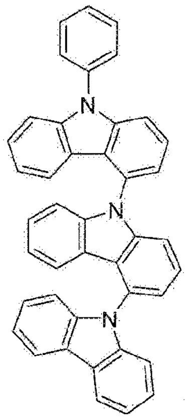
7-62

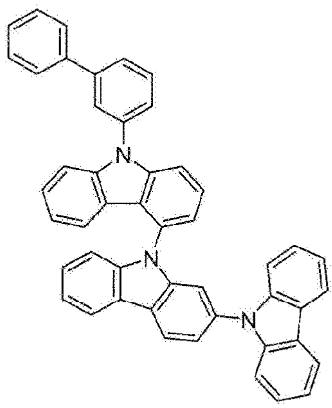
7-63

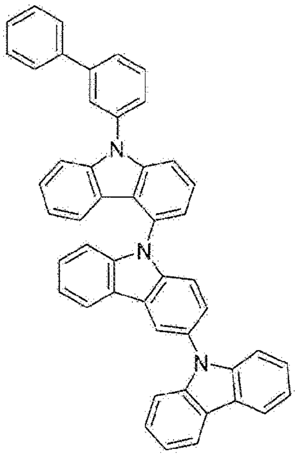
7-64

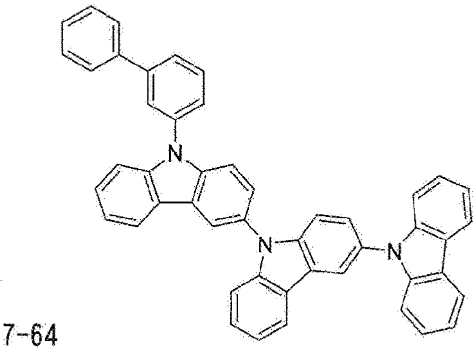
7-65

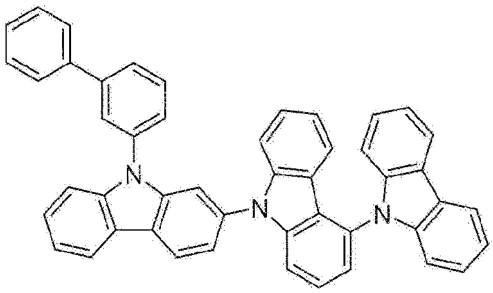
7-66

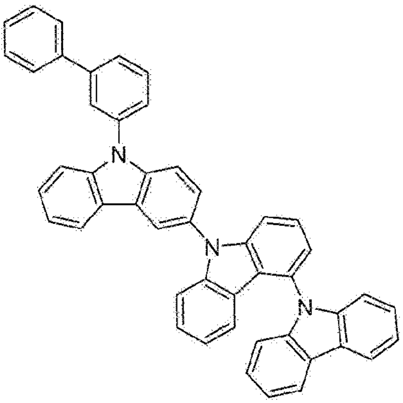
7-67

[C108]

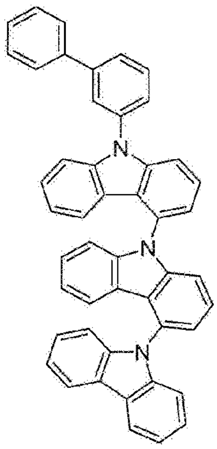
7-68

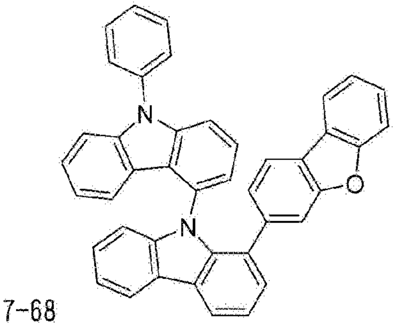
7-69

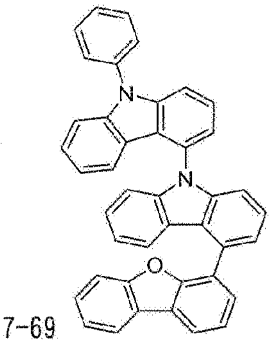
7-70

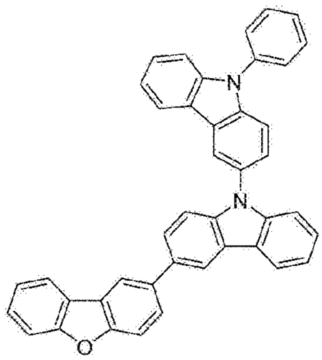
7-71

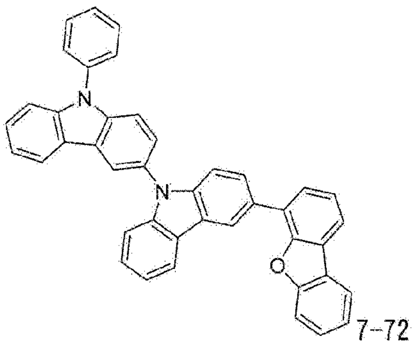
7-72

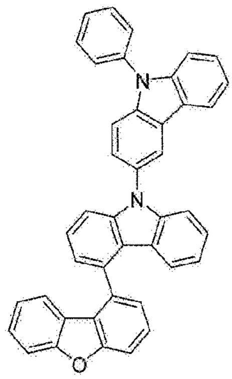
7-73

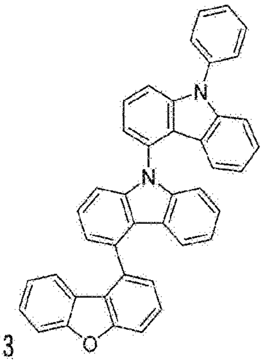
7-74

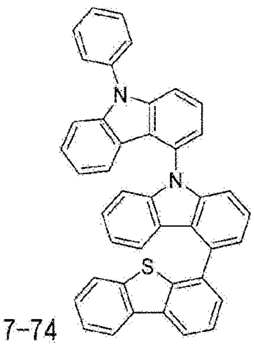
7-75

[C109]

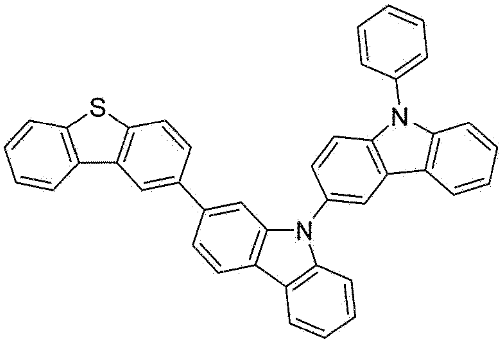

7-76

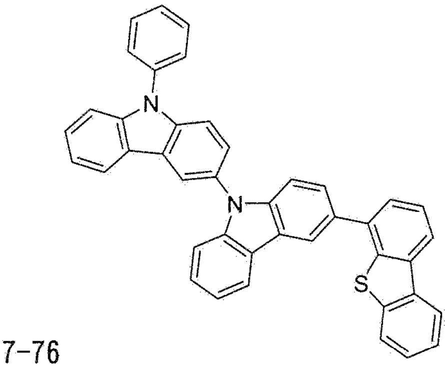

7-77

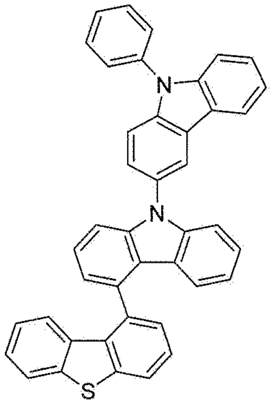

7-78

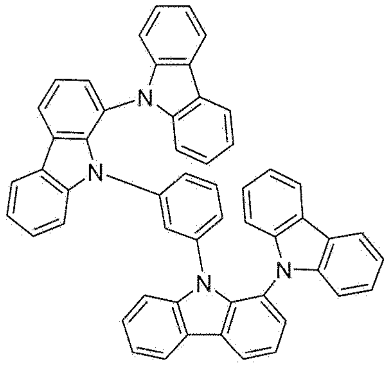

7-79

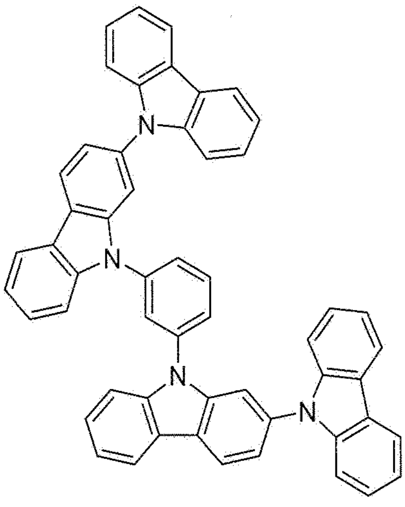

7-80

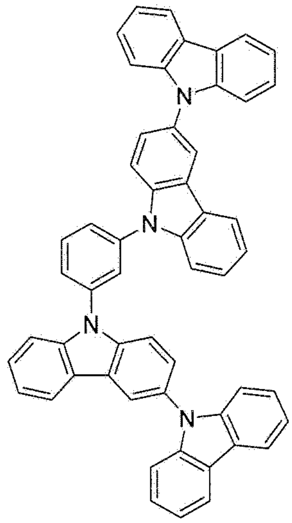

7-81

[C110]

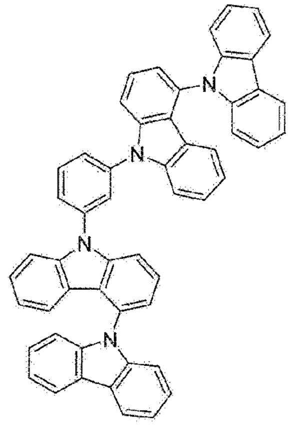

7-82

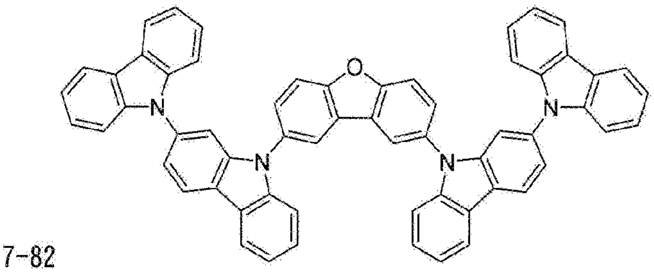

7-83

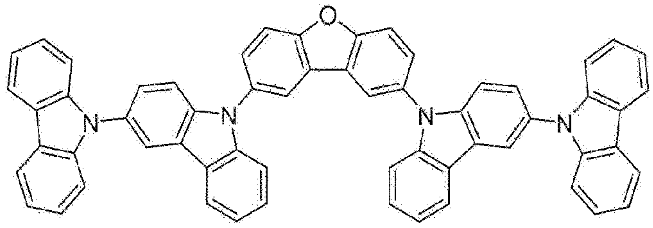

7-84

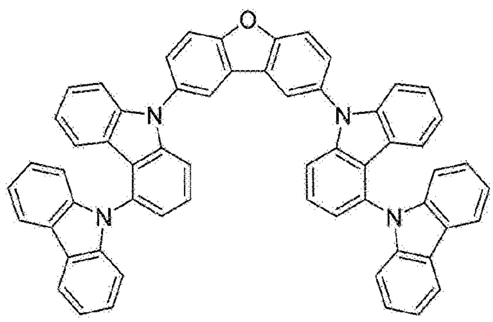

7-85

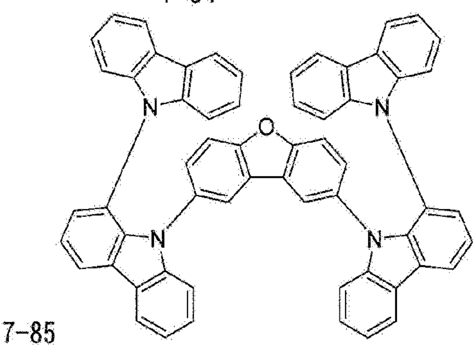

7-86

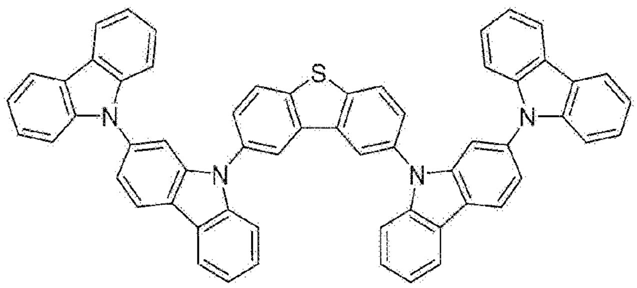

7-87

[C111]

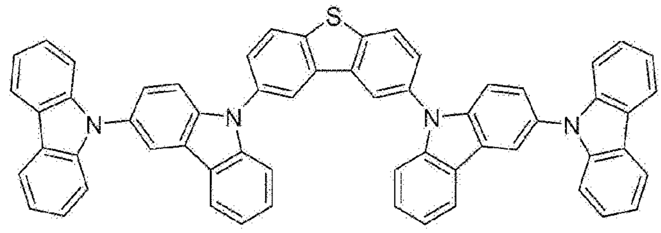

7-88

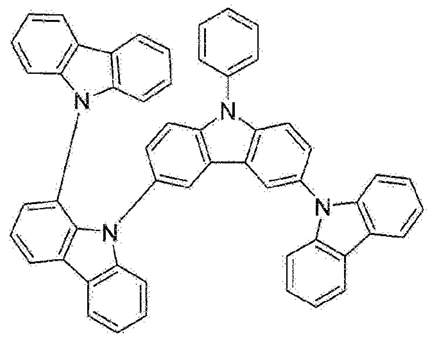

7-89

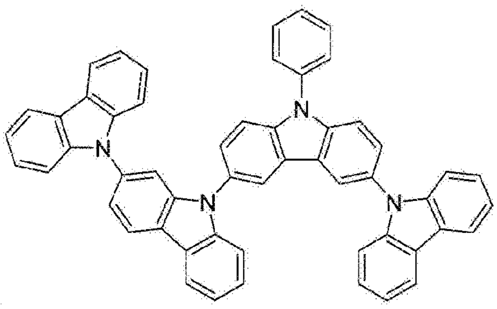

7-90

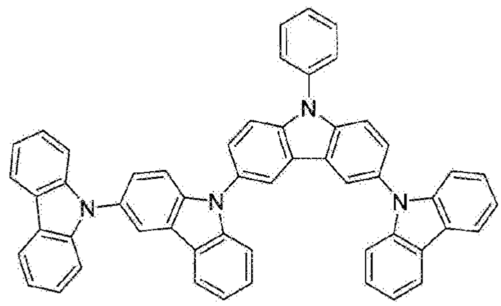

7-91

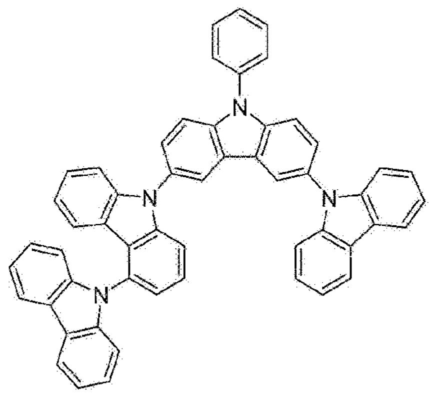

7-92

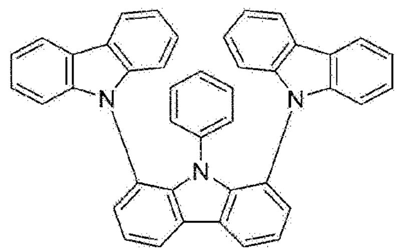

7-93

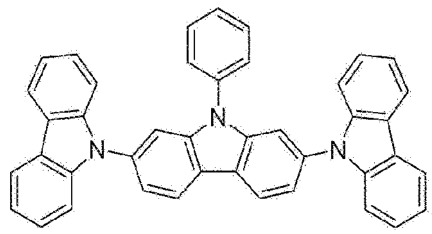

7-94

[C112]

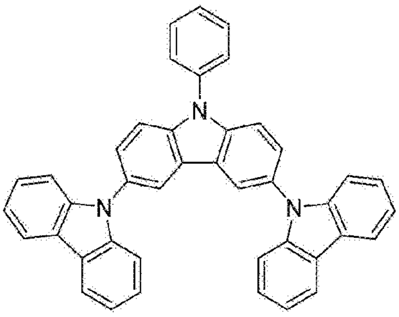

7-95

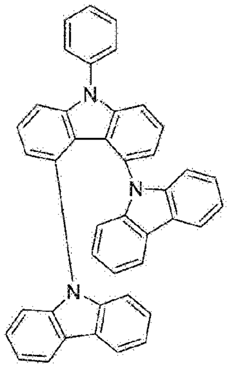

7-96

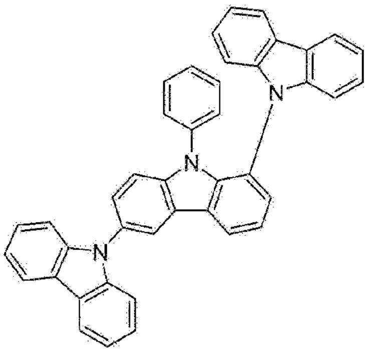

7-97

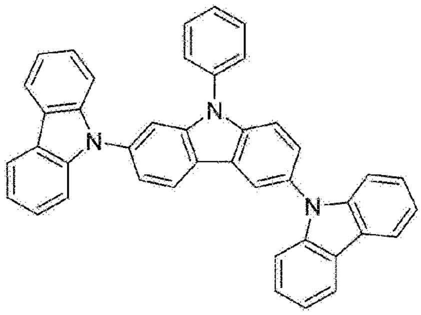

7-98

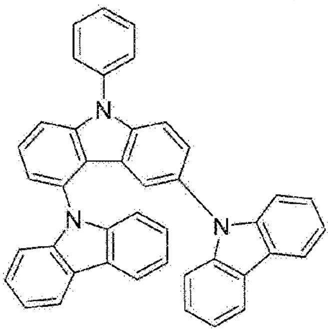

7-99

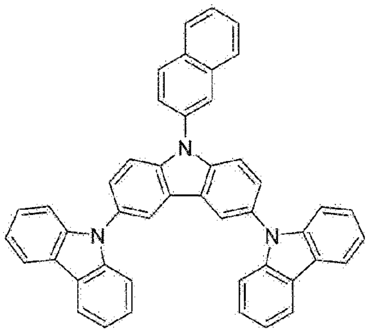

7-100

[C113]

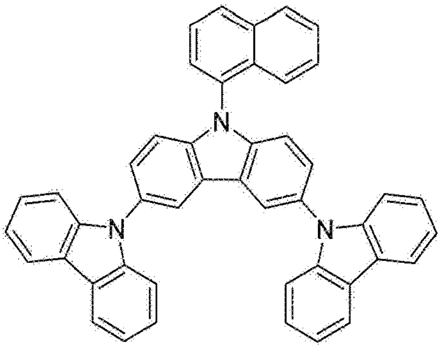
7-101

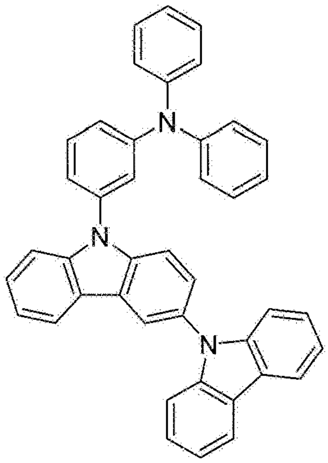
7-102

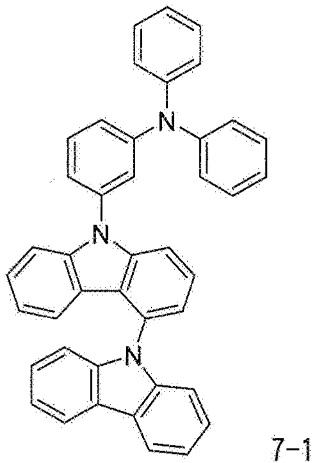
7-103

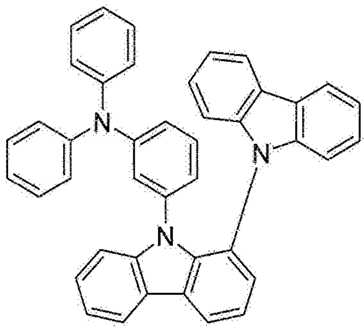
7-104

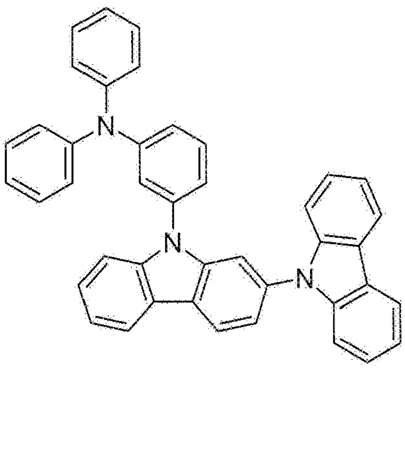
7-105

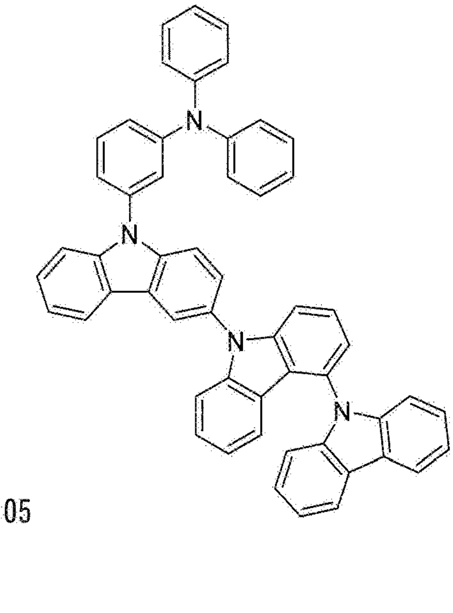
7-106

[C114]

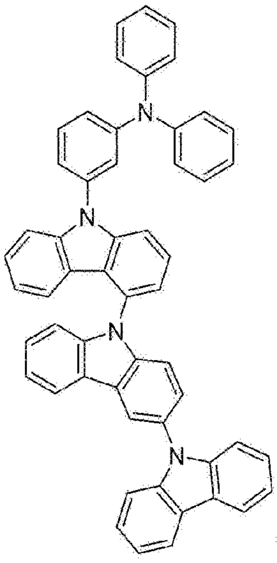

7-107

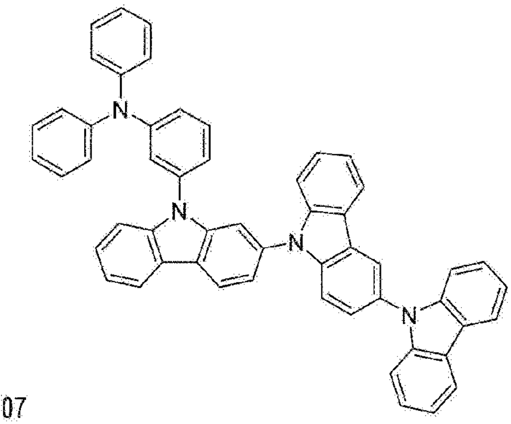

7-108

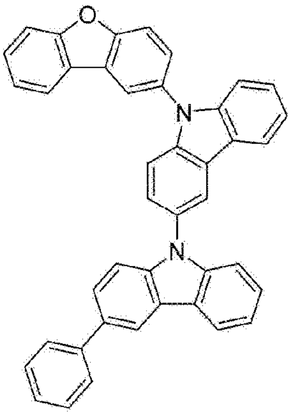

7-109

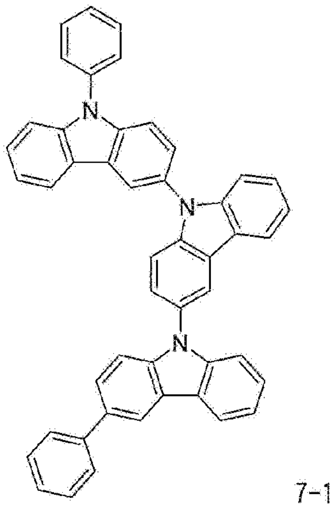

7-110

[C115]

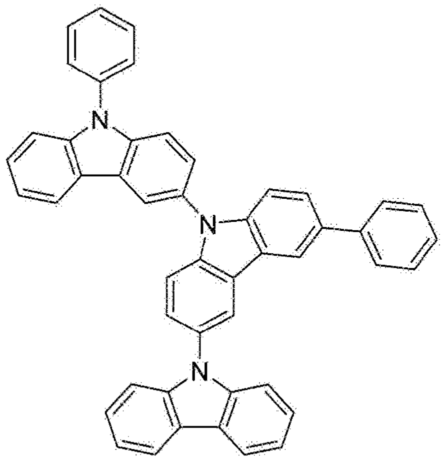

7-111

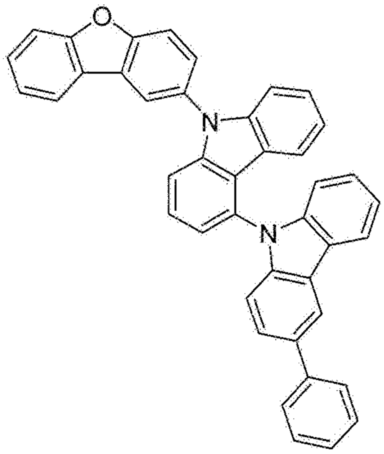

7-112

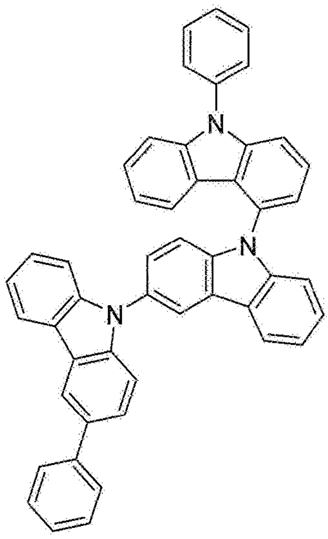

7-113

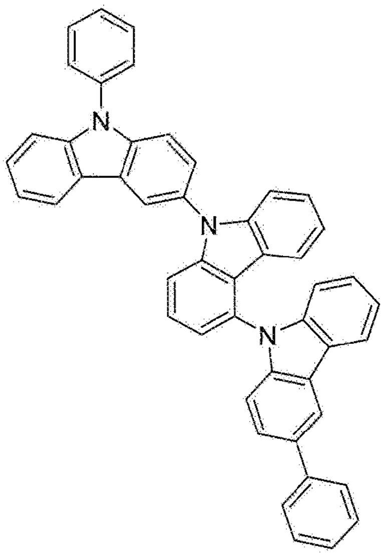

7-114

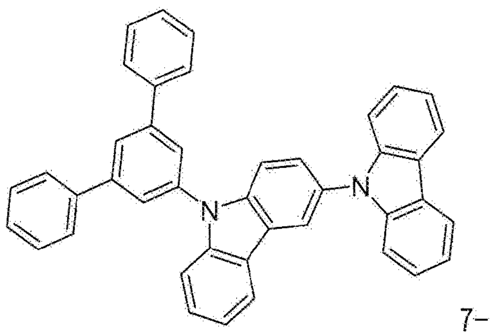

7-115

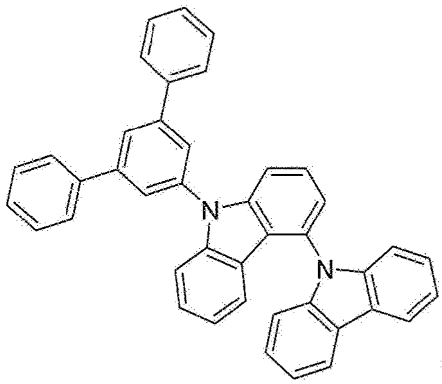

7-116

[C116]

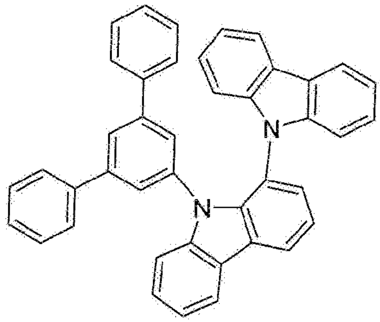

7-117

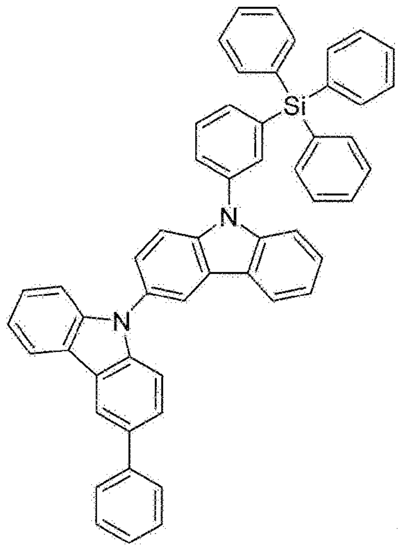

7-118

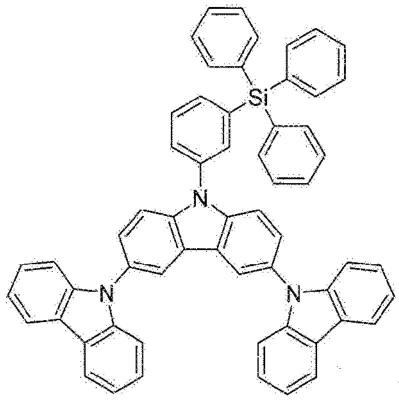

7-119

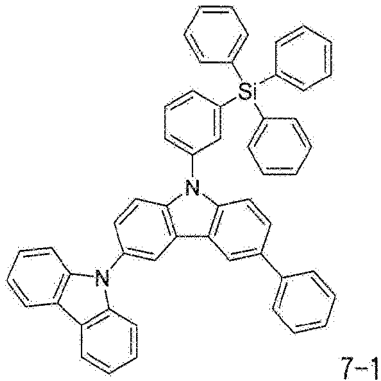

7-120

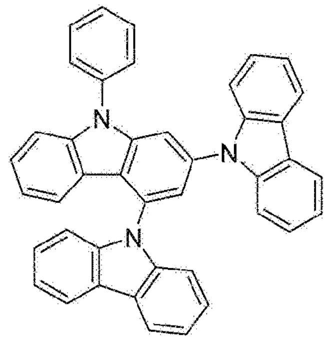

7-121

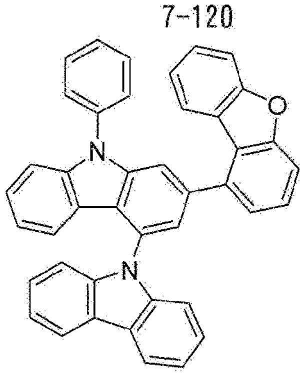

7-122

[C117]

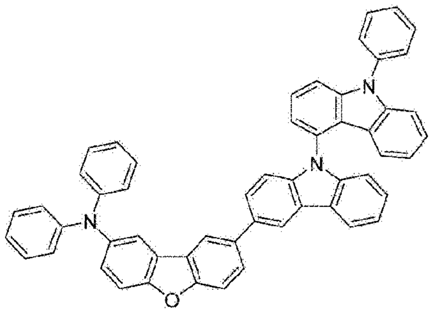
7-123

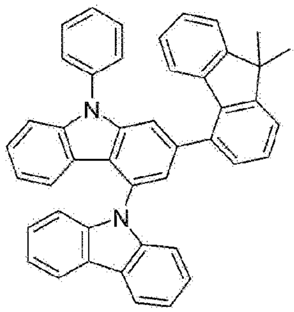
7-124

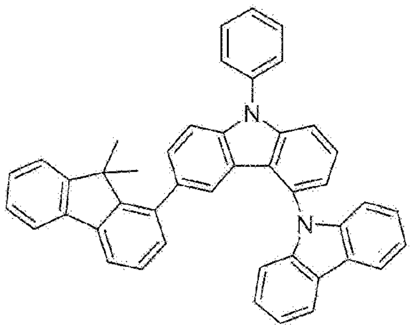
7-125

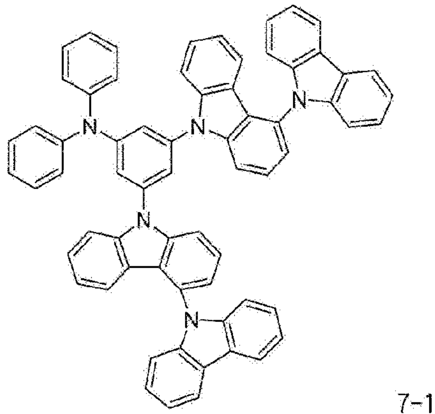
7-126

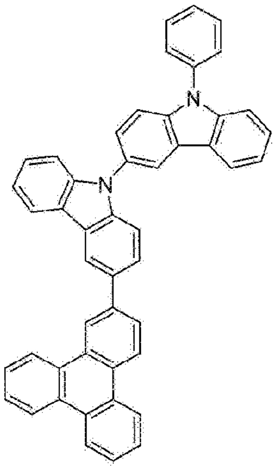
7-125 -b

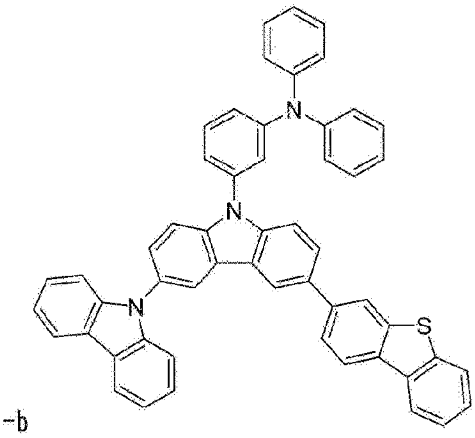
7-126-c

[C118]

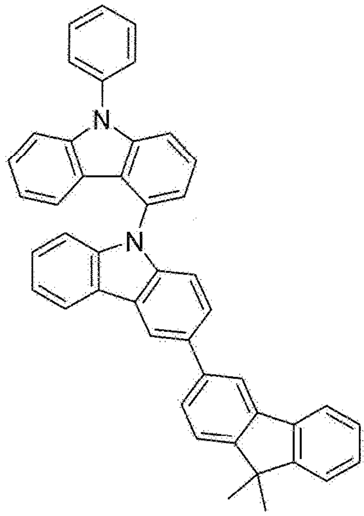

7-127

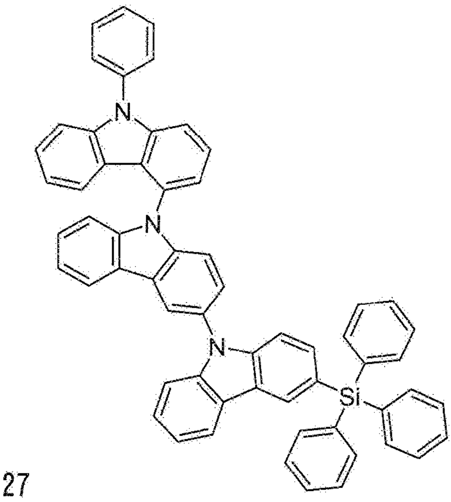

7-128

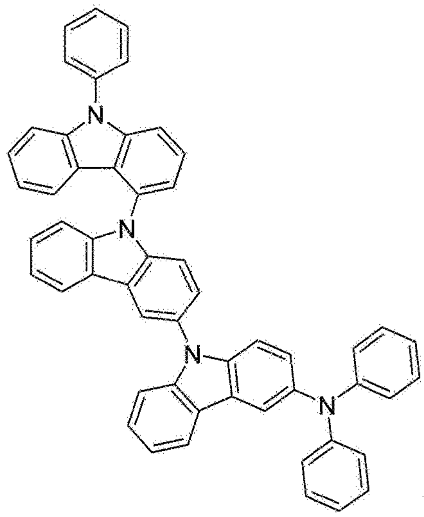

7-129

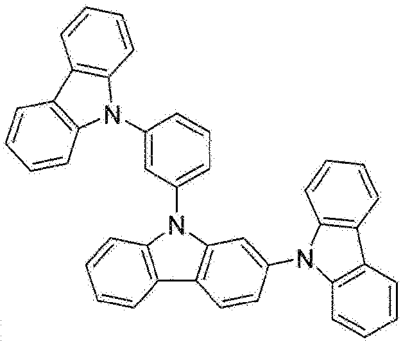

7-130

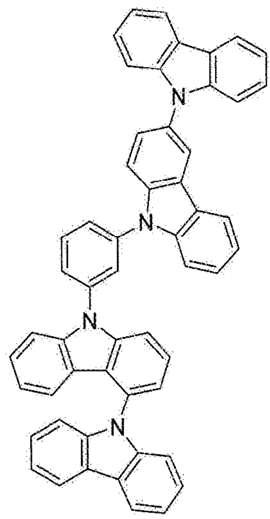

7-131

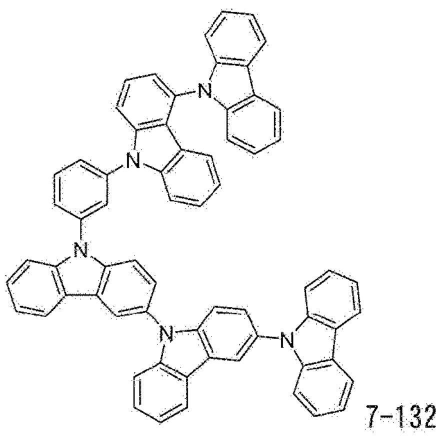

7-132

[C119]

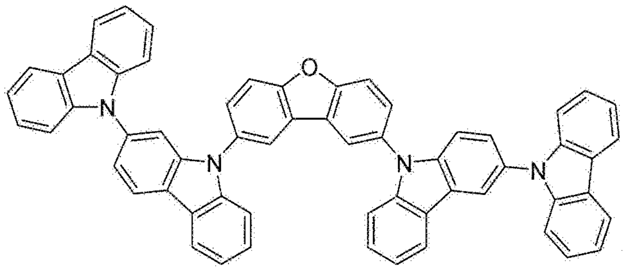

7-133

The polycyclic aromatic compounds represented by the general formulae (7a), (7b), (8a), and (8b) above used as light emitting dopants in the organic EL device of the present invention preferably have a ΔEST of 0.20 eV or less. They more preferably have a ΔEST of 0.15 eV or less and still more preferably 0.10 eV or less.

The ΔEST represents a difference between excited singlet energy (S1) and excited triplet energy (T1). Here, S1 and T1 are measured under the conditions in the method described in the Examples.

By using a material selected from the polycyclic aromatic compounds represented by the general formulae (7a), (7b), (8a), and (8b) above (hereinafter also referred to as a polycyclic aromatic compound material) or a phosphorescent dopant as a light emitting dopant, and using a material selected from the compounds represented by the general formula (1) above as the first host and a material selected from the compounds represented by the general formulae (9), (9a), (9b), (12), or (13) above as the second host, an excellent organic EL device can be provided.

Next, a structure of the organic EL device of the present invention will be described with reference to drawings, but the structure of the organic EL device of the present invention is not limited thereto.

Figure 1 shows a cross-sectional view of a structure example of a typical organic EL device used in the present invention. Reference numeral 1 denotes a substrate, reference numeral 2 denotes an anode, reference numeral 3 denotes a hole injection layer, reference numeral 4 denotes a hole transport layer, reference numeral 5 denotes a light emitting layer, reference numeral 6 denotes an electron transport layer, and reference numeral 7 denotes a cathode. The organic EL device of the present invention may have an exciton blocking layer adjacent to the light emitting layer, or may have an electron blocking layer between the light emitting layer and the hole injection layer. The exciton blocking layer may be inserted on either the anode side or the cathode side of the light emitting layer or may be inserted on both sides at the same time. The organic EL device of the present invention has the anode, the light emitting layer, and the cathode as essential layers, but preferably has a hole injection/transport layer and an electron injection/transport layer in addition to the essential layers, and further preferably has a hole blocking layer between the light emitting layer and the electron injection/transport layer. The hole injection/transport layer means either or both of the hole injection layer and the hole transport layer, and the electron injection/transport layer means either or both of the electron injection layer and electron transport layer.

It is also possible to have a structure that is the reverse of the structure shown in Figure 1, that is, the cathode 7, the electron transport layer 6, the light emitting layer 5, the hole transport layer 4, the hole injection layer 3, and the anode 2 can be laminated on the substrate 1, in the order presented. Also, in this case, layers can be added or omitted, as necessary.

-Substrate-

The organic EL device of the present invention is preferably supported on a substrate. The substrate is not particularly limited and may be a substrate conventionally used for organic EL devices, and for example, a substrate made of glass, transparent plastic, or quartz can be used.

-Anode-

As the anode material in the organic EL device, a material made of a metal, alloy, or conductive compound having a high work function (4 eV or more), or a mixture thereof is preferably used. Specific examples of such an electrode material include metals such as Au, and conductive transparent materials such as CuI, indium tin oxide (ITO), $SnO_2$, and ZnO. An amorphous material capable of producing a transparent conductive film such as IDIXO ($In_2O_3$-ZnO) may also be used. As the anode, these electrode materials may be formed into a thin film by a method such as vapor deposition or sputtering, and then a pattern of a desired form may be formed by photolithography. Alternatively, when a highly precise pattern is not required (about 100 μm or more), a pattern may be formed through a mask of a desired form at the time of vapor deposition or sputtering of the above electrode materials. Alternatively, when a coatable material such as an organic conductive

compound is used, a wet film forming method such as a printing method and a coating method can also be used. When light is extracted from the anode, the transmittance is desirably more than 10%, and the sheet resistance as the anode is preferably several hundred Ω/square or less. The film thickness is selected within a range of usually 10 to 1,000 nm, and preferably 10 to 200 nm, although it depends on the material.

-Cathode-

On the other hand, a material made of a metal (referred to as an electron injection metal), alloy, or conductive compound having a low work function (4 eV or less) or a mixture thereof is used as the cathode material. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide ($Al_2O_3$) mixture, indium, a lithium/aluminum mixture, and a rare earth metal. Among them, in terms of electron injection properties and durability against oxidation and the like, a mixture of an electron injection metal with a second metal that has a higher work function value than the electron injection metal and is stable, for example, a magnesium/silver mixture, a magnesium/aluminum mixture, a magnesium/indium mixture, an aluminum/aluminum oxide ($Al_2O_3$) mixture, a lithium/aluminum mixture, or aluminum is suitable. The cathode can be produced by forming a thin film from these cathode materials by a method such as vapor deposition and sputtering. The sheet resistance as the cathode is preferably several hundred Ω/square or less, and the film thickness is selected within a range of usually 10 nm to 5 μm, and preferably 50 to 200 nm. To transmit the light emitted, either one of the anode and the cathode of the organic EL device is favorably transparent or translucent because light emission brightness is improved.

The above metal is formed to have a film thickness of 1 to 20 nm on the cathode, and then a conductive transparent material mentioned in the description of the anode is formed on the metal, so that a transparent or translucent cathode can be produced. By applying this process, a device in which both anode and cathode have transmittance can be produced.

-Light Emitting Layer-

The light emitting layer is a layer that emits light after holes and electrons respectively injected from the anode and the cathode are recombined to form excitons, and the light emitting layer includes the light emitting dopant and the hosts.

The light emitting dopant and the host can be used so that the amount of the light emitting dopant is, for example, 0.10 to 10% and the amount of the host is 99.9% to 90%. Preferably the amount of the light emitting dopant is 1.0 to 5.0% and the amount of the host is 99 to 95%. More preferably the amount of the light emitting dopant is 1.0 to 3.0% and the amount of the host is 99 to 97%.

In the present description, % denotes % by mass unless otherwise noted.

As the host in the light emitting layer, the compound of the present invention represented by the general formula (1) above can be used. When the first host and the second host described above are used, the compound represented by the general formula (1) above can be used as the first host, and the compound represented by the general formula (9), (12) or (13) above can be suitably used as the second host. For the first host and the second host, for example, the first host can be used in an amount of 10 to 90% and the second host can be used in an amount of 90 to 10%. Preferably, the amount of the first host is 30 to 70% and the amount of the second host is 70 to 30%. More preferably, the amount of the first host is 30 to 50% and the amount of the second host is 70 to 50%.

Furthermore, one or more known hosts as other hosts may be used in combination with those described above, and the amount thereof used may be 50% or less and preferably 25% or less based on the total amount of the host materials.

The other known host that can be used is a compound having the ability to transport hole, the ability to transport electron, and a high glass transition temperature, and preferably has a higher T1 than the T1 of the light emitting dopant. Specifically, the host has a higher T1 than the T1 of the light emitting dopant preferably by 0.010 eV or more, more preferably by 0.030 eV or more, and still more preferably by 0.10 eV or more. A TADF-active compound may also be used as the host material, and this compound preferably has a ΔEST of 0.20 eV or less.

The other hosts are known in a large number of patent literatures and the like, and hence may be selected from them. Specific examples of the host include, but are not particularly limited to, various metal complexes typified by metal complexes of indole derivatives, carbazole derivatives, indolocarbazole derivatives, triazole derivatives, oxazole derivatives, oxadiazole derivatives, imidazole derivatives, phenylenediamine derivatives, arylamine derivatives, styrylanthracene derivatives, fluorenone derivatives, stilbene derivatives, triphenylene derivatives, carborane derivatives, porphyrin derivatives, phthalocyanine derivatives, and 8-quinolinol derivatives, and metal phthalocyanine, and metal complexes of benzoxazole and benzothiazole derivatives; and polymer compounds such as poly(N-vinyl carbazole) derivatives, aniline-based copolymers, thiophene oligomers, polythiophene derivatives, polyphenylene derivatives, polyphenylene vinylene derivatives, and polyfluorene derivatives.

When a plurality of hosts is used, each host is deposited from different deposition sources, or a plurality of hosts is premixed before vapor deposition to form a premix, whereby a plurality of hosts can be simultaneously deposited from one deposition source.

As the method of premixing, a method by which hosts can be mixed as uniformly as possible is desirable, and examples thereof include, but are not limited to, milling, a method of heating and melting hosts under reduced pressure or under an inert gas atmosphere such as nitrogen, and sublimation.

The premixed mixture may be in the form of powder, a stick, or a granule.

When the compound of the present invention is used as a host, the energy level of the highest occupied molecular orbital (HOMO) obtained by structure optimization calculation using density functional calculation B3LYP/6-31G(D) is preferably -4.7 eV or less and more preferably in the range of -5.5 eV to -4.7 eV.

Also the energy level of the lowest unoccupied molecular orbital (LUMO) obtained by the above structure optimization calculation is preferably -2.5 eV or more and more preferably in the range of -2.5 eV to -1.5 eV.

When the compound of the present invention is used as a host, the difference (absolute value) between the HOMO energy level and the LUMO energy level is preferably in the range of 2.2 to 3.5 eV and more preferably in the range of 2.5 to 3.3 eV.

As the light emitting dopant in the light emitting layer, the polycyclic aromatic compound material represented by the general formula (1) above, or the general formula (7a) or (7b) above, specifically the general formula (8a) or (8b) above, can be used, and in addition thereto, a phosphorescent dopant can also be used.

The phosphorescent dopant material is not particularly limited, but specific examples thereof include the following materials:

[C120]

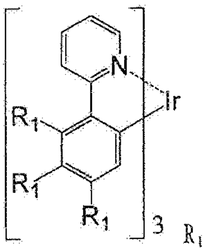

$R_1$:H, $CH_3$, $CF_3$, F

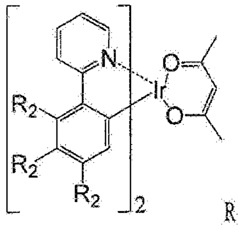

$R_2$:H, F

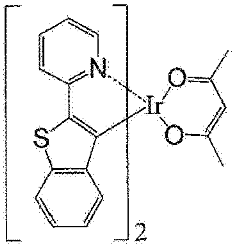
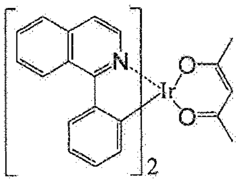
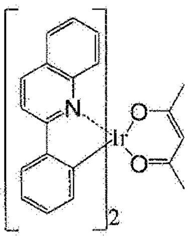
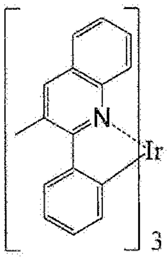
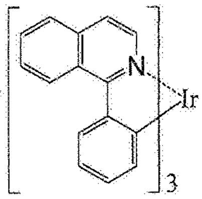

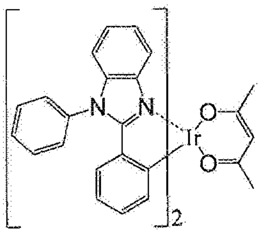
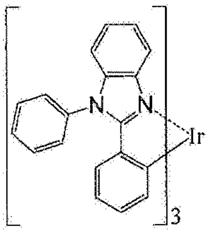
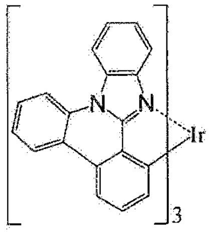
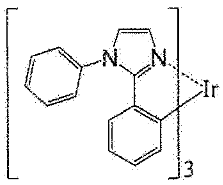

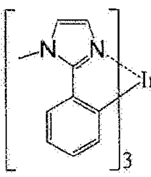
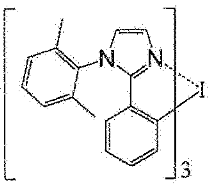
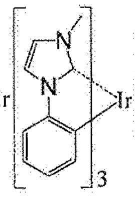
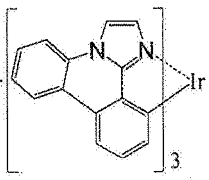

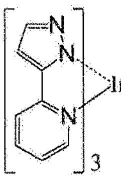
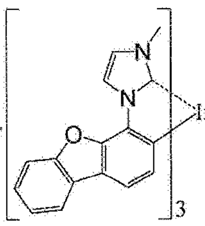
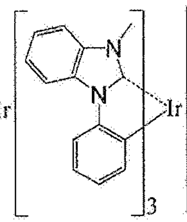
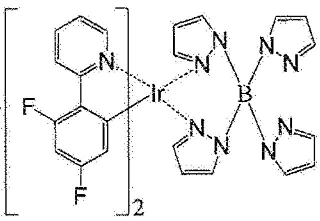

[C121]

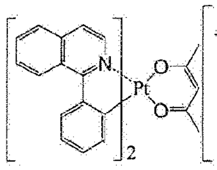
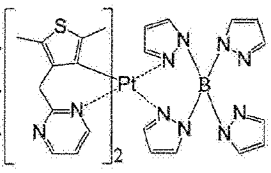

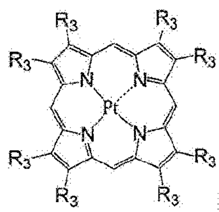

$R_3$: $CH_3$, $CH_2CH_3$

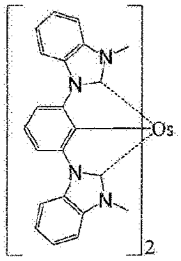

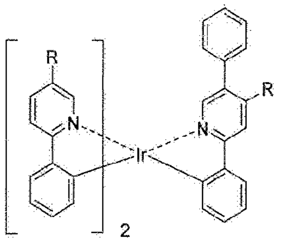

R: H, $CH_3$, $CD_3$

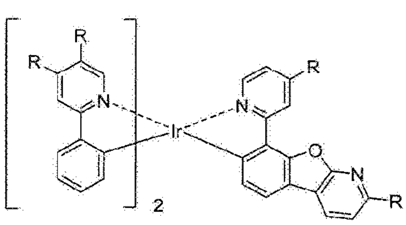

R: H, $CH_3$, $CD_3$

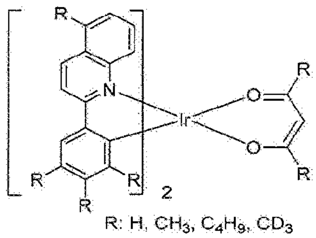

R: H, $CH_3$, $C_4H_9$, $CD_3$

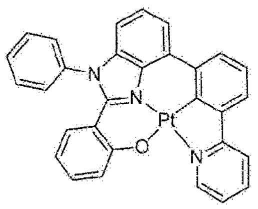
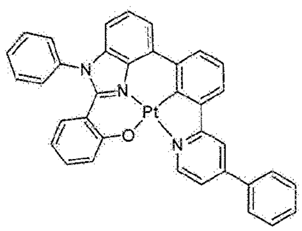
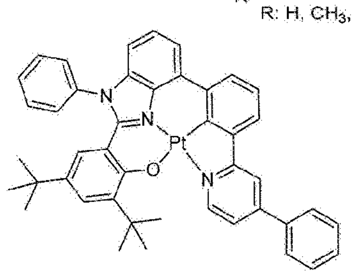

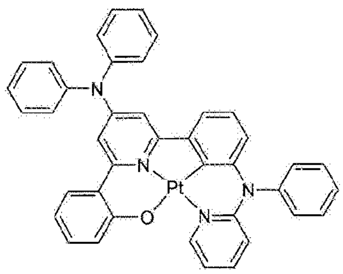
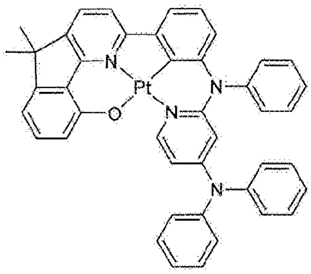
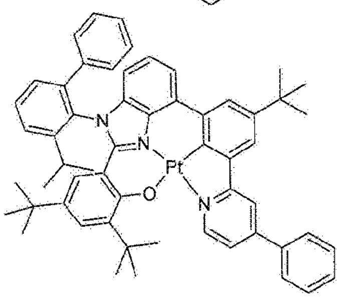

[C122]

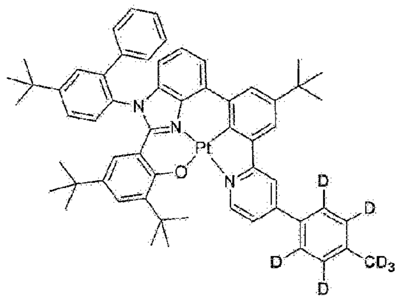
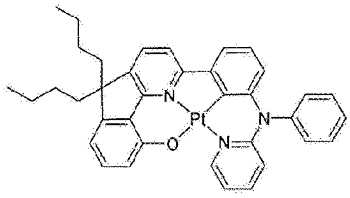
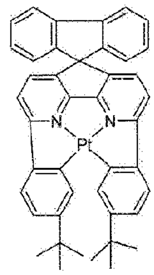
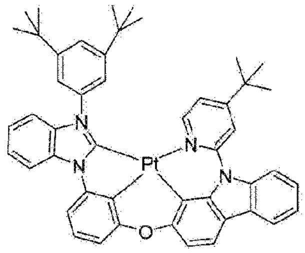
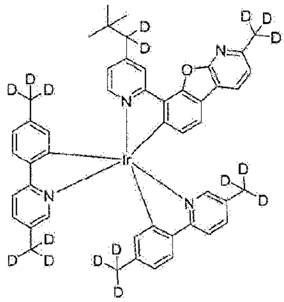
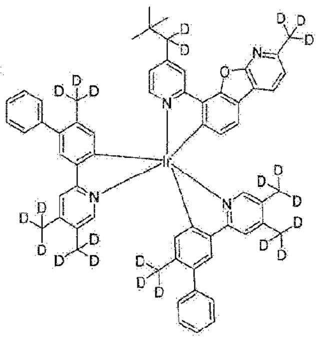
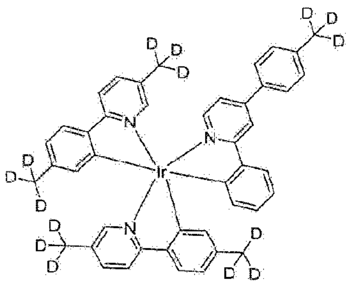
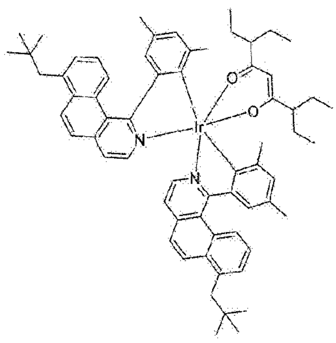
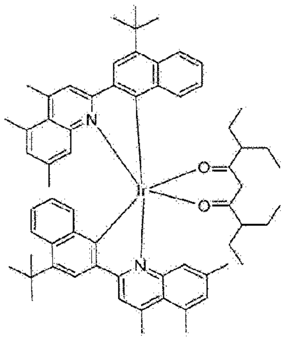
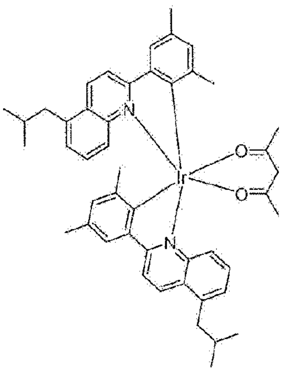

[C123]

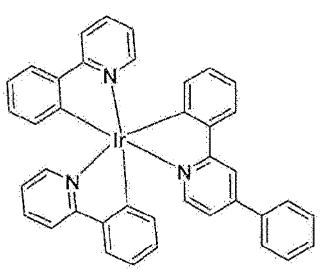
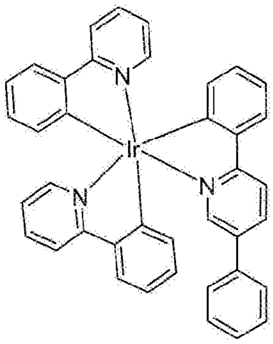
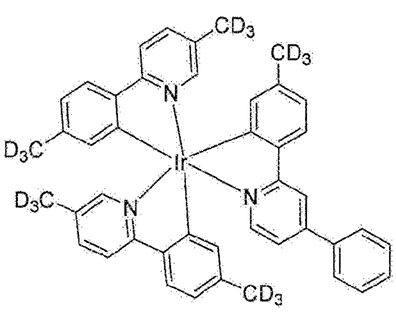

The light emitting layer can contain two or more light emitting dopants. For example, the compound represented by the above-described general formula (1), or the polycyclic aromatic compound material represented by the general formula (7a) or (7b), more specifically the general formula (8a) or (8b) above, can be used in combination of two or more thereof, or these polycyclic aromatic compound materials can be combined for use with a phosphorescent dopant, or further, a light emitting dopant including other compounds can be combined for use to allow the light emitting layer to include two or more light emitting dopants. In a case in which the light emitting layer includes the compound represented by the above-described general formula (1), or the polycyclic aromatic compound material represented by the general formula (7a) or (7b), more specifically the general formula (8a) or (8b) above, it is preferred that the light emitting layer includes the compound represented by the above-described general formula (1) as a host material, and includes the polycyclic aromatic compound material represented by the general formula (7a) or (7b) above, more specifically the general formula (8a) or (8b) above as a light emitting dopant.

When containing two or more light emitting dopants in the light emitting layer, the first dopant is the compound represented by the general formulae (7a), (7b), (8a), and (8b) above or a fluorescent dopant, and for the second dopant, a known compound may be combined for use as the light emitting dopant. The content of the first dopant is preferably 0.050 to 50% relative to the host materials, and the content of the second dopant is preferably 0.050 to 50% relative to the host materials. The total content of the first dopant and the second dopant do not exceed 50% relative to the host materials.

Such other light emitting dopants are known in a large number of patent literatures and the like, and may be selected therefrom. Specific examples of the dopants include, but not limited to, fused ring derivatives such as phenanthrene, anthracene, pyrene, tetracene, pentacene, perylene, naphthopyren, dibenzopyren, rubrene, and chrysene, benzoxazole derivatives, benzothiazole derivatives, benzoimidazole derivatives, benzotriazole derivatives, oxazole derivatives, oxadiazole derivatives, thiazole derivatives, imidazole derivatives, thiadiazole derivatives, triazole derivatives, pyrazoline derivatives, stilbene derivatives, thiophene derivatives, tetraphenylbutadiene derivatives, cyclopentadiene derivatives, bisstyryl derivatives such as bisstyrylanthracene derivatives and distyrylbenzene derivatives, bisstyrylarylene derivatives, diazaindacene derivatives, furan derivatives, benzofuran derivatives, isobenzofuran derivatives, dibenzofuran derivatives, coumarin derivatives, dicyanomethylenopyran derivatives, dicyanomethylenethiopyran derivatives, polymethine derivatives, cyanine derivatives, oxobenzoanthracene derivatives, xanthene derivatives, rhodamine derivatives, fluorescein derivatives, pyrylium derivatives, carbostyryl derivatives, acridine derivatives, oxazine derivatives, phenylene oxide derivatives, quinacridone derivatives, quinazoline derivatives, pyrrolopyridine derivatives, fluoropyridine derivatives, 1,2,5-thiadiazolopyrene derivatives, pyrromethene derivatives, perinone derivatives, pyrrolopyrrole derivatives, squarylium derivatives, violanthrone derivatives, phenazine derivatives, acridone derivatives, deazaflavin derivatives, fluorene derivatives, benzofluorene derivatives, etc.

The light emitting dopant and the first or second host can be vapor deposited from different vapor deposition sources, or the light emitting dopant and the first or second host can be premixed before vapor deposition to form a premix, whereby they can be simultaneously deposited from one deposition source.

-Injection Layer-

The injection layer refers to a layer provided between the electrode and the organic layer to reduce the driving voltage and improve the light emission brightness, and includes the hole injection layer and the electron injection layer. The injection layer may be present between the anode and the light emitting layer or the hole transport layer, as well as between the cathode and the light emitting layer or the electron transport layer. The injection layer may be provided as necessary.

-Hole Blocking Layer-

The hole blocking layer has the function of the electron transport layer in a broad sense, is made of a hole blocking material having a very small ability to transport holes while having the function of transporting electrons, and can improve the recombination probability between the electrons and the holes in the light emitting layer by blocking the holes while transporting the electrons. For the hole blocking layer, a known hole blocking material can be used. In order to exhibit the characteristics of the light emitting dopant, the material used as the first host can also be used as a material of the hole blocking layer, and the compound of the present invention represented by the general formula (1) above can be used. A plurality of hole blocking materials can be used in combination.

-Electron Blocking Layer-

The electron blocking layer has the function of the hole transport layer in a broad sense, and can improve the recombination probability between the electrons and the holes in the light emitting layer by blocking the electrons while transporting the holes. As the material for the electron blocking layer, a known material for the electron blocking layer can be used. To exhibit the characteristics of the light emitting dopant, the material used as the second host can also be used as

the material for the electron blocking layer. The thickness of the electron blocking layer is preferably 3 to 100 nm, and more preferably 5 to 30 nm.

-Exciton Blocking Layer-

The exciton blocking layer is a layer to block the diffusion of the excitons generated by recombination of the holes and the electrons in the light emitting layer into a charge transport layer, and insertion of this layer makes it possible to efficiently keep the excitons in the light emitting layer, so that the emission efficiency of the device can be improved. The exciton blocking layer can be inserted between two light emitting layers adjacent to each other in the device in which two or more light emitting layers are adjacent to each other.
As the material for the exciton blocking layer, a known material for the exciton blocking layer can be used.
The layer adjacent to the light emitting layer includes the hole blocking layer, the electron blocking layer, and the exciton blocking layer, and when these layers are not provided, the adjacent layer is the hole transport layer, the electron transport layer, and the like.

-Hole Transport Layer-

The hole transport layer is made of a hole transport material having the function of transporting holes, and the hole transport layer may be provided as a single layer or a plurality of layers.
The hole transport material has any of hole injection properties, hole transport properties, or electron barrier properties, and may be either an organic material or an inorganic material. As the hole transport layer, any of conventionally known compounds may be selected and used. Examples of such a hole transport material include porphyrin derivatives, triazole derivatives, oxadiazole derivatives, imidazole derivatives, polyarylalkane derivatives, phenylenediamine derivatives, arylamine derivatives, amino-substituted chalcone derivatives, oxazole derivatives, styrylanthracene derivatives, fluorenone derivatives, hydrazone derivatives, stilbene derivatives, silazane derivatives, aniline-based copolymers, and conductive polymer oligomers, particularly, thiophene oligomers. Porphyrin derivatives, arylamine derivatives, and styrylamine derivatives are preferably used, and arylamine derivatives are more preferably used.

-Electron Transport Layer-

The electron transport layer is made of a material having the function of transporting electrons, and the electron transport layer may be provided as a single layer or a plurality of layers.
The electron transport material (may also serve as the hole blocking material) has the function of transmitting electrons injected from the cathode to the light emitting layer. As the electron transport layer, any of conventionally known compounds may be selected and used, and examples thereof include polycyclic aromatic derivatives such as naphthalene, anthracene, and phenanthroline, tris(8-quinolinolato)aluminum (III) derivatives, phosphine oxide derivatives, nitro-substituted fluorene derivatives, diphenylquinone derivatives, thiopyran dioxide derivatives, carbodiimides, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. Further, polymer materials in which these materials are introduced in the polymer chain or these materials constitute the main chain of the polymer can also be used.
When the organic EL device of the present invention is produced, the film formation method of each layer is not particularly limited, and the layers may be produced by either a dry process or a wet process.

## Examples

### Calculation example

### Calculation of HOMO and LUMO values

HOMO and LUMO were calculated for the above compounds 1-1, 1-10, 1-11, 1-51, 1-52, 1-55, 1-105, 1-106, 1-122, 1-124, 1-126, and 1-127. The calculation was performed using the density functional theory (DFT), Gaussian as a calculation program, and structural optimization calculation by density functional calculation B3LYP/6-31G(d). The results are shown in Table 1 below. All of the materials of the present invention can be said to have preferable HOMO and LUMO values.

[Table 1]

| Compound | HOMO(eV) | LUMO(eV) |
|---|---|---|
| 1-1 | -5.2 | -1.9 |
| 1-10 | -5.3 | -2.0 |
| 1-11 | -5.1 | -2.0 |
| 1-51 | -5.2 | -2.0 |
| 1-52 | -5.1 | -2.0 |
| 1-55 | -5.1 | -2.0 |
| 1-105 | -5.2 | -1.9 |

Hereinafter, the present invention will be described in further detail with reference to Examples, but the present invention is not limited to these Examples. First, as representative examples, some synthesis examples of the compounds will be described. The other compounds were also synthesized in a similar manner.

Synthesis Example 1 (synthesis of compound 1-51)

[C124]

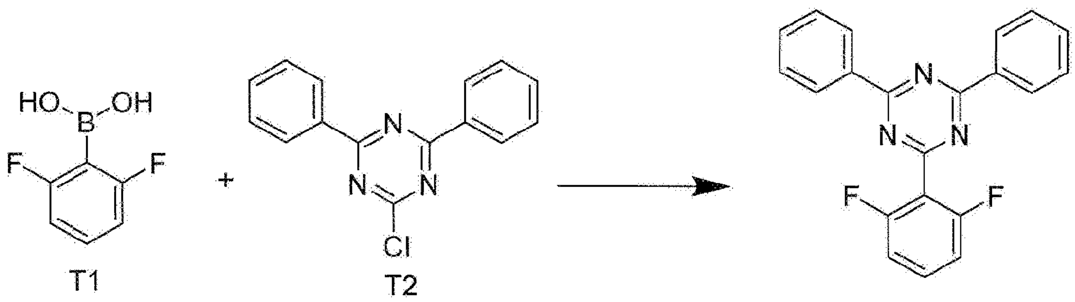

T1 T2 T3

A 2000 ml three-neck flask degassed and replaced with nitrogen was charged with T1 (56.2 mmol), T2 (37.4 mmol), tetrakis(triphenylphosphine)palladium(0) (7.5 mmol), and potassium carbonate (1.12 mol), and added with 900 ml of toluene, 200 ml of ethanol, and 200 ml of water, followed by stirring at 100°C for 4 hours. The mixture therein was cooled to room temperature, then added with 500 ml of water, and transferred to a separatory funnel and separated into an organic layer and a water layer. The organic layer was washed three times with 500 ml of water, and then the resulting organic layer was concentrated under reduced pressure. The resulting residue was purified by column chromatography, yielding a compound T3 (white solid). The yield was 21%.

[C125]

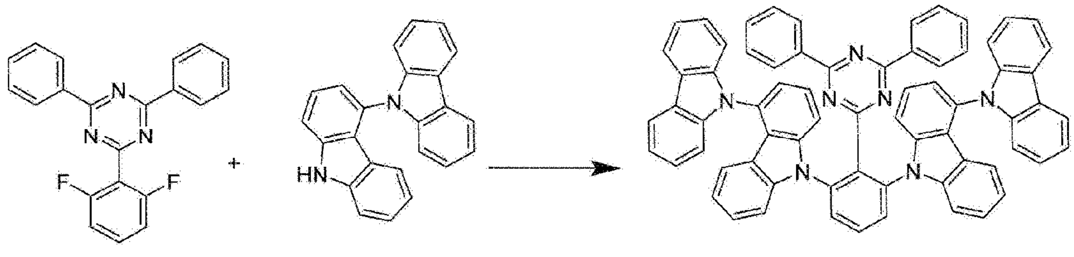

T3 T4 1-51

A 300 ml three-neck flask degassed and replaced with nitrogen was charged with T3 (27.2 mmol), T4 (59.9 mmol), and tripotassium phosphate (100.9 mmol), and added with 70 ml of 1,3-dimethyl-2-imidazolidinone (DMI), followed

by stirring at 190°C for 16 hours. The mixture was cooled to room temperature, then added with 200 ml of water, and the precipitated solid was filtered off. The resulting solid was purified by column chromatography, yielding a compound 1-51 (light yellow solid). The yield was 67%. APCI-TOFMS m/z 970 $[M+1]^+$

Synthesis Example 2 (synthesis of compound 1-68)

[C126]

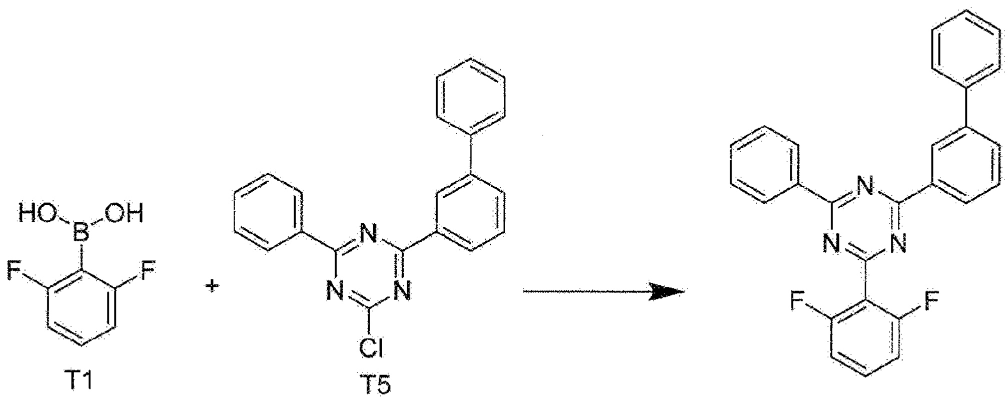

T1 T5 T6

A 1000 ml three-neck flask degassed and replaced with nitrogen was charged with T1 (28.1 mmol), T5 (16.7 mmol), tetrakis(triphenylphosphine)palladium(0) (3.8 mmol), and potassium carbonate (5.56 mol), and added with 450 ml of toluene, 100 ml of ethanol, and 100 ml of water thereto, followed by stirring at 100°C for 4 hours. The mixture was cooled to room temperature, then added with 300 ml of water, and transferred to a separatory funnel and separated into an organic layer and a water layer. The organic layer was washed three times with 300 ml of water, and then the resulting organic layer was concentrated under reduced pressure. The resulting residue was purified by column chromatography, yielding a compound T6 (white solid). The yield was 19%.

[C127]

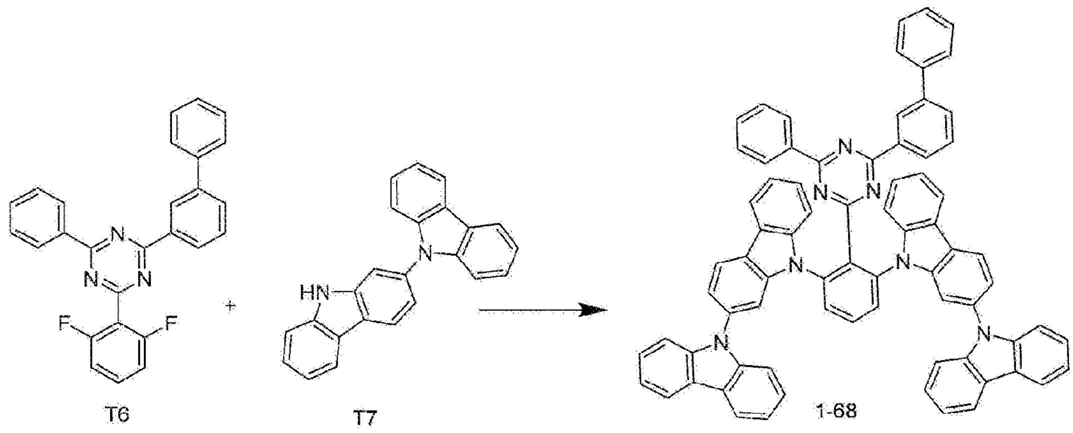

T6 T7 1-68

A 300 ml three-neck flask degassed and replaced with nitrogen was charged with T6 (10.9 mmol), T7 (24.0 mmol), and tripotassium phosphate (43.5 mmol), and added with 30 ml of 1,3-dimethyl-2-imidazolidinone (DMI), followed by stirring at 190°C for 16 hours. The mixture was cooled to room temperature, then added with 100 ml of water, and the precipitated solid was filtered off. The resulting solid was purified by column chromatography, yielding a compound 1-68 (light yellow solid). The yield was 71%. APCI-TOFMS m/z=1046 $[M+1]^+$

Synthesis Example 3 (synthesis of compound 1-106)

[C128]

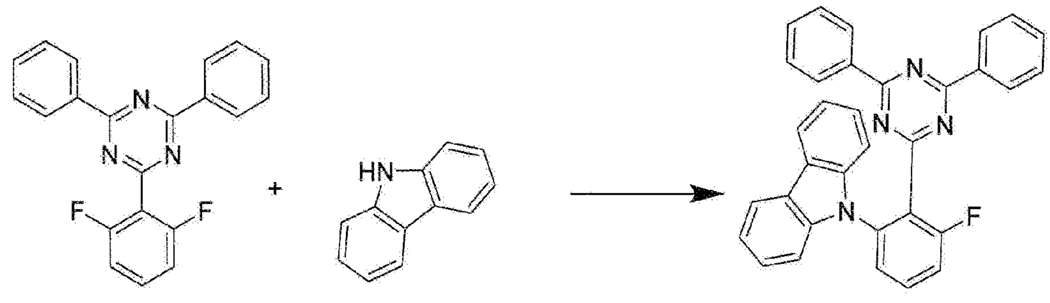

T3 T8 T9

A 300 ml three-neck flask degassed and replaced with nitrogen was charged with T3 (9.9 mmol), T8 (9.9 mmol), and tripotassium phosphate (43.5 mmol), and added with 30 ml of DMI, followed by stirring at 170°C for 16 hours. The mixture was cooled to room temperature, then added with 100 ml of water, and the precipitated solid was filtered off. The resulting solid was purified by column chromatography, yielding a compound 1-68 (light yellow solid). The yield was 54%.

[C129]

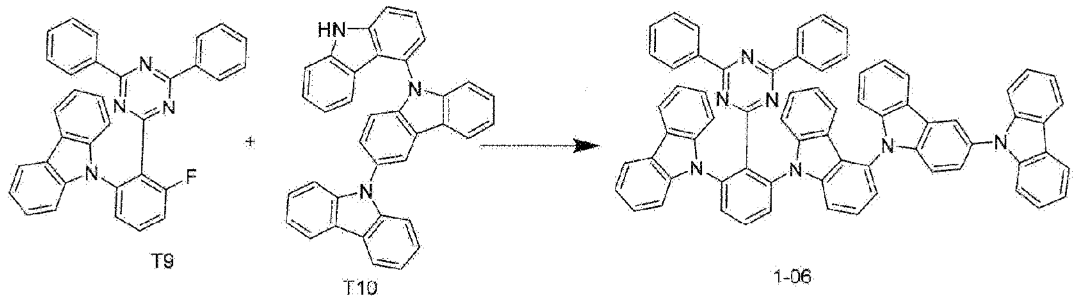

T9 T10 1-06

A 300 ml three-neck flask degassed and replaced with nitrogen was charged with T3 (3.0 mmol), T8 (3.1 mmol), and tripotassium phosphate (20.5 mmol), and added with 10 ml of 1,3-dimethyl-2-imidazolidinone (DMI), followed by stirring at 190°C for 16 hours. The mixture was cooled to room temperature, then added with 50 ml of water, and the precipitated solid was filtered off. The resulting solid was purified by column chromatography, yielding a compound 1-106 (light yellow solid). The yield was 70%. APCI-TOFMS m/z 970 $[M+1]^+$

Synthesis Example 4 (synthesis of compound 1-127)

[C130]

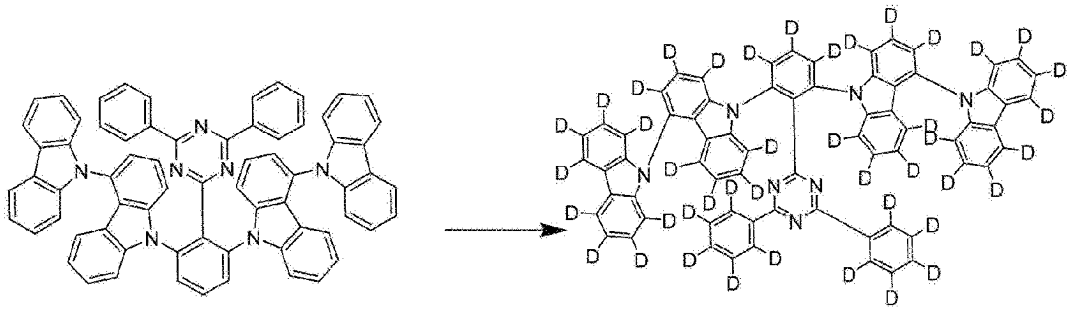

1-51 1-127

A 300 ml three-neck flask degassed and replaced with nitrogen was added with 1-51 (10.1 mmol), 150 mL of deuterated benzene (C6D6) and 9.0 g of deuterated trifluoromethanesulfonic acid (TfOD), and the mixture was heated and stirred at 50°C for 6.5 hours under a nitrogen atmosphere. The reaction liquid was added in a deuterium oxide solution (200 mL) of sodium carbonate (7.0 g) and quenched to obtain a solid. The obtained solid was purified by column chromatography, yielding a compound 1-127 (light yellow solid) (average deuteration rate 78%). The yield was 44%. APCI-TOFMS m/z 1013 $[M+1]^+$

Here, an average deuteration ratio can be determined by mass spectrometry or proton nuclear magnetic resonance spectroscopy. For example, when the ratio is determined by proton nuclear magnetic resonance spectroscopy, first a compound and an internal standard were added and dissolved in a deuterated solvent to prepare a measurement sample, and a proton concentration [mol/g] of the compound included in the measurement sample is calculated from the ratio of the integrated intensity of the internal standard and that of the compound. Next, the ratio between the proton concentration of the deuterated compound and the proton concentration of the corresponding non-deuterated compound is calculated and subtracted from 1 to enable an average deuteration ratio of the deuterated compound to be calculated. A method for having determined an average deuteration ratio of the compound 1-127 by proton nuclear magnetic resonance spectroscopy is shown below. First, the compound 1-127 (5.0 mg) and dimethyl sulfone (2.0 mg) as an internal standard were dissolved in deuterated tetrahydrofuran (1.0 ml) to prepare a measurement sample. An average proton concentration [mol/g] of the compound 1-127 included in the measurement sample was calculated from the ratio of the integrated intensity derived from the internal standard and that of the compound 1-127. Similarly, an average proton concentration [mol/g] was calculated for a non-deuterated compound of the compound 1-127 (corresponding to the compound 1-51). Next, the ratio between the proton concentration of the compound 1-127 and the proton concentration of the non-deuterated compound of the compound 1-127 was calculated and subtracted from 1 to calculate an average deuteration rate of the compound 1-127.

The compound 1-127 denotes an example of the structural formula when all hydrogen atoms of the compound represented by formula (1) are deuterated.

The compounds used in Examples and Comparative Examples are shown below.

[C131]

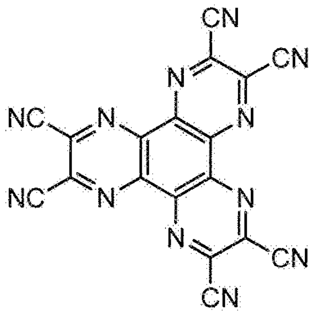
HAT－CN

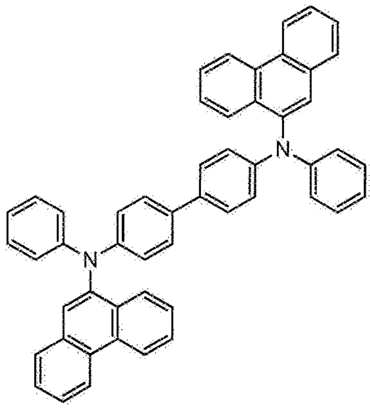
HT－1

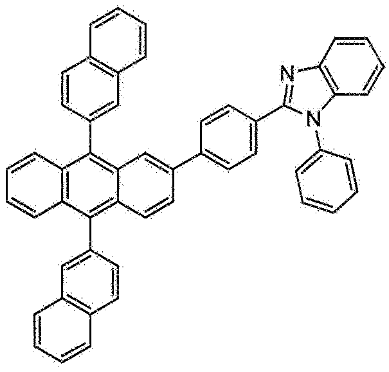
ET－1

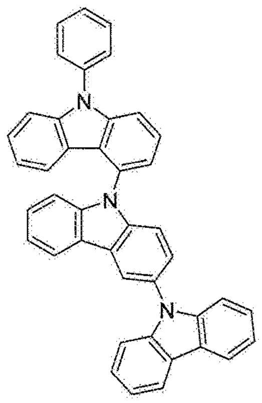
7－58

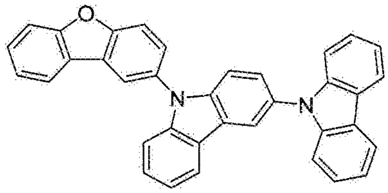
7－31

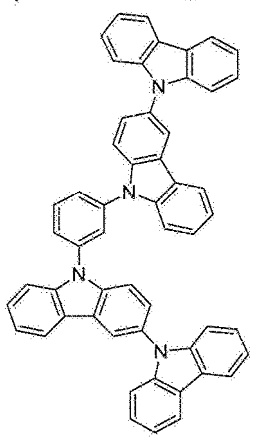
7－81

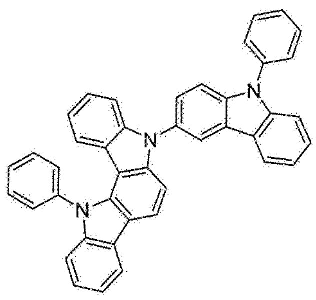
5－148

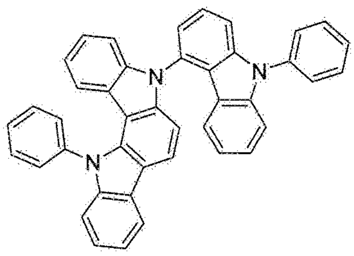
5－115

[C132]

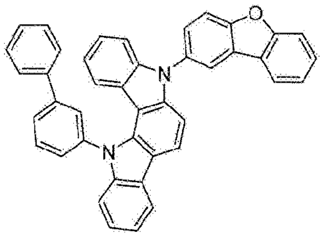

5－179

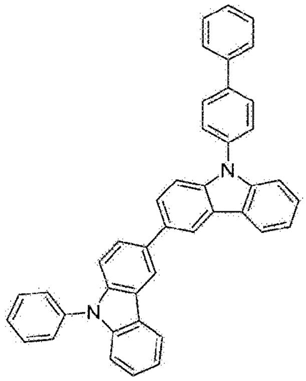

6－2

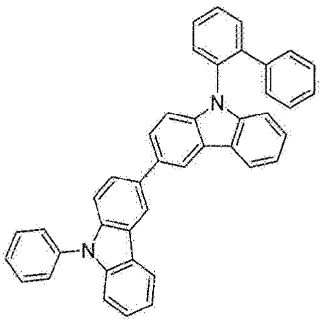

6－4

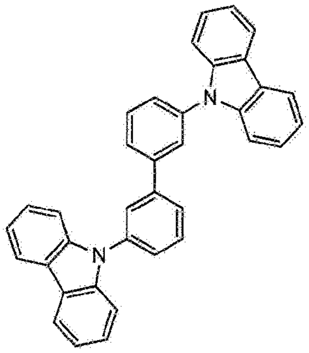

mCBP

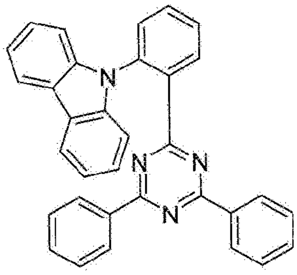

H1

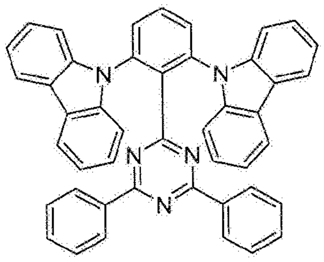

H2

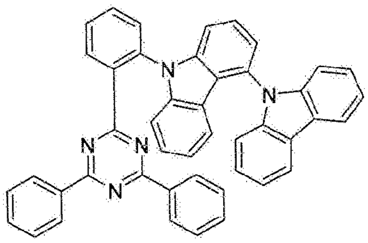

H3

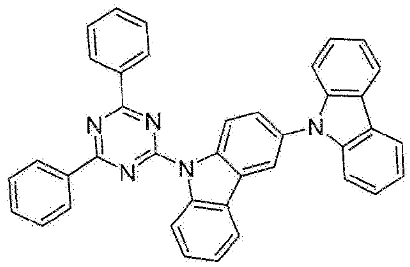

H4

[C133]

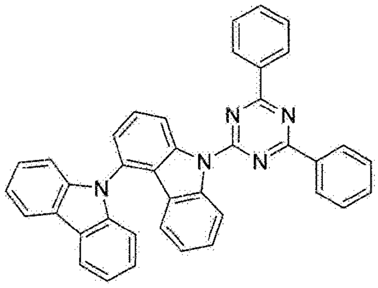

H 5

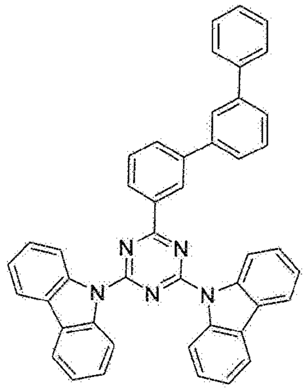

H 6

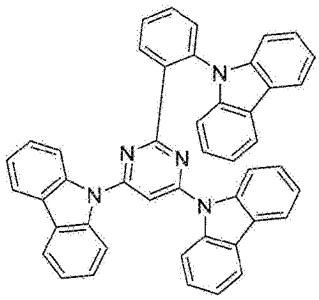

H 7

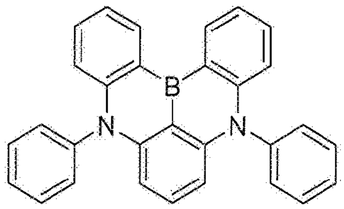

2 − 2

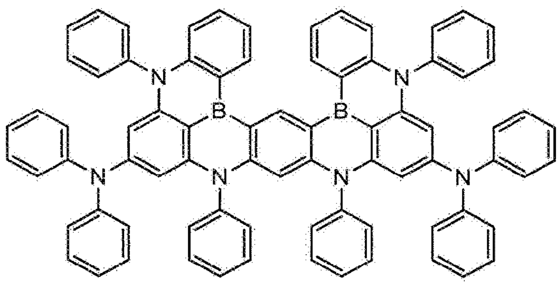

4 − 2

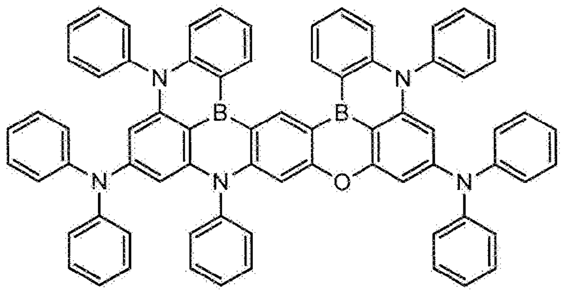

4 − 1 3

S1 and T1 were measured for the compounds 2-2, 4-2 and 4-13.

S1 and T1 were measured as follows.

The compound (7-58) as a host and the compound 2-2, the compound 4-2 or the compound 4-13 as a light emitting dopant were vapor co-deposited on a quartz substrate from different vapor deposition sources by a vacuum deposition method under the conditions of a degree of vacuum of $10^{-4}$ Pa or less to form a vapor deposited film with 100 nm thickness. At this time, they were vapor co-deposited under the vapor deposition conditions such that the concentration of the light emitting dopant was 3%.

For S1, the emission spectrum of this deposition film was measured, a tangent was drawn to the rise of the emission spectrum on the short-wavelength side, and the wavelength value λedge [nm] of the point of intersection of the tangent and the horizontal axis was substituted into the following equation (i) to calculate S1.

$$S1\ [eV] = 1239.85/\lambda edge \qquad (i)$$

For T1, the phosphorescence spectrum of the above deposition film was measured, a tangent was drawn to the rise of the phosphorescence spectrum on the short-wavelength side, and the wavelength value λedge [nm] of the point of intersection of the tangent and the horizontal axis was substituted into the following equation (ii) to calculate T1.

-

$$T1\ [eV] = 1239.85/\lambda edge \qquad (ii)$$

The measurement results are shown in Table 2.

[Table 2]

| Compound | S1(eV) | T1(eV) | S1-T1(eV) |
|---|---|---|---|
| 2-2 | 2.79 | 2.61 | 0.18 |
| 4-2 | 2.71 | 2.67 | 0.04 |
| 4-13 | 2.76 | 2.71 | 0.05 |

Example 1

Each thin film was laminated on the glass substrate on which an anode made of ITO having a film thickness of 70 nm was formed by a vacuum deposition method at a degree of vacuum of $4.0 \times 10^{-5}$ Pa. First, HAT-CN was formed on ITO to a thickness of 10 nm as a hole injection layer, and then HT-1 was formed to a thickness of 25 nm as a hole transport layer. Then, the compound 7-58 was formed to a thickness of 5 nm as an electron blocking layer. Then, the compound 1-1 as the first host, a compound 5-148 as the second host, and the compound 4-2 as the light emitting dopant were vapor co-deposited from different vapor deposition sources to form a light emitting layer to a thickness of 30 nm. At this time, they were vapor co-deposited under the vapor deposition conditions such that the concentration of the compound 4-2 was 2% and the mass ratio of the first host to the second host was 30:70. Then, compound H6 was formed to a thickness of 5 nm as a hole blocking layer. Then, ET-1 was formed to a thickness of 40 nm as an electron transport layer. Lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device was produced.

Examples 2 to 6, 8 to 10, 12 to 22, and Comparative Examples 1 to 11

Each organic EL device was produced in the same manner as in Example 1, except that the light emitting dopant, first host, second host, and hole blocking layer as well as the mass ratio between the first host and the second host were changed to the compounds as shown in Table 3. The mass ratio denotes the first host : the second host.

Examples 7 and 11

Each organic EL device was produced in the same manner as in Example 6, except that the light emitting dopant, first host, second host, and hole blocking layer as well as the mass ratio between the first host and the second host were changed to the compounds as shown in Table 3. The mass ratio denotes the first host : the second host.

[Table 3]

| | Light emitting dopant | First host | Second host | Mass ratio | Hole blocking layer |
|---|---|---|---|---|---|
| Example 1 | 4-2 | 1-1 | 5-148 | 30:70 | H6 |
| Example 2 | 4-2 | 1-10 | 5-148 | 30:70 | H6 |
| Example 3 | 4-2 | 1-11 | 5-148 | 30:70 | H6 |
| Example 4 | 4-2 | 1-51 | 5-148 | 30:70 | H6 |
| Example 5 | 4-2 | 1-51 | 6-2 | 50:50 | H6 |
| Example 6 | 4-2 | 1-51 | 6-4 | 50:50 | H6 |
| Example 7 | 4-2 | 1-51 | 6-4 | 50:50 | 1-51 |

(continued)

| | Light emitting dopant | First host | Second host | Mass ratio | Hole blocking layer |
|---|---|---|---|---|---|
| Example 8 | 4-13 | 1-51 | 7-58 | 50:50 | H6 |
| Example 9 | 4-2 | 1-52 | 5-148 | 30:70 | H6 |
| Example 10 | 4-2 | 1-55 | 5-148 | 30:70 | H6 |
| Example 11 | 4-2 | 1-55 | 5-148 | 30:70 | 1-51 |
| Example 12 | 4-2 | 1-65 | 5-148 | 30:70 | H6 |
| Example 13 | 4-2 | 1-68 | 5-115 | 30:70 | H6 |
| Example 14 | 4-2 | 1-105 | 5-179 | 30:70 | H6 |
| Example 15 | 4-2 | 1-106 | 5-148 | 30:70 | H6 |
| Example 16 | 4-2 | 1-122 | 5-148 | 30:70 | H6 |
| Example 17 | 4-2 | 1-124 | 5-148 | 30:70 | H6 |
| Example 18 | 4-2 | 1-126 | 5-148 | 30:70 | H6 |
| Example 19 | 4-2 | 1-127 | 5-148 | 30:70 | H6 |
| Example 20 | 2-2 | 1-6 | 7-58 | 30:70 | H6 |
| Example 21 | 2-2 | 1-6 | 7-31 | 30:70 | H6 |
| Example 22 | 2-2 | 1-6 | 7-81 | 30:70 | H6 |
| Comparative Example 1 | 4-2 | H1 | 5-148 | 30:70 | H6 |
| Comparative Example 2 | 4-2 | H2 | 5-148 | 30:70 | H6 |
| Comparative Example 3 | 4-2 | H3 | 5-148 | 30:70 | H6 |
| Comparative Example 4 | 4-2 | H4 | 5-148 | 30:70 | H6 |
| Comparative Example 5 | 4-2 | H5 | 5-148 | 30:70 | H6 |
| Comparative Example 6 | 4-2 | H6 | 5-148 | 30:70 | H6 |
| Comparative Example 7 | 4-2 | H6 | 6-2 | 50:50 | H6 |
| Comparative Example 8 | 4-2 | H6 | 6-4 | 50:50 | H6 |
| Comparative Example 9 | 4-13 | H6 | 7-58 | 30:70 | H6 |
| Comparative Example 10 | 4-13 | H6 | mCBP | 30:70 | H6 |
| Comparative Example 11 | 2-2 | H6 | 7-58 | 30:70 | H6 |
| Comparative Example 12 | 4-2 | H7 | 5-148 | 30:70 | H6 |

Table 4 shows the maximum emission wavelength of the light emission spectrum, the external quantum efficiency, and the device lifetime of each organic EL device produced in Examples and Comparative Examples. The maximum emission wavelength and the external quantum efficiency were values at a current density of 2.5 mA/cm$^2$ and were initial properties. As the device lifetime, the time taken for the luminance to reduce to 70% of the initial luminance at a current density of 2.5 mA/cm$^2$ was measured.

[Table 4]

| | Maximum emission wavelength (nm) | Voltage (V) | Eternal quantum efficiency (%) | Device lifetime (h) |
|---|---|---|---|---|
| Example 1 | 472 | 3.8 | 24 | 112 |
| Example 2 | 472 | 3.9 | 24 | 104 |
| Example 3 | 471 | 4.2 | 23 | 106 |
| Example 4 | 472 | 4.0 | 25 | 125 |

(continued)

| | Maximum emission wavelength (nm) | Voltage (V) | Eternal quantum efficiency (%) | Device lifetime (h) |
|---|---|---|---|---|
| Example 5 | 472 | 3.8 | 21 | 130 |
| Example 6 | 472 | 3.9 | 24 | 120 |
| Example 7 | 472 | 4.0 | 28 | 126 |
| Example 8 | 465 | 4.2 | 15 | 65 |
| Example 9 | 472 | 4.0 | 24 | 119 |
| Example 10 | 472 | 3.8 | 24 | 100 |
| Example 11 | 472 | 4.0 | 28 | 128 |
| Example 12 | 473 | 3.7 | 23 | 112 |
| Example 13 | 473 | 3.9 | 20 | 102 |
| Example 14 | 471 | 3.9 | 25 | 105 |
| Example 15 | 471 | 4.2 | 25 | 99 |
| Example 16 | 472 | 4.0 | 25 | 131 |
| Example 17 | 472 | 4.0 | 25 | 137 |
| Example 18 | 472 | 4.0 | 25 | 142 |
| Example 19 | 472 | 4.0 | 25 | 147 |
| Example 20 | 461 | 4.3 | 12 | 47 |
| Example 21 | 461 | 4.2 | 12 | 44 |
| Example 22 | 461 | 4.0 | 11 | 40 |
| Comparative Example 1 | 472 | 3.6 | 24 | 45 |
| Comparative Example 2 | 471 | 3.5 | 25 | 56 |
| Comparative Example 3 | 472 | 3.6 | 25 | 83 |
| Comparative Example 4 | 473 | 3.5 | 20 | 69 |
| Comparative Example 5 | 473 | 3.6 | 25 | 76 |
| Comparative Example 6 | 472 | 3.7 | 23 | 75 |
| Comparative Example 7 | 472 | 3.5 | 20 | 89 |
| Comparative Example 8 | 472 | 3.6 | 23 | 72 |
| Comparative Example 9 | 465 | 4.0 | 12 | 42 |
| Comparative Example 10 | 461 | 4.5 | 11 | 32 |
| Comparative Example 11 | 461 | 4.1 | 11 | 27 |
| Comparative Example 12 | 471 | 4.2 | 23 | 12 |

It is found from Table 4 that the organic EL devices of Examples 1 to 6, 8 to 10, 12 to 22, and Comparative Examples 1 to 12 are characterized by the higher efficiency and the longer lifetime, and also exhibit blue light emission in view of the maximum emission wavelength, whereby they exhibit excellent characteristics compared to known compounds as host materials. When comparing Examples 6 and 7, and Example 10 and Example 11, respectively, the device using the compound represented by general formula (1) in the hole blocking layer is found to also exhibit longer lifetime characteristics compared to known compounds.

Example 23

Each thin film was laminated on the glass substrate on which an anode made of ITO having a film thickness of 70 nm was formed by a vacuum deposition method at a degree of vacuum of $4.0 \times 10^{-5}$ Pa. First, HAT-CN was formed on ITO

to a thickness of 10 nm as a hole injection layer, and then HT-1 was formed to a thickness of 25 nm as a hole transport layer. Then, the compound 7-58 was formed to a thickness of 5 nm as an electron blocking layer. Then, the compound 7-58 as the host and the compound 1-51 as the light emitting dopant were co-deposited from different deposition sources to form a light emitting layer having a thickness of 30 nm. At this time, they were co-deposited under deposition conditions such that the concentration of the compound 1-51 was 30%. Then, the compound H6 was formed to a thickness of 5 nm as a hole blocking layer. Then, ET-1 was formed to a thickness of 40 nm as an electron transport layer. Further, lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device was produced.

Comparative Examples 13 and 14

Organic EL devices were produced in the same manner as in Example 23, except that the light emitting dopant was changed to the compounds as shown in Table 5.

The maximum emission wavelength of the emission spectrum, external quantum efficiency, and lifetime of each organic EL device produced in Examples and Comparative Examples, are shown in Table 5. The maximum emission wavelength and the external quantum efficiency were values at a current density of 2.5 mA/cm$^2$ and were initial properties. The lifetime was measured as the time taken for the luminance to reduce to 70% of the initial luminance at a current density of 2.5 mA/cm$^2$.

[Table 5]

| | Light emitting dopant | Maximum emission wavelength (nm) | Voltage (V) | Eternal quantum efficiency (%) | lifetime (h) |
|---|---|---|---|---|---|
| Example 23 | 1-51 | 467 | 4.6 | 9 | 21 |
| Comparative Example 13 | H1 | 474 | 4.5 | 9 | 10 |
| Comparative Example 14 | H3 | 470 | 4.5 | 3 | 5 |

It is found from Table 5 that the organic EL devices of the Examples are characterized by the high efficiency and long lifetime and exhibit blue light emission in view of the maximum emission wavelength.

Compounds to be used in the Examples and Comparative Examples will be shown below.

[C134]

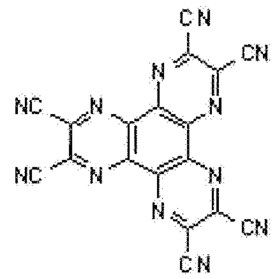

HAT-CN

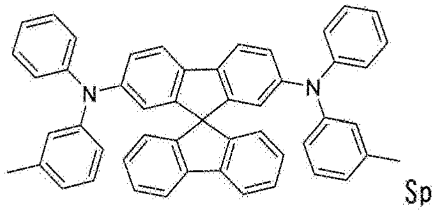

Spiro-TPD

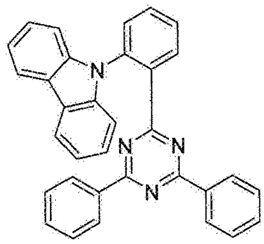

H 1

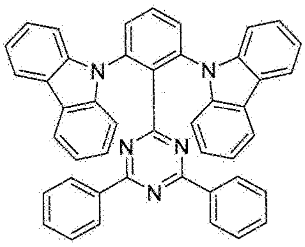

H 2

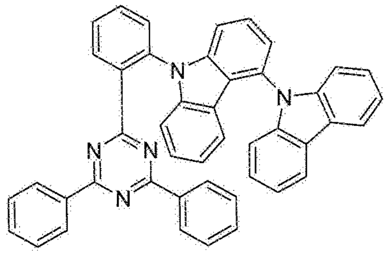

H 3

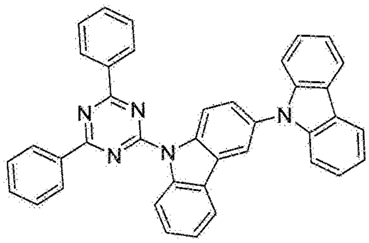

H 4

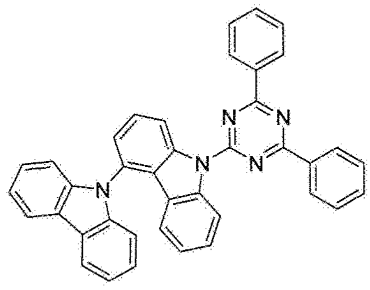

H 5

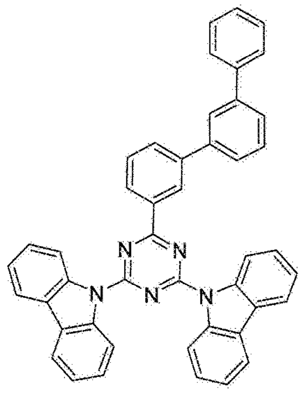

H 6

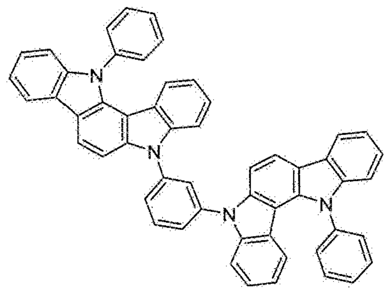

H T − 2

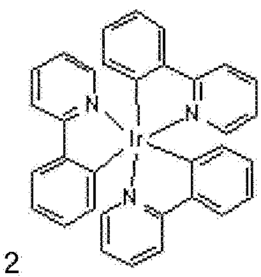

I r (p p y)₃

[C135]

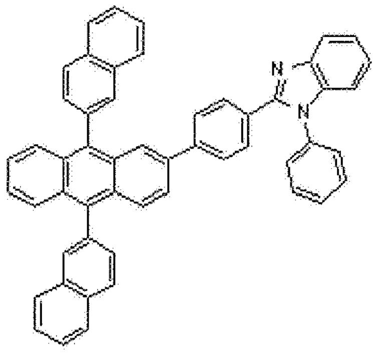

ET-1

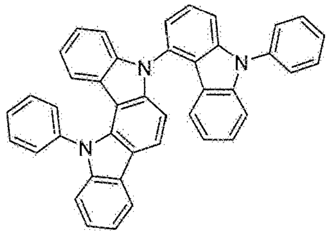

5-115

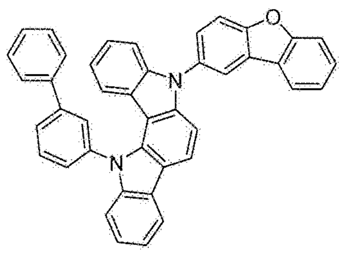

5-179

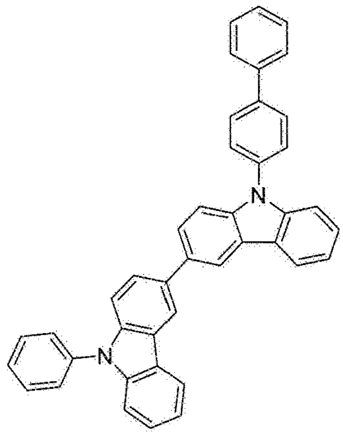

6-2

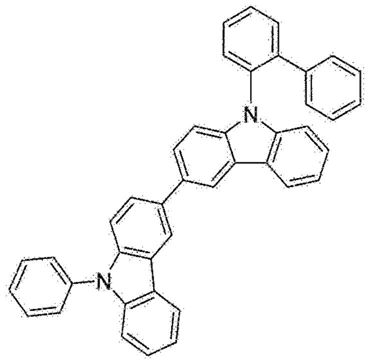

6-4

Example 24

Each thin film was laminated on a glass substrate on which an anode made of ITO having a film thickness of 70 nm was formed, by a vacuum deposition method at a degree of vacuum of $4.0 \times 10^{-5}$ Pa. First, HAT-CN was formed on ITO to a thickness of 25 nm as a hole injection layer, and then Spiro-TPD was formed to a thickness of 30 nm as a hole transport layer. Then, HT-2 was formed to a thickness of 10 nm as an electron blocking layer. Next, the compound 1-1 as the host and $Ir(ppy)_3$ as the light emitting dopant were vapor co-deposited from different vapor deposition sources to form a light emitting layer to a thickness of 40 nm. At this time, they were vapor co-deposited under the vapor deposition conditions such that the concentration of $Ir(ppy)_3$ was 10 wt%. Then, ET-1 was formed to a thickness of 20 nm as an electron transport layer. Further, LiF was formed on the electron transport layer to a thickness of 1 nm as an electron injection layer. Finally, Al was formed on the electron injection layer to a thickness of 70 nm as a cathode, whereby an organic EL device was produced.

Examples 25 to 39 and Comparative Examples 15 to 20

Each organic EL device was produced in the same manner as in Example 24, except that the compound shown in Table 6 was used as the host.

The evaluation results of the organic EL devices produced are shown in Table 6. In the table, the luminance, the voltage, and the power efficiency are values when a driving current was 10 mA/cm$^2$, and are initial properties. LT97 is the time taken for the luminance to reduce to 97% when the initial luminance was 100% at a driving current of 20 mA/cm$^2$ and denotes a device lifetime. The numbers of the host compounds are those assigned to the exemplified compounds above.

[Table 6]

| | Host compound | Voltage (V) | Luminance (cd/m$^2$) | Power efficiency (lm/W) | LT97 (h) |
|---|---|---|---|---|---|
| Example 24 | 1-1 | 4.1 | 7710 | 60 | 31 |
| Example 25 | 1-2 | 3.9 | 7670 | 62 | 33 |
| Example 26 | 1-10 | 4.2 | 7740 | 59 | 30 |
| Example 27 | 1-11 | 4.3 | 7750 | 59 | 29 |
| Example 28 | 1-25 | 3.7 | 7630 | 63 | 25 |
| Example 29 | 1-51 | 3.8 | 7640 | 63 | 46 |
| Example 30 | 1-52 | 3.8 | 7650 | 63 | 40 |
| Example 31 | 1-55 | 3.8 | 7650 | 63 | 36 |
| Example 32 | 1-65 | 3.8 | 7640 | 63 | 37 |
| Example 33 | 1-68 | 4.1 | 7700 | 60 | 37 |
| Example 34 | 1-105 | 4.3 | 7750 | 58 | 39 |
| Example 35 | 1-106 | 4.2 | 7740 | 59 | 39 |
| Example 36 | 1-122 | 3.8 | 7650 | 63 | 49 |
| Example 37 | 1-124 | 3.8 | 7650 | 62 | 54 |
| Example 38 | 1-126 | 3.8 | 7650 | 62 | 60 |
| Example 39 | 1-127 | 3.8 | 7650 | 63 | 63 |
| Comparative Example 15 | H1 | 4.2 | 7430 | 55 | 7 |
| Comparative Example 16 | H2 | 4.0 | 7640 | 61 | 16 |
| Comparative Example 17 | H3 | 4.1 | 7530 | 59 | 19 |
| Comparative Example 18 | H4 | 3.6 | 6980 | 61 | 15 |
| Comparative Example 19 | H5 | 3.9 | 7110 | 58 | 18 |
| Comparative Example 20 | H6 | 5.0 | 7350 | 46 | 12 |

Example 40

Each thin film was laminated on the glass substrate on which an anode made of ITO having a film thickness of 110 nm was formed, by a vacuum deposition method at a degree of vacuum of $4.0 \times 10^{-5}$ Pa. First, HAT-CN was formed on ITO to a thickness of 25 nm as a hole injection layer, and then Spiro-TPD was formed to a thickness of 30 nm as a hole transport layer. Next, HT-2 was formed to a thickness of 10 nm as an electron blocking layer. Then, the compound 1-1 as the first host, the compound 6-2 as the second host, and $Ir(ppy)_3$ as a light emitting dopant were vapor co-deposited from different vapor deposition sources to form a light emitting layer to a thickness of 40 nm. At this time, they were vapor co-deposited under the vapor deposition conditions such that the concentration of $Ir(ppy)_3$ was 10 wt% and the weight ratio of the first host and the second host was 30 : 70. Then, ET-1 was formed to a thickness of 20 nm as an electron transport layer. Further, LiF was formed on the electron transport layer to a thickness of 1 nm as an electron injection layer. Finally, Al was formed on the electron injection layer to a thickness of 70 nm as a cathode, whereby an organic EL device was produced.

Examples 41 to 57 and Comparative Examples 21 to 26

Each organic EL device was produced in the same manner as in Example 40, except that the first host, the second host, and the mass ratio of the first host and the second host were changed to the compounds as shown in Table 7.

The evaluation results of the organic EL devices produced are shown in Table 7. In the table, the luminance, the voltage, and the power efficiency are values when a driving current was 10 mA/cm$^2$, and are initial properties. LT97 is the time taken for the luminance to reduce to 97% when the initial luminance was 100% at a driving current of 20 mA/cm$^2$ and represents a device lifetime. The mass ratio denotes the first host : the second host.

[Table 7]

| | First host | Second host | Mass ratio | Voltage (V) | Luminance (cd/m$^2$) | Power efficiency (lm/W) | LT97 (h) |
|---|---|---|---|---|---|---|---|
| Example 40 | 1-1 | 6-2 | 30:70 | 4.8 | 8090 | 54 | 148 |
| Example 41 | 1-2 | 6-2 | 30:70 | 4.5 | 8020 | 56 | 160 |
| Example 42 | 1-10 | 6-2 | 30:70 | 5.2 | 8230 | 50 | 137 |
| Example 43 | 1-11 | 5-179 | 30:70 | 5.3 | 8250 | 50 | 135 |
| Example 44 | 1-25 | 6-2 | 30:70 | 4.1 | 7900 | 59 | 126 |
| Example 45 | 1-51 | 6-2 | 30:70 | 4.4 | 7940 | 58 | 243 |
| Example 46 | 1-51 | 6-2 | 50:50 | 4.2 | 7700 | 59 | 221 |
| Example 47 | 1-52 | 6-2 | 30:70 | 4.4 | 7960 | 58 | 205 |
| Example 48 | 1-55 | 6-2 | 30:70 | 4.4 | 7970 | 57 | 180 |
| Example 49 | 1-65 | 6-2 | 30:70 | 4.4 | 7940 | 58 | 187 |
| Example 50 | 1-68 | 5-115 | 30:70 | 4.9 | 8113 | 53 | 132 |
| Example 51 | 1-105 | 6-4 | 30:70 | 5.3 | 8260 | 50 | 194 |
| Example 52 | 1-106 | 6-4 | 30:70 | 5.1 | 8220 | 51 | 195 |
| Example 53 | 1-122 | 6-2 | 30:70 | 4.4 | 7970 | 57 | 261 |
| Example 54 | 1-124 | 6-2 | 30:70 | 4.5 | 7970 | 57 | 292 |
| Example 55 | 1-126 | 6-2 | 30:70 | 4.5 | 7970 | 57 | 329 |
| Example 56 | 1-127 | 6-2 | 30:70 | 4.4 | 7950 | 58 | 348 |
| Example 57 | 1-127 | 6-2 | 50:50 | 4.2 | 7700 | 59 | 307 |
| Comparative Example 21 | H1 | 6-2 | 30:70 | 5.4 | 7880 | 46 | 42 |
| Comparative Example 22 | H2 | 6-2 | 30:70 | 4.7 | 8060 | 54 | 97 |
| Comparative Example 23 | H3 | 6-2 | 30:70 | 4.7 | 7940 | 53 | 119 |
| Comparative Example 24 | H4 | 6-2 | 30:70 | 4.4 | 7380 | 53 | 92 |
| Comparative Example 25 | H5 | 6-2 | 30:70 | 4.7 | 7510 | 51 | 112 |
| Comparative Example 26 | H6 | 6-2 | 30:70 | 6.2 | 7770 | 39 | 73 |

It is found from the results in Tables 6 and 7 that Examples 40 to 57 improve the lifetime more and exhibit more favorable properties than Comparative Examples.

Reference Signs List

1 substrate, 2 anode, 3 hole injection layer, 4 hole transport layer, 5 light emitting layer, 6 electron transport layer, 7 cathode

## Claims

**1.** A compound represented by the following general formula (1):

[C1]

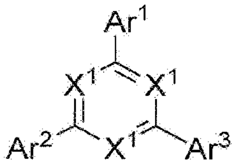

(1)

wherein $Ar^1$ represents the following general formula (2) :

[C2]

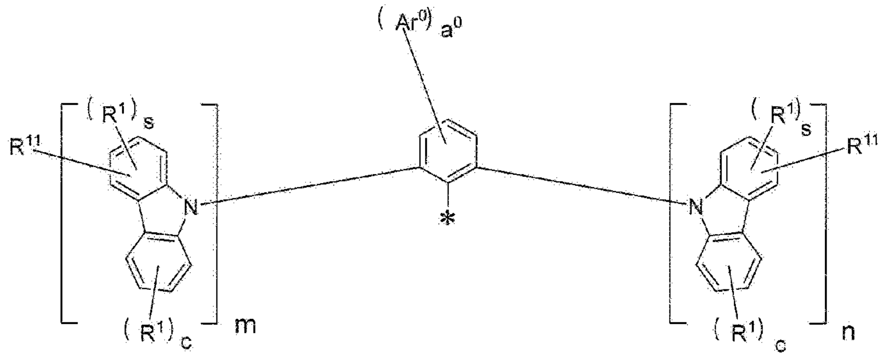

(2)

wherein $R^1$ independently represents deuterium, an aliphatic hydrocarbon group having 1 to 10 carbon atoms, or a substituted or unsubstituted aromatic hydrocarbon group having 6 to 18 carbon atoms; $R^{11}$ independently represents hydrogen, deuterium, an aliphatic hydrocarbon group having 1 to 10 carbon atoms, or a substituted or unsubstituted aromatic hydrocarbon group having 6 to 18 carbon atoms; $a^0$ represents the number of substitutions and represents an integer of 0 to 3; s and c represent the number of substitutions, s independently represents an integer of 0 to 3, and c independently represents an integer of 0 to 4; n and m represent the number of repetitions and each independently represent an integer of 1 to 3, and at least one of n or m represents 2 or 3; and "*" represents a bonding site to the general formula (1),

$X^1$ independently represents N or C-$R^2$, and at least one $X^1$ represents N; and $R^2$ independently represents hydrogen, deuterium, an aliphatic hydrocarbon group having 1 to 10 carbon atoms, a substituted or unsubstituted aromatic hydrocarbon group having 6 to 18 carbon atoms, or a substituted or unsubstituted linked aromatic group formed by linking 2 to 8 of the aromatic hydrocarbon groups,

$Ar^2$ and $Ar^3$ represent hydrogen, deuterium, an aliphatic hydrocarbon group having 1 to 10 carbon atoms, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups, and

$Ar^0$ represents deuterium, an aliphatic hydrocarbon group having 1 to 10 carbon atoms, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups.

**2.** The compound according to claim 1, wherein the general formula (1) is represented by the following formula (3):

[C3]

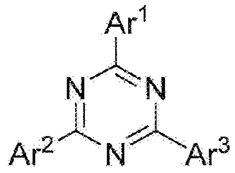

(3)

wherein $Ar^1$ to $Ar^3$ are as defined for the general formula (1).

**3.** The compound according to claim 1, wherein $Ar^1$ contains at least one linked carbazole group represented by any of the following general formulae (4a) to (4c):

[C4]

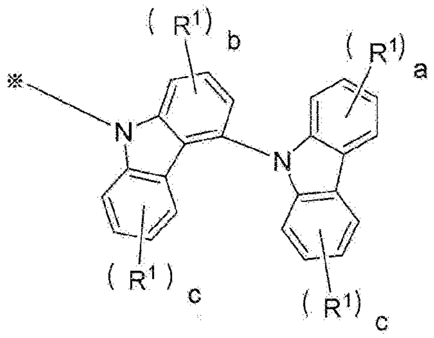

(4 a)

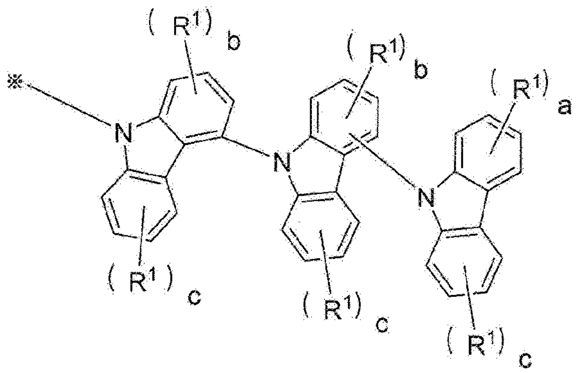

(4 b)

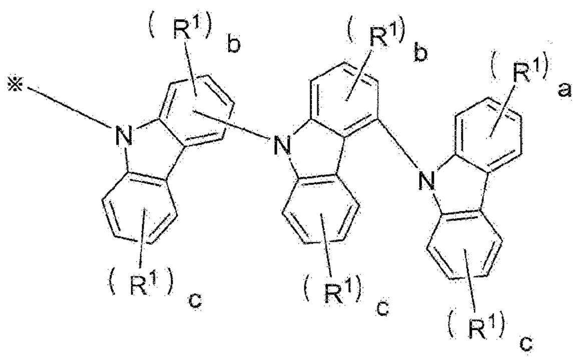

(4 c)

wherein $R^1$ and c are as defined for the general formula (2); a and b represent the number of substitutions, a independently represents an integer of 0 to 4, and b independently represents an integer of 0 to 3; and "※" represents

a bonding site to a benzene ring in the general formula (2).

**4.** The compound according to claim 1, wherein $Ar^1$ is represented by any of the following general formulae (5a) to (5e):

[C5]

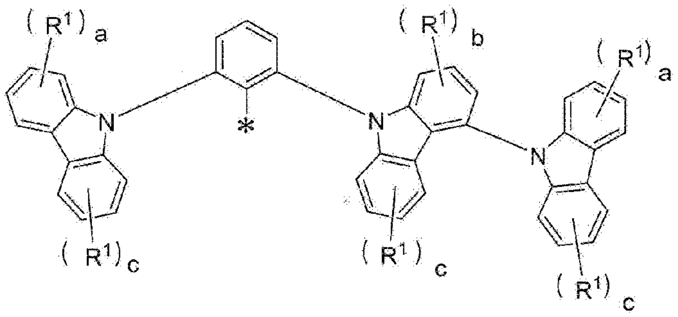

(5 a)

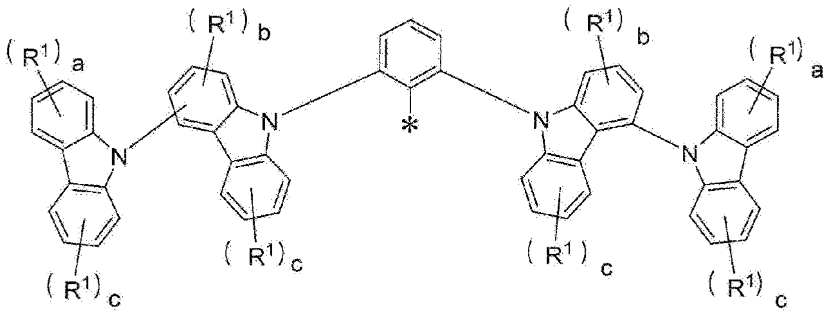

(5 b)

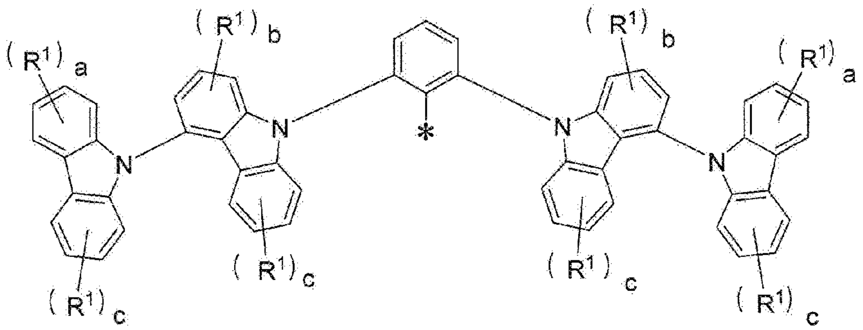

(5 c)

[C6]

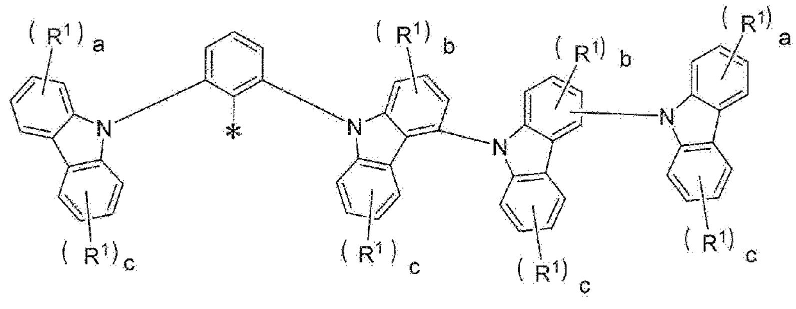

(5d)

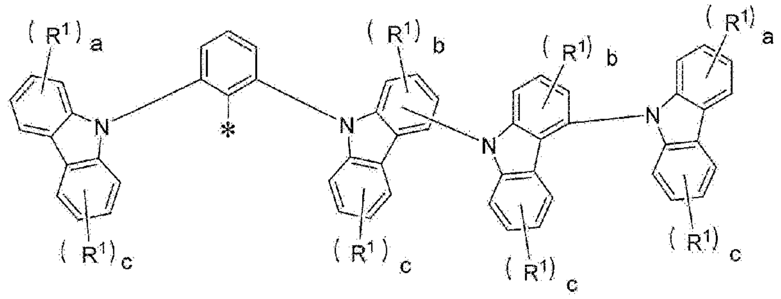

(5e)

wherein $R^1$, c, and "*" are as defined for the general formula (2); and a and b are as defined for the general formulae (4a) to (4c).

**5.** The compound according to claim 1, wherein $Ar^2$ and $Ar^3$ are represented by hydrogen, deuterium, a substituted or unsubstituted aromatic hydrocarbon group having 6 to 18 carbon atoms, or a substituted or unsubstituted linked aromatic group formed by linking 2 to 8 of the aromatic hydrocarbon groups.

**6.** The compound according to claim 1, wherein the general formula (1) is represented by any of the following general formulae (6a) to (6c):

[C7]

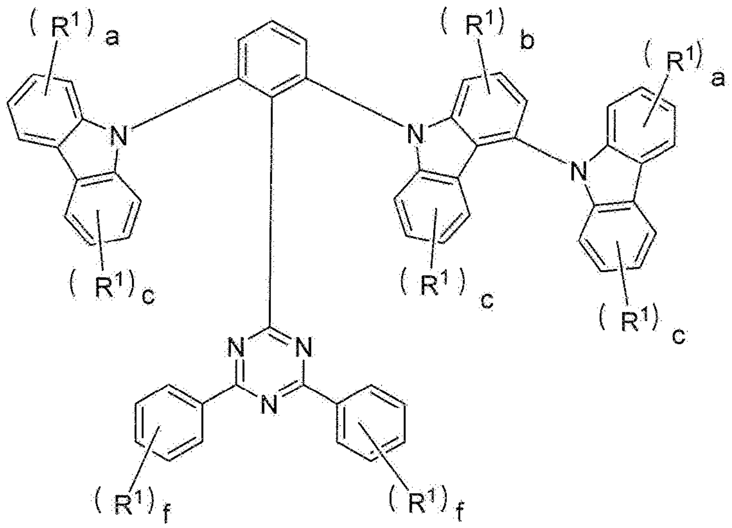

(6 a)

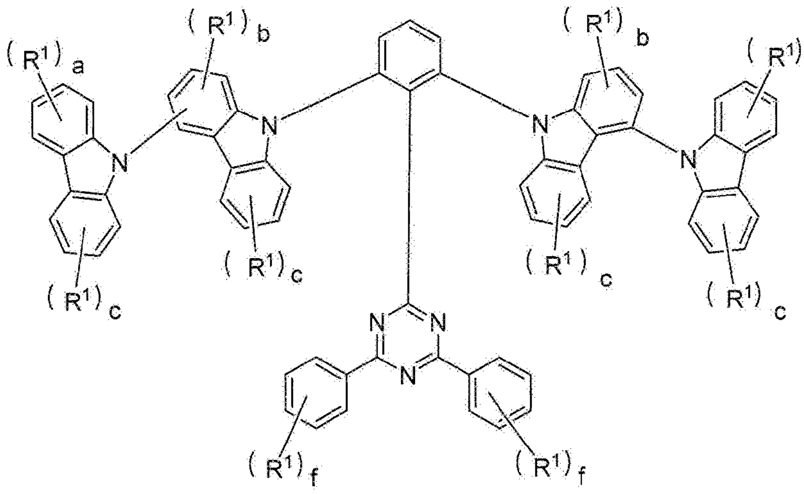

(6 b)

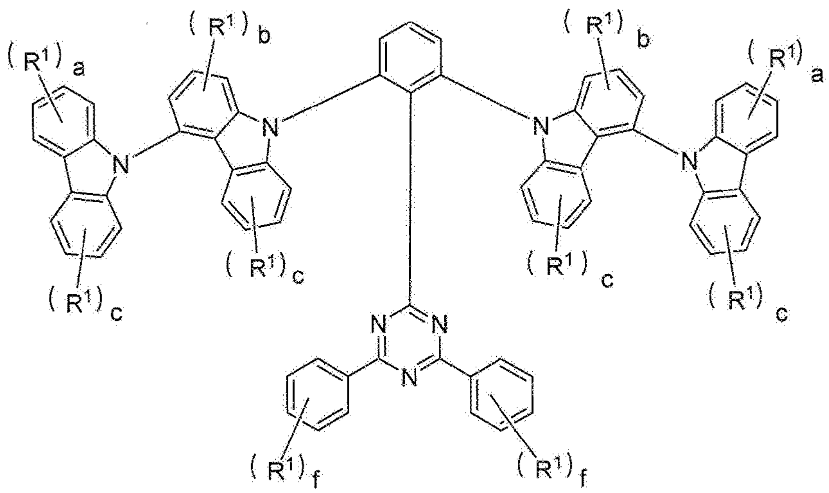

(6 c)

wherein $R^1$ and c are as defined for the general formula (2) and are independent of each other; a and b are as defined for the general formulae (4a) to (4c); and f independently represents the number of substitutions and represents an integer of 0 to 5.

**7.** The compound according to claim 1, wherein at least one of $Ar^1$ to $Ar^3$ contains a deuterium atom.

8. The compound according to claim 1, wherein at least one of $A^2$ and $Ar^3$ contains a deuterium atom.

9. The compound according to claim 1, wherein at least one $R^1$ is deuterium.

10. A material for an organic electroluminescent device comprising the compound represented by the general formula (1).

11. An organic electroluminescent device, comprising one or more light emitting layers between an anode and a cathode opposite to each other, wherein at least one light emitting layer comprises a host selected from the compounds represented by the general formula (1) and a light emitting dopant.

12. The organic electroluminescent device according to claim 11, comprising a host selected from the compounds represented by the general formula (1) and a light emitting dopant selected from polycyclic aromatic compounds represented by the following general formula (7a) or (7b):

[C8]

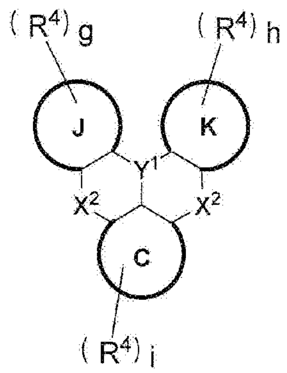
(7 a)
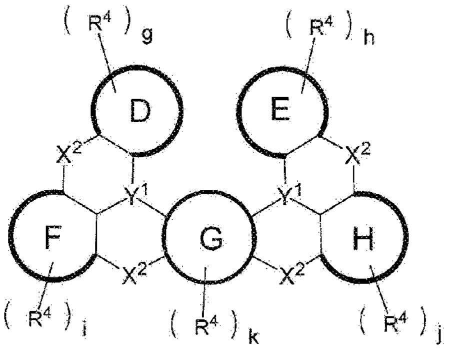
(7 b)

wherein a ring J, a ring K, a ring C, a ring D, a ring E, a ring F, a ring G, and a ring H are each independently a substituted or unsubstituted aromatic hydrocarbon ring having 6 to 24 carbon atoms, or a substituted or unsubstituted aromatic heterocyclic ring having 3 to 17 carbon atoms,
$Y^1$ is each independently B, P, P=O, P=S, Al, Ga, As, Si-$R^3$, or Ge-$R^3$,
$R^3$ is each independently an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms,
$X^2$ is each independently O, N-$Ar^4$, S, or Se,
$Ar^4$ is each independently a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these groups, and N-$Ar^4$ is optionally bonded to any of the ring J, the ring K, the ring C, the ring D, the ring E, the ring F, the ring G, or the ring H to form a heterocyclic ring containing N,
$R^4$ each independently represents a cyano group, deuterium, a diarylamino group having 12 to 44 carbon atoms, an arylheteroarylamino group having 12 to 44 carbon atoms, a diheteroarylamino group having 12 to 44 carbon atoms, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms, and
g and h represent the number of substitutions and each independently represent an integer of 0 to 4, i and j represent the number of substitutions and each independently represent an integer of 0 to 3, and k represents the number of substitutions and represents an integer of 0 to 2.

13. The organic electroluminescent device according to claim 11, comprising a host selected from the compounds represented by the general formula (1) and a light emitting dopant selected from polycyclic aromatic compounds represented by the following general formula (8a) or (8b):

[C9]

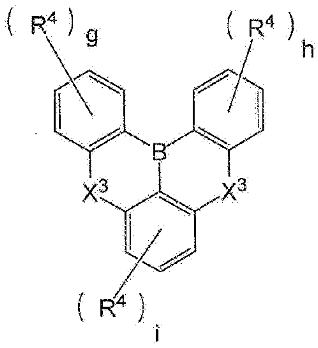

(8 a)

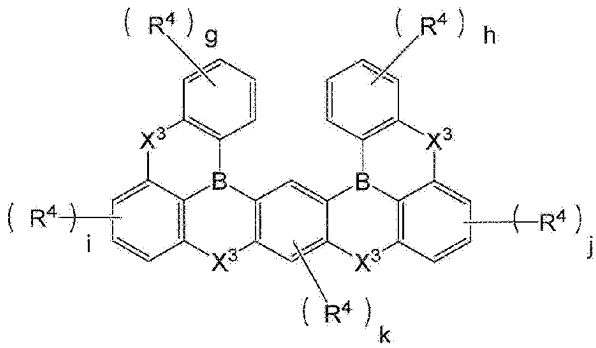

(8 b)

wherein $X^3$ each independently represents N-$Ar^4$, O, or S, and at least one $X^3$ represents N-$Ar^4$; and $Ar^4$, $R^4$, g, h, i, j, and k are as defined for the general formula (7a) or (7b).

**14.** The organic electroluminescent device according to claim 11, wherein the light emitting dopant has a difference between excited singlet energy (S1) and excited triplet energy (T1) (ΔEST) of 0.20 eV or less.

**15.** The organic electroluminescent device according to claim 14, wherein the ΔEST is 0.10 eV or less.

**16.** The organic electroluminescent device according to claim 11, wherein the light emitting dopant is a phosphorescent dopant.

**17.** The organic electroluminescent device according to any one of claims 11 to 16, comprising a first host selected from the compounds represented by the general formula (1), a light emitting dopant, and a second host.

**18.** The organic electroluminescent device according to claim 17, wherein the second host is selected from compounds represented by the following general formula (9), or is selected from compounds represented by the general formula (9a) or general formula (9b),

[C10]

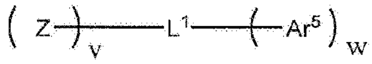

(9)

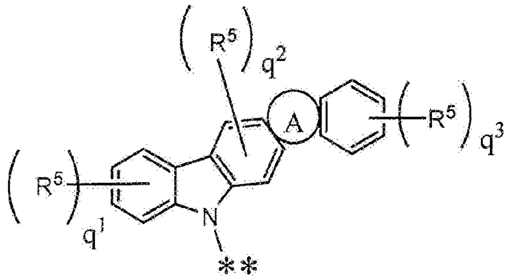

(10)

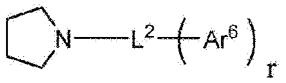

(11)

wherein in the general formula (9), Z is an indolocarbazole ring-containing group represented by the general formula (10), and "* *" represents a bonding site to $L^1$; the ring A in the general formula (10) is a heterocyclic ring represented by the general formula (11), and the ring A is condensed with the adjacent ring at any position,
$L^1$ and $L^2$ are each independently a substituted or unsubstituted aromatic hydrocarbon group having 6 to 18 carbon atoms or a substituted or unsubstituted aromatic heterocyclic group having 3 to 17 carbon atoms,
$Ar^5$ and $Ar^6$ are each independently deuterium, a substituted or unsubstituted aromatic hydrocarbon group having 6 to 18 carbon atoms, a substituted or unsubstituted aromatic heterocyclic ring having 3 to 17 carbon atoms, or a substituted or unsubstituted linked aromatic group formed by linking 2 to 8 of these aromatic groups,
$R^5$ is independently deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic ring having 3 to 17 carbon atoms,
v represents the number of substitutions and represents an integer of 1 to 3; w represents the number of substitutions and represents an integer of 0 to 3; $q^1$ and $q^3$ represent the number of substitutions and each independently represent an integer of 0 to 4; $q^2$ represents the number of substitutions and represents an integer of 0 to 2; and r represents the number of substitutions and represents an integer of 0 to 3,

[C11]

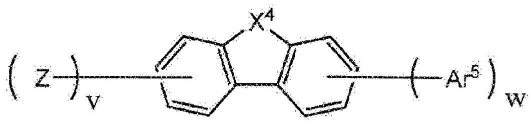

(9 a)

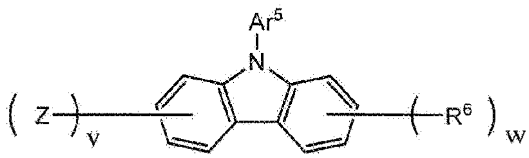

(9 b)

wherein Z, $Ar^5$, v, and w are as defined for the general formula (9), and $X^4$ represents O or S; $R^6$ is each independently deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms.

**19.** The organic electroluminescent device according to claim 17, wherein the second host is selected from compounds represented by the following general formula (12) or general formula (13),

[C12]

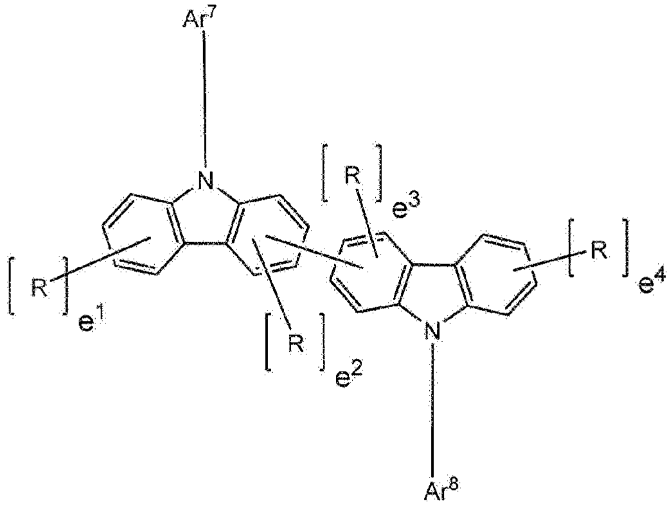

(1 2)

wherein $Ar^7$ and $Ar^8$ each independently represent a substituted or unsubstituted aromatic hydrocarbon group having 6 to 18 carbon atoms, a substituted or unsubstituted aromatic heterocyclic ring having 3 to 17 carbon atoms, or a substituted or unsubstituted linked aromatic group formed by linking 2 to 8 of these aromatic groups, R each independently represents deuterium, or an aliphatic hydrocarbon group having 1 to 10 carbon atoms, $e^1$ to $e^4$ each represent the number of substitutions, $e^1$ and $e^4$ independently represent an integer of 0 to 4, and $e^2$ and $e^3$ independently represent an integer of 0 to 3,

[C13]

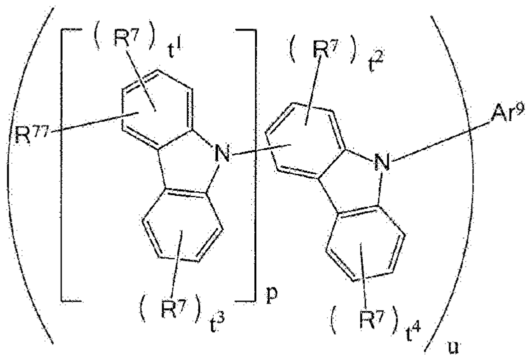

(1 3)

wherein $Ar^9$ represents a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group formed by linking 2 to 8 of these aromatic groups,
$R^7$ each independently represents deuterium, an aliphatic hydrocarbon group having 1 to 10 carbon atoms, a substituted or unsubstituted aromatic hydrocarbon group having 6 to 18 carbon atoms, or a substituted or unsubstituted aromatic heterocyclic group having 3 to 17 carbon atoms,
$R^{77}$ independently represents hydrogen, deuterium, an aliphatic hydrocarbon group having 1 to 10 carbon atoms, or a substituted or unsubstituted aromatic hydrocarbon group having 6 to 18 carbon atoms,
$t^1$ to $t^4$ each represent the number of substitutions, $t^3$ and $t^4$ independently represent an integer of 0 to 4, and $t^1$ and $t^2$ independently represent an integer of 0 to 3; p represents the number of repetitions and each independently represents an integer of 1 to 4; u represents the number of substitutions and represents an integer of 1 or 2; when u is 2, the general formula (13) is symmetric or asymmetric.

Fig. 1

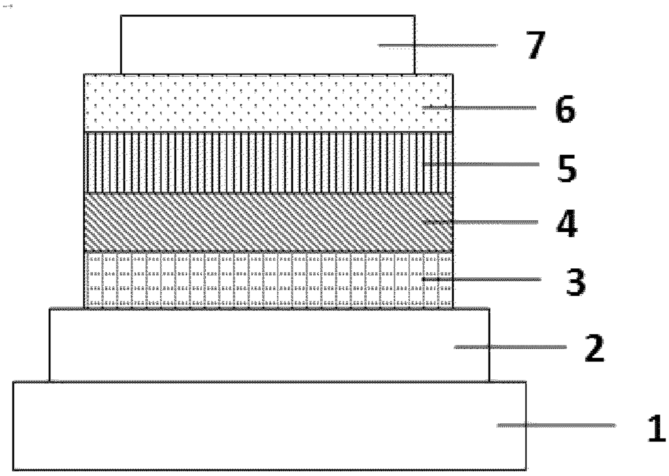

## INTERNATIONAL SEARCH REPORT

International application No.
**PCT/JP2023/042938**

### A. CLASSIFICATION OF SUBJECT MATTER

***C07D 403/14***(2006.01)i; ***C07D 405/14***(2006.01)i; ***C09K 11/06***(2006.01)i; ***H10K 50/00***(2023.01)i; ***H10K 50/10***(2023.01)i; ***H10K 50/11***(2023.01)i; ***H10K 50/12***(2023.01)i; ***H10K 59/00***(2023.01)i; ***H10K 59/10***(2023.01)i; ***H10K 85/00***(2023.01)i; ***H10K 85/60***(2023.01)i; *H10K 101/10*(2023.01)n

FI: C07D403/14 CSP; C07D405/14; C09K11/06 660; C09K11/06 690; H05B33/14 B; H10K50/00; H10K50/10; H10K50/11; H10K50/12; H10K59/00; H10K59/10; H10K85/00; H10K85/60; H10K101:10

According to International Patent Classification (IPC) or to both national classification and IPC

### B. FIELDS SEARCHED

Minimum documentation searched (classification system followed by classification symbols)

C07D403/14; C07D405/14; C09K11/06; H10K50/00; H10K50/10; H10K50/11; H10K50/12; H10K59/00; H10K59/10; H10K85/00; H10K85/60; H10K101/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | WO 2019/076965 A1 (CYNORA GMBH) 25 April 2019 (2019-04-25) claims, pp. 50, 77, 78 | 1-5, 7-11, 17 |
| Y | claims, pp. 50, 77, 78 | 12-16, 18, 19 |
| A | claims, pp. 50, 77, 78 | 6 |
| Y | WO 2021/200252 A1 (NIPPON STEEL CHEMICAL & MATERIAL CO., LTD.) 07 October 2021 (2021-10-07) claims, paragraphs [0039]-[0117], examples | 12-16, 18, 19 |
| A | DE 102016112377 A1 (CYNORA GMBH) 11 January 2018 (2018-01-11) claims, examples | 1-19 |
| A | KR 10-1926771 B1 (JINWOONG IND CO., LTD.) 07 December 2018 (2018-12-07) claims, examples | 1-19 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 January 2024** | **06 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/042938**

| Patent document cited in search report | Publication date (day/month/year) | Patent family member(s) | Publication date (day/month/year) |
|---|---|---|---|
| WO 2019/076965 A1 | 25 April 2019 | EP 3697782 A1 | |
| WO 2021/200252 A1 | 07 October 2021 | US 2023/0139757 A1<br>claims, paragraphs [0075]-[0151], examples<br>EP 4130191 A1<br>CN 115280533 A<br>KR 10-2022-0160573 A | |
| DE 102016112377 A1 | 11 January 2018 | (Family: none) | |
| KR 10-1926771 B1 | 07 December 2018 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010134350 A
- WO 2011070963 A
- WO 2015102118 A
- WO 2017115833 A
- WO 2018212169 A
- WO 2018181188 A
- WO 2020040298 A
- JP 2020120096 A
- WO 2016159479 A
- US 20110309345 A
- WO 2008117826 A
- US 20220177492 A
- WO 2016158191 A
- WO 2012077520 A
- US 20140158992 A
- WO 2016158540 A
- US 20200168812 A